# EUROPEAN PATENT APPLICATION

(11) **EP 4 817 632 A2**
(43) Date of publication of application: **07.10.2026**
(21) Application number: 26184524.2
(22) Date of filing: 16.09.2016
(51) Int. Cl.: A61K 40/42

(54) **CAR T CELL THERAPIES WITH ENHANCED EFFICACY**

(30) Priority: 17.09.2015 US 201562220196 P
(62) Document number(s) of the earlier application(s) in accordance with Art. 76 EPC: 16779217.5 / 3 350 320
(71) Applicant: Novartis AG, 4056 Basel (CH); The Trustees of The University of Pennsylvania, Philadelphia, PA 19104 (US)
(72) Inventor: BUSHMAN, Frederic Dixon, Rose Valley, PA 19086 (US); FRAIETTA, Joseph A., Williamstown, NJ 08094 (US); JUNE, Carl H., Merion Station, PA 19066 (US); MELENHORST, Jan J., Cherry Hill, NJ 08003 (US); NOBLES, Christopher Loren, Philadelphia, PA 19146 (US); YOUNG, Regina M., Bryn Mawr, PA 19010 (US); MOTZ, Gregory, Cambridge, MA 02140 (US)
(74) Representative: Mathys & Squire

(57) **Abstract**

The invention provides compositions and methods improved CAR T cell therapies. Specifically, the invention provides cells with reduced Tet, e.g., Tet2 function or expression, and methods of use therefore. The invention further provides Tet2 inhibitors and methods of use therefore in connection with CAR T cells.

## Description

### CROSS REFERENCE TO RELATED APPLICATIONS

This application claims priority to U.S. Application Serial No. 62/220,196, filed September 17, 2015, the contents of which are incorporated herein by reference in their entireties.

### SEQUENCE LISTING

The instant application contains a Sequence Listing which has been submitted electronically in ASCII format and is hereby incorporated by reference in its entirety. Said ASCII copy, created on September 14, 2016, is named N2067-7098WO_SL.txt and is 507,996 bytes in size.

### FIELD OF THE INVENTION

The present invention relates generally to the use of immune effector cells (e.g., T cells, NK cells) engineered to express a Chimeric Antigen Receptor (CAR) to treat a disease associated with expression of a tumor antigen.

### BACKGROUND OF THE INVENTION

Adoptive cell transfer (ACT) therapy with autologous T-cells, especially with T-cells transduced with Chimeric Antigen Receptors (CARs), has shown promise in hematologic cancer trials. There is a medical need for T cell therapies, especially CAR T cell therapies with improved efficacy.

### SUMMARY OF THE INVENTION

The present invention provides compositions and methods that disrupt methylcytosine dioxygenase genes (e.g., Tet1, Tet2, Tet3), and uses of such compositions and methods for increasing the functional activities of engineered cells (e.g., gene-modified antigen-specific T cells, such as CAR T cells). In particular, the present invention provides methods and compositions for bolstering the therapeutic efficacy of chimeric antigen receptor (CAR) T cells. While not to be bound by the theory, disruption of a single allele of a Tet gene (e.g., a Tet1, Tet2, or Tet3) leads to decreased total levels of 5-hydroxymethylcytosine in association with enhanced proliferation, regulation of effector cytokine production and degranulation, and thereby increases CAR T cell proliferation and/or function.

Accordingly, the present invention provides a cell (e.g., a population of cells, such as a population of immune effector cells) engineered to express a chimeric antigen receptor (CAR), wherein the CAR comprises an antigen-binding domain, a transmembrane domain, and an intracellular signaling domain, and wherein expression and/or function of Tet1, Tet2 and/or Tet3 in said cell has been reduced or eliminated. In one embodiment, the expression and/or function of Tet2 in said cell has been reduced or eliminated. In some embodiments, the antigen-binding domain binds to a tumor antigen is selected from a group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1. In one embodiment, the tumor antigen is CD19. In some embodiments, the antigen-binding domain is an antibody or antibody fragment as described in, e.g., WO2012/079000 or WO2014/153270.

In one aspect, the present invention provides a cell (e.g., a population of cells, such as a population of immune effector cells) engineered to express a CAR, and wherein expression and/or function of Tet1, Tet2 and/or Tet3 in said cell has been reduced or eliminated. In one embodiment, the expression and/or function of Tet2 in said cell has been reduced or eliminated. In some embodiments, the transmembrane domain of said CAR comprises: (i) an amino acid sequence having at least one, two or three modifications but not more than 20, 10 or 5 modifications of an amino acid sequence of SEQ ID NO: 12, or a sequence with 95-99% identity to an amino acid sequence of SEQ ID NO: 12; or (ii) the sequence of SEQ ID NO: 12.

In one aspect, the present invention provides a cell (e.g., a population of cells, such as a population of immune effector cells) engineered to express a CAR, and wherein expression and/or function of Tet1, Tet2 and/or Tet3 in said cell has been reduced or eliminated. In one embodiment, the antigen binding domain of said CAR is connected to the transmembrane domain by a hinge region, wherein said hinge region comprises SEQ ID NO: 2 or SEQ ID NO: 6, or a sequence with 95-99% identity thereof. In some embodiments, the intracellular signaling domain of said CAR comprises a primary signaling domain and/or a costimulatory signaling domain, wherein the primary signaling domain comprises a functional signaling domain of a protein chosen from CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, or DAP12.

In some embodiments, the primary signaling domain of said CAR comprises: (i) an amino acid sequence having at least one, two or three modifications but not more than 20, 10 or 5 modifications of an amino acid sequence of SEQ ID NO: 18 or SEQ ID NO: 20, or a sequence with 95-99% identity to an amino acid sequence of SEQ ID NO: 18 or SEQ ID NO: 20; or (ii) the amino acid sequence of SEQ ID NO:18 or SEQ ID NO: 20. In some embodiments, the intracellular signaling domain of said CAR comprises a costimulatory signaling domain, or a primary signaling domain and a costimulatory signaling domain, wherein the costimulatory signaling domain comprises a functional signaling domain of a protein selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D.

In some embodiments, the costimulatory signaling domain of said CAR comprises an amino acid sequence having at least one, two or three modifications but not more than 20, 10 or 5 modifications of an amino acid sequence of SEQ ID NO: 14 or SEQ ID NO: 16, or a sequence with 95-99% identity to an amino acid sequence of SEQ ID NO: 14 or SEQ ID NO: 16. In some embodiments, the intracellular domain of said CAR comprises the sequence of SEQ ID NO: 14 or SEQ ID NO: 16, and the sequence of SEQ ID NO: 18 or SEQ ID NO: 20, wherein the sequences comprising the intracellular signaling domain are expressed in the same frame and as a single polypeptide chain. In some embodiments, the CAR of the present invention further comprises a leader sequence comprises the sequence of SEQ ID NO: 2.

In some embodiments, the immune effector cell of the present invention is a T cell or an NK cell. In some embodiments, the T cell is a CD4+ T cell, a CD8+ T cell, or a combination thereof. In one aspect, the cells of the present invention are human cells. In one aspect, the cells (e.g., engineered immune effector cells, e.g., CAR T cells) of the present invention comprise an inhibitor of Tet1, Tet2, and/or Tet3. In some embodiments, the cells of the present invention comprise a CAR, and an inhibitor of Tet1, Tet2 and/or Tet3, wherein said inhibitor is (1) a gene editing system targeted to one or more sites within the gene encoding Tet1, Tet2 and/or Tet3, or its regulatory elements, e.g., Tet2, or its regulatory elements; (2) nucleic acid encoding one or more components of said gene editing system; or (3) combinations thereof.

In some embodiments, the cells of the present invention comprise a CAR, and an inhibitor of Tet1, Tet2 and/or Tet3, wherein said inhibitor is a gene editing system targeted to one or more sites within the gene encoding Tet1, Tet2 and/or Tet3, or its regulatory elements, e.g., Tet2, or its regulatory elements, and wherein the gene editing system is selected from the group consisting of: a CRISPR/Cas9 system, a zinc finger nuclease system, a TALEN system and a meganuclease system.

In some embodiments, the cells of the present invention comprise a CAR, and an inhibitor of Tet1, Tet2 and/or Tet3, wherein said inhibitor is a gene editing system targeted to one or more sites within the gene encoding Tet1, Tet2 and/or Tet3, or its regulatory elements, e.g., Tet2, or its regulatory elements, and wherein the gene editing system binds to a target sequence in an early exon or intron of a gene encoding Tet1, Tet2 and/or Tet3, e.g., Tet2.

In some embodiments, the cells of the present invention comprise a CAR, and an inhibitor of Tet1, Tet2 and/or Tet3, wherein said inhibitor is a gene editing system targeted to one or more sites within the gene encoding Tet1, Tet2 and/or Tet3, or its regulatory elements, e.g., Tet2, or its regulatory elements, and wherein the gene editing system binds a target sequence of a gene encoding tet2, and the target sequence is upstream of exon 4, e.g., in exon1, exon2, or exon3, e.g. in exon 3.

In some embodiments, the cells of the present invention comprise a CAR, and an inhibitor of Tet1, Tet2 and/or Tet3, wherein said inhibitor is a gene editing system targeted to one or more sites within the gene encoding Tet1, Tet2 and/or Tet3, or its regulatory elements, e.g., Tet2, or its regulatory elements, and wherein the gene editing system binds to a target sequence in a late exon or intron of a gene encoding Tet1, Tet2 and/or Tet3, e.g., Tet2.

In some embodiments, the cells of the present invention comprise a CAR, and an inhibitor of Tet1, Tet2 and/or Tet3, wherein said inhibitor is a gene editing system targeted to one or more sites within the gene encoding Tet1, Tet2 and/or Tet3, or its regulatory elements, e.g., Tet2, or its regulatory elements, and wherein the gene editing system binds a target sequence of a gene encoding tet2, and the target sequence is downstream of exon 8, e.g., is in exon9, exon10, or exon11, e.g. is in exon 9.

In some embodiments, the cells of the present invention comprise a CAR, and an inhibitor of Tet1, Tet2 and/or Tet3, wherein said inhibitor is a gene editing system targeted to one or more sites within the gene encoding Tet1, Tet2 and/or Tet3, or its regulatory elements, e.g., Tet2, or its regulatory elements, and wherein the gene editing system is a CRISPR/Cas system comprising a gRNA molecule comprising a targeting sequence which hybridize to a target sequence of a Tet2 gene. In some embodiments, the targeting sequence is a targeting sequence listed in Table 3. In some embodiments, the target sequence is a targeting sequence listed in Table 5.

In some embodiments, the cells of the present invention comprise a CAR, and an inhibitor of Tet1, Tet2 and/or Tet3, wherein said inhibitor is an siRNA or shRNA specific for Tet1, Tet2, Tet3, or nucleic acid encoding said siRNA or shRNA. In some embodiments, the siRNA or shRNA comprises a sequence complementary to a sequence of a Tet2 mRNA, e.g., comprises a target sequence of shRNA listed in Table 4.

In some embodiments, the cells of the present invention comprise a CAR, and an inhibitor of Tet1, Tet2 and/or Tet3, wherein said inhibitor a small molecule.

In some embodiments, the cells of the present invention comprise a CAR, and an inhibitor of Tet1, Tet2 and/or Tet3, wherein the inhibitor is a protein, e.g., is a dominant negative binding partner of Tet1, Tet2, and/or Tet3 (e.g., a histone deacetylase (HDAC) that interacts with Tet1, Tet2, and/or Tet3), or nucleic acid encoding said dominant negative binding partner of Tet1, Tet2, and Tet3.

In some embodiments, the cells of the present invention comprise a CAR, and an inhibitor of Tet1, Tet2 and/or Tet3, wherein the inhibitor is a protein, e.g., is a dominant negative (e.g., catalytically inactive) Tet1, Tet2, or Tet3, or nucleic acid encoding said dominant negative Tet1, Tet2, or Tet3.

In one aspect, the present invention provides a method of increasing the therapeutic efficacy of a CAR-expressing cell, e.g., a cell of any of the previous claims, e.g., a CAR19-expressing cell (e.g., CTL019), comprising a step of decreasing the level of 5-hydroxymethylcytosine in said cell. In some embodiments, said step comprises contacting said cells with a Tet (e.g., Tet1, Tet2, and/or Tet3) inhibitor. In some embodiments, said Tet inhibitor is a Tet2 inhibitor. In some embodiments, a Tet (e.g., Tet1, Tet2, and/or Tet3) inhibitor of the present invention is selected from the group consisting of: (1) a gene editing system targeted to one or more sites within the gene encoding Tet1, Tet2, or Tet3, or its corresponding regulatory elements; (2) a nucleic acid (e.g., an siRNA or shRNA) that inhibits expression of Tet1, Tet2, or Tet3; (3) a protein (e.g., a dominant negative, e.g., catalytically inactive) Tet1, Tet2, or Tet3, or a binding partner of Tet1, Tet2, or Tet3; (4) a small molecule that inhibits expression and/or function of Tet1, Tet2, or Tet3; (5) a nucleic acid encoding any of (1)-(3); and (6) any combination of (1) -(5). In some embodiments, the Tet inhibitor of the present invention is a Tet2 inhibitor.

In one aspect, the present invention provides a method of increasing the therapeutic efficacy of a CAR-expressing cell, e.g., a cell of any of the previous claims, e.g., a CAR19-expressing cell (e.g., CTL019), comprising a step of decreasing the level of 5-hydroxymethylcytosine in said cell. In some embodiments, said step comprises contacting said cells with a Tet (e.g., Tet1, Tet2, and/or Tet3) inhibitor. In some embodiments, said contacting occurs *ex vivo.* In some embodiments, said contacting occurs *in vivo.* In some embodiments, said contacting occurs *in vivo* prior to delivery of nucleic acid encoding a CAR into the cell. In some embodiments, said contacting occurs *in vivo* after the cells have been administered to a subject in need thereof.

In one aspet, the present invention provides a method of increasing the therapeutic efficacy of a CAR-expressed cell, e.g., a cell of any of the previous claims, e.g., a CAR19-expressing cell (e.g., CTL019), comprising a step of contacting said cell with a Tet inhibitor, e.g., a Tet1, Tet2 and/or Tet3 inhibitor. In some embodiments, said Tet inhibitor is a Tet2 inhibitor. In some embodiments, a Tet (e.g., Tet1, Tet2, and/or Tet3) inhibitor of the present invention is selected from the group consisting of: (1) a gene editing system targeted to one or more sites within the gene encoding Tet1, Tet2, or Tet3, or its corresponding regulatory elements; (2) a nucleic acid (e.g., an siRNA or shRNA) that inhibits expression of Tet1, Tet2, or Tet3; (3) a protein (e.g., a dominant negative, e.g., catalytically inactive) Tet1, Tet2, or Tet3, or a binding partner of Tet1, Tet2, or Tet3; (4) a small molecule that inhibits expression and/or function of Tet1, Tet2, or Tet3; (5) a nucleic acid encoding any of (1)-(3); and (6) any combination of (1) -(5). In some embodiments, the Tet inhibitor of the present invention is a Tet2 inhibitor.

In one aspet, the present invention provides a method of increasing the therapeutic efficacy of a CAR-expressed cell, e.g., a cell of any of the previous claims, e.g., a CAR19-expressing cell (e.g., CTL019), comprising a step of contacting said cell with a Tet inhibitor, e.g., a Tet1, Tet2 and/or Tet3 inhibitor. In some embodiments, said step comprises contacting said cells with a Tet (e.g., Tet1, Tet2, and/or Tet3) inhibitor. In some embodiments, said contacting occurs *ex vivo.* In some embodiments, said contacting occurs *in vivo.* In some embodiments, said contacting occurs *in vivo* prior to delivery of nucleic acid encoding a CAR into the cell. In some embodiments, said contacting occurs *in vivo* after the cells have been administered to a subject in need thereof.

In one aspects, the present invention provides a method of treating a subject in need thereof, comprising administering to said subject an effective amount of the cells as described herein, e.g., an immune effector cell (e.g., T cell or NK cell) comprising a CAR, and, optionally, administering to said subject a Tet1, Tet2, and/or Tet3 inhibitor. In some embodiments, the subject receives a pre-treatment of the Tet1, Tet2 and/or Tet3 inhibitor, and prior to the initiation of the CAR-expressing cell therapy. In some embodiments, the subject receives concurrent treatment with a Tet1, Tet2, and/or Tet3 inhibitor and the CAR expressing cell therapy. In some embodiments, the subject receives treatment with a Tet1, Tet2, and/or Tet3 inhibitor post-CAR-expressing cell therapy. In some embodiments, the subject has a disease associated with expression of a tumor antigen, e.g., a proliferative disease, a precancerous condition, a cancer, and a non-cancer related indication associated with expression of the tumor antigen. In some embodiments, the subject has a hematologic cancer chosen from one or more of chronic lymphocytic leukemia (CLL), acute leukemias, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or preleukemia.

The present invention provides uses of the compositions and/or methods described here for treatment of cancer, wherein the cancer is selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

The present invention provides Tet1, Tet2 and/or Tet3 inhibitors for use in the treatment of a subject, wherein said subject has received, is receiving, or is about to receive therapy comprising a CAR-expressing cell.

The present invention further provides a method of manufacturing a CAR-expressing cell, comprising introducing nucleic acid encoding a CAR into a cell such that said nucleic acid (or CAR-encoding portion thereof) integrates into the genome of the cell within a Tet1, Tet2 and/or Tet3 gene (e.g., within an intron or exon of a Tet1, Tet2 and/or Tet3 gene), such that Tet1, Tet2 and/or Tet3 expression and/or function is reduced or eliminated.

The present invention further provides a method of manufacturing a CAR-expressing cell, comprising contacting said CAR-expressing cell *ex vivo* with a Tet1, Tet2 and/or Tet3 inhibitor. In some embodiments, the inhibitor is a Tet2 inhibitor.

The present invention further provides a vector comprising sequence encoding a CAR and sequence encoding a Tet inhibitor, e.g., a Tet1, Tet2, and/or Tet3 inhibitor. In some embodiments, the Tet inhibitor is a (1) a gene editing system targeted to one or more sites within the gene encoding Tet1, Tet2, or Tet3, or its corresponding regulatory elements; (2) a nucleic acid (e.g., an siRNA or shRNA) that inhibits expression of Tet1, Tet2, or Tet3; (3) a protein (e.g., a dominant negative, e.g., catalytically inactive) Tet1, Tet2, or Tet3, or a binding partner of Tet1, Tet2, or Tet3; and (4) a nucleic acid encoding any of (1)-(3), or combinations thereof. In some embodiments, the sequence encoding a CAR and the sequence encoding a Tet inhibitor are separated by a 2A site.

The present invention further provides a gene editing system that is specific for a sequence of a Tet gene or its regulatory elements, e.g., a Tet1, Tet2 or Tet3 gene or its regulatory elements. In some embodiments, the gene editing system is specific for a sequence of a Tet2 gene. In some embodiments, the gene editing system is (1) a CRISPR/Cas gene editing system, (2) a zinc finger nuclease system, a TALEN system and a meganuclease system. In some embodiments, the gene editing system is a CRISPR/Cas gene editing system. In some embodiments, the gene editing system comprises: a gRNA molecule comprising a targeting sequence specific to a sequence of a Tet2 gene or its regulatory elements, and a Cas9 protein; a gRNA molecule comprising a targeting sequence specific to a sequence of a Tet2 gene or its regulatory elements, and a nucleic acid encoding a Cas9 protein; a nucleic acid encoding a gRNA molecule comprising a targeting sequence specific to a sequence of a Tet2 gene or its regulatory elements, and a Cas9 protein; or a nucleic acid encoding a gRNA molecule comprising a targeting sequence specific to a sequence of a Tet2 gene or its regulatory elements, and a nucleic acid encoding a Cas9 protein. In some embodiments, the gene editing system further comprises a template DNA. In some embodiments, the template DNA comprises nucleic acid sequence encoding a CAR, e.g., a CAR as described herein.

The present invention further provides a composition for the *ex vivo* manufacture of a CAR-expressing cell, comprising a Tet inhibitor, e.g., a Tet1, Tet2, and/or Tet3 inhibitor, e.g., a Tet2 inhibitor. In some embodiments, the Tet inhibitor is selected from N-[3-[7-(2,5-dimethyl-2H-pyrazol-3-ylamino)-1-methyl-2-oxo-1,4-dihydro-2H-pyrimido[4,5-d]pyrimidin-3-yl]-4-methylphenyl]-3-trifluoromethyl-benzamide, 2-[(2,6-dichloro-3-methylphenyl)amino]benzoic acid and 2-hydroxyglutarate.

The present invention further provides a population of cells comprising one or more cells described herein, wherein the population of cells comprises a higher percentage of Tscm cells (e.g., CD45RA+CD62L+CCR7+CD27+CD95+ T cells) than a population of cells which does not comprise one or more cells in which expression and/or function of Tet1, Tet2 and/or Tet3 in said cell has been reduced or eliminated.

### BRIEF DESCRIPTION OF THE DRAWINGS

**FIG. 1****:** CD19-expressing CART cells were administered to a patient (UPCC04409-10) for the treatment of CLL. CART cells in patient UPCC04409-10 were monitored over time by sampling blood. The amount of BBZ expression in cells was determined (red). The number of copies of sequence from the Vbeta5.1 TCR family was determined (blue). Both measurements were made from samples collected on the indicated days after the second infusion of CART cells.
**FIGS. 2A and 2B****:** The T-cell receptor repertoire from patient UPCC04409-10 was determined from a sample collected on day 28 (FIG. 2A) or day 51 (FIG. 2B) after CART infusion. This demonstrates the abundance of the TCRBV05-01 family of T-cell receptors at day 51 indicating clonal expansion over time.
**FIG. 3****:** The T-cells isolated from patient UPCC04409-10 were analyzed for the simultaneous expression of CAR19 and 2 different TCR family genes over time (day 50 and day 51) and compared to the input dosed material (product): upper panel is TCR family Vb13.1; the lower panel shows TCR family Vb5.1. The data demonstrate that the CAR19 positive cells contain a single TCR family gene (Vb5.1) that becomes rapidly enriched between days 50 and 51.
**FIG. 4****:** The T-cell receptor repertoire of CD8 positive cells from patient UPCC04409-10 was determined from a sample collected on day 51 after CART infusion. This demonstrates the abundance of the TCRBV05-01 family of T-cell receptors at day 51 indicating clonal expansion of CD8 positive cells over time.

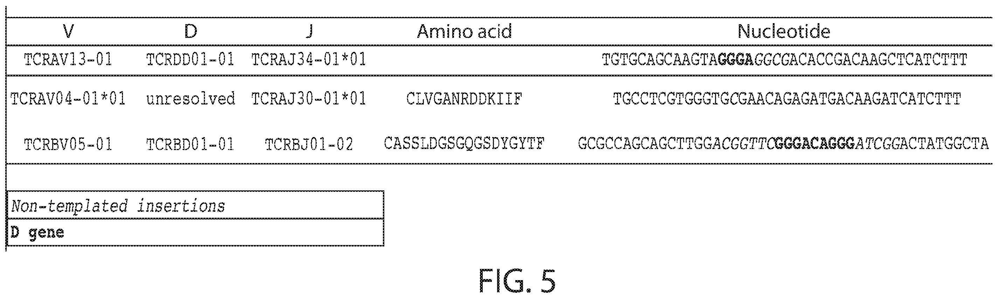

**FIG. 5****:** The T-cell receptor from patient UPCC04409-10 was sequenced and the sequence of the alpha and beta chains are shown (Amino Acid sequences disclosed as SEQ ID NOS: 1297-1298 and Nucleotide sequences disclosed as SEQ ID NOS: 1299-1301, all respectively, in order of appearance).
**FIG. 6****:** Sonically fragmented DNA was generated from T-cells from Patient UPCC04409-10. This material was used to amplify genomic sequences adjacent to the CAR19 insertion. The genes indicated were identified as being enriched relative to the infused product (D0) adjacent to CAR19 in the genome. At the different time points after CART infusion indicated (d=day; m=month), a different relative abundance of adjacent genes was seen, with Tet2 abundance peaking in both peripheral blood (PBMC) and CAR+CD8+ T-cells samples at day 51.
**FIG. 7****:** The site of insertion of the CAR19 gene was mapped to the Tet2 gene. More specifically, the insertion occurred between exons 9 and 10 of the Tet2 gene. The catalytic domain for Tet2 resides in exon 11. The insertion at this location may lead to expression of aberrant mRNA transcripts or decrease the expression of functional (wild-type) Tet2.

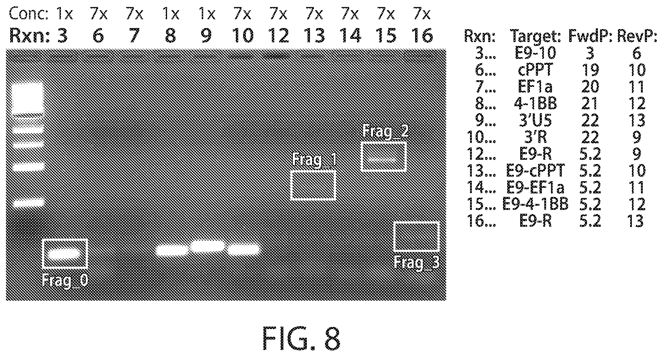

**FIG. 8****:** Transcripts of the Tet2 gene from mRNA isolated from patient UPCC04409-10 were evaluated by RTPCR using primers spanning the indicated regions of Tet2 or CAR19 or both as indicated in the right hand side of the figure. Rxn 3 contains primers designed to amplify the region of the Tet2 transcript spanning exons 9 and 10. Rxn, 6, 7, 8, 9, and 10 are primers designed to amplify the indicated portions of the CAR19 lentivirus. Rxn 12-16 are pairs of primers that contain exon 9 sequence of the Tet2 transcript as well as sequence from the CAR19 lentiviral construct. These data show that transcripts are made from the Tet2 locus that contains both Tet2 sequence as well as CAR19 sequence.

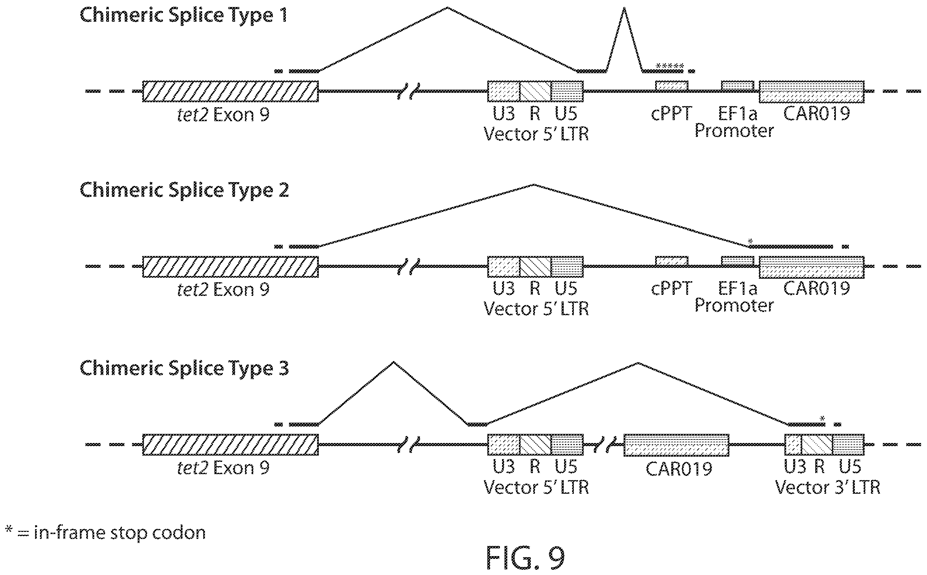

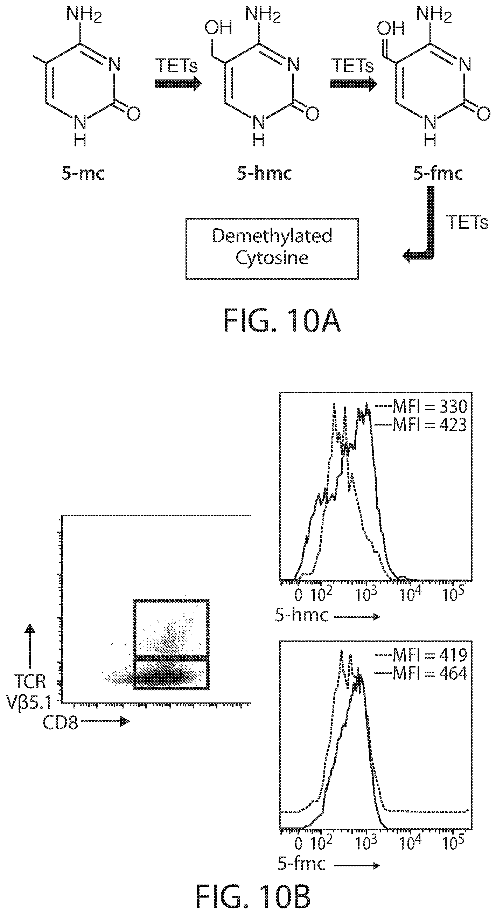

**FIG. 9****:** A schematic representation of the transcripts derived from the Tet2 locus discovered in FIGS. 10A and 10B is shown. This figure indicates splice variants of this Tet2/CAR19 fusion that were detected in the patient sample. This analysis has revealed that the CAR19 insertion into Tet2 has resulted in transcripts containing stop codons upstream of exon 11. Exon 11 has been demonstrated to be important for Tet2 function. This suggests Tet2 function has been disrupted by the insertion of the CAR19. This also suggests that the disruption of Tet2 function has resulted in favorable expansion of this individual CART clones.
**FIGS. 10A and 10B****:** The enzymatic activity of Tet2 is schematized (FIG. 10A). Tet family protein convert 5-methylcytosine (5-mc) to 5-hydroxymethylcytosine (5-hmc) and then into 5-formylcytosine (5-fmc) resulting in demethylated cytosine. Methylated DNA is an epigenetic state that is known to affect transcriptional profiles. The methylation state of the T-cells from patient UPCC04409-10 was evaluated (FIG. 10B). The patient's T-cells were stained for TCRVb5.1 (which contain the CAR19 insertion at Tet2) and the 5-hmc and 5-fmc were evaluated in TCRVb5.1 positive (red) and TCRVb5.1 negative (blue) populations by flow cytometry. This data indicates that the cells containing the insertion of CAR19 in the Tet2 gene are defective in demethylation.
**FIG.11****:** TET2 shRNAs reduce 5-hmc levels in normal human T cells. TET2 and scramble control shRNA constructs expressing mCherry were introduced into normal human T cells. 5-hmc levels were determined by intracellular staining by FACS on day 6 following expansion with anti-CD3/CD28 beads. Knockdown of TET2 reduced overall 5-hmc levels.
**FIG. 12****:** TET2 shRNAs expand Tscm T cells. TET2 and scramble control shRNA constructs expressing mCherry were introduced into normal human T cells. CD45RA+CD62L+CCR7+CD27+CD95+ Tscm T cells were determined by FACS staining on day 11 following expansion with anti-CD3/CD28 beads. Knockdown of TET2 promoted the expansion of T cells with a Tscm phenotype.
**FIG. 13A****:** Gating strategy for quantification of CAR+ cells.
**FIG. 13B****:** CAR expression levels in cells electroporated with CRISPR/Cas systems targeting Tet2, as compared with untransfected cells.
**FIG. 14****:** Quantitation of CD4+ and CD8+ cells after CAR transduction and Tet2 editing.
**FIG. 15****:** Effect of Tet2 inhibition on CD3/CD28 bead expansion of CAR T cells.
**FIG. 16****:** Effect of Tet2 inhibition on antigen-dependendent interleukin-2 (IL-2) production by CAR T cells.
**FIG. 17****:** Effect of Tet2 inhibition on antigen-dependendent interferon gamma production by CAR T cells.
**FIG. 18****:** Effect of Tet2 inhibition on antigen-driven CAR+ T cell proliferation.
**FIG. 19****:** Effect of Tet2 inhibition on antigen-driven T cell proliferation.
**FIG. 20****:** Effect of Tet2 inhibition on antigen-driven CD4+ T cell proliferation.
**FIG. 21****:** Effect of Tet2 inhibition on antigen-driven CAR+ CD4+ T cell proliferation.
**FIG. 22****:** Effect of Tet2 inhibition on antigen-driven CD8+ T cell proliferation.
**FIG. 23****:** Effect of Tet2 inhibition on antigen-driven CAR+ CD8+ T cell proliferation.
**FIG. 24****:** % editing, and % frame shift edit by introduction of CRISPR/Cas systems targeting Tet2 as measured by NGS.
**FIG. 25****:** Top 5 most frequent indels observed in T cells after addition of RNP that included the indicated TET2 Exon 3-targeting gRNAs (SEQ ID NOS: 1302-1326, respectively, in order of appearance). Changes from the unmodified wt sequence are shown, with insertions represented with lowercase letters ("a". "t", "g" and "c") and deletions shown with a dash ("-"). Indel frequency is shown in the right-most column.
**FIG. 26****:** Top 5 most frequent indels observed in T cells after addition of RNP that included the indicated TET2 Exon 9 -targeting gRNAs (SEQ ID NOS: 1327-1356, respectively, in order of appearance). Changes from the unmodified wt sequence are shown, with insertions represented with lowercase letters ("a," "t," "g," and "c") and deletions shown with a dash ("-"). Indel frequency is shown in the right-most column.
**FIG. 27****:** Schematic experimental protocol for determination of TET2 knockdown in Jurkat cells in response to lentivirus encoding shRNA TET2 inhibitors.
**FIG. 28****:** RFP expression in shRNA infected Jurkat cells. RFP expression was determined by FACS on day 6 after puromycin treatment. Based on RFP expression, greater than 99% shRNA introduced jurkat cells were selected by puromycin treatment.
**FIG. 29****:** Knockdown efficiency of *tet*2 in TET2 shRNAs infected Jurkat cells. qRT-PCR experiment was performed. The expression levels of tet1 and tet3 were also measured. β-actin serves as an internal control to quantify relative gene expression among samples tested. To increase reliability of qRT-PCR, two β-actin primers and one RPLP1 primer were used in this experiment.
**FIG. 30****:** Knockdown of TET2 protein in response to shRNAs in Jurkat cells. A western blot experiment was performed.

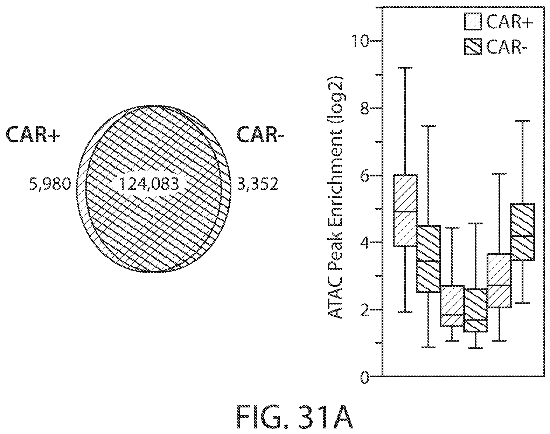

**FIG. 31A****:** Venn diagrams of ATAC peaks in the CAR+CD8+ T cells from a patient with a Tet2 disruption compared to CAR-CD8+ T cells from the same patient at the matched time point without the Tet2 disruption. The box plots show differences in ATAC enrichment between the two cell populations.

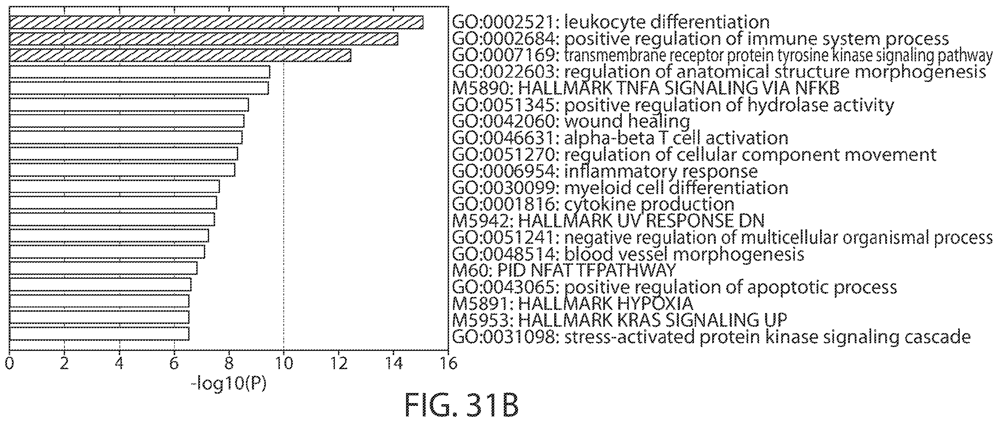

**FIG. 31B****:** GO terms associated with ATAC peaks more closed in the cell population with the Tet2 disruption, compared to its counterpart.
**FIG. 32A****:** Silencing of Tet2 by shRNA in primary CD8+ T cells from healthy donors as measured by quantitative PCR. Expression (mean, SEM) normalized to GAPDH is presented as fold change relative to non-targeting control shRNA.
**FIGS. 32B and 32C****:** Relative frequencies of central memory (FIG. 32B) and effector CD8+ T cells (FIG. 32C) at day 14 post-expansion via CD3/CD28 stimulation in the same healthy donors as presented in A.

### DETAILED DESCRIPTION

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some aspects, the set of polypeptides are contiguous with eachother. In some embodiments, the set of polypeptides include a dimerization switch that, upon the presence of a dimerization molecule, can couple the polypeptides to one another, e.g., can couple an antigen binding domain to an intracellular signaling domain. In one aspect, the stimulatory molecule is the zeta chain associated with the T cell receptor complex. In one aspect, the cytoplasmic signaling domain further comprises one or more functional signaling domains derived from at least one costimulatory molecule as defined below. In one aspect, the costimulatory molecule is chosen from the costimulatory molecules described herein, e.g., 4-1BB (i.e., CD137), CD27 and/or CD28. In one aspect, the CAR comprises a chimeric fusion protein comprising an extracellular antigen binding domain, a transmembrane domain and an intracellular signaling domain comprising a functional signaling domain derived from a stimulatory molecule. In one aspect, the CAR comprises a chimeric fusion protein comprising an extracellular antigen binding domain, a transmembrane domain and an intracellular signaling domain comprising a functional signaling domain derived from a costimulatory molecule and a functional signaling domain derived from a stimulatory molecule. In one aspect, the CAR comprises a chimeric fusion protein comprising an extracellular antigen binding domain, a transmembrane domain and an intracellular signaling domain comprising two functional signaling domains derived from one or more costimulatory molecule(s) and a functional signaling domain derived from a stimulatory molecule. In one aspect, the CAR comprises a chimeric fusion protein comprising an extracellular antigen binding domain, a transmembrane domain and an intracellular signaling domain comprising at least two functional signaling domains derived from one or more costimulatory molecule(s) and a functional signaling domain derived from a stimulatory molecule. In one aspect, the CAR comprises an optional leader sequence at the amino-terminus (N-ter) of the CAR fusion protein. In one aspect, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane.

A CAR that comprises an antigen binding domain (e.g., a scFv, or TCR) that targets a specific tumor maker X, such as those described herein, is also referred to as XCAR. For example, a CAR that comprises an antigen binding domain that targets CD19 is referred to as CD19CAR.

The term "signaling domain" refers to the functional portion of a protein which acts by transmitting information within the cell to regulate cellular activity via defined signaling pathways by generating second messengers or functioning as effectors by responding to such messengers.

The term "antibody," as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be tetramers of immunoglobulin molecules.

The term "antibody fragment" refers to at least one portion of an antibody, that retains the ability to specifically interact with (e.g., by binding, steric hinderance, stabilizing/destabilizing, spatial distribution) an epitope of an antigen. Examples of antibody fragments include, but are not limited to, Fab, Fab', F(ab')₂, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide brudge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The portion of the CAR of the invention comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the invention comprises an antibody fragment. In a further aspect, the CAR comprises an antibody fragment that comprises a scFv. The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof.

As used herein, the term "binding domain" or "antibody molecule" refers to a protein, e.g., an immunoglobulin chain or fragment thereof, comprising at least one immunoglobulin variable domain sequence. The term "binding domain" or "antibody molecule" encompasses antibodies and antibody fragments. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The portion of the CAR of the invention comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody, or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the invention comprises an antibody fragment. In a further aspect, the CAR comprises an antibody fragment that comprises a scFv.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (K) and lambda (λ) light chains refer to the two major antibody light chain isotypes.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated synthesized or can be derived from a biological sample, or might be macromolecule besides a polypeptide. Such a biological sample can include, but is not limited to a tissue sample, a tumor sample, a cell or a fluid with other biological components.

The term "anti-cancer effect" refers to a biological effect which can be manifested by various means, including but not limited to, e.g., a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition. An "anti-cancer effect" can also be manifested by the ability of the peptides, polynucleotides, cells and antibodies in prevention of the occurrence of cancer in the first place. The term "anti-tumor effect" refers to a biological effect which can be manifested by various means, including but not limited to, e.g., a decrease in tumor volume, a decrease in the number of tumor cells, a decrease in tumor cell proliferation, or a decrease in tumor cell survival.

The term "autologous" refers to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically

The term "xenogeneic" refers to a graft derived from an animal of a different species.

The term "cancer" refers to a disease characterized by the uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

"Derived from" as that term is used herein, indicates a relationship between a first and a second molecule. It generally refers to structural similarity between the first molecule and a second molecule and does not connotate or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connotate or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

The phrase "disease associated with expression of a tumor antigen as described herein" includes, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation. In some embodiments, the tumor antigen-expressing cells express, or at any time expressed, mRNA encoding the tumor antigen. In an embodiment, the tumor antigen -expressing cells produce the tumor antigen protein (e.g., wild-type or mutant), and the tumor antigen protein may be present at normal levels or reduced levels. In an embodiment, the tumor antigen -expressing cells produced detectable levels of a tumor antigen protein at one point, and subsequently produced substantially no detectable tumor antigen protein.

The term "conservative sequence modifications" refers to amino acid modifications that do not significantly affect or alter the binding characteristics of the antibody or antibody fragment containing the amino acid sequence. Such conservative modifications include amino acid substitutions, additions and deletions. Modifications can be introduced into an antibody or antibody fragment of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a CAR of the invention can be replaced with other amino acid residues from the same side chain family and the altered CAR can be tested using the functional assays described herein.

The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules.

The term "stimulatory molecule," refers to a molecule expressed by an immune cell (e.g., T cell, NK cell, B cell) that provides the cytoplasmic signaling sequence(s) that regulate activation of the immune cell in a stimulatory way for at least some aspect of the immune cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing cytoplasmic signaling sequence that is of particular use in the invention includes, but is not limited to, those derived from CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12. In a specific CAR of the invention, the intracellular signaling domain in any one or more CARS of the invention comprises an intracellular signaling sequence, e.g., a primary signaling sequence of CD3-zeta. In a specific CAR of the invention, the primary signaling sequence of CD3-zeta is the sequence provided as SEQ ID NO: 18, or the equivalent residues from a non-human species, e.g., mouse, rodent, monkey, ape and the like. In a specific CAR of the invention, the primary signaling sequence of CD3-zeta is the sequence as provided in SEQ ID NO: 20, or the equivalent residues from a non-human species, e.g., mouse, rodent, monkey, ape and the like.

The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

An "intracellular signaling domain," as the term is used herein, refers to an intracellular portion of a molecule. The intracellular signaling domain generates a signal that promotes an immune effector function of the CAR containing cell, e.g., a CART cell. Examples of immune effector function, e.g., in a CART cell, include cytolytic activity and helper activity, including the secretion of cytokines.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CART, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12.

The term "zeta" or alternatively "zeta chain", "CD3-zeta" or "TCR-zeta" is defined as the protein provided as GenBan Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain, or functional derivatives thereof, that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO: 18. In one aspect, the "zeta stimulatory domain" or a "CD3-zeta stimulatory domain" is the sequence provided as SEQ ID NO: 20.

The term a "costimulatory molecule" refers to a cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are contribute to an efficient immune response. Costimulatory molecules include, but are not limited to an MHC class I molecule, BTLA and a Toll ligand receptor, as well as OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

A costimulatory intracellular signaling domain can be the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, NKG2D, SLAMF7, NKp80, NKp30, NKp44, NKp46, CD160, B7-H3, and a ligand that specifically binds with CD83, and the like.

The intracellular signaling domain can comprise the entire intracellular portion, or the entire native intracellular signaling domain, of the molecule from which it is derived, or a functional fragment or derivative thereof.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc. No. AAA62478.2, or the equivalent residues from a non-human species, e.g., mouse, rodent, monkey, ape and the like. In one aspect, the "4-1BB costimulatory domain" is the sequence provided as SEQ ID NO: 14 or the equivalent residues from a non-human species, e.g., mouse, rodent, monkey, ape and the like.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloic-derived phagocytes.

"Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and co-stimulation are examples of immune effector function or response.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "effective amount" or "therapeutically effective amount" are used interchangeably herein, and refer to an amount of a compound, formulation, material, or composition, as described herein effective to achieve a particular biological result.

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, e.g., the LENTIVECTOR^{®} gene delivery technology from Oxford BioMedica, the LENTIMAX^{™} vector system from Lentigen and the like. Nonclinical types of lentiviral vectors are also available and would be known to one skilled in the art.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody or antibody fragment, where the whole molecule is of human origin or consists of an amino acid sequence identical to a human form of the antibody or immunoglobulin.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

The terms "cancer associated antigen" or "tumor antigen" interchangeably refers to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some enbodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the CARs of the present invention includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Blood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library.

The term "tumor-supporting antigen" or "cancer-supporting antigen" interchangeably refer to a molecule (typically a protein, carbohydrate or lipid) that is expressed on the surface of a cell that is, itself, not cancerous, but supports the cancer cells, e.g., by promoting their growth or survival e.g., resistance to immune cells. Exemplary cells of this type include stromal cells and myeloid-derived suppressor cells (MDSCs). The tumor-supporting antigen itself need not play a role in supporting the tumor cells so long as the antigen is present on a cell that supports cancer cells.

The term "flexible polypeptide linker" or "linker" as used in the context of a scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser)n, where n is a positive integer equal to or greater than 1. For example, n=1, n=2, n=3. n=4, n=5 and n=6, n=7, n=8, n=9 and n=10 (SEQ ID NO:28). In one embodiment, the flexible polypeptide linkers include, but are not limited to, (Gly4 Ser)4 (SEQ ID NO:29) or (Gly4 Ser)3 (SEQ ID NO:30). In another embodiment, the linkers include multiple repeats of (Gly2Ser), (GlySer) or (Gly3Ser) (SEQ ID NO:31). Also included within the scope of the invention are linkers described in WO2012/138475, incorporated herein by reference).

As used herein, a 5' cap (also termed an RNA cap, an RNA 7-methylguanosine cap or an RNA m⁷G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a capsynthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, preferably mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In the preferred embodiment of a construct for transient expression, the polyA is between 50 and 5000 (SEQ ID NO: 34), preferably greater than 64, more preferably greater than 100, most preferably greater than 300 or 400. poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

As used herein, the terms "treat", "treatment" and "treating" refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a CAR of the invention). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human).

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

The term "therapeutic" as used herein means a treatment. A therapeutic effect is obtained by reduction, suppression, remission, or eradication of a disease state.

The term "prophylaxis" as used herein means the prevention of or protective treatment for a disease or disease state.

In the context of the present invention, "tumor antigen" or "hyperproliferative disorder antigen" or "antigen associated with a hyperproliferative disorder" refers to antigens that are common to specific hyperproliferative disorders. In certain aspects, the hyperproliferative disorder antigens of the present invention are derived from, cancers including but not limited to primary or metastatic melanoma, thymoma, lymphoma, sarcoma, lung cancer, liver cancer, non-Hodgkin lymphoma, Hodgkin lymphoma, leukemias, uterine cancer, cervical cancer, bladder cancer, kidney cancer and adenocarcinomas such as breast cancer, prostate cancer, ovarian cancer, pancreatic cancer, and the like.

The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The term "specifically binds," refers to an antibody, or a ligand, which recognizes and binds with a binding partner (e.g., a tumor antigen) protein present in a sample, but which antibody or ligand does not substantially recognize or bind other molecules in the sample.

"Regulatable chimeric antigen receptor (RCAR),"as that term is used herein, refers to a set of polypeptides, typically two in the simplest embodiments, which when in a RCARX cell, provides the RCARX cell with specificity for a target cell, typically a cancer cell, and with regulatable intracellular signal generation or proliferation, which can optimize an immune effector property of the RCARX cell. An RCARX cell relies at least in part, on an antigen binding domain to provide specificity to a target cell that comprises the antigen bound by the antigen binding domain. In an embodiment, an RCAR includes a dimerization switch that, upon the presence of a dimerization molecule, can couple an intracellular signaling domain to the antigen binding domain.

"Membrane anchor" or "membrane tethering domain", as that term is used herein, refers to a polypeptide or moiety, e.g., a myristoyl group, sufficient to anchor an extracellular or intracellular domain to the plasma membrane.

"Switch domain," as that term is used herein, e.g., when referring to an RCAR, refers to an entity, typically a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the first and second switch domains are different from one another, e.g., they are polypeptides having different primary amino acid sequences, and are referred to collectively as a heterodimerization switch. In embodiments, the switch is intracellular. In embodiments, the switch is extracellular. In embodiments, the switch domain is a polypeptide-based entity, e.g., FKBP or FRB-based, and the dimerization molecule is small molecule, e.g., a rapalogue. In embodiments, the switch domain is a polypeptide-based entity, e.g., an scFv that binds a myc peptide, and the dimerization molecule is a polypeptide, a fragment thereof, or a multimer of a polypeptide, e.g., a myc ligand or multimers of a myc ligand that bind to one or more myc scFvs. In embodiments, the switch domain is a polypeptide-based entity, e.g., myc receptor, and the dimerization molecule is an antibody or fragments thereof, e.g., myc antibody.

"Dimerization molecule," as that term is used herein, e.g., when referring to an RCAR, refers to a molecule that promotes the association of a first switch domain with a second switch domain. In embodiments, the dimerization molecule does not naturally occur in the subject, or does not occur in concentrations that would result in significant dimerization. In embodiments, the dimerization molecule is a small molecule, e.g., rapamycin or a rapalogue, e.g, RAD001.

The term "bioequivalent" refers to an amount of an agent other than the reference compound (e.g., RAD001), required to produce an effect equivalent to the effect produced by the reference dose or reference amount of the reference compound (e.g., RAD001). In an embodiment the effect is the level of mTOR inhibition, e.g., as measured by P70 S6 kinase inhibition, e.g., as evaluated in an in vivo or in vitro assay, e.g., as measured by an assay described herein, e.g., the Boulay assay. In an embodiment, the effect is alteration of the ratio of PD-1 positive/PD-1 negative T cells, as measured by cell sorting. In an embodiment a bioequivalent amount or dose of an mTOR inhibitor is the amount or dose that achieves the same level of P70 S6 kinase inhibition as does the reference dose or reference amount of a reference compound. In an embodiment, a bioequivalent amount or dose of an mTOR inhibitor is the amount or dose that achieves the same level of alteration in the ratio of PD-1 positive/PD-1 negative T cells as does the reference dose or reference amount of a reference compound.

The term "low, immune enhancing, dose" when used in conjunction with an mTOR inhibitor, e.g., an allosteric mTOR inhibitor, e.g., RAD001 or rapamycin, or a catalytic mTOR inhibitor, refers to a dose of mTOR inhibitor that partially, but not fully, inhibits mTOR activity, e.g., as measured by the inhibition of P70 S6 kinase activity. Methods for evaluating mTOR activity, e.g., by inhibition of P70 S6 kinase, are discussed herein. The dose is insufficient to result in complete immune suppression but is sufficient to enhance the immune response. In an embodiment, the low, immune enhancing, dose of mTOR inhibitor results in a decrease in the number of PD-1 positive T cells and/or an increase in the number of PD-1 negative T cells, or an increase in the ratio of PD-1 negative T cells/PD-1 positive T cells. In an embodiment, the low, immune enhancing, dose of mTOR inhibitor results in an increase in the number of naive T cells. In an embodiment, the low, immune enhancing, dose of mTOR inhibitor results in one or more of the following:
an increase in the expression of one or more of the following markers: CD62L^{high} CD127^{high}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{high}, increased CD127^{high}, increased CD27⁺, decreased KLRG1, and increased BCL2;
wherein any of the changes described above occurs, e.g., at least transiently, e.g., as compared to a non-treated subject.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become resistant during a treatment. A refractory cancer is also called a resistant cancer.

"Relapsed" as used herein refers to the return of a disease (e.g., cancer) or the signs and symptoms of a disease such as cancer after a period of improvement, e.g., after prior treatment of a therapy, e.g., cancer therapy
Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

"Tet" as the term is used herein, refers to the family of genes, and the proteins encoded by said genes, of the ten-eleven translocation methlcytosine dioxygenase family. Tet includes, for example, Tet1, Tet2 and Tet3.

"Tet2" as the term is used herein, refers to gene, tet methylcytosine dioxygenase 2, and the protein encoded by said gene, the tet2 methylcytosine dioxygenase, which catalyzes the conversion of methylcytosine to 5-hydroxymethylcytosine. It is sometimes also referred to as "KIAA1546," "FLJ20032" and "tet oncogene family member 2" The encoded protein is involved in myelopoiesis, and defects in this gene have been associated with several myeloproliferative disorders. In the human genome, TET2 is located on chromosome 4q24. Currently six TET2 isoforms have been described and their Genebank numbers are: NM_001127208.2; XM_005263082.1; XM_006714242.2; NM_017628.4; XM_011532044.1; and XM_011532043.1.

An example of the protein sequence of human Tet2 is provided as UniProt accession number Q6N021:

10 20 30 40 50
MEQDRTNHVE GNRLSPFLIP SPPICQTEPL ATKLQNGSPL PERAHPEVNG
60 70 80 90 100
DTKWHSFKSY YGIPCMKGSQ NSRVSPDFTQ ESRGYSKCLQ NGGIKRTVSE
110 120 130 140 150
PSLSGLLQIK KLKQDQKANG ERRNFGVSQE RNPGESSQPN VSDLSDKKES
160 170 180 190 200
VSSVAQENAV KDFTSFSTHN CSGPENPELQ ILNEQEGKSA NYHDKNIVLL
210 220 230 240 250
KNKAVLMPNG ATVSASSVEH THGELLEKTL SQYYPDCVSI AVQKTTSHIN
260 270 280 290 300
AINSQATNEL SCEITHPSHT SGQINSAQTS NSELPPKPAA VVSEACDADD
310 320 330 340 350
ADNASKLAAM LNTCSFQKPE QLQQQKSVFE ICPSPAENNI QGTTKLASGE
360 370 380 390 400
EFCSGSSSNL QAPGGSSERY LKQNEMNGAY FKQSSVFTKD SFSATTTPPP
410 420 430 440 450
PSQLLLSPPP PLPQVPQLPS EGKSTLNGGV LEEHHHYPNQ SNTTLLREVK
460 470 480 490 500
IEGKPEAPPS QSPNPSTHVC SPSPMLSERP QNNCVNRNDI QTAGTMTVPL
510 520 530 540 550
CSEKTRPMSE HLKHNPPIFG SSGELQDNCQ QLMRNKEQEI LKGRDKEQTR
560 570 580 590 600
DLVPPTQHYL KPGWIELKAP RFHQAESHLK RNEASLPSIL QYQPNLSNQM
610 620 630 640 650
TSKQYTGNSN MPGGLPRQAY TQKTTQLEHK SQMYQVEMNQ GQSQGTVDQH
660 670 680 690 700
LQFQKPSHQV HFSKTDHLPK AHVQSLCGTR FHFQQRADSQ TEKLMSPVLK
710 720 730 740 750
QHLNQQASET EPFSNSHLLQ HKPHKQAAQT QPSQSSHLPQ NQQQQKLQI
760 770 780 790 800
KNKEEILQTF PHPQSNNDQQ REGSFFGQTK VEECFHGENQ YSKSSEFETH
810 820 830 840 850
NVQMGLEEVQ NINRRNSPYS QTMKSSACKI QVSCSNNTHL VSENKEQTTH
860 870 880 890 900
PELFAGNKTQ NLHHMQYFPN NVIPKQDLLH RCFQEQEQKS QQASVLQGYK

910 920 930 940 950
NRNQDMSGQQ AAQLAQQRYL IHNHANVFPV PDQGGSHTQT PPQKDTQKHA
960 970 980 990 1000
ALRWHLLQKQ EQQQTQQPQT ESCHSQMHRP IKVEPGCKPH ACMHTAPPEN
1010 1020 1030 1040 1050
KTWKKVTKQE NPPASCDNVQ QKSIIETMEQ HLKQFHAKSL FDHKALTLKS
1060 1070 1080 1090 1100
QKQVKVEMSG PVTVLTRQTT AAELDSHTPA LEQQTTSSEK TPTKRTAASV
1110 1120 1130 1140 1150
LNNFIESPSK LLDTPIKNLL DTPVKTQYDF PSCRCVEQII EKDEGPFYTH
1160 1170 1180 1190 1200
LGAGPNVAAI REIMEERFGQ KGKAIRIERV IYTGKEGKSS QGCPIAKWVV
1210 1220 1230 1240 1250
RRSSSEEKLL CLVRERAGHT CEAAVIVILI LVWEGIPLSL ADKLYSELTE
1260 1270 1280 1290 1300
TLRKYGTLTN RRCALNEERT CACQGLDPET CGASFSFGCS WSMYYNGCKF
1310 1320 1330 1340 1350
ARSKIPRKFK LLGDDPKEEE KLESHLQNLS TLMAPTYKKL APDAYNNQIE
1360 1370 1380 1390 1400
YEHRAPECRL GLKEGRPFSG VTACLDFCAH AHRDLHNMQN GSTLVCTLTR
1410 1420 1430 1440 1450
EDNREFGGKP EDEQLHVLPL YKVSDVDEFG SVEAQEEKKR SGAIQVLSSF
1460 1470 1480 1490 1500
RRKVRMLAEP VKTCRQRKLE AKKAAAEKLS SLENSSNKNE KEKSAPSRTK
1510 1520 1530 1540 1550
QTENASQAKQ LAELLRLSGP VMQQSQQPQP LQKQPPQPQQ QQRPQQQQPH
1560 1570 1580 1590 1600
HPQTESVNSY SASGSTNPYM RRPNPVSPYP NSSHTSDIYG STSPMNFYST
1610 1620 1630 1640 1650
SSQAAGSYLN SSNPMNPYPG LLNQNTQYPS YQCNGNLSVD NCSPYLGSYS
1660 1670 1680 1690 1700
PQSQPMDLYR YPSQDPLSKL SLPPIHTLYQ PRFGNSQSFT SKYLGYGNQN
1710 1720 1730 1740 1750
MQGDGFSSCT IRPNVHHVGK LPPYPTHEMD GHFMGATSRL PPNLSNPNMD
1760 1770 1780 1790 1800
YKNGEHHSPS HIIHNYSAAP GMFNSSLHAL HLQNKENDML SHTANGLSKM
1810 1820 1830 1840 1850
LPALNHDRTA CVQGGLHKLS DANGQEKQPL ALVQGVASGA EDNDEVWSDS
1860 1870 1880 1890 1900
EQSFLDPDIG GVAVAPTHGS ILIECAKREL HATTPLKNPN RNHPTRISLV
1910 1920 1930 1940 1950
FYQHKSMNEP KHGLALWEAK MAEKAREKEE ECEKYGPDYV PQKSHGKKVK
1960 1970 1980 1990 2000
REPAEPHETS EPTYLRFIKS LAERTMSVTT DSTVTTSPYA FTRVTGPYNR
2002
YI
[SEQ ID NO: 1357]

The tet2 gene is located on chromosome 4, location GRCh38.p2 (GCF_000001405.28) (NC_000004.12 (105145875 to 105279803); Gene ID 54790.

Examples of nucleic acid sequences encoding Tet2 are provided below. There are 6 identified isoforms of human Tet2 have been identified. The mRNA sequences are provided below (In embodiments, in each sequence, T may be replaced with U). In embodiments, Tet2 includes the proteins encoded by each of the sequences below:

| **NName** | **NCBI Reference Sequence** | **Sequence** |
|---|---|---|
| HHomo sapiens tet methylcytosine dioxygenase 2 (TET2), transcript variant 1, mRNA | NNM_00112 7208.2 | GGCAGTGGCAGCGGCGAGAGCTTGGGCGGCCGCCGCC TCCTCGCGAGCGCCGCGCGCCCGGGTCCCG CTCGCATGCAAGTCACGTCCGCCCCCTCGGCGCGGCCGCCC CGAGACGCCGGCCCCGCTGAGTGATGAGA ACAGACGTCAAACTGCCTTATGAATATTGATGCGGAGGCTA GGCTGCTTTCGTAGAGAAGCAGAAGGAAG CAAGATGGCTGCCCTTTAGGATTTGTTAGAAAGGAGACCCG ACTGCAACTGCTGGATTGCTGCAAGGCTG AGGGACGAGAACGAGGCTGGCAAACATTCAGCAGCACACC CTCTCAAGATTGTTTACTTGCCTTTGCTCC TGTTGAGTTACAACGCTTGGAAGCAGGAGATGGGCTCAGCA GCAGCCAATAGGACATGATCCAGGAAGAG CAGTAAGGGACTGAGCTGCTGAATTCAACTAGAGGGCAGC CTTGTGGATGGCCCCGAAGCAAGCCTGATG GAACAGGATAGAACCAACCATGTTGAGGGCAACAGACTAA GTCCATTCCTGATACCATCACCTCCCATTT GCCAGACAGAACCTCTGGCTACAAAGCTCCAGAATGGAAG CCCACTGCCTGAGAGAGCTCATCCAGAAGT AAATGGAGACACCAAGTGGCACTCTTTCAAAAGTTATTATG GAATACCCTGTATGAAGGGAAGCCAGAAT AGTCGTGTGAGTCCTGACTTTACACAAGAAAGTAGAGGGTA TTCCAAGTGTTTGCAAAATGGAGGAATAA AACGCACAGTTAGTGAACCTTCTCTCTCTGGGCTCCTTCAGA TCAAGAAATTGAAACAAGACCAAAAGGC TAATGGAGAAAGACGTAACTTCGGGGTAAGCCAAGAAAGA AATCCAGGTGAAAGCAGTCAACCAAATGTC TCCGATTTGAGTGATAAGAAAGAATCTGTGAGTTCTGTAGC CCAAGAAAATGCAGTTAAAGATTTCACCA GTTTTTCAACACATAACTGCAGTGGGCCTGAAAATCCAGAG CTTCAGATTCTGAATGAGCAGGAGGGGAA AAGTGCTAATTACCATGACAAGAACATTGTATTACTTAAAA ACAAGGCAGTGCTAATGCCTAATGGTGCT ACAGTTTCTGCCTCTTCCGTGGAACACACACATGGTGAACT CCTGGAAAAAACACTGTCTCAATATTATC CAGATTGTGTTTCCATTGCGGTGCAGAAAACCACATCTCAC ATAAATGCCATTAACAGTCAGGCTACTAA TGAGTTGTCCTGTGAGATCACTCACCCATCGCATACCTCAG GGCAGATCAATTCCGCACAGACCTCTAAC TCTGAGCTGCCTCCAAAGCCAGCTGCAGTGGTGAGTGAGGC CTGTGATGCTGATGATGCTGATAATGCCA GTAAACTAGCTGCAATGCTAAATACCTGTTCCTTTCAGAAA |
| [SEQ ID NO: 1358] | | |
| | | CCAGAACAACTACAACAACAAAAATCAGT TTTTGAGATATGCCCATCTCCTGCAGAAAATAACATCCAGG GAACCACAAAGCTAGCGTCTGGTGAAGAA TTCTGTTCAGGTTCCAGCAGCAATTTGCAAGCTCCTGGTGGC AGCTCTGAACGGTATTTAAAACAAAATG AAATGAATGGTGCTTACTTCAAGCAAAGCTCAGTGTTCACT AAGGATTCCTTTTCTGCCACTACCACACC ACCACCACCATCACAATTGCTTCTTTCTCCCCCTCCTCCTCT TCCACAGGTTCCTCAGCTTCCTTCAGAA GGAAAAAGCACTCTGAATGGTGGAGTTTTAGAAGAACACC ACCACTACCCCAACCAAAGTAACACAACAC TTTTAAGGGAAGTGAAAATAGAGGGTAAACCTGAGGCACC ACCTTCCCAGAGTCCTAATCCATCTACACA TGTATGCAGCCCTTCTCCGATGCTTTCTGAAAGGCCTCAGA ATAATTGTGTGAACAGGAATGACATACAG ACTGCAGGGACAATGACTGTTCCATTGTGTTCTGAGAAAAC AAGACCAATGTCAGAACACCTCAAGCATA ACCCACCAATTTTTGGTAGCAGTGGAGAGCTACAGGACAAC TGCCAGCAGTTGATGAGAAACAAAGAGCA AGAGATTCTGAAGGGTCGAGACAAGGAGCAAACACGAGAT CTTGTGCCCCCAACACAGCACTATCTGAAA CCAGGATGGATTGAATTGAAGGCCCCTCGTTTTCACCAAGC GGAATCCCATCTAAAACGTAATGAGGCAT CACTGCCATCAATTCTTCAGTATCAACCCAATCTCTCCAATC AAATGACCTCCAAACAATACACTGGAAA TTCCAACATGCCTGGGGGGCTCCCAAGGCAAGCTTACACCC AGAAAACAACACAGCTGGAGCACAAGTCA CAAATGTACCAAGTTGAAATGAATCAAGGGCAGTCCCAAG GTACAGTGGACCAACATCTCCAGTTCCAAA AACCCTCACACCAGGTGCACTTCTCCAAAACAGACCATTTA CCAAAAGCTCATGTGCAGTCACTGTGTGG CACTAGATTTCATTTTCAACAAAGAGCAGATTCCCAAACTG AAAAACTTATGTCCCCAGTGTTGAAACAG CACTTGAATCAACAGGCTTCAGAGACTGAGCCATTTTCAAA CTCACACCTTTTGCAACATAAGCCTCATA AACAGGCAGCACAAACACAACCATCCCAGAGTTCACATCTC CCTCAAAACCAGCAACAGCAGCAAAAATT ACAAATAAAGAATAAAGAGGAAATACTCCAGACTTTTCCTC ACCCCCAAAGCAACAATGATCAGCAAAGA GAAGGATCATTCTTTGGCCAGACTAAAGTGGAAGAATGTTT TCATGGTGAAAATCAGTATTCAAAATCAA GCGAGTTCGAGACTCATAATGTCCAAATGGGACTGGAGGA AGTACAGAATATAAATCGTAGAAATTCCCC TTATAGTCAGACCATGAAATCAAGTGCATGCAAAATACAGG TTTCTTGTTCAAACAATACACACCTAGTT TCAGAGAATAAAGAACAGACTACACATCCTGAACTTTTTGC AGGAAACAAGACCCAAAACTTGCATCACA TGCAATATTTTCCAAATAATGTGATCCCAAAGCAAGATCTT CTTCACAGGTGCTTTCAAGAACAGGAGCA GAAGTCACAACAAGCTTCAGTTCTACAGGGATATAAAAATA |
| | | GAAACCAAGATATGTCTGGTCAACAAGCT GCGCAACTTGCTCAGCAAAGGTACTTGATACATAACCATGC AAATGTTTTTCCTGTGCCTGACCAGGGAG GAAGTCACACTCAGACCCCTCCCCAGAAGGACACTCAAAA GCATGCTGCTCTAAGGTGGCATCTCTTACA GAAGCAAGAACAGCAGCAAACACAGCAACCCCAAACTGAG TCTTGCCATAGTCAGATGCACAGGCCAATT AAGGTGGAACCTGGATGCAAGCCACATGCCTGTATGCACAC AGCACCACCAGAAAACAAAACATGGAAAA AGGTAACTAAGCAAGAGAATCCACCTGCAAGCTGTGATAAT GTGCAGCAAAAGAGCATCATTGAGACCAT GGAGCAGCATCTGAAGCAGTTTCACGCCAAGTCGTTATTTG ACCATAAGGCTCTTACTCTCAAATCACAG AAGCAAGTAAAAGTTGAAATGTCAGGGCCAGTCACAGTTTT GACTAGACAAACCACTGCTGCAGAACTTG ATAGCCACACCCCAGCTTTAGAGCAGCAAACAACTTCTTCA GAAAAGACACCAACCAAAAGAACAGCTGC TTCTGTTCTCAATAATTTTATAGAGTCACCTTCCAAATTACT AGATACTCCTATAAAAAATTTATTGGAT ACACCTGTCAAGACTCAATATGATTTCCCATCTTGCAGATGT GTAGAGCAAATTATTGAAAAAGATGAAG GTCCTTTTTATACCCATCTAGGAGCAGGTCCTAATGTGGCA GCTATTAGAGAAATCATGGAAGAAAGGTT TGGACAGAAGGGTAAAGCTATTAGGATTGAAAGAGTCATCT ATACTGGTAAAGAAGGCAAAAGTTCTCAG GGATGTCCTATTGCTAAGTGGGTGGTTCGCAGAAGCAGCAG TGAAGAGAAGCTACTGTGTTTGGTGCGGG AGCGAGCTGGCCACACCTGTGAGGCTGCAGTGATTGTGATT CTCATCCTGGTGTGGGAAGGAATCCCGCT GTCTCTGGCTGACAAACTCTACTCGGAGCTTACCGAGACGC TGAGGAAATACGGCACGCTCACCAATCGC CGGTGTGCCTTGAATGAAGAGAGAACTTGCGCCTGTCAGGG GCTGGATCCAGAAACCTGTGGTGCCTCCT TCTCTTTTGGTTGTTCATGGAGCATGTACTACAATGGATGTA AGTTTGCCAGAAGCAAGATCCCAAGGAA GTTTAAGCTGCTTGGGGATGACCCAAAAGAGGAAGAGAAA CTGGAGTCTCATTTGCAAAACCTGTCCACT CTTATGGCACCAACATATAAGAAACTTGCACCTGATGCATA TAATAATCAGATTGAATATGAACACAGAG CACCAGAGTGCCGTCTGGGTCTGAAGGAAGGCCGTCCATTC TCAGGGGTCACTGCATGTTTGGACTTCTG TGCTCATGCCCACAGAGACTTGCACAACATGCAGAATGGCA GCACATTGGTATGCACTCTCACTAGAGAA GACAATCGAGAATTTGGAGGAAAACCTGAGGATGAGCAGC TTCACGTTCTGCCTTTATACAAAGTCTCTG ACGTGGATGAGTTTGGGAGTGTGGAAGCTCAGGAGGAGAA AAAACGGAGTGGTGCCATTCAGGTACTGAG TTCTTTTCGGCGAAAAGTCAGGATGTTAGCAGAGCCAGTCA AGACTTGCCGACAAAGGAAACTAGAAGCC AAGAAAGCTGCAGCTGAAAAGCTTTCCTCCCTGGAGAACAG |
| | | CTCAAATAAAAATGAAAAGGAAAAGTCAG CCCCATCACGTACAAAACAAACTGAAAACGCAAGCCAGGC TAAACAGTTGGCAGAACTTTTGCGACTTTC AGGACCAGTCATGCAGCAGTCCCAGCAGCCCCAGCCTCTAC AGAAGCAGCCACCACAGCCCCAGCAGCAG CAGAGACCCCAGCAGCAGCAGCCACATCACCCTCAGACAG AGTCTGTCAACTCTTATTCTGCTTCTGGAT CCACCAATCCATACATGAGACGGCCCAATCCAGTTAGTCCT TATCCAAACTCTTCACACACTTCAGATAT CTATGGAAGCACCAGCCCTATGAACTTCTATTCCACCTCATC TCAAGCTGCAGGTTCATATTTGAATTCT TCTAATCCCATGAACCCTTACCCTGGGCTTTTGAATCAGAAT ACCCAATATCCATCATATCAATGCAATG GAAACCTATCAGTGGACAACTGCTCCCCATATCTGGGTTCC TATTCTCCCCAGTCTCAGCCGATGGATCT GTATAGGTATCCAAGCCAAGACCCTCTGTCTAAGCTCAGTC TACCACCCATCCATACACTTTACCAGCCA AGGTTTGGAAATAGCCAGAGTTTTACATCTAAATACTTAGG TTATGGAAACCAAAATATGCAGGGAGATG GTTTCAGCAGTTGTACCATTAGACCAAATGTACATCATGTA GGGAAATTGCCTCCTTATCCCACTCATGA GATGGATGGCCACTTCATGGGAGCCACCTCTAGATTACCAC CCAATCTGAGCAATCCAAACATGGACTAT AAAAATGGTGAACATCATTCACCTTCTCACATAATCCATAA CTACAGTGCAGCTCCGGGCATGTTCAACA GCTCTCTTCATGCCCTGCATCTCCAAAACAAGGAGAATGAC ATGCTTTCCCACACAGCTAATGGGTTATC AAAGATGCTTCCAGCTCTTAACCATGATAGAACTGCTTGTG TCCAAGGAGGCTTACACAAATTAAGTGAT GCTAATGGTCAGGAAAAGCAGCCATTGGCACTAGTCCAGG GTGTGGCTTCTGGTGCAGAGGACAACGATG AGGTCTGGTCAGACAGCGAGCAGAGCTTTCTGGATCCTGAC ATTGGGGGAGTGGCCGTGGCTCCAACTCA TGGGTCAATTCTCATTGAGTGTGCAAAGCGTGAGCTGCATG CCACAACCCCTTTAAAGAATCCCAATAGG AATCACCCCACCAGGATCTCCCTCGTCTTTTACCAGCATAA GAGCATGAATGAGCCAAAACATGGCTTGG CTCTTTGGGAAGCCAAAATGGCTGAAAAAGCCCGTGAGAA AGAGGAAGAGTGTGAAAAGTATGGCCCAGA CTATGTGCCTCAGAAATCCCATGGCAAAAAAGTGAAACGG GAGCCTGCTGAGCCACATGAAACTTCAGAG CCCACTTACCTGCGTTTCATCAAGTCTCTTGCCGAAAGGACC ATGTCCGTGACCACAGACTCCACAGTAA CTACATCTCCATATGCCTTCACTCGGGTCACAGGGCCTTACA ACAGATATATATGATATCACCCCCTTTT GTTGGTTACCTCACTTGAAAAGACCACAACCAACCTGTCAG TAGTATAGTTCTCATGACGTGGGCAGTGG GGAAAGGTCACAGTATTCATGACAAATGTGGTGGGAAAAA CCTCAGCTCACCAGCAACAAAAGAGGTTAT CTTACCATAGCACTTAATTTTCACTGGCTCCCAAGTGGTCAC |
| | | AGATGGCATCTAGGAAAAGACCAAAGCA TTCTATGCAAAAAGAAGGTGGGAAGAAAGTGTTCCGCAA TTTACATTTTTAAACACTGGTTCTATTATT GGACGAGATGATATGTAAATGTGATCCCCCCCCCCGCTTA CAACTCTACACATCTGTGACCACTTTTAA TAATATCAAGTTTGCATAGTCATGGAACACAAATCAAACAA GTACTGTAGTATTACAGTGACAGGAATCT TAAAATACCATCTGGTGCTGAATATATGATGTACTGAAATA CTGGAATTATGGCTTTTTGAAATGCAGTT TTTACTGTAATCTTAACTTTTATTTATCAAAATAGCTACAGG AAACATGAATAGCAGGAAAACACTGAAT TTGTTTGGATGTTCTAAGAAATGGTGCTAAGAAAATGGTGT CTTTAATAGCTAAAAATTTAATGCCTTTA TATCATCAAGATGCTATCAGTGTACTCCAGTGCCCTTGAAT AATAGGGGTACCTTTTCATTCAAGTTTTT ATCATAATTACCTATTCTTACACAAGCTTAGTTTTTAAAATG TGGACATTTTAAAGGCCTCTGGATTTTG CTCATCCAGTGAAGTCCTTGTAGGACAATAAACGTATATAT GTACATATATACACAAACATGTATATGTG CACACACATGTATATGTATAAATATTTTAAATGGTGTTTTAG AAGCACTTTGTCTACCTAAGCTTTGACA ACTTGAACAATGCTAAGGTACTGAGATGTTTAAAAAACAAG TTTACTTTCATTTTAGAATGCAAAGTTGA TTTTTTTAAGGAAACAAAGAAAGCTTTTAAAATATTTTTGCT TTTAGCCATGCATCTGCTGATGAGCAAT TGTGTCCATTTTTAACACAGCCAGTTAAATCCACCATGGGG CTTACTGGATTCAAGGGAATACGTTAGTC CACAAAACATGTTTTCTGGTGCTCATCTCACATGCTATACTG TAAAACAGTTTTATACAAAATTGTATGA CAAGTTCATTGCTCAAAAATGTACAGTTTTAAGAATTTTCTA TTAACTGCAGGTAATAATTAGCTGCATG CTGCAGACTCAACAAAGCTAGTTCACTGAAGCCTATGCTAT TTTATGGATCATAGGCTCTTCAGAGAACT GAATGGCAGTCTGCCTTTGTGTTGATAATTATGTACATTGTG ACGTTGTCATTTCTTAGCTTAAGTGTCC TCTTTAACAAGAGGATTGAGCAGACTGATGCCTGCATAAGA TGAATAAACAGGGTTAGTTCCATGTGAAT CTGTCAGTTAAAAAGAAACAAAAACAGGCAGCTGGTTTGCT GTGGTGGTTTTAAATCATTAATTTGTATA AAGAAGTGAAAGAGTTGTATAGTAAATTAAATTGTAAACA AAACTTTTTTAATGCAATGCTTTAGTATTT TAGTACTGTAAAAAAATTAAATATATACATATATATATATA TATATATATATATATATATGAGTTTGAAG CAGAATTCACATCATGATGGTGCTACTCAGCCTGCTACAAA TATATCATAATGTGAGCTAAGAATTCATT AAATGTTTGAGTGATGTTCCTACTTGTCATATACCTCAACAC TAGTTTGGCAATAGGATATTGAACTGAG AGTGAAAGCATTGTGTACCATCATTTTTTTCCAAGTCCTTTT TTTTATTGTTAAAAAAAAAAGCATACCT TTTTTCAATACTTGATTTCTTAGCAAGTATAACTTGAACTTC |
| | | AACCTTTTTGTTCTAAAAATTCAGGGAT ATTTCAGCTCATGCTCTCCCTATGCCAACATGTCACCTGTGT TTATGTAAAATTGTTGTAGGTTAATAAA TATATTCTTTGTCAGGGATTTAACCCTTTTATTTTGAATCCCT TCTATTTTACTTGTACATGTGCTGATG TAACTAAAACTAATTTTGTAAATCTGTTGGCTCTTTTTATTG TAAAGAAAAGCATTTTAAAAGTTTGAGG AATCTTTTGACTGTTTCAAGCAGGAAAAAAAAATTACATGA AAATAGAATGCACTGAGTTGATAAAGGGA AAAATTGTAAGGCAGGAGTTTGGCAAGTGGCTGTTGGCCAG AGACTTACTTGTAACTCTCTAAATGAAGT TTTTTTGATCCTGTAATCACTGAAGGTACATACTCCATGTGG ACTTCCCTTAAACAGGCAAACACCTACA GGTATGGTGTGCAACAGATTGTACAATTACATTTTGGCCTA AATACATTTTTGCTTACTAGTATTTAAAA TAAATTCTTAATCAGAGGAGGCCTTTGGGTTTTATTGGTCAA ATCTTTGTAAGCTGGCTTTTGTCTTTTT AAAAAATTTCTTGAATTTGTGGTTGTGTCCAATTTGCAAACA TTTCCAAAAATGTTTGCTTTGCTTACAA ACCACATGATTTTAATGTTTTTGTATACCATAATATCTAGC CCCAAACATTTGATTACTACATGTGCAT TGGTGATTTTGATCATCCATTCTTAATATTTGATTTCTGTGTC ACCTACTGTCATTTGTTAAACTGCTGG CCAACAAGAACAGGAAGTATAGTTTGGGGGGTTGGGGAGA GTTTACATAAGGAAGAGAAGAAATTGAGTG GCATATTGTAAATATCAGATCTATAATTGTAAATATAAAAC CTGCCTCAGTTAGAATGAATGGAAAGCAG ATCTACAATTTGCTAATATAGGAATATCAGGTTGACTATAT AGCCATACTTGAAAATGCTTCTGAGTGGT GTCAACTTTACTTGAATGAATTTTTCATCTTGATTGACGCAC AGTGATGTACAGTTCACTTCTGAAGCTA GTGGTTAACTTGTGTAGGAAACTTTTGCAGTTTGACACTAA GATAACTTCTGTGTGCATTTTTCTATGCT TTTTTAAAAACTAGTTTCATTTCATTTTCATGAGATGTTTGG TTTATAAGATCTGAGGATGGTTATAAAT ACTGTAAGTATTGTAATGTTATGAATGCAGGTTATTTGAAA GCTGTTTATTATTATATCATTCCTGATAA TGCTATGTGAGTGTTTTTAATAAAATTTATATTTATTTAATG CACTCTAAAAAAAAAAAAAAAAAA |
| PPREDICTED: Homo sapiens tet methylcytosine dioxygenase 2 (TET2), transcript variant X1, mRNA | XXM_00526 3082.1 | AAGCAGAAGGAAGCAAGATGGCTGCCCTTTAGGATTTGTTA GAAAGGAGACCCGACTGCAACTGCTGGAT TGCTGCAAGGCTGAGGGACGAGAACGAGAATTCAACTAGA GGGCAGCCTTGTGGATGGCCCGAAGCAAG CCTGATGGAACAGGATAGAACCAACCATGTTGAGGGCAAC AGACTAAGTCCATTCCTGATACCATCACCT CCCATTTGCCAGACAGAACCTCTGGCTACAAAGCTCCAGAA TGGAAGCCCACTGCCTGAGAGAGCTCATC CAGAAGTAAATGGAGACACCAAGTGGCACTCTTTCAAAAGT TATTATGGAATACCCTGTATGAAGGGAAG CCAGAATAGTCGTGTGAGTCCTGACTTTACACAAGAAAGTA GAGGGTATTCCAAGTGTTTGCAAAATGGA GGAATAAAACGCACAGTTAGTGAACCTTCTCTCTCTGGGCT CCTTCAGATCAAGAAATTGAAACAAGACC AAAAGGCTAATGGAGAAAGACGTAACTTCGGGGTAAGCCA AGAAAGAAATCCAGGTGAAAGCAGTCAACC AAATGTCTCCGATTTGAGTGATAAGAAAGAATCTGTGAGTT CTGTAGCCCAAGAAAATGCAGTTAAAGAT TTCACCAGTTTTTCAACACATAACTGCAGTGGGCCTGAAAA TCCAGAGCTTCAGATTCTGAATGAGCAGG AGGGGAAAAGTGCTAATTACCATGACAAGAACATTGTATTA CTTAAAAACAAGGCAGTGCTAATGCCTAA TGGTGCTACAGTTTCTGCCTCTTCCGTGGAACACACACATG GTGAACTCCTGGAAAAAACACTGTCTCAA TATTATCCAGATTGTGTTTCCATTGCGGTGCAGAAAACCAC ATCTCACATAAATGCCATTAACAGTCAGG CTACTAATGAGTTGTCCTGTGAGATCACTCACCCATCGCAT ACCTCAGGGCAGATCAATTCCGCACAGAC CTCTAACTCTGAGCTGCCTCCAAAGCCAGCTGCAGTGGTGA GTGAGGCCTGTGATGCTGATGATGCTGAT AATGCCAGTAAACTAGCTGCAATGCTAAATACCTGTTCCTT TCAGAAACCAGAACAACTACAACAACAAA AATCAGTTTTTGAGATATGCCCATCTCCTGCAGAAAATAAC ATCCAGGGAACCACAAAGCTAGCGTCTGG TGAAGAATTCTGTTCAGGTTCCAGCAGCAATTTGCAAGCTC CTGGTGGCAGCTCTGAACGGTATTTAAAA CAAAATGAAATGAATGGTGCTTACTTCAAGCAAAGCTCAGT GTTCACTAAGGATTCCTTTTCTGCCACTA CCACACCACCACCACCATCACAATTGCTTCTTTCTCCCCCTC CTCCTCTTCCACAGGTTCCTCAGCTTCC TTCAGAAGGAAAAAGCACTCTGAATGGTGGAGTTTTAGAAG AACACCACCACTACCCCAACCAAAGTAAC ACAACACTTTTAAGGGAAGTGAAAATAGAGGGTAAACCTG AGGCACCACCTTCCCAGAGTCCTAATCCAT CTACACATGTATGCAGCCCTTCTCCGATGCTTTCTGAAAGGC CTCAGAATAATTGTGTGAACAGGAATGA CATACAGACTGCAGGGACAATGACTGTTCCATTGTGTTCTG AGAAAACAAGACCAATGTCAGAACACCTC AAGCATAACCCACCAATTTTTGGTAGCAGTGGAGAGCTACA GGACAACTGCCAGCAGTTGATGAGAAACA |
| [SEQ ID NO: 1359] | | |
| | | AAGAGCAAGAGATTCTGAAGGGTCGAGACAAGGAGCAAAC ACGAGATCTTGTGCCCCAACACAGCACTA TCTGAAACCAGGATGGATTGAATTGAAGGCCCCTCGTTTTC ACCAAGCGGAATCCCATCTAAAACGTAAT GAGGCATCACTGCCATCAATTCTTCAGTATCAACCCAATCT CTCCAATCAAATGACCTCCAAACAATACA CTGGAAATTCCAACATGCCTGGGGGCTCCCAAGGCAAGCT TACACCCAGAAAACAACACAGCTGGAGCA CAAGTCACAAATGTACCAAGTTGAAATGAATCAAGGGCAG TCCCAAGGTACAGTGGACCAACATCTCCAG TTCCAAAAACCCTCACACCAGGTGCACTTCTCCAAAACAGA CCATTTACCAAAAGCTCATGTGCAGTCAC TGTGTGGCACTAGATTTCATTTTCAACAAAGAGCAGATTCC CAAACTGAAAAACTTATGTCCCAGTGTT GAAACAGCACTTGAATCAACAGGCTTCAGAGACTGAGCCAT TTTCAAACTCACACCTTTTGCAACATAAG CCTCATAAACAGGCAGCACAAACACAACCATCCCAGAGTTC ACATCTCCCTCAAAACCAGCAACAGCAGC AAAAATTACAAATAAAGAATAAAGAGGAAATACTCCAGAC TTTTCCTCACCCCCAAAGCAACAATGATCA GCAAAGAGAAGGATCATTCTTTGGCCAGACTAAAGTGGAA GAATGTTTTCATGGTGAAAATCAGTATTCA AAATCAAGCGAGTTCGAGACTCATAATGTCCAAATGGGACT GGAGGAAGTACAGAATATAAATCGTAGAA ATTCCCCTTATAGTCAGACCATGAAATCAAGTGCATGCAAA ATACAGGTTTCTTGTTCAAACAATACACA CCTAGTTTCAGAGAATAAAGAACAGACTACACATCCTGAAC TTTTTGCAGGAAACAAGACCCAAAACTTG CATCACATGCAATATTTTCCAAATAATGTGATCCCAAAGCA AGATCTTCTTCACAGGTGCTTTCAAGAAC AGGAGCAGAAGTCACAACAAGCTTCAGTTCTACAGGGATAT AAAAATAGAAACCAAGATATGTCTGGTCA ACAAGCTGCGCAACTTGCTCAGCAAAGGTACTTGATACATA ACCATGCAAATGTTTTTCCTGTGCCTGAC CAGGGAGGAAGTCACACTCAGACCCCTCCCCAGAAGGACA CTCAAAAGCATGCTGCTCTAAGGTGGCATC TCTTACAGAAGCAAGAACAGCAGCAAACACAGCAACCCCA AACTGAGTCTTGCCATAGTCAGATGCACAG GCCAATTAAGGTGGAACCTGGATGCAAGCCACATGCCTGTA TGCACACAGCACCACCAGAAACAAAACA TGGAAAAAGGTAACTAAGCAAGAGAATCCACCTGCAAGCT GTGATAATGTGCAGCAAAAGAGCATCATTG AGACCATGGAGCAGCATCTGAAGCAGTTTCACGCCAAGTCG TTATTTGACCATAAGGCTCTTACTCTCAA ATCACAGAAGCAAGTAAAAGTTGAAATGTCAGGGCCAGTC ACAGTTTTGACTAGACAAACCACTGCTGCA GAACTTGATAGCCACACCCCAGCTTTAGAGCAGCAAACAAC TTCTTCAGAAAAGACACCAACCAAAAGAA CAGCTGCTTCTGTTCTCAATAATTTTATAGAGTCACCTTCCA AATTACTAGATACTCCTATAAAAAATTT |
| | | ATTGGATACACCTGTCAAGACTCAATATGATTTCCCATCTTG CAGATGTGTAGAGCAAATTATTGAAAAA GATGAAGGTCCTTTTTATACCCATCTAGGAGCAGGTCCTAA TGTGGCAGCTATTAGAGAAATCATGGAAG AAAGGTTTGGACAGAAGGGTAAAGCTATTAGGATTGAAAG AGTCATCTATACTGGTAAAGAAGGCAAAAG TTCTCAGGGATGTCCTATTGCTAAGTGGGTGGTTCGCAGAA GCAGCAGTGAAGAGAAGCTACTGTGTTTG GTGCGGGAGCGAGCTGGCCACACCTGTGAGGCTGCAGTGAT TGTGATTCTCATCCTGGTGTGGGAAGGAA TCCCGCTGTCTCTGGCTGACAAACTCTACTCGGAGCTTACCG AGACGCTGAGGAAATACGGCACGCTCAC CAATCGCCGGTGTGCCTTGAATGAAGAGAGAACTTGCGCCT GTCAGGGGCTGGATCCAGAAACCTGTGGT GCCTCCTTCTCTTTTGGTTGTTCATGGAGCATGTACTACAAT GGATGTAAGTTTGCCAGAAGCAAGATCC CAAGGAAGTTTAAGCTGCTTGGGGATGACCCAAAAGAGGA AGAGAAACTGGAGTCTCATTTGCAAAACCT GTCCACTCTTATGGCACCAACATATAAGAAACTTGCACCTG ATGCATATAATAATCAGATTGAATATGAA CACAGAGCACCAGAGTGCCGTCTGGGTCTGAAGGAAGGCC GTCCATTCTCAGGGGTCACTGCATGTTTGG ACTTCTGTGCTCATGCCCACAGAGACTTGCACAACATGCAG AATGGCAGCACATTGGTATGCACTCTCAC TAGAGAAGACAATCGAGAATTTGGAGGAAAACCTGAGGAT GAGCAGCTTCACGTTCTGCCTTTATACAAA GTCTCTGACGTGGATGAGTTTGGGAGTGTGGAAGCTCAGGA GGAGAAAAAACGGAGTGGTGCCATTCAGG TACTGAGTTCTTTTCGGCGAAAAGTCAGGATGTTAGCAGAG CCAGTCAAGACTTGCCGACAAAGGAAACT AGAAGCCAAGAAAGCTGCAGCTGAAAAGCTTTCCTCCCTGG AGAACAGCTCAAATAAAAATGAAAAGGAA AAGTCAGCCCCATCACGTACAAAACAAACTGAAAACGCAA GCCAGGCTAAACAGTTGGCAGAACTTTTGC GACTTTCAGGACCAGTCATGCAGCAGTCCCAGCAGCCCCAG CCTCTACAGAAGCAGCCACCACAGCCCCA GCAGCAGCAGAGACCCCAGCAGCAGCAGCCACATCACCCT CAGACAGAGTCTGTCAACTCTTATTCTGCT TCTGGATCCACCAATCCATACATGAGACGGCCCAATCCAGT TAGTCCTTATCCAAACTCTTCACACACTT CAGATATCTATGGAAGCACCAGCCCTATGAACTTCTATTCC ACCTCATCTCAAGCTGCAGGTTCATATTT GAATTCTTCTAATCCCATGAACCCTTACCCTGGGCTTTTGAA TCAGAATACCCAATATCCATCATATCAA TGCAATGGAAACCTATCAGTGGACAACTGCTCCCCATATCT GGGTTCCTATTCTCCCCAGTCTCAGCCGA TGGATCTGTATAGGTATCCAAGCCAAGACCCTCTGTCTAAG CTCAGTCTACCACCCATCCATACACTTTA CCAGCCAAGGTTTGGAAATAGCCAGAGTTTTACATCTAAAT ACTTAGGTTATGGAAACCAAAATATGCAG |
| | | GGAGATGGTTTCAGCAGTTGTACCATTAGACCAAATGTACA TCATGTAGGGAAATTGCCTCCTTATCCCA CTCATGAGATGGATGGCCACTTCATGGGAGCCACCTCTAGA TTACCACCCAATCTGAGCAATCCAAACAT GGACTATAAAAATGGTGAACATCATTCACCTTCTCACATAA TCCATAACTACAGTGCAGCTCCGGGCATG TTCAACAGCTCTCTTCATGCCCTGCATCTCCAAAACAAGGA GAATGACATGCTTTCCCACACAGCTAATG GGTTATCAAAGATGCTTCCAGCTCTTAACCATGATAGAACT GCTTGTGTCCAAGGAGGCTTACACAAATT AAGTGATGCTAATGGTCAGGAAAAGCAGCCATTGGCACTA GTCCAGGGTGTGGCTTCTGGTGCAGAGGAC AACGATGAGGTCTGGTCAGACAGCGAGCAGAGCTTTCTGGA TCCTGACATTGGGGGAGTGGCCGTGGCTC CAACTCATGGGTCAATTCTCATTGAGTGTGCAAAGCGTGAG CTGCATGCCACAACCCCTTTAAAGAATCC CAATAGGAATCACCCCACCAGGATCTCCCTCGTCTTTTACC AGCATAAGAGCATGAATGAGCCAAAACAT GGCTTGGCTCTTTGGGAAGCCAAAATGGCTGAAAAAGCCCG TGAGAAAGAGGAAGAGTGTGAAAAGTATG GCCCAGACTATGTGCCTCAGAAATCCCATGGCAAAAAAGTG AAACGGGAGCCTGCTGAGCCACATGAAAC TTCAGAGCCCACTTACCTGCGTTTCATCAAGTCTCTTGCCGA AAGGACCATGTCCGTGACCACAGACTCC ACAGTAACTACATCTCCATATGCCTTCACTCGGGTCACAGG GCCTTACAACAGATATATATGATATCACC CCCTTTTGTTGGTTACCTCACTTGAAAAGACCACAACCAAC CTGTCAGTAGTATAGTTCTCATGACGTGG GCAGTGGGGAAAGGTCACAGTATTCATGACAAATGTGGTG GGAAAAACCTCAGCTCACCAGCAACAAAAG AGGTTATCTTACCATAGCACTTAATTTTCACTGGCTCCCAAG TGGTCACAGATGGCATCTAGGAAAAGAC CAAAGCATTCTATGCAAAAAGAAGGTGGGGAAGAAAGTGT TCCGCAATTTACATTTTTAAACACTGGTTC TATTATTGGACGAGATGATATGTAAATGTGATCCCCCCCCC CCGCTTACAACTCTACACATCTGTGACCA CTTTTAATAATATCAAGTTTGCATAGTCATGGAACACAAAT CAAACAAGTACTGTAGTATTACAGTGACA GGAATCTTAAAATACCATCTGGTGCTGAATATATGATGTAC TGAAATACTGGAATTATGGCTTTTTGAAA TGCAGTTTTTACTGTAATCTTAACTTTTATTTATCAAAATAG CTACAGGAAACATGAATAGCAGGAAAAC ACTGAATTTGTTTGGATGTTCTAAGAAATGGTGCTAAGAAA ATGGTGTCTTTAATAGCTAAAAATTTAAT GCCTTTATATCATCAAGATGCTATCAGTGTACTCCAGTGCCC TTGAATAATAGGGGTACCTTTTCATTCA AGTTTTTATCATAATTACCTATTCTTACACAAGCTTAGTTTT TAAAATGTGGACATTTTAAAGGCCTCTG GATTTTGCTCATCCAGTGAAGTCCTTGTAGGACAATAAACG TATATATGTACATATATACACAAACATGT |
| | | ATATGTGCACACACATGTATATGTATAAATATTTTAAATGG TGTTTTAGAAGCACTTTGTCTACCTAAGC TTTGACAACTTGAACAATGCTAAGGTACTGAGATGTTTAAA AAACAAGTTTACTTTCATTTTAGAATGCA AAGTTGATTTTTTTAAGGAAACAAAGAAAGCTTTTAAAATA TTTTTGCTTTTAGCCATGCATCTGCTGAT GAGCAATTGTGTCCATTTTTAACACAGCCAGTTAAATCCAC CATGGGGCTTACTGGATTCAAGGGAATAC GTTAGTCCACAAAACATGTTTTCTGGTGCTCATCTCACATGC TATACTGTAAAACAGTTTTATACAAAAT TGTATGACAAGTTCATTGCTCAAAAATGTACAGTTTTAAGA ATTTTCTATTAACTGCAGGTAATAATTAG CTGCATGCTGCAGACTCAACAAAGCTAGTTCACTGAAGCCT ATGCTATTTTATGGATCATAGGCTCTTCA GAGAACTGAATGGCAGTCTGCCTTTGTGTTGATAATTATGT ACATTGTGACGTTGTCATTTCTTAGCTTA AGTGTCCTCTTTAACAAGAGGATTGAGCAGACTGATGCCTG CATAAGATGAATAAACAGGGTTAGTTCCA TGTGAATCTGTCAGTTAAAAAGAAACAAAAACAGGCAGCT GGTTTGCTGTGGTGGTTTTAAATCATTAAT TTGTATAAAGAAGTGAAAGAGTTGTATAGTAAATTAAATTG TAAACAAAACTTTTTTAATGCAATGCTTT AGTATTTTAGTACTGTAAAAAAATTAAATATATACATATAT ATATATATATATATATATATATATATGAG TTTGAAGCAGAATTCACATCATGATGGTGCTACTCAGCCTG CTACAAATATATCATAATGTGAGCTAAGA ATTCATTAAATGTTTGAGTGATGTTCCTACTTGTCATATACC TCAACACTAGTTTGGCAATAGGATATTG AACTGAGAGTGAAAGCATTGTGTACCATCATTTTTTTCCAA GTCCTTTTTTTTATTGTTAAAAAAAAAAG CATACCTTTTTTCAATACTTGATTTCTTAGCAAGTATAACTT GAACTTCAACCTTTTTGTTCTAAAAATT CAGGGATATTTCAGCTCATGCTCTCCCTATGCCAACATGTCA CCTGTGTTTATGTAAAATTGTTGTAGGT TAATAAATATATTCTTTGTCAGGGATTTAACCCTTTTATTTT GAATCCCTTCTATTTTACTTGTACATGT GCTGATGTAACTAAAACTAATTTTGTAAATCTGTTGGCTCTT TTTATTGTAAAGAAAAGCATTTTAAAAG TTTGAGGAATCTTTTGACTGTTTCAAGCAGGAAAAAAAAAT TACATGAAAATAGAATGCACTGAGTTGAT AAAGGGAAAAATTGTAAGGCAGGAGTTTGGCAAGTGGCTG TTGGCCAGAGACTTACTTGTAACTCTCTAA ATGAAGTTTTTTTGATCCTGTAATCACTGAAGGTACATACTC CATGTGGACTTCCCTTAAACAGGCAAAC ACCTACAGGTATGGTGTGCAACAGATTGTACAATTACATTT TGGCCTAAATACATTTTTGCTTACTAGTA TTTAAAATAAATTCTTAATCAGAGGAGGCCTTTGGGTTTTAT TGGTCAAATCTTTGTAAGCTGGCTTTTG TCTTTTTAAAAAATTTCTTGAATTTGTGGTTGTGTCCAATTT GCAAACATTTCCAAAAATGTTTGCTTTG |
| | | CTTACAAACCACATGATTTTAATGTTTTTGTATACCATAAT ATCTAGCCCCAAACATTTGATTACTACA TGTGCATTGGTGATTTTGATCATCCATTCTTAATATTTGATT TCTGTGTCACCTACTGTCATTTGTTAAA CTGCTGGCCAACAAGAACAGGAAGTATAGTTTGGGGGGTTG GGGAGAGTTTACATAAGGAAGAGAAGAAA TTGAGTGGCATATTGTAAATATCAGATCTATAATTGTAAAT ATAAAACCTGCCTCAGTTAGAATGAATGG AAAGCAGATCTACAATTTGCTAATATAGGAATATCAGGTTG ACTATATAGCCATACTTGAAAATGCTTCT GAGTGGTGTCAACTTTACTTGAATGAATTTTTCATCTTGATT GACGCACAGTGATGTACAGTTCACTTCT GAAGCTAGTGGTTAACTTGTGTAGGAAACTTTTGCAGTTTG ACACTAAGATAACTTCTGTGTGCATTTTT CTATGCTTTTTTAAAAACTAGTTTCATTTCATTTTCATGAGA TGTTTGGTTTATAAGATCTGAGGATGGT TATAAATACTGTAAGTATTGTAATGTTATGAATGCAGGTTA TTTGAAAGCTGTTTATTATTATATCATTC CTGATAATGCTATGTGAGTGTTTTTAATAAAATTTATATTTA TTTAATGCACTCTAA |
| PPREDICTED: Homo sapiens tet methylcytosine dioxygenase 2 (TET2), transcript variant X2, mRNA | XXM_00671 4242.2 | GTAGAGAAGCAGAAGGAAGCAAGATGGCTGCCCTTTAGGA TTTGTTAGAAAGGAGACCCGACTGCAACTG CTGGATTGCTGCAAGGCTGAGGGACGAGAACGAGGCTGGC AAACATTCAGCAGCACACCCTCTCAAGATT GTTTACTTGCCTTTGCTCCTGTTGAGTTACAACGCTTGGAAG CAGGAGATGGGCTCAGCAGCAGCCAATA GGACATGATCCAGGAAGAGCAGTAAGGGACTGAGCTGCTG AATTCAACTAGAGGGCAGCCTTGTGGATGG CCCCGAAGCAAGCCTGATGGAACAGGATAGAACCAACCAT GTTGAGGGCAACAGACTAAGTCCATTCCTG ATACCATCACCTCCCATTTGCCAGACAGAACCTCTGGCTAC AAAGCTCCAGAATGGAAGCCCACTGCCTG AGAGAGCTCATCCAGAAGTAAATGGAGACACCAAGTGGCA CTCTTTCAAAAGTTATTATGGAATACCCTG TATGAAGGGAAGCCAGAATAGTCGTGTGAGTCCTGACTTTA CACAAGAAAGTAGAGGGTATTCCAAGTGT TTGCAAAATGGAGGAATAAAACGCACAGTTAGTGAACCTTC TCTCTCTGGGCTCCTTCAGATCAAGAAAT TGAAACAAGACCAAAAGGCTAATGGAGAAAGACGTAACTT CGGGGTAAGCCAAGAAAGAAATCCAGGTGA AAGCAGTCAACCAAATGTCTCCGATTTGAGTGATAAGAAAG AATCTGTGAGTTCTGTAGCCCAAGAAAAT GCAGTTAAAGATTTCACCAGTTTTTCAACACATAACTGCAG TGGGCCTGAAAATCCAGAGCTTCAGATTC TGAATGAGCAGGAGGGGAAAAGTGCTAATTACCATGACAA GAACATTGTATTACTTAAAAACAAGGCAGT GCTAATGCCTAATGGTGCTACAGTTTCTGCCTCTTCCGTGGA ACACACACATGGTGAACTCCTGGAAAAA ACACTGTCTCAATATTATCCAGATTGTGTTTCCATTGCGGTG CAGAAAACCACATCTCACATAAATGCCA |
| [SEQ ID NO: 1360] | | |
| | | TTAACAGTCAGGCTACTAATGAGTTGTCCTGTGAGATCACT CACCCATCGCATACCTCAGGGCAGATCAA TTCCGCACAGACCTCTAACTCTGAGCTGCCTCCAAAGCCAG CTGCAGTGGTGAGTGAGGCCTGTGATGCT GATGATGCTGATAATGCCAGTAAACTAGCTGCAATGCTAAA TACCTGTTCCTTTCAGAAACCAGAACAAC TACAACAACAAAAATCAGTTTTGAGATATGCCCATCTCCT GCAGAAAATAACATCCAGGGAACCACAAA GCTAGCGTCTGGTGAAGAATTCTGTTCAGGTTCCAGCAGCA ATTTGCAAGCTCCTGGTGGCAGCTCTGAA CGGTATTTAAAACAAAATGAAATGAATGGTGCTTACTTCAA GCAAAGCTCAGTGTTCACTAAGGATTCCT TTTCTGCCACTACCACACCACCACCACCATCACAATTGCTTC TTTCTCCCCCTCCTCCTCTTCCACAGGT TCCTCAGCTTCCTTCAGAAGGAAAAGCACTCTGAATGGTG GAGTTTTAGAAGAACACCACCACTACCCC AACCAAAGTAACACAACACTTTTAAGGGAAGTGAAAATAG AGGGTAAACCTGAGGCACCACCTTCCCAGA GTCCTAATCCATCTACACATGTATGCAGCCCTTCTCCGATGC TTTCTGAAAGGCCTCAGAATAATTGTGT GAACAGGAATGACATACAGACTGCAGGGACAATGACTGTT CCATTGTGTTCTGAGAAAACAAGACCAATG TCAGAACACCTCAAGCATAACCCACCAATTTTTGGTAGCAG TGGAGAGCTACAGGACAACTGCCAGCAGT TGATGAGAAACAAAGAGCAAGAGATTCTGAAGGGTCGAGA CAAGGAGCAAACACGAGATCTTGTGCCCCC AACACAGCACTATCTGAAACCAGGATGGATTGAATTGAAG GCCCCTCGTTTTCACCAAGCGGAATCCCAT CTAAAACGTAATGAGGCATCACTGCCATCAATTCTTCAGTA TCAACCCAATCTCTCCAATCAAATGACCT CCAAACAATACACTGGAAATTCCAACATGCCTGGGGGCTC CCAAGGCAAGCTTACACCCAGAAAACAAC ACAGCTGGAGCACAAGTCACAAATGTACCAAGTTGAAATG AATCAAGGGCAGTCCCAAGGTACAGTGGAC CAACATCTCCAGTTCCAAAAACCCTCACACCAGGTGCACTT CTCCAAAACAGACCATTTACCAAAAGCTC ATGTGCAGTCACTGTGTGGCACTAGATTTCATTTTCAACAA AGAGCAGATTCCCAAACTGAAAAACTTAT GTCCCCAGTGTTGAAACAGCACTTGAATCAACAGGCTTCAG AGACTGAGCCATTTTCAAACTCACACCTT TTGCAACATAAGCCTCATAAACAGGCAGCACAAACACAAC CATCCCAGAGTTCACATCTCCCTCAAAACC AGCAACAGCAGCAAAAATTACAAATAAAGAATAAAGAGGA AATACTCCAGACTTTTCCTCACCCCCAAAG CAACAATGATCAGCAAAGAGAAGGATCATTCTTTGGCCAGA CTAAAGTGGAAGAATGTTTTCATGGTGAA AATCAGTATTCAAAATCAAGCGAGTTCGAGACTCATAATGT CCAAATGGGACTGGAGGAAGTACAGAATA TAAATCGTAGAAATTCCCCTTATAGTCAGACCATGAAATCA AGTGCATGCAAAATACAGGTTTCTTGTTC |
| | | AAACAATACACACCTAGTTTCAGAGAATAAAGAACAGACT ACACATCCTGAACTTTTTGCAGGAAACAAG ACCCAAAACTTGCATCACATGCAATATTTCCAAATAATGT GATCCCAAAGCAAGATCTTCTTCACAGGT GCTTTCAAGAACAGGAGCAGAAGTCACAACAAGCTTCAGTT CTACAGGGATATAAAAATAGAAACCAAGA TATGTCTGGTCAACAAGCTGCGCAACTTGCTCAGCAAAGGT ACTTGATACATAACCATGCAAATGTTTTT CCTGTGCCTGACCAGGGAGGAAGTCACACTCAGACCCCTCC CCAGAAGGACACTCAAAAGCATGCTGCTC TAAGGTGGCATCTCTTACAGAAGCAAGAACAGCAGCAAAC ACAGCAACCCCAAACTGAGTCTTGCCATAG TCAGATGCACAGGCCAATTAAGGTGGAACCTGGATGCAAG CCACATGCCTGTATGCACACAGCACCACCA GAAAACAAAACATGGAAAAAGGTAACTAAGCAAGAGAATC CACCTGCAAGCTGTGATAATGTGCAGCAAA AGAGCATCATTGAGACCATGGAGCAGCATCTGAAGCAGTTT CACGCCAAGTCGTTATTTGACCATAAGGC TCTTACTCTCAAATCACAGAAGCAAGTAAAAGTTGAAATGT CAGGGCCAGTCACAGTTTTGACTAGACAA ACCACTGCTGCAGAACTTGATAGCCACACCCCAGCTTTAGA GCAGCAAACAACTTCTTCAGAAAAGACAC CAACCAAAAGAACAGCTGCTTCTGTTCTCAATAATTTTATA GAGTCACCTTCCAAATTACTAGATACTCC TATAAAAAATTTATTGGATACACCTGTCAAGACTCAATATG ATTTCCCATCTTGCAGATGTGTAGGTTTG GACAGAAGGGTAAAGCTATTAGGATTGAAAGAGTCATCTAT ACTGGTAAAGAAGGCAAAAGTTCTCAGGG ATGTCCTATTGCTAAGTGGGAGAACTTGCGCCTGTCAGGGG CTGGATCCAGAAACCTGTGGTGCCTCCTT CTCTTTTGGTTGTTCATGGAGCATGTACTACAATGGATGTAA GTTTGCCAGAAGCAAGATCCCAAGGAAG TTTAAGCTGCTTGGGGATGACCCAAAAGAGGAAGAGAAAC TGGAGTCTCATTTGCAAAACCTGTCCACTC TTATGGCACCAACATATAAGAAACTTGCACCTGATGCATAT AATAATCAGATTGAATATGAACACAGAGC ACCAGAGTGCCGTCTGGGTCTGAAGGAAGGCCGTCCATTCT CAGGGGTCACTGCATGTTTGGACTTCTGT GCTCATGCCCACAGAGACTTGCACAACATGCAGAATGGCAG CACATTGGTATGCACTCTCACTAGAGAAG ACAATCGAGAATTTGGAGGAAAACCTGAGGATGAGCAGCT TCACGTTCTGCCTTTATACAAAGTCTCTGA CGTGGATGAGTTTGGGAGTGTGGAAGCTCAGGAGGAGAAA AAACGGAGTGGTGCCATTCAGGTACTGAGT TCTTTTCGGCGAAAAGTCAGGATGTTAGCAGAGCCAGTCAA GACTTGCCGACAAAGGAAACTAGAAGCCA AGAAAGCTGCAGCTGAAAAGCTTTCCTCCCTGGAGAACAGC TCAAATAAAAATGAAAAGGAAAAGTCAGC CCCATCACGTACAAAACAAACTGAAAACGCAAGCCAGGCT AAACAGTTGGCAGAACTTTTGCGACTTTCA |
| | | GGACCAGTCATGCAGCAGTCCCAGCAGCCCCAGCCTCTACA GAAGCAGCCACCACAGCCCCAGCAGCAGC AGAGACCCCAGCAGCAGCAGCCACATCACCCTCAGACAGA GTCTGTCAACTCTTATTCTGCTTCTGGATC CACCAATCCATACATGAGACGGCCCAATCCAGTTAGTCCTT ATCCAAACTCTTCACACACTTCAGATATC TATGGAAGCACCAGCCCTATGAACTTCTATTCCACCTCATCT CAAGCTGCAGGTTCATATTTGAATTCTT CTAATCCCATGAACCCTTACCCTGGGCTTTTGAATCAGAAT ACCCAATATCCATCATATCAATGCAATGG AAACCTATCAGTGGACAACTGCTCCCCATATCTGGGTTCCT ATTCTCCCCAGTCTCAGCCGATGGATCTG TATAGGTATCCAAGCCAAGACCCTCTGTCTAAGCTCAGTCT ACCACCCATCCATACACTTTACCAGCCAA GGTTTGGAAATAGCCAGAGTTTTACATCTAAATACTTAGGT TATGGAAACCAAAATATGCAGGGAGATGG TTTCAGCAGTTGTACCATTAGACCAAATGTACATCATGTAG GGAAATTGCCTCCTTATCCCACTCATGAG ATGGATGGCCACTTCATGGGAGCCACCTCTAGATTACCACC CAATCTGAGCAATCCAAACATGGACTATA AAAATGGTGAACATCATTCACCTTCTCACATAATCCATAAC TACAGTGCAGCTCCGGGCATGTTCAACAG CTCTCTTCATGCCCTGCATCTCCAAAACAAGGAGAATGACA TGCTTTCCCACACAGCTAATGGGTTATCA AAGATGCTTCCAGCTCTTAACCATGATAGAACTGCTTGTGT CCAAGGAGGCTTACACAAATTAAGTGATG CTAATGGTCAGGAAAAGCAGCCATTGGCACTAGTCCAGGGT GTGGCTTCTGGTGCAGAGGACAACGATGA GGTCTGGTCAGACAGCGAGCAGAGCTTTCTGGATCCTGACA TTGGGGGAGTGGCCGTGGCTCCAACTCAT GGGTCAATTCTCATTGAGTGTGCAAAGCGTGAGCTGCATGC CACAACCCCTTTAAAGAATCCCAATAGGA ATCACCCCACCAGGATCTCCCTCGTCTTTTACCAGCATAAG AGCATGAATGAGCCAAAACATGGCTTGGC TCTTTGGGAAGCCAAAATGGCTGAAAAAGCCCGTGAGAAA GAGGAAGAGTGTGAAAAGTATGGCCCAGAC TATGTGCCTCAGAAATCCCATGGCAAAAAAGTGAAACGGG AGCCTGCTGAGCCACATGAAACTTCAGAGC CCACTTACCTGCGTTTCATCAAGTCTCTTGCCGAAAGGACC ATGTCCGTGACCACAGACTCCACAGTAAC TACATCTCCATATGCCTTCACTCGGGTCACAGGGCCTTACA ACAGATATATATGATATCACCCCCTTTTG TTGGTTACCTCACTTGAAAAGACCACAACCAACCTGTCAGT AGTATAGTTCTCATGACGTGGGCAGTGGG GAAAGGTCACAGTATTCATGACAAATGTGGTGGGAAAAAC CTCAGCTCACCAGCAACAAAAGAGGTTATC TTACCATAGCACTTAATTTTCACTGGCTCCCAAGTGGTCACA GATGGCATCTAGGAAAAGACCAAAGCAT TCTATGCAAAAAGAAGGTGGGGAAGAAAGTGTTCCGCAAT TTACATTTTTAAACACTGGTTCTATTATTG |
| | | GACGAGATGATATGTAAATGTGATCCCCCCCCCCCGCTTAC AACTCTACACATCTGTGACCACTTTTAAT AATATCAAGTTTGCATAGTCATGGAACACAAATCAAACAAG TACTGTAGTATTACAGTGACAGGAATCTT AAAATACCATCTGGTGCTGAATATATGATGTACTGAAATAC TGGAATTATGGCTTTTTGAAATGCAGTTT TTACTGTAATCTTAACTTTATTTATCAAAATAGCTACAGGA AACATGAATAGCAGGAAAACACTGAATT TGTTTGGATGTTCTAAGAAATGGTGCTAAGAAAATGGTGTC TTTAATAGCTAAAAATTTAATGCCTTTAT ATCATCAAGATGCTATCAGTGTACTCCAGTGCCCTTGAATA ATAGGGGTACCTTTTCATTCAAGTTTTTA TCATAATTACCTATTCTTACACAAGCTTAGTTTTTAAAATGT GGACATTTTAAAGGCCTCTGGATTTTGC TCATCCAGTGAAGTCCTTGTAGGACAATAAACGTATATATG TACATATATACACAAACATGTATATGTGC ACACACATGTATATGTATAAATATTTTAAATGGTGTTTTAGA AGCACTTTGTCTACCTAAGCTTTGACAA CTTGAACAATGCTAAGGTACTGAGATGTTTAAAAAACAAGT TTACTTTCATTTTAGAATGCAAAGTTGAT TTTTTTAAGGAAACAAAGAAAGCTTTTAAAATATTTTTGCTT TTAGCCATGCATCTGCTGATGAGCAATT GTGTCCATTTTTAACACAGCCAGTTAAATCCACCATGGGGC TTACTGGATTCAAGGGAATACGTTAGTCC ACAAAACATGTTTTCTGGTGCTCATCTCACATGCTATACTGT AAAACAGTTTTATACAAAATTGTATGAC AAGTTCATTGCTCAAAAATGTACAGTTTTAAGAATTTTCTAT TAACTGCAGGTAATAATTAGCTGCATGC TGCAGACTCAACAAAGCTAGTTCACTGAAGCCTATGCTATT TTATGGATCATAGGCTCTTCAGAGAACTG AATGGCAGTCTGCCTTTGTGTTGATAATTATGTACATTGTGA CGTTGTCATTTCTTAGCTTAAGTGTCCT CTTTAACAAGAGGATTGAGCAGACTGATGCCTGCATAAGAT GAATAAACAGGGTTAGTTCCATGTGAATC TGTCAGTTAAAAAGAAACAAAAACAGGCAGCTGGTTTGCTG TGGTGGTTTTAAATCATTAATTTGTATAA AGAAGTGAAAGAGTTGTATAGTAAATTAAATTGTAAACAA AACTTTTTTAATGCAATGCTTTAGTATTTT AGTACTGTAAAAAAATTAAATATATACATATATATATATAT ATATATATATATATATATGAGTTTGAAGC AGAATTCACATCATGATGGTGCTACTCAGCCTGCTACAAAT ATATCATAATGTGAGCTAAGAATTCATTA AATGTTTGAGTGATGTTCCTACTTGTCATATACCTCAACACT AGTTTGGCAATAGGATATTGAACTGAGA GTGAAAGCATTGTGTACCATCATTTTTTTCCAAGTCCTTTTT TTTATTGTTAAAAAAAAAAGCATACCTT TTTTCAATACTTGATTTCTTAGCAAGTATAACTTGAACTTCA ACCTTTTTGTTCTAAAAATTCAGGGATA TTTCAGCTCATGCTCTCCCTATGCCAACATGTCACCTGTGTT TATGTAAAATTGTTGTAGGTTAATAAAT |
| | | ATATTCTTTGTCAGGGATTTAACCCTTTTATTTTGAATCCCTT CTATTTTACTTGTACATGTGCTGATGT AACTAAAACTAATTTTGTAAATCTGTTGGCTCTTTTTATTGT AAAGAAAAGCATTTTAAAAGTTTGAGGA ATCTTTTGACTGTTTCAAGCAGGAAAAAAAAATTACATGAA AATAGAATGCACTGAGTTGATAAAGGGAA AAATTGTAAGGCAGGAGTTTGGCAAGTGGCTGTTGGCCAGA GACTTACTTGTAACTCTCTAAATGAAGTT TTTTTGATCCTGTAATCACTGAAGGTACATACTCCATGTGGA CTTCCCTTAAACAGGCAAACACCTACAG GTATGGTGTGCAACAGATTGTACAATTACATTTTGGCCTAA ATACATTTTTGCTTACTAGTATTTAAAAT AAATTCTTAATCAGAGGAGGCCTTTGGGTTTTATTGGTCAA ATCTTTGTAAGCTGGCTTTTGTCTTTTTA AAAAATTTCTTGAATTTGTGGTTGTGTCCAATTTGCAAACAT TTCCAAAAATGTTTGCTTTGCTTACAAA CCACATGATTTTAATGTTTTTTGTATACCATAATATCTAGCC CCAAACATTTGATTACTACATGTGCATT GGTGATTTTGATCATCCATTCTTAATATTTGATTTCTGTGTC ACCTACTGTCATTTGTTAAACTGCTGGC CAACAAGAACAGGAAGTATAGTTTGGGGGGTTGGGGAGAG TTTACATAAGGAAGAGAAGAAATTGAGTGG CATATTGTAAATATCAGATCTATAATTGTAAATATAAAACC TGCCTCAGTTAGAATGAATGGAAAGCAGA TCTACAATTTGCTAATATAGGAATATCAGGTTGACTATATA GCCATACTTGAAAATGCTTCTGAGTGGTG TCAACTTTACTTGAATGAATTTTTCATCTTGATTGACGCACA GTGATGTACAGTTCACTTCTGAAGCTAG TGGTTAACTTGTGTAGGAAACTTTTGCAGTTTGACACTAAG ATAACTTCTGTGTGCATTTTTCTATGCTT TTTTAAAAACTAGTTTCATTTCATTTTCATGAGATGTTTGGT TTATAAGATCTGAGGATGGTTATAAATA CTGTAAGTATTGTAATGTTATGAATGCAGGTTATTTGAAAG CTGTTTATTATTATATCATTCCTGATAAT GCTATGTGAGTGTTTTTAATAAAATTTATATTTATTTAATGC ACTCTAA |
| HHomo sapiens tet methylcytosine dioxygenase 2 (TET2), transcript variant 2, mRNA | NM_017628. 4 | AAACAGAAGGTGGGCCGGGGCGGGAGAAACAGAACTCGG TCAATTTCCCAGTTTGTCGGGTCTTTAAAA ATACAGGCCCCTAAAGCACTAAGGGCATGCCCTCGGTGAAA CAGGGGAGCGCTTCTGCTGAATGAGATTA AAGCGACAGAAAAGGGAAAGGAGAGCGCGGGCAACGGGA TCTAAAGGGAGATAGAGACGCGGGCCTCTGA GGGCTGGCAAACATTCAGCAGCACACCCTCTCAAGATTGTT TACTTGCCTTTGCTCCTGTTGAGTTACAA CGCTTGGAAGCAGGAGATGGGCTCAGCAGCAGCCAATAGG ACATGATCCAGGAAGAGCAGTAAGGGACTG AGCTGCTGAATTCAACTAGAGGGCAGCCTTGTGGATGGCCC CGAAGCAAGCCTGATGGAACAGGATAGAA CCAACCATGTTGAGGGCAACAGACTAAGTCCATTCCTGATA CCATCACCTCCCATTTGCCAGACAGAACC |
| [SEQ ID NO: 1361] | | |
| | | TCTGGCTACAAAGCTCCAGAATGGAAGCCACTGCCTGAGA GAGCTCATCCAGAAGTAAATGGAGACACC AAGTGGCACTCTTTCAAAAGTTATTATGGAATACCCTGTAT GAAGGGAAGCCAGAATAGTCGTGTGAGTC CTGACTTTACACAAGAAAGTAGAGGGTATTCCAAGTGTTTG CAAAATGGAGGAATAAAACGCACAGTTAG TGAACCTTCTCTCTCTGGGCTCCTTCAGATCAAGAAATTGAA ACAAGACCAAAAGGCTAATGGAGAAAGA CGTAACTTCGGGGTAAGCCAAGAAAGAAATCCAGGTGAAA GCAGTCAACCAAATGTCTCCGATTTGAGTG ATAAGAAAGAATCTGTGAGTTCTGTAGCCCAAGAAAATGCA GTTAAAGATTTCACCAGTTTTTCAACACA TAACTGCAGTGGGCCTGAAAATCCAGAGCTTCAGATTCTGA ATGAGCAGGAGGGGAAAAGTGCTAATTAC CATGACAAGAACATTGTATTACTTAAAAACAAGGCAGTGCT AATGCCTAATGGTGCTACAGTTTCTGCCT CTTCCGTGGAACACACACATGGTGAACTCCTGGAAAAACA CTGTCTCAATATTATCCAGATTGTGTTTC CATTGCGGTGCAGAAAACCACATCTCACATAAATGCCATTA ACAGTCAGGCTACTAATGAGTTGTCCTGT GAGATCACTCACCCATCGCATACCTCAGGGCAGATCAATTC CGCACAGACCTCTAACTCTGAGCTGCCTC CAAAGCCAGCTGCAGTGGTGAGTGAGGCCTGTGATGCTGAT GATGCTGATAATGCCAGTAAACTAGCTGC AATGCTAAATACCTGTTCCTTTCAGAAACCAGAACAACTAC AACAACAAAAATCAGTTTTGAGATATGC CCATCTCCTGCAGAAAATAACATCCAGGGAACCACAAAGCT AGCGTCTGGTGAAGAATTCTGTTCAGGTT CCAGCAGCAATTTGCAAGCTCCTGGTGGCAGCTCTGAACGG TATTTAAAACAAAATGAAATGAATGGTGC TTACTTCAAGCAAAGCTCAGTGTTCACTAAGGATTCCTTTTC TGCCACTACCACACCACCACCACCATCA CAATTGCTTCTTTCTCCCCCTCCTCCTCTTCCACAGGTTCCTC AGCTTCCTTCAGAAGGAAAAAGCACTC TGAATGGTGGAGTTTTAGAAGAACACCACCACTACCCCAAC CAAAGTAACACAACACTTTTAAGGGAAGT GAAAATAGAGGGTAAACCTGAGGCACCACCTTCCCAGAGT CCTAATCCATCTACACATGTATGCAGCCCT TCTCCGATGCTTTCTGAAAGGCCTCAGAATAATTGTGTGAA CAGGAATGACATACAGACTGCAGGGACAA TGACTGTTCCATTGTGTTCTGAGAAAACAAGACCAATGTCA GAACACCTCAAGCATAACCCACCAATTTT TGGTAGCAGTGGAGAGCTACAGGACAACTGCCAGCAGTTG ATGAGAAACAAAGAGCAAGAGATTCTGAAG GGTCGAGACAAGGAGCAAACACGAGATCTTGTGCCCCCAA CACAGCACTATCTGAAACCAGGATGGATTG AATTGAAGGCCCCTCGTTTTCACCAAGCGGAATCCCATCTA AAACGTAATGAGGCATCACTGCCATCAAT TCTTCAGTATCAACCCAATCTCTCCAATCAAATGACCTCCAA ACAATACACTGGAAATTCCAACATGCCT |
| | | GGGGGGCTCCCAAGGCAAGCTTACACCCAGAAAACAACAC AGCTGGAGCACAAGTCACAAATGTACCAAG TTGAAATGAATCAAGGGCAGTCCCAAGGTACAGTGGACCA ACATCTCCAGTTCCAAAAACCCTCACACCA GGTGCACTTCTCCAAAACAGACCATTTACCAAAAGCTCATG TGCAGTCACTGTGTGGCACTAGATTTCAT TTTCAACAAAGAGCAGATTCCCAAACTGAAAAACTTATGTC CCCAGTGTTGAAACAGCACTTGAATCAAC AGGCTTCAGAGACTGAGCCATTTTCAAACTCACACCTTTTG CAACATAAGCCTCATAAACAGGCAGCACA AACACAACCATCCCAGAGTTCACATCTCCCTCAAAACCAGC AACAGCAGCAAAAATTACAAATAAAGAAT AAAGAGGAAATACTCCAGACTTTTCCTCACCCCCAAAGCAA CAATGATCAGCAAAGAGAAGGATCATTCT TTGGCCAGACTAAAGTGGAAGAATGTTTTCATGGTGAAAAT CAGTATTCAAAATCAAGCGAGTTCGAGAC TCATAATGTCCAAATGGGACTGGAGGAAGTACAGAATATA AATCGTAGAAATTCCCCTTATAGTCAGACC ATGAAATCAAGTGCATGCAAAATACAGGTTTCTTGTTCAAA CAATACACACCTAGTTTCAGAGAATAAAG AACAGACTACACATCCTGAACTTTTTGCAGGAAACAAGACC CAAAACTTGCATCACATGCAATATTTTCC AAATAATGTGATCCCAAAGCAAGATCTTCTTCACAGGTGCT TTCAAGAACAGGAGCAGAAGTCACAACAA GCTTCAGTTCTACAGGGATATAAAAATAGAAACCAAGATAT GTCTGGTCAACAAGCTGCGCAACTTGCTC AGCAAAGGTACTTGATACATAACCATGCAAATGTTTTTCCT GTGCCTGACCAGGGAGGAAGTCACACTCA GACCCCTCCCCAGAAGGACACTCAAAAGCATGCTGCTCTAA GGTGGCATCTCTTACAGAAGCAAGAACAG CAGCAAACACAGCAACCCCAAACTGAGTCTTGCCATAGTCA GATGCACAGGCCAATTAAGGTGGAACCTG GATGCAAGCCACATGCCTGTATGCACACAGCACCACCAGAA AACAAAACATGGAAAAAGGTAACTAAGCA AGAGAATCCACCTGCAAGCTGTGATAATGTGCAGCAAAAG AGCATCATTGAGACCATGGAGCAGCATCTG AAGCAGTTTCACGCCAAGTCGTTATTTGACCATAAGGCTCT TACTCTCAAATCACAGAAGCAAGTAAAAG TTGAAATGTCAGGGCCAGTCACAGTTTTGACTAGACAAACC ACTGCTGCAGAACTTGATAGCCACACCCC AGCTTTAGAGCAGCAAACAACTTCTTCAGAAAAGACACCAA CCAAAAGAACAGCTGCTTCTGTTCTCAAT AATTTTATAGAGTCACCTTCCAAATTACTAGATACTCCTATA AAAAATTTATTGGATACACCTGTCAAGA CTCAATATGATTTCCCATCTTGCAGATGTGTAGGTAAGTGCC AGAAATGTACTGAGACACATGGCGTTTA TCCAGAATTAGCAAATTTATCTTCAGATATGGGATTTTCCTT CTTTTTTTAAATCTTGAGTCTGGCAGCA ATTTGTAAAGGCTCATAAAAATCTGAAGCTTACATTTTTTGT CAAGTTACCGATGCTTGTGTCTTGTGAA |
| | | AGAGAACTTCACTTACATGCAGTTTTTCCAAAAGAATTAAA TAATCGTGCATGTTTATTTTTCCCTCTCT TCAGATCCTGTAAAATTTGAATGTATCTGTTTTAGATCAATT CGCCTATTTAGCTCTTTGTATATTATCT CCTGGAGAGACAGCTAGGCAGCAAAAAAACAATCTATTAA AATGAGAAAATAACGACCATAGGCAGTCTA ATGTACGAACTTTAAATATTTTTAATTCAAGGTAAAATATA TTAGTTTCACAAGATTTCTGGCTAATAG GGAAATTATTATCTTCAGTCTTCATGAGTTGGGGGAAATGA TAATGCTGACACTCTTAGTGCTCCTAAAG TTTCCTTTTCTCCATTTATACATTTGGAATGTTGTGATTTATA TTCATTTTGATTCCCTTTTCTCTAAAA TTTCATCTTTTTGATTAAAAAATATGATACAGGCATACCTCA GAGATATTGTGGGTTTGGCTCCATACCA CAATAAAATGAATATTACAATAAAGCAAGTTGTAAGGACTT TTTGGTTTCTCACTGTATGTAAAAGTTAT TTATATACTATACTGTAACATACTAAGTGTGCAATAGCATT GTGTCTAAAAAATATATACTTTAAAAATA ATTTATTGTTAAAAAAATGCCAACAATTATCTGGGCCTTTA GTGAGTGCTAATCTTTTTGCTGGTGGAGG GTCGTGCTTCAGTATTGATCGCTGTGGACTGATCATGGTGGT AGTTGCTGAAGGTTGCTGGGATGGCTGT GTGTGTGGCAATTTCTTAAAATAAGACAACAGTGAAGTGCT GTATCAATTGATTTTTCCATTCACAAAAG ATTTCTCTGTAGCATGCAATGCTGTTTGATAGCATTTAACCC ACAGCAGAATTTCTTTGAAAATTGGACT CAGTCCTCTCAAACTGTGCTGCTGCTTTATCAACTAAGTTTT TGTAATTTTCTGAATCCTTTGTTGTCAT TTCAGCAGTTTACAGCATCTTCATTGGAAGTATATTCCATCT CAAACATTCTTTGTTCATCCATAAGAAG CAACTTCTTATCAAGTTTTTCATGACATTGCAGTAACTCAG CCCCATCTTCAGGCTCTACTTCTAATTC TGGTTCTCTTGCTACATCTCCCTCATCTGCAGTGACCTCTCC ACGGAAGTCTTGAACTCCTCAAAGTAAT CCATGAGGGTTGGAATCAACTTCTAAACTCCTGTTAATGTT GATATATTGACCCCCTCCCATGAATTATG AATGTTCTTAATAACTTCTAAATGGTGATACCTTTCCAGAAG GCTTTCAATGTACTTTGCCCGGATCCAT CAGAAGACTATCTTGGCAGCTGTAGACTAACAATATATTTC TTAAATGATAAGACTTGAAAGTCAAAAGT ACTCCTTAATCCATAGGCTGCAGAATCAATGTTGTATTAAC AGGCACGAAAACAGCATTAATCTTGTGCA TCTCCATCGGAGCTCTTGGGTGACTAGGTGCCTTGAGCAGT AATATTTTGAAAGGAGGTTTTGGTTTTGT TTTTTGTTTTTTTTTTTGTTTTTAGCAGTAAGTCTCAACAC TGGGCTTAAAATATTCAGTAAACTATG TTGTAAAAAGATGTGTTATCATCCAGACTTTGTTGTTCCATT ACTCTACACAAGCAGGGTACACTTAGCA TAATTCTTAAGGGCCTTGGAATTTTCAGAATGGTAAATGAG TATGGGCTTCAACTTAAAATCATCAACTG |
| | | CATTAGCCTGTAACAAGAGAGTCAGCCTGTCCTTTGAAGCA AGGCATTGACTTCTATCTATGAAAGTCTT AGATGGCACCTTGTTTCAATAGTAGGCTGTTTAGTACAGCC ACCTTCATCAGTGATCTTAGCTAGATCTT CTGCATAACTTGCTGCAGCTTCTACATCAGCACTTGCTGCCT CACCTTGTCCTTTTATGTTATAGAGACA GCTGCGCTTCTTAAACTTTATAAACCAACTTCTGCTAGCTTC CAACTTCTCTTCTGCAGCTTCCTCATTC TCTTCATAGAACTGAAGGGAGTCAAGGCCTTGCTCTGGATT AAGCTTTGGCTTAAGGAATGTTGTGGCTG ACGTGATCTTCTATCCAGACCACTAAAGCGCTCTCCATATC AGCAATAAGGCCGTTTTGCTTTCTTACCT TTCATGTGTTCACTGGAGTAATTTCCTTCAAGAATTTTTCCT TTACATTCACAACTTGGCTAACTGGCAT GCAAGGCCTAGCTTTCAGCCTGTCTTGGCTTTTGACATGCCT TCCTCACTTAGCTCGTCATATCTAGCTT TTGATTTAAAGTGGCAGGCATACAACTCTTCCTTTCACTTGA ACACTTAGAGGCCACTGTAGGGTTATTA ATTGGCCTAATTTCAATATTGTTGTGTTTTAGGGAATAGAGA GGCCCAGGGAGAGGGAGAGAGCCCAAAC GGCTGGTTGATAGAGCAGGCAGAATGCACACAACATTTATC AGATTATGTTTGCACCATTTACCAGATTA TGGGTACGGTTTGTGGCACCCCCCAAAAATTAGAATAGTAA CATCAAAGATCACTGATCACAGATCGCCA TAACATAAATAATAATAAACTTTAAAATACTGTGAGAATTA CCAAAATGTGATACAGAGACATGAAGTGA GCACATGCTGTTGAAAAAAATGACACTGATAGACATACTTA ACACGTGGGATTGCCACAAACCTTCAGTT TGTAAAAGTCACAGTAACTGTGACTCACAAAAGAACAAAG CACAATAAAACGAGGTATGCCTGTATTTTT AAAAAAAGCTTTTTGTTAAAATTCAGGATATGTAATAGGTC TGTAGGAATAGTGAAATATTTTTGCTGAT GGATGTAGATATATACGTGGATAGAGATGAAGATCTTAATT ATAGCTATGCAGCATAGATTTAGTCAAAG ACATTTGAAAAGACAAATGTTAAATTAGTGTGGCTAATGAC CTACCCGTGCCATGTTTTCCCTCTTGCAA TGAGATACCCCACACTGTGTAGAAGGATGGAGGGAGGACT CCTACTGTCCCTCTTTGCGTGTGGTTATTA AGTTGCCTCACTGGGCTAAAACACCACACATCTCATAGATA ATATTTGGTAAGTTGTAATCGTCTTCACT CTTCTCTTATCACCCACCCCTATCTTCCCACTTTTCCATCTTT GTTGGTTTGCAACAGCCCCTTCTTTTT GCCTGACTCTCCAGGATTTTCTCTCATCATAAATTGTTCTAA AGTACATACTAATATGGGTCTGGATTGA CTATTCTTATTTGCAAAACAGCAATTAAATGTTATAGGGAA GTAGGAAGAAAAAGGGGTATCCTTGACAA TAAACCAAGCAATATTCTGGGGGTGGGATAGAGCAGGAAA TTTTATTTTTAATCTTTTAAAATCCAAGTA ATAGGTAGGCTTCCAGTTAGCTTTAAATGTTTTTTTTTCCA GCTCAAAAAATTGGATTGTAGTTGATAC |
| | | TACATATAATACATTCTAATTCCCTCACTGTATTCTTTGTTT AGTTTCATTTATTTGGTTTAAAATAATT TTTTATCCCATATCTGAAATGTAATATATTTTTATCCAACAA CCAGCATGTACATATACTTAATTATGTG GCACATTTTCTAATAGATCAGTCCATCAATCTACTCATTTTA AAGAAAAAAAAATTTTAAAGTCACTTTT AGAGCCCTTAATGTGTAGTTGGGGGTTAAGCTTTGTGGATG TAGCCTTTATATTTAGTATAATTGAGGTC TAAAATAATAATCTTCTATTATCTCAACAGAGCAAATTATT GAAAAAGATGAAGGTCCTTTTTATACCCA TCTAGGAGCAGGTCCTAATGTGGCAGCTATTAGAGAAATCA TGGAAGAAAGGTAATTAACGCAAAGGCAC AGGGCAGATTAACGTTTATCCTTTTGTATATGTCAGAATTTT TCCAGCCTTCACACACAAAGCAGTAAAC AATTGTAAATTGAGTAATTATTAGTAGGCTTAGCTATTCTAG GGTTGCCAACACTACACACTGTGCTATT CACCAGAGAGTCACAATATTTGACAGGACTAATAGTCTGCT AGCTGGCACAGGCTGCCCACTTTGCGATG GATGCCAGAAAACCCAGGCATGAACAGGAATCGGCCAGCC AGGCTGCCAGCCACAAGGTACTGGCACAGG CTCCAACGAGAGGTCCCACTCTGGCTTTCCCACCTGATAAT AAAGTGTCAAAGCAGAAAGACTGGTAAAG TGTGGTATAAGAAAAGAACCACTGAATTAAATTCACCTAGT GTTGCAAATGAGTACTTATCTCTAAGTTT TCTTTTACCATAAAAAGAGAGCAAGTGTGATATGTTGAATA GAAAGAGAAACATACTATTTACAGCTGCC TTTTTTTTTTTTTTCGCTATCAATCACAGGTATACAAGTACT TGCCTTTACTCCTGCATGTAGAAGACT CTTATGAGCGAGATAATGCAGAGAAGGCCTTTCATATAAAT TTATACAGCTCTGAGCTGTTCTTCTTCTA GGGTGCCTTTTCATTAAGAGGTAGGCAGTATTATTATTAAA GTACTTAGGATACATTGGGGCAGCTAGGA CATATTCAGTATCATTCTTGCTCCATTTCCAAATTATTCATTT CTAAATTAGCATGTAGAAGTTCACTAA ATAATCATCTAGTGGCCTGGCAGAAATAGTGAATTTCCCTA AGTGCCTTTTTTTGTTGTTTTTTGTTT TGTTTTTTAAACAAGCAGTAGGTGGTGCTTTGGTCATAAGG GAAGATATAGTCTATTTCTAGGACTATTC CATATTTTCCATGTGGCTGGATACTAACTATTTGCCAGCCTC CTTTTCTAAATTGTGAGACATTCTTGGA GGAACAGTTCTAACTAAAATCTATTATGACTCCCCAAGTTTT AAAATAGCTAAATTTAGTAAGGAAAAA ATAGTTTATGTTTTAGAAGACTGAACTTAGCAAACTAACCT GAATTTTGTGCTTTGTGAAATTTTATATC GAAATGAGCTTTCCCATTTTCACCCACATGTAATTTACAAA ATAGTTCATTACAATTATCTGTACATTTT GATATTGAGGAAAAACAAGGCTTAAAAACCATTATCCAGTT TGCTTGGCGTAGACCTGTTTAAAAAATAA TAAACCGTTCATTTCTCAGGATGTGGTCATAGAATAAAGTT ATGCTCAAATGTTCAAATATTTAAA |
| PPREDICTED: Homo sapiens tet methylcytosine dioxygenase 2 (TET2), transcript variant X9, mRNA | XXM_01153 2044.1 | TCAGGCTCTACTTCTAATTCTGGTTCTCTTGCTACATCTCCCT CATCTGCAGTGACCTCTCCACGGAAGT CTTGAACTCCTCAAAAGCAAATTATTGAAAAAGATGAAGGT CCTTTTTATACCCATCTAGGAGCAGGTCC TAATGTGGCAGCTATTAGAGAAATCATGGAAGAAAGGTTTG GACAGAAGGGTAAAGCTATTAGGATTGAA AGAGTCATCTATACTGGTAAAGAAGGCAAAAGTTCTCAGGG ATGTCCTATTGCTAAGTGGGTGGTTCGCA GAAGCAGCAGTGAAGAGAAGCTACTGTGTTTGGTGCGGGA GCGAGCTGGCCACACCTGTGAGGCTGCAGT GATTGTGATTCTCATCCTGGTGTGGGAAGGAATCCCGCTGT CTCTGGCTGACAAACTCTACTCGGAGCTT ACCGAGACGCTGAGGAAATACGGCACGCTCACCAATCGCC GGTGTGCCTTGAATGAAGAGAGAACTTGCG CCTGTCAGGGGCTGGATCCAGAAACCTGTGGTGCCTCCTTC TCTTTTGGTTGTTCATGGAGCATGTACTA CAATGGATGTAAGTTTGCCAGAAGCAAGATCCCAAGGAAG TTTAAGCTGCTTGGGGATGACCCAAAAGAG GAAGAGAAACTGGAGTCTCATTTGCAAAACCTGTCCACTCT TATGGCACCAACATATAAGAAACTTGCAC CTGATGCATATAATAATCAGATTGAATATGAACACAGAGCA CCAGAGTGCCGTCTGGGTCTGAAGGAAGG CCGTCCATTCTCAGGGGTCACTGCATGTTTGGACTTCTGTGC TCATGCCCACAGAGACTTGCACAACATG CAGAATGGCAGCACATTGGTATGCACTCTCACTAGAGAAGA CAATCGAGAATTTGGAGGAAAACCTGAGG ATGAGCAGCTTCACGTTCTGCCTTTATACAAAGTCTCTGACG TGGATGAGTTTGGGAGTGTGGAAGCTCA GGAGGAGAAAAAACGGAGTGGTGCCATTCAGGTACTGAGT TCTTTTCGGCGAAAAGTCAGGATGTTAGCA GAGCCAGTCAAGACTTGCCGACAAAGGAAACTAGAAGCCA AGAAAGCTGCAGCTGAAAAGCTTTCCTCCC TGGAGAACAGCTCAAATAAAAATGAAAAGGAAAAGTCAGC CCCATCACGTACAAAACAAACTGAAAACGC AAGCCAGGCTAAACAGTTGGCAGAACTTTTGCGACTTTCAG GACCAGTCATGCAGCAGTCCCAGCAGCCC CAGCCTCTACAGAAGCAGCCACCACAGCCCCAGCAGCAGC AGAGACCCCAGCAGCAGCAGCCACATCACC CTCAGACAGAGTCTGTCAACTCTTATTCTGCTTCTGGATCCA CCAATCCATACATGAGACGGCCCAATCC AGTTAGTCCTTATCCAAACTCTTCACACACTTCAGATATCTA TGGAAGCACCAGCCCTATGAACTTCTAT TCCACCTCATCTCAAGCTGCAGGTTCATATTTGAATTCTTCT AATCCCATGAACCCTTACCCTGGGCTTT TGAATCAGAATACCCAATATCCATCATATCAATGCAATGGA AACCTATCAGTGGACAACTGCTCCCCATA TCTGGGTTCCTATTCTCCCCAGTCTCAGCCGATGGATCTGTA TAGGTATCCAAGCCAAGACCCTCTGTCT AAGCTCAGTCTACCACCCATCCATACACTTTACCAGCCAAG GTTTGGAAATAGCCAGAGTTTTACATCTA |
| [SEQ ID NO: 1362] | | |
| | | AATACTTAGGTTATGGAAACCAAAATATGCAGGGAGATGGT TTCAGCAGTTGTACCATTAGACCAAATGT ACATCATGTAGGGAAATTGCCTCCTTATCCCACTCATGAGA TGGATGGCCACTTCATGGGAGCCACCTCT AGATTACCACCCAATCTGAGCAATCCAAACATGGACTATAA AAATGGTGAACATCATTCACCTTCTCACA TAATCCATAACTACAGTGCAGCTCCGGGCATGTTCAACAGC TCTCTTCATGCCCTGCATCTCCAAAACAA GGAGAATGACATGCTTTCCCACACAGCTAATGGGTTATCAA AGATGCTTCCAGCTCTTAACCATGATAGA ACTGCTTGTGTCCAAGGAGGCTTACACAAATTAAGTGATGC TAATGGTCAGGAAAAGCAGCCATTGGCAC TAGTCCAGGGTGTGGCTTCTGGTGCAGAGGACAACGATGAG GTCTGGTCAGACAGCGAGCAGAGCTTTCT GGATCCTGACATTGGGGGAGTGGCCGTGGCTCCAACTCATG GGTCAATTCTCATTGAGTGTGCAAAGCGT GAGCTGCATGCCACAACCCCTTTAAAGAATCCCAATAGGAA TCACCCCACCAGGATCTCCCTCGTCTTTT ACCAGCATAAGAGCATGAATGAGCCAAAACATGGCTTGGC TCTTTGGGAAGCCAAAATGGCTGAAAAAGC CCGTGAGAAAGAGGAAGAGTGTGAAAAGTATGGCCCAGAC TATGTGCCTCAGAAATCCCATGGCAAAAAA GTGAAACGGGAGCCTGCTGAGCCACATGAAACTTCAGAGC CCACTTACCTGCGTTTCATCAAGTCTCTTG CCGAAAGGACCATGTCCGTGACCACAGACTCCACAGTAACT ACATCTCCATATGCCTTCACTCGGGTCAC AGGGCCTTACAACAGATATATATGATATCACCCCCTTTTGTT GGTTACCTCACTTGAAAAGACCACAACC AACCTGTCAGTAGTATAGTTCTCATGACGTGGGCAGTGGGG AAAGGTCACAGTATTCATGACAAATGTGG TGGGAAAAACCTCAGCTCACCAGCAACAAAAGAGGTTATCT TACCATAGCACTTAATTTTCACTGGCTCC CAAGTGGTCACAGATGGCATCTAGGAAAAGACCAAAGCAT TCTATGCAAAAAGAAGGTGGGAAGAAAGT GTTCCGCAATTTACATTTTTAAACACTGGTTCTATTATTGGA CGAGATGATATGTAAATGTGATCCCCCC CCCCCGCTTACAACTCTACACATCTGTGACCACTTTTAATAA TATCAAGTTTGCATAGTCATGGAACACA AATCAAACAAGTACTGTAGTATTACAGTGACAGGAATCTTA AAATACCATCTGGTGCTGAATATATGATG TACTGAAATACTGGAATTATGGCTTTTTGAAATGCAGTTTTT ACTGTAATCTTAACTTTTATTTATCAAA ATAGCTACAGGAAACATGAATAGCAGGAAAACACTGAATT TGTTTGGATGTTCTAAGAAATGGTGCTAAG AAAATGGTGTCTTTAATAGCTAAAAATTTAATGCCTTTATAT CATCAAGATGCTATCAGTGTACTCCAGT GCCCTTGAATAATAGGGGTACCTTTTCATTCAAGTTTTTATC ATAATTACCTATTCTTACACAAGCTTAG TTTTTAAAATGTGGACATTTTAAAGGCCTCTGGATTTTGCTC ATCCAGTGAAGTCCTTGTAGGACAATAA |
| | | ACGTATATATGTACATATATACACAAACATGTATATGTGCA CACACATGTATATGTATAAATATTTTAAA TGGTGTTTTAGAAGCACTTTGTCTACCTAAGCTTTGACAACT TGAACAATGCTAAGGTACTGAGATGTTT AAAAAACAAGTTTACTTTCATTTTAGAATGCAAAGTTGATT TTTTTAAGGAAACAAAGAAAGCTTTTAAA ATATTTTTGCTTTTAGCCATGCATCTGCTGATGAGCAATTGT GTCCATTTTTAACACAGCCAGTTAAATC CACCATGGGGCTTACTGGATTCAAGGGAATACGTTAGTCCA CAAAACATGTTTTCTGGTGCTCATCTCAC ATGCTATACTGTAAAACAGTTTTATACAAAATTGTATGACA AGTTCATTGCTCAAAAATGTACAGTTTTA AGAATTTTCTATTAACTGCAGGTAATAATTAGCTGCATGCT GCAGACTCAACAAAGCTAGTTCACTGAAG CCTATGCTATTTTATGGATCATAGGCTCTTCAGAGAACTGA ATGGCAGTCTGCCTTTGTGTTGATAATTA TGTACATTGTGACGTTGTCATTTCTTAGCTTAAGTGTCCTCT TTAACAAGAGGATTGAGCAGACTGATGC CTGCATAAGATGAATAAACAGGGTTAGTTCCATGTGAATCT GTCAGTTAAAAAGAAACAAAAACAGGCAG CTGGTTTGCTGTGGTGGTTTTAAATCATTAATTTGTATAAAG AAGTGAAAGAGTTGTATAGTAAATTAAA TTGTAAACAAAACTTTTTTAATGCAATGCTTTAGTATTTTAG TACTGTAAAAAAATTAAATATATACATA TATATATATATATATATATATATATATATGAGTTTGAAGCAG AATTCACATCATGATGGTGCTACTCAGC CTGCTACAAATATATCATAATGTGAGCTAAGAATTCATTAA ATGTTTGAGTGATGTTCCTACTTGTCATA TACCTCAACACTAGTTTGGCAATAGGATATTGAACTGAGAG TGAAAGCATTGTGTACCATCATTTTTTTC CAAGTCCTTTTTTTTATTGTTAAAAAAAAAAGCATACCTTTT TTCAATACTTGATTTCTTAGCAAGTATA ACTTGAACTTCAACCTTTTTGTTCTAAAAATTCAGGGATATT TCAGCTCATGCTCTCCCTATGCCAACAT GTCACCTGTGTTTATGTAAAATTGTTGTAGGTTAATAAATAT ATTCTTTGTCAGGGATTTAACCCTTTTA TTTTGAATCCCTTCTATTTTACTTGTACATGTGCTGATGTAA CTAAAACTAATTTTGTAAATCTGTTGGC TCTTTTTATTGTAAAGAAAAGCATTTTAAAAGTTTGAGGAA TCTTTTGACTGTTTCAAGCAGGAAAAAAA AATTACATGAAAATAGAATGCACTGAGTTGATAAAGGGAA AAATTGTAAGGCAGGAGTTTGGCAAGTGGC TGTTGGCCAGAGACTTACTTGTAACTCTCTAAATGAAGTTTT TTTGATCCTGTAATCACTGAAGGTACAT ACTCCATGTGGACTTCCCTTAAACAGGCAAACACCTACAGG TATGGTGTGCAACAGATTGTACAATTACA TTTTGGCCTAAATACATTTTTGCTTACTAGTATTTAAAATAA ATTCTTAATCAGAGGAGGCCTTTGGGTT TTATTGGTCAAATCTTTGTAAGCTGGCTTTTGTCTTTTTAAA AAATTTCTTGAATTTGTGGTTGTGTCCA |
| | | ATTTGCAAACATTTCCAAAAATGTTTGCTTTGCTTACAAACC ACATGATTTTAATGTTTTTGTATACCA TAATATCTAGCCCCAAACATTTGATTACTACATGTGCATTGG TGATTTTGATCATCCATTCTTAATATTT GATTTCTGTGTCACCTACTGTCATTTGTTAAACTGCTGGCCA ACAAGAACAGGAAGTATAGTTTGGGGGG TTGGGGAGAGTTTACATAAGGAAGAGAAGAAATTGAGTGG CATATTGTAAATATCAGATCTATAATTGTA AATATAAAACCTGCCTCAGTTAGAATGAATGGAAAGCAGAT CTACAATTTGCTAATATAGGAATATCAGG TTGACTATATAGCCATACTTGAAAATGCTTCTGAGTGGTGTC AACTTTACTTGAATGAATTTTTCATCTT GATTGACGCACAGTGATGTACAGTTCACTTCTGAAGCTAGT GGTTAACTTGTGTAGGAAACTTTTGCAGT TTGACACTAAGATAACTTCTGTGTGCATTTTTCTATGCTTTT TTAAAAACTAGTTTCATTTCATTTTCAT GAGATGTTTGGTTTATAAGATCTGAGGATGGTTATAAATAC TGTAAGTATTGTAATGTTATGAATGCAGG TTATTTGAAAGCTGTTTATTATTATATCATTCCTGATAATGC TATGTGAGTGTTTTTAATAAAATTTATA TTTATTTAATGCACTCTAA |
| PPREDICTED: Homo sapiens tet methylcytosine dioxygenase 2 (TET2), transcript variant X7, mRNA | XXM_01153 2043.1 | GTAGAGAAGCAGAAGGAAGCAAGATGGCTGCCCTTTAGGA TTTGTTAGAAAGGAGACCCGACTGCAACTG CTGGATTGCTGCAAGGCTGAGGGACGAGAACGAGGCTGGC AAACATTCAGCAGCACACCCTCTCAAGATT GTTTACTTGCCTTTGCTCCTGTTGAGTTACAACGCTTGGAAG CAGGAGATGGGCTCAGCAGCAGCCAATA GGACATGATCCAGGAAGAGCAGTAAGGGACTGAGCTGCTG AATTCAACTAGAGGGCAGCCTTGTGGATGG CCCCGAAGCAAGCCTGATGGAACAGGATAGAACCAACCAT GTTGAGGGCAACAGACTAAGTCCATTCCTG ATACCATCACCTCCCATTTGCCAGACAGAACCTCTGGCTAC AAAGCTCCAGAATGGAAGCCCACTGCCTG AGAGAGCTCATCCAGAAGTAAATGGAGACACCAAGTGGCA CTCTTTCAAAAGTTATTATGGAATACCCTG TATGAAGGGAAGCCAGAATAGTCGTGTGAGTCCTGACTTTA CACAAGAAAGTAGAGGGTATTCCAAGTGT TTGCAAAATGGAGGAATAAAACGCACAGTTAGTGAACCTTC TCTCTCTGGGCTCCTTCAGATCAAGAAAT TGAAACAAGACCAAAAGGCTAATGGAGAAAGACGTAACTT CGGGGTAAGCCAAGAAAGAAATCCAGGTGA AAGCAGTCAACCAAATGTCTCCGATTTGAGTGATAAGAAAG AATCTGTGAGTTCTGTAGCCCAAGAAAAT GCAGTTAAAGATTTCACCAGTTTTTCAACACATAACTGCAG TGGGCCTGAAAATCCAGAGCTTCAGATTC TGAATGAGCAGGAGGGGAAAAGTGCTAATTACCATGACAA GAACATTGTATTACTTAAAAACAAGGCAGT GCTAATGCCTAATGGTGCTACAGTTTCTGCCTCTTCCGTGGA ACACACACATGGTGAACTCCTGGAAAAA ACACTGTCTCAATATTATCCAGATTGTGTTTCCATTGCGGTG |
| [SEQ ID NO: 1363] | | |
| | | CAGAAAACCACATCTCACATAAATGCCA TTAACAGTCAGGCTACTAATGAGTTGTCCTGTGAGATCACT CACCCATCGCATACCTCAGGGCAGATCAA TTCCGCACAGACCTCTAACTCTGAGCTGCCTCCAAAGCCAG CTGCAGTGGTGAGTGAGGCCTGTGATGCT GATGATGCTGATAATGCCAGTAAACTAGCTGCAATGCTAAA TACCTGTTCCTTTCAGAAACCAGAACAAC TACAACAACAAAAATCAGTTTTTGAGATATGCCCATCTCCT GCAGAAAATAACATCCAGGGAACCACAAA GCTAGCGTCTGGTGAAGAATTCTGTTCAGGTTCCAGCAGCA ATTTGCAAGCTCCTGGTGGCAGCTCTGAA CGGTATTTAAAACAAAATGAAATGAATGGTGCTTACTTCAA GCAAAGCTCAGTGTTCACTAAGGATTCCT TTTCTGCCACTACCACACCACCACCACCATCACAATTGCTTC TTTCTCCCCCTCCTCCTCTTCCACAGGT TCCTCAGCTTCCTTCAGAAGGAAAAAGCACTCTGAATGGTG GAGTTTTAGAAGAACACCACCACTACCCC AACCAAAGTAACACAACACTTTTAAGGGAAGTGAAAATAG AGGGTAAACCTGAGGCACCACCTTCCCAGA GTCCTAATCCATCTACACATGTATGCAGCCCTTCTCCGATGC TTTCTGAAAGGCCTCAGAATAATTGTGT GAACAGGAATGACATACAGACTGCAGGGACAATGACTGTT CCATTGTGTTCTGAGAAAACAAGACCAATG TCAGAACACCTCAAGCATAACCCACCAATTTTTGGTAGCAG TGGAGAGCTACAGGACAACTGCCAGCAGT TGATGAGAAACAAAGAGCAAGAGATTCTGAAGGGTCGAGA CAAGGAGCAAACACGAGATCTTGTGCCCCC AACACAGCACTATCTGAAACCAGGATGGATTGAATTGAAG GCCCCTCGTTTTCACCAAGCGGAATCCCAT CTAAAACGTAATGAGGCATCACTGCCATCAATTCTTCAGTA TCAACCCAATCTCTCCAATCAAATGACCT CCAAACAATACACTGGAAATTCCAACATGCCTGGGGGCTC CCAAGGCAAGCTTACACCCAGAAAACAAC ACAGCTGGAGCACAAGTCACAAATGTACCAAGTTGAAATG AATCAAGGGCAGTCCCAAGGTACAGTGGAC CAACATCTCCAGTTCCAAAAACCCTCACACCAGGTGCACTT CTCCAAAACAGACCATTTACCAAAAGCTC ATGTGCAGTCACTGTGTGGCACTAGATTTCATTTTCAACAA AGAGCAGATTCCCAAACTGAAAAACTTAT GTCCCCAGTGTTGAAACAGCACTTGAATCAACAGGCTTCAG AGACTGAGCCATTTTCAAACTCACACCTT TTGCAACATAAGCCTCATAAACAGGCAGCACAAACACAAC CATCCCAGAGTTCACATCTCCCTCAAAACC AGCAACAGCAGCAAAAATTACAAATAAAGAATAAAGAGGA AATACTCCAGACTTTTCCTCACCCCCAAAG CAACAATGATCAGCAAAGAGAAGGATCATTCTTTGGCCAGA CTAAAGTGGAAGAATGTTTTCATGGTGAA AATCAGTATTCAAAATCAAGCGAGTTCGAGACTCATAATGT CCAAATGGGACTGGAGGAAGTACAGAATA TAAATCGTAGAAATTCCCCTTATAGTCAGACCATGAAATCA |
| | | AGTGCATGCAAAATACAGGTTTCTTGTTC AAACAATACACACCTAGTTTCAGAGAATAAAGAACAGACT ACACATCCTGAACTTTTTGCAGGAAACAAG ACCCAAAACTTGCATCACATGCAATATTTTCCAAATAATGT GATCCCAAAGCAAGATCTTCTTCACAGGT GCTTTCAAGAACAGGAGCAGAAGTCACAACAAGCTTCAGTT CTACAGGGATATAAAAATAGAAACCAAGA TATGTCTGGTCAACAAGCTGCGCAACTTGCTCAGCAAAGGT ACTTGATACATAACCATGCAAATGTTTTT CCTGTGCCTGACCAGGGAGGAAGTCACACTCAGACCCCTCC CCAGAAGGACACTCAAAAGCATGCTGCTC TAAGGTGGCATCTCTTACAGAAGCAAGAACAGCAGCAAAC ACAGCAACCCCAAACTGAGTCTTGCCATAG TCAGATGCACAGGCCAATTAAGGTGGAACCTGGATGCAAG CCACATGCCTGTATGCACACAGCACCACCA GAAAACAAAACATGGAAAAAGGTAACTAAGCAAGAGAATC CACCTGCAAGCTGTGATAATGTGCAGCAAA AGAGCATCATTGAGACCATGGAGCAGCATCTGAAGCAGTTT CACGCCAAGTCGTTATTTGACCATAAGGC TCTTACTCTCAAATCACAGAAGCAAGTAAAAGTTGAAATGT CAGGGCCAGTCACAGTTTTGACTAGACAA ACCACTGCTGCAGAACTTGATAGCCACACCCCAGCTTTAGA GCAGCAAACAACTTCTTCAGAAAAGACAC CAACCAAAAGAACAGCTGCTTCTGTTCTCAATAATTTTATA GAGTCACCTTCCAAATTACTAGATACTCC TATAAAAAATTTATTGGATACACCTGTCAAGACTCAATATG ATTTCCCATCTTGCAGATGTGTAGAGCAA ATTATTGAAAAAGATGAAGGTCCTTTTTATACCCATCTAGG AGCAGGTCCTAATGTGGCAGCTATTAGAG AAATCATGGAAGAAAGGTATACAAGTACTTGCCTTTACTCC TGCATGTAGAAGACTCTTATGAGCGAGAT AATGCAGAGAAGGCCTTTCATATAAATTTATACAGCTCTGA GCTGTTCTTCTTCTAGGGTGCCTTTTCAT TAAGAGGTAGGCAGTATTATTATTAAAGTACTTAGGATACA TTGGGGCAGCTAGGACATATTCAGTATCA TTCTTGCTCCATTTCCAAATTATTCATTTCTAAATTAGCATG TAGAAGTTCACTAAATAATCATCTAGTG GCCTGGCAGAAATAGTGAATTTCCCTAAGTGCCTTTTTTTTG TTGTTTTTTTGTTTTGTTTTTTAAACAA GCAGTAGGTGGTGCTTTGGTCATAAGGGAAGATATAGTCTA TTTCTAGGACTATTCCATATTTTCCATGT GGCTGGATACTAACTATTTGCCAGCCTCCTTTTCTAAATTGT GAGACATTCTTGGAGGAACAGTTCTAAC TAAAATCTATTATGACTCCCCAAGTTTTAAAATAGCTAAATT TAGTAAGGGAAAAAATAGTTTATGTTTT AGAAGACTGAACTTAGCAAACTAACCTGAATTTTGTGCTTT GTGAAATTTTATATCGAAATGAGCTTTCC CATTTTCACCCACATGTAATTTACAAAATAGTTCATTACAAT TATCTGTACATTTTGATATTGAGGAAAA ACAAGGCTTAAAAACCATTATCCAGTTTGCTTGGCGTAGAC |
| | | CTGTTTAAAAATAATAAACCGTTCATTT CTCAGGATGTGGTCATAGAATAAAGTTATGCTCAAATGTTC AAA |

"Tet inhibitor" or "Tet[x] inhibitor" (e.g., "Tet1 inhibitor," "Tet2 inhibitor", or "Tet3 inhibitor") as the terms are used herein, refers to a molecule, or group of molecules (e.g., a system) that reduces or eliminates the function and/or expression of the corresponding Tet, e.g., Tet1, Tet2 and/or Tet3, e.g., Tet2. In embodiments, a Tet, e.g., Tet1, Tet2 and/or Tet3, e.g., Tet2 inhibitor is a molecule that inhibits the expression of Tet, e.g., Tet1, Tet2 and/or Tet3, e.g., Tet2, e.g., reduces or eliminates expression of Tet, e.g., Tet1, Tet2 and/or Tet3, e.g., Tet2. In embodiments, the Tet, e.g., Tet1, Tet2 and/or Tet3, e.g., Tet2 inhibitor is a molecule that inhibits the function of Tet, e.g., Tet1, Tet2 and/or Tet3, e.g., Tet2. An example of Tet, e.g., Tet1, Tet2 and/or Tet3, e.g., Tet2 inhibitor that inhibits the expression of Tet, e.g., Tet1, Tet2 and/or Tet3, e.g., Tet2 is a gene editing system, e.g., as described herein, that is targeted to nucleic acid within the Tet, e.g., Tet1, Tet2 and/or Tet3, e.g., Tet2 gene, or its regulatory elements, such that modification of the nucleic acid at or near the gene editing system binding site(s) is modified to reduce or eliminate expression of Tet, e.g., Tet1, Tet2 and/or Tet3, e.g., Tet2. Another example of a Tet, e.g., Tet1, Tet2 and/or Tet3, e.g., Tet2 inhibitor that inhibits the expression of Tet, e.g., Tet1, Tet2 and/or Tet3, e.g., Tet2 is a nucleic acid molecule, e.g., RNA molecule, e.g., a short hairpin RNA (shRNA) or short interfering RNA (siRNA), capable of hybridizing with Tet, e.g., Tet1, Tet2 and/or Tet3, e.g., Tet2 mRNA and causing a reduction or elimination of Tet, e.g., Tet1, Tet2 and/or Tet3, e.g., Tet2 translation. Tet, e.g., Tet1, Tet2 and/or Tet3, e.g., Tet2 inhibitors also include nucleic acids encoding molecules which inhibit Tet, e.g., Tet1, Tet2 and/or Tet3, e.g., Tet2 expression (e.g., nucleic acid encoding an anti-Tet, e.g., Tet1, Tet2 and/or Tet3, e.g., Tet2 shRNA or siRNA, or nucleic acid encoding one or more, e.g., all, components of an anti-Tet, e.g., Tet1, Tet2 and/or Tet3, e.g., Tet2 gene editing system). An example of a molecule that inhibits the function of Tet, e.g., Tet1, Tet2 and/or Tet3, e.g., Tet2 is a molecule, e.g., a protein or small molecule which inhibits one or more activities of Tet, e.g., Tet1, Tet2 and/or Tet3, e.g., Tet2. An example is a small molecule inhibitor of Tet, e.g., Tet1, Tet2 and/or Tet3, e.g., Tet2. Another example is a dominant negative Tet, e.g., Tet1, Tet2 and/or Tet3, e.g., Tet2 protein. Another example is a dominant negative version of a Tet, e.g., Tet1, Tet2 and/or Tet3, e.g., Tet2 binding partner, e.g., an associated histone deacetylase (HDAC). Another example is a molecule, e.g., a small molecule, which inhibits a Tet, e.g., Tet1, Tet2 and/or Tet3, e.g., Tet2 binding partner, e.g., a Tet, e.g., Tet1, Tet2 and/or Tet3, e.g., Tet2-associated HDAC inhibitor. Tet, e.g., Tet1, Tet2 and/or Tet3, e.g., Tet2 inhibitors also include nucleic acids encoding inhibitors of Tet, e.g., Tet1, Tet2 and/or Tet3, e.g., Tet2 function.

A "system" as the term is used herein in connection with gene editing or Tet, e.g., Tet1, Tet2 and/or Tet3, e.g., Tet2 inhibition, refers to a group of molecules, e.g., one or more molecules, which together act to effect a desired function.

A "gene editing system" as the term is used herein, refers to a system, e.g., one or more molecules, that direct and effect an alteration, e.g., a deletion, of one or more nucleic acids at or near a site of genomic DNA targeted by said system. Gene editing systems are known in the art, and are described more fully below.

"binding partner" as the term is used herein in the context of a Tet, e.g., Tet1, Tet2 and/or Tet3, e.g., Tet2 binding partner, refers to a molecule, e.g., a protein, which interacts, e.g., binds to, Tet, e.g., Tet1, Tet2 and/or Tet3, e.g., Tet2 protein. Without being bound by theory, it is believed that Tet, e.g., Tet1, Tet2 and/or Tet3, e.g., Tet2 binds to one or more HDAC proteins. Such HDAC proteins are considered examples of Tet, e.g., Tet1, Tet2 and/or Tet3, e.g., Tet2 binding partners.

A "dominant negative" gene product or protein is one that interferes with the function of another gene product or protein. The other gene product affected can be the same or different from the dominant negative protein. Dominant negative gene products can be of many forms, including truncations, full length proteins with point mutations or fragments thereof, or fusions of full length wild type or mutant proteins or fragments thereof with other proteins. The level of inhibition observed can be very low. For example, it may require a large excess of the dominant negative protein compared to the functional protein or proteins involved in a process in order to see an effect. It may be difficult to see effects under normal biological assay conditions. In one embodiment, a dominant negative Tet, e.g., Tet1, Tet2 and/or Tet3, e.g., Tet2 is a catalytically inactive Tet, e.g., Tet1, Tet2 and/or Tet3, e.g., Tet2. In another embodiment, a dominant negative Tet, e.g., Tet1, Tet2 and/or Tet3, e.g., Tet2 binding partner is a catalytically inactive Tet, e.g., Tet1, Tet2 and/or Tet3, e.g., Tet2-binding HDAC inhibitor.

### Description

The present invention provides Tet, e.g., Tet1, Tet2 and/or Tet3, e.g., Tet2, inhibitors and methods of use therefore. In particular, the invention provides CAR-expressing T cells comprising Tet, e.g., Tet1, Tet2 and/or Tet3, e.g., Tet2, inhibitors, and use of Tet, e.g., Tet1, Tet2 and/or Tet3, e.g., Tet2, in connection with CAR T cells. Tet, e.g., Tet1, Tet2 and/or Tet3, e.g., Tet2, inhibitor of the present invention, together with their methods of use, are described in more detail below. CARs, CAR T cells, and methods of use are further described below.

### Tet, e.g., Tet1, Tet2 and/or Tet3, e.g., Tet2 Inhibitors

The present invention provides compositions, e.g., Tet, e.g., Tet1, Tet2 and/or Tet3, e.g., Tet2 inhibitors, and methods for enhancing immune effector cell functions, e.g., CAR-expressing cell functions, by using such compositions and/or other means as described herein. Any Tet, e.g., Tet1, Tet2 and/or Tet3, e.g., Tet2, inhibitors known in the art can be used as a Tet, e.g., Tet1, Tet2 and/or Tet3, e.g., Tet2, inhibitor according to the present invention. Examples of Tet, e.g., Tet1, Tet2 and/or Tet3, e.g., Tet2, inhibitors are described below.

### Gene Editing Systems

According to the present invention, gene editing systems can be used as Tet, e.g., Tet1, Tet2 and/or Tet3, e.g., Tet2, inhibitors. Also contemplated by the present invention are the uses of nucleic acid encoding one or more components of a Tet, e.g., Tet1, Tet2 and/or Tet3, e.g., Tet2, gene editing system.

### CRISPR/Cas9 Gene Editing Systems

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845.

The CRISPR/Cas system has been modified for use in gene editing (silencing, enhancing or changing specific genes) in eukaryotes such as mice or primates. Wiedenheft et al. (2012) Nature 482: 331-8. This is accomplished by, for example, introducing into the eukaryotic cell a plasmid containing a specifically designed CRISPR and one or more appropriate Cas.

The CRISPR sequence, sometimes called a CRISPR locus, comprises alternating repeats and spacers. In a naturally-occurring CRISPR, the spacers usually comprise sequences foreign to the bacterium such as a plasmid or phage sequence; in an exemplary Tet, e.g., Tet1, Tet2 and/or Tet3, e.g., Tet2, CRISPR/Cas system, the spacers are derived from the Tet, e.g., Tet1, Tet2 and/or Tet3, e.g., Tet2, gene sequence, or a sequence of its regulatory elements.

RNA from the CRISPR locus is constitutively expressed and processed into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836.

As these naturally occur in many different types of bacteria, the exact arrangements of the CRISPR and structure, function and number of Cas genes and their product differ somewhat from species to species. Haft et al. (2005) PLoS Comput. Biol. 1: e60; Kunin et al. (2007) Genome Biol. 8: R61; Mojica et al. (2005) J. Mol. Evol. 60: 174-182; Bolotin et al. (2005) Microbiol. 151: 2551-2561; Pourcel et al. (2005) Microbiol. 151: 653-663; and Stern et al. (2010) Trends. Genet. 28: 335-340. For example, the Cse (Cas subtype, *E. coli*) proteins (e.g., CasA) form a functional complex, Cascade, that processes CRISPR RNA transcripts into spacer-repeat units that Cascade retains. Brouns et al. (2008) Science 321: 960-964. In other prokaryotes, Cas6 processes the CRISPR transcript. The CRISPR-based phage inactivation in E. coli requires Cascade and Cas3, but not Cas1 or Cas2. The Cmr (Cas RAMP module) proteins in Pyrococcus furiosus and other prokaryotes form a functional complex with small CRISPR RNAs that recognizes and cleaves complementary target RNAs. A simpler CRISPR system relies on the protein Cas9, which is a nuclease with two active cutting sites, one for each strand of the double helix. Combining Cas9 and modified CRISPR locus RNA can be used in a system for gene editing. Pennisi (2013) Science 341: 833-836.

The CRISPR/Cas system can thus be used to modify, e.g., delete one or more nucleic acids, the Tet, e.g., Tet1, Tet2 and/or Tet3, e.g., Tet2, gene, or a Tet, e.g., Tet1, Tet2 and/or Tet3, e.g., Tet2, gene regulatory element, or introduce a premature stop which thus decreases expression of a functional Tet, e.g., Tet1, Tet2 and/or Tet3, e.g., Tet2,. The CRISPR/Cas system can alternatively be used like RNA interference, turning off the Tet, e.g., Tet1, Tet2 and/or Tet3, e.g., Tet2, gene in a reversible fashion. In a mammalian cell, for example, the RNA can guide the Cas protein to a Tet, e.g., Tet1, Tet2 and/or Tet3, e.g., Tet2, promoter, sterically blocking RNA polymerases.

CRISPR/Cas systems for gene editing in eukaryotic cells typically involve (1) a guide RNA molecule (gRNA) comprising a targeting sequence (which is capable of hybridizing to the genomic DNA target sequence), and sequence which is capable of binding to a Cas, e.g., Cas9 enzyme, and (2) a Cas, e.g., Cas9, protein. The targeting sequence and the sequence which is capable of binding to a Cas, e.g., Cas9 enzyme, may be disposed on the same or different molecules. If disposed on different molecules, each includes a hybridization domain which allows the molecules to associate, e.g., through hybridization.

An exemplary gRNA molecule of the present invention comprises, e.g., consists of a first nucleic acid having the sequence (where the "n'" s refer to the residues of the targeting sequence (e.g., as described herein, e.g., in Table 3), and may consist of 15-25 nucelotides, e.g., consist of 20 nucleotides):
nnnnnnnnnnnnnnnnnnnnGUUUUAGAGCUAUGCUGUUUUG (SEQ ID NO: 40);
and a second nucleic acid sequence having the sequence:
   AACUUACCAAGGAACAGCAUAGCAAGUUAAAAUAAGGCUAGUCCGUUAUC AACUUGAAAAAGUGGCACCGAGUCGGUGC, optionally with 1, 2, 3, 4, 5, 6, or 7 (e.g., 4 or 7, e.g., 7) additional U nucleotides at the 3' end (SEQ ID NO: 41).

The second nucleic acid molecule may alternatively consist of a fragment of the sequence above, wherein such fragment is capable of hybridizing to the first nucleic acid. An example of such second nucleic acid molecule is:
AACAGCAUAGCAAGUUAAAAUAAGGCUAGUCCGUUAUCAACUUGAAAAAG UGGCACCGAGUCGGUGC, optionally with 1, 2, 3, 4, 5, 6, or 7 (e.g., 4 or 7, e.g., 7) additional U nucleotides at the 3' end (SEQ ID NO: 42).

Another exemplary gRNA molecule of the present invention comprises, e.g., consists of a first nucleic acid having the sequence (where the "n"'s refer to the residues of the targeting sequence (e.g., as described herein, e.g., in Table 3), and may consist of 15-25 nucelotides, e.g., consist of 20 nucleotides): 
nnnnnnnnnnnnnnnnnnnGUUUUAGAGCUAGAAAUAGCAAGUUAAAAUAAGGCU
AGUCCGUUAUCAACUUGAAAAAGUGGCACCGAGUCGGUGC (SEQ ID NO: 43),
 optionally with 1, 2, 3, 4, 5, 6, or 7 (e.g., 4 or 7, e.g., 4) additional U nucleotides at the 3' end.Artificial CRISPR/Cas systems can be generated which inhibit Tet, e.g., Tet1, Tet2 and/or Tet3, e.g., Tet2, using technology known in the art, e.g., that are described in U.S. Publication No.20140068797, WO2015/048577, and Cong (2013) Science 339: 819-823. Other artificial CRISPR/Cas systems that are known in the art may also be generated which inhibit Tet, e.g., Tet1, Tet2 and/or Tet3, e.g., Tet2,, e.g., that described in Tsai (2014) Nature Biotechnol., 32:6 569-576, U.S. Patent No.: 8,871,445; 8,865,406; 8,795,965; 8,771,945; and 8,697,359, the contents of which are hereby incorporated by reference in their entirety. Such systems can be generated which inhibit Tet, e.g., Tet1, Tet2 and/or Tet3, e.g., Tet2, by, for example, engineering a CRISPR/Cas system to include a gRNA molecule comprising a targeting sequence that hybridizes to a sequence of a tet gene, e.g., a Tet1, Tet2 and/or Tet3, e.g., Tet2 gene. In embodiments, the gRNA comprises a targeting sequence which is fully complementarity to 15-25 nucleotides, e.g., 20 nucleotides, of a tet gene, e.g., a Tet1, Tet2 and/or Tet3, e.g., Tet2 gene. In embodiments, the 15-25 nucleotides, e.g., 20 nucleotides, of a tet gene, e.g., a Tet1, Tet2 and/or Tet3, e.g., Tet2 gene, are disposed immediately 5' to a protospacer adjacent motif (PAM) sequence recognized by the Cas protein of the CRISPR/Cas system (e.g., where the system comprises a *S*. *pyogenes* Cas9 protein, the PAM sequence comprises NGG, where N can be any of A, T, G or C). In embodiments, the targeting sequence of the gRNA comprises, e.g., consists of, a RNA sequence complementary to a sequence listed in Table 2. In embodiments, the gRNA comprises a targeting sequence listed in Table 3.

In one embodiment, foreign DNA can be introduced into the cell along with the CRISPR/Cas system, e.g., DNA encoding a CAR, e.g., as described herein; depending on the sequences of the foreign DNA and chromosomal sequence, this process can be used to integrate the DNA encoding the CAR, e.g., as described herein, at or near the site targeted by the CRISPR/Cas system. As shown herein, in the examples, but without being bound by theory, such integration may lead to the expression of the CAR as well as disruption of the Tet, e.g., Tet1, Tet2 and/or Tet3, e.g., Tet2, gene. Such foreign DNA molecule is referred to herein as "template DNA." In embodiments, the template DNA further comprises homology arms 5' to, 3' to, or both 5' and 3' to the nucleic acid of the template DNA which encodes the molecule or molecules of interest (e.g., which encodes a CAR described herein), wherein said homology arms are complementary to genomic DNA sequence flanking the target sequence.

In an embodiment, the CRISPR/Cas system of the present invention comprises Cas9, e.g., S. pyogenes Cas9, and a gRNA comprising a targeting sequence which hybridizes to a sequence of the Tet, e.g., Tet1, Tet2 and/or Tet3, e.g., Tet2, gene. In an embodiment, the CRISPR/Cas system comprises nucleic acid encoding a Tet, e.g., Tet1, Tet2 and/or Tet3, e.g., Tet2, gRNA and nucleic acid encoding a Cas protein, e.g., Cas9, e.g., S. pyogenes Cas9. In an embodiment, the CRISPR/Cas system comprises a Tet, e.g., Tet1, Tet2 and/or Tet3, e.g., Tet2, gRNA and nucleic acid encoding a Cas protein, e.g., Cas9, e.g., S. pyogenes Cas9.

Examples of genomic target sequences for Tet2, for which gRNAs comprising complementary targeting sequences can be generated for use in the present invention are listed in the table 2 below. In embodiments, the gRNA comprises an RNA complement of a Target Sequence of the table below (e.g., for sgTET2_1, the gRNA would comprise CCUUGGACACCUUCUCCUCC (SEQ ID NO: 44)). In embodiments, the gRNA comprises the RNA analog of a Target sequence of the table 2 below (e.g., for sgTET2_1, the gRNA would comprise GGAACCUGUGGAAGAGGAGG (SEQ ID NO: 45). In embodiments, the Tet2 inhibitor is nucleic acid encoding a gRNA molecule specific for Tet2, wherein the nucleic acid comprises the sequence of a Target Sequence from the 2 table below, e.g., under the control of a U6- or H1- promoter:

**Table 2**

| **gRNA ID** | **Gene Symbol** | **Chromosome** | **Position** | **Strand** | **Target Sequence within the Tet2 gene sequence** |
|---|---|---|---|---|---|
| sgTET2_1 | TET2 | chr4 | 106156327 | - | GGAACCTGTGGAAGAGGAGG (SEQ ID NO: 46) |
| sgTET2_2 | TET2 | chr4 | 106156339 | - | GAAGGAAGCTGAGGAACCTG (SEQ ID NO: 47) |
| sgTET2_3 | TET2 | chr4 | 106156897 | + | ATGACCTCCAAACAATACAC (SEQ ID NO: 48) |
| sgTET2_4 | TET2 | chr4 | 106157189 | - | CAAGTGCTGTTTCAACACTG (SEQ ID NO: 49) |
| sgTET2_5 | TET2 | chr4 | 106157296 | - | GGGAGATGTGAACTCTGGGA (SEQ ID NO: 50) |
| sgTET2_6 | TET2 | chr4 | 106155148 | - | GGAGGTGATGGTATCAGGAA (SEQ ID NO: 51) |
| sgTET2_7 | TET2 | chr4 | 106155166 | - | GGTTCTGTCTGGCAAATGGG (SEQ ID NO: 52) |
| sgTET2_8 | TET2 | chr4 | 106155217 | - | GGATGAGCTCTCTCAGGCAG (SEQ ID NO: 53) |
| sgTET2_9 | TET2 | chr4 | 106155403 | - | TGAAGGAGCCCAGAGAGAGA (SEQ ID NO: 65) |
| sgTET2_10 | TET2 | chr4 | 106155478 | + | GTAAGCCAAGAAAGAAATCC (SEQ ID NO: 66) |

Examples of gRNA targeting sequences which are useful in the various embodiments of the present invention to inhibit a Tet, e.g., Tet2, are provided below in Table 3. In embodiments a CRISPR/Cas system of the present invention comprises a gRNA molecule comprising a targeting sequence comprising a sequence listed in Table 3. In embodiments, a CRISPR/Cas system of the present invention comprises a gRNA molecule comprising a targeting sequence that is a sequence listed in Table 3.

**Table 3**

| ID | TARGET | TARGET REGION | STRAND | Location of Genomic Target Sequence (hg38) | gRNA Targeting sequence | SEQ ID NO: |
|---|---|---|---|---|---|---|
| 54790_1_1 | TET2 | EXON | + | chr4:105145928 -105145948 | UGUCGGGUCUUUAAAAAUAC | 73 |
| 54790_1_3 | TET2 | EXON | + | chr4:105145945 -105145965 | UACAGGCCCCUAAAGCACUA | 74 |
| 54790_1_4 | TET2 | EXON | + | chr4:105145946 -105145966 | ACAGGCCCCUAAAGCACUAA | 75 |
| 54790_1_5 | TET2 | EXON | + | chr4:105145957 -105145977 | AAGCACUAAGGGCAUGCCCU | 76 |
| 54790_1_8 | TET2 | EXON | + | chr4:105145966 -105145986 | GGGCAUGCCCUCGGUGAAAC | 77 |
| 54790_1_10 | TET2 | EXON | + | chr4:105145967 -105145987 | GGCAUGCCCUCGGUGAAACA | 78 |
| 54790_1_12 | TET2 | EXON | + | chr4:105145968 -105145988 | GCAUGCCCUCGGUGAAACAG | 79 |
| 54790_1_20 | TET2 | EXON | + | chr4:105146006 -105146026 | UGAGAUUAAAGCGACAGAAA | 80 |
| 54790_1_23 | TET2 | EXON | + | chr4:105146007 -105146027 | GAGAUUAAAGCGACAGAAAA | 81 |
| 54790_1_25 | TET2 | EXON | + | chr4:105146012 -105146032 | UAAAGCGACAGAAAAGGGAA | 82 |
| 54790_1_30 | TET2 | EXON | + | chr4:105146021 -105146041 | AGAAAAGGGAAAGGAGAGCG | 83 |
| 54790_1_31 | TET2 | EXON | + | chr4:105146022 -105146042 | GAAAAGGGAAAGGAGAGCGC | 84 |
| 54790_1_33 | TET2 | EXON | + | chr4:105146028 -105146048 | GGAAAGGAGAGCGCGGGCAA | 85 |
| 54790_1_35 | TET2 | EXON | + | chr4:105146029 -105146049 | GAAAGGAGAGCGCGGGCAAC | 86 |
| 54790_1_38 | TET2 | EXON | + | chr4:105146038 -105146058 | GCGCGGGCAACGGGAUCUAA | 87 |
| 54790_1_39 | TET2 | EXON | + | chr4:105146039 -105146059 | CGCGGGCAACGGGAUCUAAA | 88 |
| 54790_1_43 | TET2 | EXON | + | chr4:105146053 -105146073 | UCUAAAGGGAGAUAGAGACG | 89 |
| 54790_1_44 | TET2 | EXON | + | chr4:105146054 -105146074 | CUAAAGGGAGAUAGAGACGC | 90 |
| 54790_1_47 | TET2 | EXON | + | chr4:105146063 -105146083 | GAUAGAGACGCGGGCCUCUG | 91 |
| 54790_1_48 | TET2 | EXON | + | chr4:105146064 -105146084 | AUAGAGACGCGGGCCUCUGA | 92 |
| 54790_1_49 | TET2 | EXON | + | chr4:105146069 -105146089 | GACGCGGGCCUCUGAGGGUA | 93 |
| 54790_1_51 | TET2 | EXON | + | chr4:105146072 -105146092 | GCGGGCCUCUGAGGGUAAGG | 94 |
| 54790_1_52 | TET2 | EXON | + | chr4:105146073 -105146093 | CGGGCCUCUGAGGGUAAGGU | 95 |
| 54790_1_54 | TET2 | EXON | + | chr4:105146082 -105146102 | GAGGGUAAGGUGGGCGCAAG | 96 |
| 54790_1_61 | TET2 | EXON | - | chr4:105145954 -105145974 | GCAUGCCCUUAGUGCUUUAG | 97 |
| 54790_1_62 | TET2 | EXON | - | chr4:105145955 -105145975 | GGCAUGCCCUUAGUGCUUUA | 98 |
| 54790_1_64 | TET2 | EXON | - | chr4:105145956 -105145976 | GGGCAUGCCCUUAGUGCUUU | 99 |
| 54790_1_68 | TET2 | EXON | - | chr4:105145976 -105145996 | GCGCUCCCCUGUUUCACCGA | 100 |
| 54790_1_69 | TET2 | EXON | - | chr4:105145977 -105145997 | AGCGCUCCCCUGUUUCACCG | 101 |
| 54790_1_87 | TET2 | EXON | - | chr4:105146080 -105146100 | UGCGCCCACCUUACCCUCAG | 102 |
| 54790_2_1 | TET2 | EXON | + | chr4:105146669 -105146689 | AGAGCCGGCGGUAGCGGCAG | 103 |
| 54790_2_2 | TET2 | EXON | + | chr4:105146675 -105146695 | GGCGGUAGCGGCAGUGGCAG | 104 |
| 54790_2_6 | TET2 | EXON | + | chr4:105146686 -105146706 | CAGUGGCAGCGGCGAGAGCU | 105 |
| 54790_2_7 | TET2 | EXON | + | chr4:105146687 -105146707 | AGUGGCAGCGGCGAGAGCUU | 106 |
| 54790_2_8 | TET2 | EXON | + | chr4:105146690 -105146710 | GGCAGCGGCGAGAGCUUGGG | 107 |
| 54790_2_12 | TET2 | EXON | + | chr4:105146725 -105146745 | CCUCGCGAGCGCCGCGCGCC | 108 |
| 54790_2_13 | TET2 | EXON | + | chr4:105146726 -105146746 | CUCGCGAGCGCCGCGCGCCC | 109 |
| 54790_2_14 | TET2 | EXON | + | chr4:105146761 -105146781 | GCAAGUCACGUCCGCCCCCU | 110 |
| 54790_2_15 | TET2 | EXON | + | chr4:105146766 -105146786 | UCACGUCCGCCCCCUCGGCG | 111 |
| 54790_2_17 | TET2 | EXON | + | chr4:105146783 -105146803 | GCGCGGCCGCCCCGAGACGC | 112 |
| 54790_2_24 | TET2 | EXON | + | chr4:105146836 -105146856 | CUGCCUUAUGAAUAUUGAUG | 113 |
| 54790_2_25 | TET2 | EXON | + | chr4:105146839 -105146859 | CCUUAUGAAUAUUGAUGCGG | 114 |
| 54790_2_27 | TET2 | EXON | + | chr4:105146844 -105146864 | UGAAUAUUGAUGCGGAGGCU | 115 |
| 54790_2_34 | TET2 | EXON | + | chr4:105146868 -105146888 | UGCUUUCGUAGAGAAGCAGA | 116 |
| 54790_2_37 | TET2 | EXON | + | chr4:105146879 -105146899 | AGAAGCAGAAGGAAGCAAGA | 117 |
| 54790_2_39 | TET2 | EXON | + | chr4:105146891 -105146911 | AAGCAAGAUGGCUGCCCUUU | 118 |
| 54790_2_44 | TET2 | EXON | + | chr4:105146905 -105146925 | CCCUUUAGGAUUUGUUAGAA | 119 |
| 54790_2_51 | TET2 | EXON | + | chr4:105146926 -105146946 | GGAGACCCGACUGCAACUGC | 120 |
| 54790_2_52 | TET2 | EXON | + | chr4:105146938 -105146958 | GCAACUGCUGGAUUGCUGCA | 121 |
| 54790_2_56 | TET2 | EXON | + | chr4:105146944 -105146964 | GCUGGAUUGCUGCAAGGCUG | 122 |
| 54790_2_57 | TET2 | EXON | + | chr4:105146945 -105146965 | CUGGAUUGCUGCAAGGCUGA | 123 |
| 54790_2_62 | TET2 | EXON | + | chr4:105146957 -105146977 | AAGGCUGAGGGACGAGAACG | 124 |
| 54790_2_64 | TET2 | EXON | - | chr4:105146676 -105146696 | GCUGCCACUGCCGCUACCGC | 125 |
| 54790_2_65 | TET2 | EXON | - | chr4:105146716 -105146736 | CGCUCGCGAGGAGGCGGCGG | 126 |
| 54790_2_66 | TET2 | EXON | - | chr4:105146719 -105146739 | CGGCGCUCGCGAGGAGGCGG | 127 |
| 54790_2_67 | TET2 | EXON | - | chr4:105146722 -105146742 | GCGCGGCGCUCGCGAGGAGG | 128 |
| 54790_2_68 | TET2 | EXON | - | chr4:105146725 -105146745 | GGCGCGCGGCGCUCGCGAGG | 129 |
| 54790_2_69 | TET2 | EXON | - | chr4:105146728 -105146748 | CCGGGCGCGCGGCGCUCGCG | 130 |
| 54790_2_74 | TET2 | EXON | - | chr4:105146739 -105146759 | GCGAGCGGGACCCGGGCGCG | 131 |
| 54790_2_75 | TET2 | EXON | - | chr4:105146746 -105146766 | CUUGCAUGCGAGCGGGACCC | 132 |
| 54790_2_76 | TET2 | EXON | - | chr4:105146747 -105146767 | ACUUGCAUGCGAGCGGGACC | 133 |
| 54790_2_78 | TET2 | EXON | - | chr4:105146753 -105146773 | GACGUGACUUGCAUGCGAGC | 134 |
| 54790_2_79 | TET2 | EXON | - | chr4:105146754 -105146774 | GGACGUGACUUGCAUGCGAG | 135 |
| 54790_2_83 | TET2 | EXON | - | chr4:105146775 -105146795 | GGGCGGCCGCGCCGAGGGGG | 136 |
| 54790_2_85 | TET2 | EXON | - | chr4:105146778 -105146798 | UCGGGGCGGCCGCGCCGAGG | 137 |
| 54790_2_86 | TET2 | EXON | - | chr4:105146779 -105146799 | CUCGGGGCGGCCGCGCCGAG | 138 |
| 54790_2_88 | TET2 | EXON | - | chr4:105146780 -105146800 | UCUCGGGGCGGCCGCGCCGA | 139 |
| 54790_2_89 | TET2 | EXON | - | chr4:105146781 -105146801 | GUCUCGGGGCGGCCGCGCCG | 140 |
| 54790_2_93 | TET2 | EXON | - | chr4:105146792 -105146812 | GCGGGGCCGGCGUCUCGGGG | 141 |
| 54790_2_94 | TET2 | EXON | - | chr4:105146795 -105146815 | UCAGCGGGGCCGGCGUCUCG | 142 |
| 54790_2_95 | TET2 | EXON | - | chr4:105146796 -105146816 | CUCAGCGGGGCCGGCGUCUC | 143 |
| 54790_2_97 | TET2 | EXON | - | chr4:105146797 -105146817 | ACUCAGCGGGGCCGGCGUCU | 144 |
| 54790_2_100 | TET2 | EXON | - | chr4:105146805 -105146825 | UUCUCAUCACUCAGCGGGGC | 145 |
| 54790_2_101 | TET2 | EXON | - | chr4:105146809 -105146829 | UCUGUUCUCAUCACUCAGCG | 146 |
| 54790_2_103 | TET2 | EXON | - | chr4:105146810 -105146830 | GUCUGUUCUCAUCACUCAGC | 147 |
| 54790_2_106 | TET2 | EXON | - | chr4:105146811 -105146831 | CGUCUGUUCUCAUCACUCAG | 148 |
| 54790_2_109 | TET2 | EXON | - | chr4:105146842 -105146862 | CCUCCGCAUCAAUAUUCAUA | 149 |
| 54790_2_117 | TET2 | EXON | - | chr4:105146908 -105146928 | CCUUUCUAACAAAUCCUAAA | 150 |
| 54790_2_118 | TET2 | EXON | - | chr4:105146909 -105146929 | UCCUUUCUAACAAAUCCUAA | 151 |
| 54790_2_122 | TET2 | EXON | - | chr4:105146934 -105146954 | GCAAUCCAGCAGUUGCAGUC | 152 |
| 54790_2_123 | TET2 | EXON | - | chr4:105146935 -105146955 | AGCAAUCCAGCAGUUGCAGU | 153 |
| 54790_3_1 | TET2 | EXON | + | chr4:105190341 -105190361 | AAACUCUGUCUUCUCUAGGC | 154 |
| 54790_3_13 | TET2 | EXON | + | chr4:105190411 -105190431 | UCCUGUUGAGUUACAACGCU | 155 |
| 54790_3_16 | TET2 | EXON | + | chr4:105190418 -105190438 | GAGUUACAACGCUUGGAAGC | 156 |
| 54790_3_19 | TET2 | EXON | + | chr4:105190424 -105190444 | CAACGCUUGGAAGCAGGAGA | 157 |
| 54790_3_21 | TET2 | EXON | + | chr4:105190425 -105190445 | AACGCUUGGAAGCAGGAGAU | 158 |
| 54790_3_24 | TET2 | EXON | + | chr4:105190444 -105190464 | UGGGCUCAGCAGCAGCCAAU | 159 |
| 54790_3_26 | TET2 | EXON | + | chr4:105190456 -105190476 | CAGCCAAUAGGACAUGAUCC | 160 |
| 54790_3_30 | TET2 | EXON | + | chr4:105190469 -105190489 | AUGAUCCAGGAAGAGCAGUA | 161 |
| 54790_3_32 | TET2 | EXON | + | chr4:105190470 -105190490 | UGAUCCAGGAAGAGCAGUAA | 162 |
| 54790_3_34 | TET2 | EXON | + | chr4:105190483 -105190503 | GCAGUAAGGGACUGAGCUGC | 163 |
| 54790_3_37 | TET2 | EXON | + | chr4:105190494 -105190514 | CUGAGCUGCUGGUAAGACAG | 164 |
| 54790_3_46 | TET2 | EXON | - | chr4:105190385 -105190405 | GCAAGUAAACAAUCUUGAGA | 165 |
| 54790_3_47 | TET2 | EXON | - | chr4:105190386 -105190406 | GGCAAGUAAACAAUCUUGAG | 166 |
| 54790_3_52 | TET2 | EXON | - | chr4:105190407 -105190427 | UUGUAACUCAACAGGAGCAA | 167 |
| 54790_3_55 | TET2 | EXON | - | chr4:105190415 -105190435 | UCCAAGCGUUGUAACUCAAC | 168 |
| 54790_3_60 | TET2 | EXON | - | chr4:105190462 -105190482 | CUUCCUGGAUCAUGUCCUAU | 169 |
| 54790_3_62 | TET2 | EXON | - | chr4:105190477 -105190497 | CAGUCCCUUACUGCUCUUCC | 170 |
| 54790_4_7 | TET2 | EXON | + | chr4:105233887 -105233907 | GCUCUUUAGAAUUCAACUAG | 171 |
| 54790_4_8 | TET2 | EXON | + | chr4:105233888 -105233908 | CUCUUUAGAAUUCAACUAGA | 172 |
| 54790_4_12 | TET2 | EXON | + | chr4:105233899 -105233919 | UCAACUAGAGGGCAGCCUUG | 173 |
| 54790_4_14 | TET2 | EXON | + | chr4:105233903 -105233923 | CUAGAGGGCAGCCUUGUGGA | 174 |
| 54790_4_19 | TET2 | EXON | + | chr4:105233923 -105233943 | UGGCCCCGAAGCAAGCCUGA | 175 |
| 54790_4_21 | TET2 | EXON | + | chr4:105233929 -105233949 | CGAAGCAAGCCUGAUGGAAC | 176 |
| 54790_4_25 | TET2 | EXON | + | chr4:105233950 -105233970 | GGAUAGAACCAACCAUGUUG | 177 |
| 54790_4_26 | TET2 | EXON | + | chr4:105233951 -105233971 | GAUAGAACCAACCAUGUUGA | 178 |
| 54790_4_30 | TET2 | EXON | + | chr4:105234010 -105234030 | CAUUUGCCAGACAGAACCUC | 179 |
| 54790_4_37 | TET2 | EXON | + | chr4:105234029 -105234049 | CUGGCUACAAAGCUCCAGAA | 180 |
| 54790_4_44 | TET2 | EXON | + | chr4:105234068 -105234088 | AGAGCUCAUCCAGAAGUAAA | 181 |
| 54790_4_45 | TET2 | EXON | + | chr4:105234081 -105234101 | AAGUAAAUGGAGACACCAAG | 182 |
| 54790_4_47 | TET2 | EXON | + | chr4:105234104 -105234124 | CACUCUUUCAAAAGUUAUUA | 183 |
| 54790_4_54 | TET2 | EXON | + | chr4:105234121 -105234141 | UUAUGGAAUACCCUGUAUGA | 184 |
| 54790_4_57 | TET2 | EXON | + | chr4:105234122 -105234142 | UAUGGAAUACCCUGUAUGAA | 185 |
| 54790_4_66 | TET2 | EXON | + | chr4:105234170 -105234190 | GACUUUACACAAGAAAGUAG | 186 |
| 54790_4_67 | TET2 | EXON | + | chr4:105234171 -105234191 | ACUUUACACAAGAAAGUAGA | 187 |
| 54790_4_72 | TET2 | EXON | + | chr4:105234194 -105234214 | UAUUCCAAGUGUUUGCAAAA | 188 |
| 54790_4_74 | TET2 | EXON | + | chr4:105234197 -105234217 | UCCAAGUGUUUGCAAAAUGG | 189 |
| 54790_4_81 | TET2 | EXON | + | chr4:105234233 -105234253 | GUUAGUGAACCUUCUCUCUC | 190 |
| 54790_4_82 | TET2 | EXON | + | chr4:105234234 -105234254 | UUAGUGAACCUUCUCUCUCU | 191 |
| 54790_4_89 | TET2 | EXON | + | chr4:105234271 -105234291 | GAAAUUGAAACAAGACCAAA | 192 |
| 54790_4_93 | TET2 | EXON | + | chr4:105234278 -105234298 | AAACAAGACCAAAAGGCUAA | 193 |
| 54790_4_97 | TET2 | EXON | + | chr4:105234296 -105234316 | AAUGGAGAAAGACGUAACUU | 194 |
| 54790_4_99 | TET2 | EXON | + | chr4:105234297 -105234317 | AUGGAGAAAGACGUAACUUC | 195 |
| 54790_4_100 | TET2 | EXON | + | chr4:105234298 -105234318 | UGGAGAAAGACGUAACUUCG | 196 |
| 54790_4_106 | TET2 | EXON | + | chr4:105234320 -105234340 | GUAAGCCAAGAAAGAAAUCC | 197 |
| 54790_4_123 | TET2 | EXON | + | chr4:105234437 -105234457 | UUUUCAACACAUAACUGCAG | 198 |
| 54790_4_124 | TET2 | EXON | + | chr4:105234438 -105234458 | UUUCAACACAUAACUGCAGU | 199 |
| 54790_4_134 | TET2 | EXON | + | chr4:105234475 -105234495 | GCUUCAGAUUCUGAAUGAGC | 200 |
| 54790_4_138 | TET2 | EXON | + | chr4:105234478 -105234498 | UCAGAUUCUGAAUGAGCAGG | 201 |
| 54790_4_140 | TET2 | EXON | + | chr4:105234479 -105234499 | CAGAUUCUGAAUGAGCAGGA | 202 |
| 54790_4_141 | TET2 | EXON | + | chr4:105234480 -105234500 | AGAUUCUGAAUGAGCAGGAG | 203 |
| 54790_4_147 | TET2 | EXON | + | chr4:105234529 -105234549 | CAUUGUAUUACUUAAAAACA | 204 |
| 54790_4_151 | TET2 | EXON | + | chr4:105234548 -105234568 | AAGGCAGUGCUAAUGCCUAA | 205 |
| 54790_4_153 | TET2 | EXON | + | chr4:105234574 -105234594 | UACAGUUUCUGCCUCUUCCG | 206 |
| 54790_4_157 | TET2 | EXON | + | chr4:105234587 -105234607 | UCUUCCGUGGAACACACACA | 207 |
| 54790_4_161 | TET2 | EXON | + | chr4:105234598 -105234618 | ACACACACAUGGUGAACUCC | 208 |
| 54790_4_163 | TET2 | EXON | + | chr4:105234643 -105234663 | UCCAGAUUGUGUUUCCAUUG | 209 |
| 54790_4_171 | TET2 | EXON | + | chr4:105234685 -105234705 | CAUAAAUGCCAUUAACAGUC | 210 |
| 54790_4_177 | TET2 | EXON | + | chr4:105234734 -105234754 | ACUCACCCAUCGCAUACCUC | 211 |
| 54790_4_178 | TET2 | EXON | + | chr4:105234735 -105234755 | CUCACCCAUCGCAUACCUCA | 212 |
| 54790_4_181 | TET2 | EXON | + | chr4:105234793 -105234813 | GCCUCCAAAGCCAGCUGCAG | 213 |
| 54790_4_184 | TET2 | EXON | + | chr4:105234802 -105234822 | GCCAGCUGCAGUGGUGAGUG | 214 |
| 54790_4_200 | TET2 | EXON | + | chr4:105234943 -105234963 | UCCUGCAGAAAAUAACAUCC | 215 |
| 54790_4_201 | TET2 | EXON | + | chr4:105234944 -105234964 | CCUGCAGAAAAUAACAUCCA | 216 |
| 54790_4_203 | TET2 | EXON | + | chr4:105234965 -105234985 | GGAACCACAAAGCUAGCGUC | 217 |
| 54790_4_207 | TET2 | EXON | + | chr4:105234983 -105235003 | UCUGGUGAAGAAUUCUGUUC | 218 |
| 54790_4_211 | TET2 | EXON | + | chr4:105235010 -105235030 | AGCAGCAAUUUGCAAGCUCC | 219 |
| 54790_4_212 | TET2 | EXON | + | chr4:105235013 -105235033 | AGCAAUUUGCAAGCUCCUGG | 220 |
| 54790_4_216 | TET2 | EXON | + | chr4:105235026 -105235046 | CUCCUGGUGGCAGCUCUGAA | 221 |
| 54790_4_219 | TET2 | EXON | + | chr4:105235052 -105235072 | UUAAAACAAAAUGAAAUGAA | 222 |
| 54790_4_225 | TET2 | EXON | + | chr4:105235087 -105235107 | GCAAAGCUCAGUGUUCACUA | 223 |
| 54790_4_235 | TET2 | EXON | + | chr4:105235162 -105235182 | UCCCCCUCCUCCUCUUCCAC | 224 |
| 54790_4_240 | TET2 | EXON | + | chr4:105235184 -105235204 | GUUCCUCAGCUUCCUUCAGA | 225 |
| 54790_4_245 | TET2 | EXON | + | chr4:105235202 -105235222 | GAAGGAAAAAGCACUCUGAA | 226 |
| 54790_4_247 | TET2 | EXON | + | chr4:105235205 -105235225 | GGAAAAAGCACUCUGAAUGG | 227 |
| 54790_4_256 | TET2 | EXON | + | chr4:105235260 -105235280 | AAAGUAACACAACACUUUUA | 228 |
| 54790_4_258 | TET2 | EXON | + | chr4:105235261 -105235281 | AAGUAACACAACACUUUUAA | 229 |
| 54790_4_262 | TET2 | EXON | + | chr4:105235276 -105235296 | UUUAAGGGAAGUGAAAAUAG | 230 |
| 54790_4_263 | TET2 | EXON | + | chr4:105235277 -105235297 | UUAAGGGAAGUGAAAAUAGA | 231 |
| 54790_4_268 | TET2 | EXON | + | chr4:105235288 -105235308 | GAAAAUAGAGGGUAAACCUG | 232 |
| 54790_4_272 | TET2 | EXON | + | chr4:105235356 -105235376 | CUUCUCCGAUGCUUUCUGAA | 233 |
| 54790_4_280 | TET2 | EXON | + | chr4:105235380 -105235400 | CUCAGAAUAAUUGUGUGAAC | 234 |
| 54790_4_284 | TET2 | EXON | + | chr4:105235400 -105235420 | AGGAAUGACAUACAGACUGC | 235 |
| 54790_4_286 | TET2 | EXON | + | chr4:105235401 -105235421 | GGAAUGACAUACAGACUGCA | 236 |
| 54790_4_294 | TET2 | EXON | + | chr4:105235478 -105235498 | AAGCAUAACCCACCAAUUUU | 237 |
| 54790_4_297 | TET2 | EXON | + | chr4:105235487 -105235507 | CCACCAAUUUUUGGUAGCAG | 238 |
| 54790_4_302 | TET2 | EXON | + | chr4:105235498 -105235518 | UGGUAGCAGUGGAGAGCUAC | 239 |
| 54790_4_313 | TET2 | EXON | + | chr4:105235546 -105235566 | CAAAGAGCAAGAGAUUCUGA | 240 |
| 54790_4_314 | TET2 | EXON | + | chr4:105235547 -105235567 | AAAGAGCAAGAGAUUCUGAA | 241 |
| 54790_4_317 | TET2 | EXON | + | chr4:105235558 -105235578 | GAUUCUGAAGGGUCGAGACA | 242 |
| 54790_4_324 | TET2 | EXON | + | chr4:105235607 -105235627 | ACACAGCACUAUCUGAAACC | 243 |
| 54790_4_326 | TET2 | EXON | + | chr4:105235611 -105235631 | AGCACUAUCUGAAACCAGGA | 244 |
| 54790_4_329 | TET2 | EXON | + | chr4:105235624 -105235644 | ACCAGGAUGGAUUGAAUUGA | 245 |
| 54790_4_333 | TET2 | EXON | + | chr4:105235645 -105235665 | GGCCCCUCGUUUUCACCAAG | 246 |
| 54790_4_339 | TET2 | EXON | + | chr4:105235669 -105235689 | AUCCCAUCUAAAACGUAAUG | 247 |
| 54790_4_343 | TET2 | EXON | + | chr4:105235739 -105235759 | AUGACCUCCAAACAAUACAC | 248 |
| 54790_4_347 | TET2 | EXON | + | chr4:105235757 -105235777 | ACUGGAAAUUCCAACAUGCC | 249 |
| 54790_4_349 | TET2 | EXON | + | chr4:105235758 -105235778 | CUGGAAAUUCCAACAUGCCU | 250 |
| 54790_4_351 | TET2 | EXON | + | chr4:105235759 -105235779 | UGGAAAUUCCAACAUGCCUG | 251 |
| 54790_4_352 | TET2 | EXON | + | chr4:105235760 -105235780 | GGAAAUUCCAACAUGCCUGG | 252 |
| 54790_4_353 | TET2 | EXON | + | chr4:105235761 -105235781 | GAAAUUCCAACAUGCCUGGG | 253 |
| 54790_4_355 | TET2 | EXON | + | chr4:105235770 -105235790 | ACAUGCCUGGGGGGCUCCCA | 254 |
| 54790_4_360 | TET2 | EXON | + | chr4:105235801 -105235821 | CACCCAGAAAACAACACAGC | 255 |
| 54790_4_365 | TET2 | EXON | + | chr4:105235841 -105235861 | UACCAAGUUGAAAUGAAUCA | 256 |
| 54790_4_366 | TET2 | EXON | + | chr4:105235842 -105235862 | ACCAAGUUGAAAUGAAUCAA | 257 |
| 54790_4_368 | TET2 | EXON | + | chr4:105235853 -105235873 | AUGAAUCAAGGGCAGUCCCA | 258 |
| 54790_4_370 | TET2 | EXON | + | chr4:105235861 -105235881 | AGGGCAGUCCCAAGGUACAG | 259 |
| 54790_4_371 | TET2 | EXON | + | chr4:105235897 -105235917 | GUUCCAAAAACCCUCACACC | 260 |
| 54790_4_376 | TET2 | EXON | + | chr4:105235952 -105235972 | GCUCAUGUGCAGUCACUGUG | 261 |
| 54790_4_388 | TET2 | EXON | + | chr4:105236038 -105236058 | GAAACAGCACUUGAAUCAAC | 262 |
| 54790_4_399 | TET2 | EXON | + | chr4:105236098 -105236118 | GCAACAUAAGCCUCAUAAAC | 263 |
| 54790_4_407 | TET2 | EXON | + | chr4:105236182 -105236202 | AUUACAAAUAAAGAAUAAAG | 264 |
| 54790_4_416 | TET2 | EXON | + | chr4:105236237 -105236257 | AACAAUGAUCAGCAAAGAGA | 265 |
| 54790_4_417 | TET2 | EXON | + | chr4:105236249 -105236269 | CAAAGAGAAGGAUCAUUCUU | 266 |
| 54790_4_419 | TET2 | EXON | + | chr4:105236263 -105236283 | AUUCUUUGGCCAGACUAAAG | 267 |
| 54790_4_426 | TET2 | EXON | + | chr4:105236279 -105236299 | AAAGUGGAAGAAUGUUUUCA | 268 |
| 54790_4_435 | TET2 | EXON | + | chr4:105236332 -105236352 | CGAGACUCAUAAUGUCCAAA | 269 |
| 54790_4_438 | TET2 | EXON | + | chr4:105236333 -105236353 | GAGACUCAUAAUGUCCAAAU | 270 |
| 54790_4_440 | TET2 | EXON | + | chr4:105236338 -105236358 | UCAUAAUGUCCAAAUGGGAC | 271 |
| 54790_4_444 | TET2 | EXON | + | chr4:105236341 -105236361 | UAAUGUCCAAAUGGGACUGG | 272 |
| 54790_4_452 | TET2 | EXON | + | chr4:105236413 -105236433 | AUCAAGUGCAUGCAAAAUAC | 273 |
| 54790_4_466 | TET2 | EXON | + | chr4:105236486 -105236506 | ACACAUCCUGAACUUUUUGC | 274 |
| 54790_4_475 | TET2 | EXON | + | chr4:105236562 -105236582 | CAAAGCAAGAUCUUCUUCAC | 275 |
| 54790_4_479 | TET2 | EXON | + | chr4:105236578 -105236598 | UCACAGGUGCUUUCAAGAAC | 276 |
| 54790_4_486 | TET2 | EXON | + | chr4:105236611 -105236631 | ACAACAAGCUUCAGUUCUAC | 277 |
| 54790_4_488 | TET2 | EXON | + | chr4:105236612 -105236632 | CAACAAGCUUCAGUUCUACA | 278 |
| 54790_4_493 | TET2 | EXON | + | chr4:105236642 -105236662 | AAUAGAAACCAAGAUAUGUC | 279 |
| 54790_4_494 | TET2 | EXON | + | chr4:105236673 -105236693 | CUGCGCAACUUGCUCAGCAA | 280 |
| 54790_4_498 | TET2 | EXON | + | chr4:105236719 -105236739 | UGUUUUUCCUGUGCCUGACC | 281 |
| 54790_4_501 | TET2 | EXON | + | chr4:105236720 -105236740 | GUUUUUCCUGUGCCUGACCA | 282 |
| 54790_4_503 | TET2 | EXON | + | chr4:105236723 -105236743 | UUUCCUGUGCCUGACCAGGG | 283 |
| 54790_4_511 | TET2 | EXON | + | chr4:105236752 -105236772 | CACUCAGACCCCUCCCCAGA | 284 |
| 54790_4_512 | TET2 | EXON | + | chr4:105236778 -105236798 | CUCAAAAGCAUGCUGCUCUA | 285 |
| 54790_4_513 | TET2 | EXON | + | chr4:105236781 -105236801 | AAAAGCAUGCUGCUCUAAGG | 286 |
| 54790_4_518 | TET2 | EXON | + | chr4:105236856 -105236876 | CUUGCCAUAGUCAGAUGCAC | 287 |
| 54790_4_520 | TET2 | EXON | + | chr4:105236866 -105236886 | UCAGAUGCACAGGCCAAUUA | 288 |
| 54790_4_522 | TET2 | EXON | + | chr4:105236869 -105236889 | GAUGCACAGGCCAAUUAAGG | 289 |
| 54790_4_525 | TET2 | EXON | + | chr4:105236876 -105236896 | AGGCCAAUUAAGGUGGAACC | 290 |
| 54790_4_531 | TET2 | EXON | + | chr4:105236928 -105236948 | CACCACCAGAAAACAAAACA | 291 |
| 54790_4_532 | TET2 | EXON | + | chr4:105236935 -105236955 | AGAAAACAAAACAUGGAAAA | 292 |
| 54790_4_540 | TET2 | EXON | + | chr4:105237004 -105237024 | AAAGAGCAUCAUUGAGACCA | 293 |
| 54790_4_545 | TET2 | EXON | + | chr4:105237052 -105237072 | CAAGUCGUUAUUUGACCAUA | 294 |
| 54790_4_553 | TET2 | EXON | + | chr4:105237098 -105237118 | CAAGUAAAAGUUGAAAUGUC | 295 |
| 54790_4_554 | TET2 | EXON | + | chr4:105237099 -105237119 | AAGUAAAAGUUGAAAUGUCA | 296 |
| 54790_4_578 | TET2 | EXON | + | chr4:105237280 -105237300 | UACUCCUAUAAAAAAUUUAU | 297 |
| 54790_4_582 | TET2 | EXON | + | chr4:105237329 -105237349 | UUCCCAUCUUGCAGAUGUGU | 298 |
| 54790_4_589 | TET2 | EXON | + | chr4:105237359 -105237379 | CAGAAAUGUACUGAGACACA | 299 |
| 54790_4_596 | TET2 | EXON | + | chr4:105237397 -105237417 | AGCAAAUUUAUCUUCAGAUA | 300 |
| 54790_4_597 | TET2 | EXON | + | chr4:105237398 -105237418 | GCAAAUUUAUCUUCAGAUAU | 301 |
| 54790_4_606 | TET2 | EXON | + | chr4:105237430 -105237450 | CUUUUUUUAAAUCUUGAGUC | 302 |
| 54790_4_614 | TET2 | EXON | + | chr4:105237446 -105237466 | AGUCUGGCAGCAAUUUGUAA | 303 |
| 54790_4_657 | TET2 | EXON | + | chr4:105237650 -105237670 | GCUCUUUGUAUAUUAUCUCC | 304 |
| 54790_4_662 | TET2 | EXON | + | chr4:105237663 -105237683 | UAUCUCCUGGAGAGACAGCU | 305 |
| 54790_4_668 | TET2 | EXON | + | chr4:105237708 -105237728 | AAUGAGAAAAUAACGACCAU | 306 |
| 54790_4_670 | TET2 | EXON | + | chr4:105237748 -105237768 | UUUAAAUAUUUUUUAAUUCA | 307 |
| 54790_4_679 | TET2 | EXON | + | chr4:105237778 -105237798 | UAUUAGUUUCACAAGAUUUC | 308 |
| 54790_4_682 | TET2 | EXON | + | chr4:105237786 -105237806 | UCACAAGAUUUCUGGCUAAU | 309 |
| 54790_4_686 | TET2 | EXON | + | chr4:105237787 -105237807 | CACAAGAUUUCUGGCUAAUA | 310 |
| 54790_4_693 | TET2 | EXON | + | chr4:105237817 -105237837 | UAUCUUCAGUCUUCAUGAGU | 311 |
| 54790_4_695 | TET2 | EXON | + | chr4:105237818 -105237838 | AUCUUCAGUCUUCAUGAGUU | 312 |
| 54790_4_697 | TET2 | EXON | + | chr4:105237819 -105237839 | UCUUCAGUCUUCAUGAGUUG | 313 |
| 54790_4_700 | TET2 | EXON | + | chr4:105237820 -105237840 | CUUCAGUCUUCAUGAGUUGG | 314 |
| 54790_4_709 | TET2 | EXON | + | chr4:105237882 -105237902 | CUUUUCUCCAUUUAUACAUU | 315 |
| 54790_4_741 | TET2 | EXON | + | chr4:105240332 -105240352 | AAAGCUUUUUGUUAAAAUUC | 316 |
| 54790_4_746 | TET2 | EXON | + | chr4:105240344 -105240364 | UAAAAUUCAGGAUAUGUAAU | 317 |
| 54790_4_750 | TET2 | EXON | + | chr4:105240352 -105240372 | AGGAUAUGUAAUAGGUCUGU | 318 |
| 54790_4_754 | TET2 | EXON | + | chr4:105240377 -105240397 | UAGUGAAAUAUUUUUGCUGA | 319 |
| 54790_4_760 | TET2 | EXON | + | chr4:105240395 -105240415 | GAUGGAUGUAGAUAUAUACG | 320 |
| 54790_4_770 | TET2 | EXON | + | chr4:105240478 -105240498 | AGACAAAUGUUAAAUUAGUG | 321 |
| 54790_4_780 | TET2 | EXON | + | chr4:105240541 -105240561 | GAUACCCCACACUGUGUAGA | 322 |
| 54790_4_783 | TET2 | EXON | + | chr4:105240545 -105240565 | CCCCACACUGUGUAGAAGGA | 323 |
| 54790_4_785 | TET2 | EXON | + | chr4:105240548 -105240568 | CACACUGUGUAGAAGGAUGG | 324 |
| 54790_4_787 | TET2 | EXON | + | chr4:105240549 -105240569 | ACACUGUGUAGAAGGAUGGA | 325 |
| 54790_4_790 | TET2 | EXON | + | chr4:105240552 -105240572 | CUGUGUAGAAGGAUGGAGGG | 326 |
| 54790_4_791 | TET2 | EXON | + | chr4:105240579 -105240599 | CUACUGUCCCUCUUUGCGUG | 327 |
| 54790_4_795 | TET2 | EXON | + | chr4:105240599 -105240619 | UGGUUAUUAAGUUGCCUCAC | 328 |
| 54790_4_796 | TET2 | EXON | + | chr4:105240600 -105240620 | GGUUAUUAAGUUGCCUCACU | 329 |
| 54790_4_800 | TET2 | EXON | + | chr4:105240634 -105240654 | CACAUCUCAUAGAUAAUAUU | 330 |
| 54790_4_807 | TET2 | EXON | + | chr4:105240703 -105240723 | UCCCACUUUUCCAUCUUUGU | 331 |
| 54790_4_818 | TET2 | EXON | + | chr4:105240740 -105240760 | UUCUUUUUGCCUGACUCUCC | 332 |
| 54790_4_829 | TET2 | EXON | + | chr4:105240784 -105240804 | UUCUAAAGUACAUACUAAUA | 333 |
| 54790_4_830 | TET2 | EXON | + | chr4:105240785 -105240805 | UCUAAAGUACAUACUAAUAU | 334 |
| 54790_4_833 | TET2 | EXON | + | chr4:105240790 -105240810 | AGUACAUACUAAUAUGGGUC | 335 |
| 54790_4_841 | TET2 | EXON | + | chr4:105240833 -105240853 | AAACAGCAAUUAAAUGUUAU | 336 |
| 54790_4_842 | TET2 | EXON | + | chr4:105240834 -105240854 | AACAGCAAUUAAAUGUUAUA | 337 |
| 54790_4_845 | TET2 | EXON | + | chr4:105240841 -105240861 | AUUAAAUGUUAUAGGGAAGU | 338 |
| 54790_4_851 | TET2 | EXON | + | chr4:105240851 -105240871 | AUAGGGAAGUAGGAAGAAAA | 339 |
| 54790_4_853 | TET2 | EXON | + | chr4:105240852 -105240872 | UAGGGAAGUAGGAAGAAAAA | 340 |
| 54790_4_855 | TET2 | EXON | + | chr4:105240853 -105240873 | AGGGAAGUAGGAAGAAAAAG | 341 |
| 54790_4_858 | TET2 | EXON | + | chr4:105240885 -105240905 | CAAUAAACCAAGCAAUAUUC | 342 |
| 54790_4_861 | TET2 | EXON | + | chr4:105240886 -105240906 | AAUAAACCAAGCAAUAUUCU | 343 |
| 54790_4_862 | TET2 | EXON | + | chr4:105240887 -105240907 | AUAAACCAAGCAAUAUUCUG | 344 |
| 54790_4_863 | TET2 | EXON | + | chr4:105240888 -105240908 | UAAACCAAGCAAUAUUCUGG | 345 |
| 54790_4_865 | TET2 | EXON | + | chr4:105240891 -105240911 | ACCAAGCAAUAUUCUGGGGG | 346 |
| 54790_4_867 | TET2 | EXON | + | chr4:105240892 -105240912 | CCAAGCAAUAUUCUGGGGGU | 347 |
| 54790_4_870 | TET2 | EXON | + | chr4:105240902 -105240922 | UUCUGGGGGUGGGAUAGAGC | 348 |
| 54790_4_880 | TET2 | EXON | + | chr4:105240940 -105240960 | UCUUUUAAAAUCCAAGUAAU | 349 |
| 54790_4_881 | TET2 | EXON | + | chr4:105240944 -105240964 | UUAAAAUCCAAGUAAUAGGU | 350 |
| 54790_4_891 | TET2 | EXON | + | chr4:105240991 -105241011 | UUUUUUCCAGCUCAAAAAAU | 351 |
| 54790_4_905 | TET2 | EXON | + | chr4:105241063 -105241083 | UUUGUUUAGUUUCAUUUAUU | 352 |
| 54790_4_929 | TET2 | EXON | + | chr4:105241146 -105241166 | UGUACAUAUACUUAAUUAUG | 353 |
| 54790_4_945 | TET2 | EXON | + | chr4:105241237 -105241257 | UAGAGCCCUUAAUGUGUAGU | 354 |
| 54790_4_949 | TET2 | EXON | + | chr4:105241238 -105241258 | AGAGCCCUUAAUGUGUAGUU | 355 |
| 54790_4_951 | TET2 | EXON | + | chr4:105241239 -105241259 | GAGCCCUUAAUGUGUAGUUG | 356 |
| 54790_4_953 | TET2 | EXON | + | chr4:105241240 -105241260 | AGCCCUUAAUGUGUAGUUGG | 357 |
| 54790_4_956 | TET2 | EXON | + | chr4:105241253 -105241273 | UAGUUGGGGGUUAAGCUUUG | 358 |
| 54790_4_962 | TET2 | EXON | + | chr4:105241283 -105241303 | CUUUAUAUUUAGUAUAAUUG | 359 |
| 54790_4_973 | TET2 | EXON | + | chr4:105241340 -105241360 | CAAAUUAUUGAAAAAGAUGA | 360 |
| 54790_4_977 | TET2 | EXON | + | chr4:105241361 -105241381 | GGUCCUUUUUAUACCCAUCU | 361 |
| 54790_4_979 | TET2 | EXON | + | chr4:105241367 -105241387 | UUUUAUACCCAUCUAGGAGC | 362 |
| 54790_4_984 | TET2 | EXON | + | chr4:105241378 -105241398 | UCUAGGAGCAGGUCCUAAUG | 363 |
| 54790_4_990 | TET2 | EXON | + | chr4:105241399 -105241419 | GGCAGCUAUUAGAGAAAUCA | 364 |
| 54790_4_993 | TET2 | EXON | + | chr4:105241407 -105241427 | UUAGAGAAAUCAUGGAAGAA | 365 |
| 54790_4_995 | TET2 | EXON | + | chr4:105241422 -105241442 | AAGAAAGGUAAUUAACGCAA | 366 |
| 54790_4_997 | TET2 | EXON | + | chr4:105241428 -105241448 | GGUAAUUAACGCAAAGGCAC | 367 |
| 54790_4_998 | TET2 | EXON | + | chr4:105241429 -105241449 | GUAAUUAACGCAAAGGCACA | 368 |
| 54790_4_1014 | TET2 | EXON | + | chr4:105241523 -105241543 | UAAAUUGAGUAAUUAUUAGU | 369 |
| 54790_4_1019 | TET2 | EXON | + | chr4:105241538 -105241558 | UUAGUAGGCUUAGCUAUUCU | 370 |
| 54790_4_1020 | TET2 | EXON | + | chr4:105241539 -105241559 | UAGUAGGCUUAGCUAUUCUA | 371 |
| 54790_4_1029 | TET2 | EXON | + | chr4:105241592 -105241612 | AGAGAGUCACAAUAUUUGAC | 372 |
| 54790_4_1032 | TET2 | EXON | + | chr4:105241612 -105241632 | AGGACUAAUAGUCUGCUAGC | 373 |
| 54790_4_1033 | TET2 | EXON | + | chr4:105241618 -105241638 | AAUAGUCUGCUAGCUGGCAC | 374 |
| 54790_4_1035 | TET2 | EXON | + | chr4:105241636 -105241656 | ACAGGCUGCCCACUUUGCGA | 375 |
| 54790_4_1040 | TET2 | EXON | + | chr4:105241653 -105241673 | CGAUGGAUGCCAGAAAACCC | 376 |
| 54790_4_1043 | TET2 | EXON | + | chr4:105241663 -105241683 | CAGAAAACCCAGGCAUGAAC | 377 |
| 54790_4_1045 | TET2 | EXON | + | chr4:105241669 -105241689 | ACCCAGGCAUGAACAGGAAU | 378 |
| 54790_4_1046 | TET2 | EXON | + | chr4:105241678 -105241698 | UGAACAGGAAUCGGCCAGCC | 379 |
| 54790_4_1047 | TET2 | EXON | + | chr4:105241693 -105241713 | CAGCCAGGCUGCCAGCCACA | 380 |
| 54790_4_1048 | TET2 | EXON | + | chr4:105241699 -105241719 | GGCUGCCAGCCACAAGGUAC | 381 |
| 54790_4_1049 | TET2 | EXON | + | chr4:105241705 -105241725 | CAGCCACAAGGUACUGGCAC | 382 |
| 54790_4_1052 | TET2 | EXON | + | chr4:105241718 -105241738 | CUGGCACAGGCUCCAACGAG | 383 |
| 54790_4_1053 | TET2 | EXON | + | chr4:105241729 -105241749 | UCCAACGAGAGGUCCCACUC | 384 |
| 54790_4_1058 | TET2 | EXON | + | chr4:105241770 -105241790 | AAGUGUCAAAGCAGAAAGAC | 385 |
| 54790_4_1059 | TET2 | EXON | + | chr4:105241780 -105241800 | GCAGAAAGACUGGUAAAGUG | 386 |
| 54790_4_1092 | TET2 | EXON | + | chr4:105241946 -105241966 | UUUUUUUCGCUAUCAAUCAC | 387 |
| 54790_4_1109 | TET2 | EXON | + | chr4:105242012 -105242032 | UGAGCGAGAUAAUGCAGAGA | 388 |
| 54790_4_1117 | TET2 | EXON | + | chr4:105242057 -105242077 | CUCUGAGCUGUUCUUCUUCU | 389 |
| 54790_4_1118 | TET2 | EXON | + | chr4:105242058 -105242078 | UCUGAGCUGUUCUUCUUCUA | 390 |
| 54790_4_1123 | TET2 | EXON | + | chr4:105242076 -105242096 | UAGGGUGCCUUUUCAUUAAG | 391 |
| 54790_4_1124 | TET2 | EXON | + | chr4:105242080 -105242100 | GUGCCUUUUCAUUAAGAGGU | 392 |
| 54790_4_1130 | TET2 | EXON | + | chr4:105242105 -105242125 | GUAUUAUUAUUAAAGUACUU | 393 |
| 54790_4_1135 | TET2 | EXON | + | chr4:105242114 -105242134 | UUAAAGUACUUAGGAUACAU | 394 |
| 54790_4_1136 | TET2 | EXON | + | chr4:105242115 -105242135 | UAAAGUACUUAGGAUACAUU | 395 |
| 54790_4_1137 | TET2 | EXON | + | chr4:105242116 -105242136 | AAAGUACUUAGGAUACAUUG | 396 |
| 54790_4_1140 | TET2 | EXON | + | chr4:105242124 -105242144 | UAGGAUACAUUGGGGCAGCU | 397 |
| 54790_4_1154 | TET2 | EXON | + | chr4:105242210 -105242230 | UUCACUAAAUAAUCAUCUAG | 398 |
| 54790_4_1156 | TET2 | EXON | + | chr4:105242215 -105242235 | UAAAUAAUCAUCUAGUGGCC | 399 |
| 54790_4_1162 | TET2 | EXON | + | chr4:105242287 -105242307 | UUGUUUUUUAAACAAGCAGU | 400 |
| 54790_4_1163 | TET2 | EXON | + | chr4:105242290 -105242310 | UUUUUUAAACAAGCAGUAGG | 401 |
| 54790_4_1164 | TET2 | EXON | + | chr4:105242298 -105242318 | ACAAGCAGUAGGUGGUGCUU | 402 |
| 54790_4_1167 | TET2 | EXON | + | chr4:105242306 -105242326 | UAGGUGGUGCUUUGGUCAUA | 403 |
| 54790_4_1169 | TET2 | EXON | + | chr4:105242307 -105242327 | AGGUGGUGCUUUGGUCAUAA | 404 |
| 54790_4_1173 | TET2 | EXON | + | chr4:105242328 -105242348 | GGAAGAUAUAGUCUAUUUCU | 405 |
| 54790_4_1176 | TET2 | EXON | + | chr4:105242351 -105242371 | ACUAUUCCAUAUUUUCCAUG | 406 |
| 54790_4_1178 | TET2 | EXON | + | chr4:105242355 -105242375 | UUCCAUAUUUUCCAUGUGGC | 407 |
| 54790_4_1187 | TET2 | EXON | + | chr4:105242404 -105242424 | UCUAAAUUGUGAGACAUUCU | 408 |
| 54790_4_1193 | TET2 | EXON | + | chr4:105242407 -105242427 | AAAUUGUGAGACAUUCUUGG | 409 |
| 54790_4_1201 | TET2 | EXON | + | chr4:105242469 -105242489 | UAAAAUAGCUAAAUUUAGUA | 410 |
| 54790_4_1205 | TET2 | EXON | + | chr4:105242470 -105242490 | AAAAUAGCUAAAUUUAGUAA | 411 |
| 54790_4_1241 | TET2 | EXON | + | chr4:105242625 -105242645 | AUCUGUACAUUUUGAUAUUG | 412 |
| 54790_4_1244 | TET2 | EXON | + | chr4:105242635 -105242655 | UUUGAUAUUGAGGAAAAACA | 413 |
| 54790_4_1250 | TET2 | EXON | + | chr4:105242663 -105242683 | AAACCAUUAUCCAGUUUGCU | 414 |
| 54790_4_1258 | TET2 | EXON | + | chr4:105242705 -105242725 | UAAUAAACCGUUCAUUUCUC | 415 |
| 54790_4_1259 | TET2 | EXON | + | chr4:105242711 -105242731 | ACCGUUCAUUUCUCAGGAUG | 416 |
| 54790_4_1269 | TET2 | EXON | - | chr4:105233886 -105233906 | UAGUUGAAUUCUAAAGAGCA | 417 |
| 54790_4_1276 | TET2 | EXON | - | chr4:105233917 -105233937 | UUGCUUCGGGGCCAUCCACA | 418 |
| 54790_4_1278 | TET2 | EXON | - | chr4:105233929 -105233949 | GUUCCAUCAGGCUUGCUUCG | 419 |
| 54790_4_1279 | TET2 | EXON | - | chr4:105233930 -105233950 | UGUUCCAUCAGGCUUGCUUC | 420 |
| 54790_4_1281 | TET2 | EXON | - | chr4:105233931 -105233951 | CUGUUCCAUCAGGCUUGCUU | 421 |
| 54790_4_1285 | TET2 | EXON | - | chr4:105233941 -105233961 | UGGUUCUAUCCUGUUCCAUC | 422 |
| 54790_4_1288 | TET2 | EXON | - | chr4:105233961 -105233981 | UCUGUUGCCCUCAACAUGGU | 423 |
| 54790_4_1289 | TET2 | EXON | - | chr4:105233965 -105233985 | UUAGUCUGUUGCCCUCAACA | 424 |
| 54790_4_1290 | TET2 | EXON | - | chr4:105233990 -105234010 | GGAGGUGAUGGUAUCAGGAA | 425 |
| 54790_4_1293 | TET2 | EXON | - | chr4:105233995 -105234015 | AAAUGGGAGGUGAUGGUAUC | 426 |
| 54790_4_1296 | TET2 | EXON | - | chr4:105234002 -105234022 | GUCUGGCAAAUGGGAGGUGA | 427 |
| 54790_4_1297 | TET2 | EXON | - | chr4:105234008 -105234028 | GGUUCUGUCUGGCAAAUGGG | 428 |
| 54790_4_1298 | TET2 | EXON | - | chr4:105234011 -105234031 | AGAGGUUCUGUCUGGCAAAU | 429 |
| 54790_4_1300 | TET2 | EXON | - | chr4:105234012 -105234032 | CAGAGGUUCUGUCUGGCAAA | 430 |
| 54790_4_1305 | TET2 | EXON | - | chr4:105234019 -105234039 | UUGUAGCCAGAGGUUCUGUC | 431 |
| 54790_4_1308 | TET2 | EXON | - | chr4:105234029 -105234049 | UUCUGGAGCUUUGUAGCCAG | 432 |
| 54790_4_1310 | TET2 | EXON | - | chr4:105234046 -105234066 | CAGGCAGUGGGCUUCCAUUC | 433 |
| 54790_4_1314 | TET2 | EXON | - | chr4:105234058 -105234078 | GAUGAGCUCUCUCAGGCAGU | 434 |
| 54790_4_1315 | TET2 | EXON | - | chr4:105234059 -105234079 | GGAUGAGCUCUCUCAGGCAG | 435 |
| 54790_4_1319 | TET2 | EXON | - | chr4:105234065 -105234085 | ACUUCUGGAUGAGCUCUCUC | 436 |
| 54790_4_1322 | TET2 | EXON | - | chr4:105234080 -105234100 | UUGGUGUCUCCAUUUACUUC | 437 |
| 54790_4_1327 | TET2 | EXON | - | chr4:105234099 -105234119 | ACUUUUGAAAGAGUGCCACU | 438 |
| 54790_4_1334 | TET2 | EXON | - | chr4:105234134 -105234154 | UUCUGGCUUCCCUUCAUACA | 439 |
| 54790_4_1335 | TET2 | EXON | - | chr4:105234135 -105234155 | AUUCUGGCUUCCCUUCAUAC | 440 |
| 54790_4_1337 | TET2 | EXON | - | chr4:105234151 -105234171 | CAGGACUCACACGACUAUUC | 441 |
| 54790_4_1341 | TET2 | EXON | - | chr4:105234170 -105234190 | CUACUUUCUUGUGUAAAGUC | 442 |
| 54790_4_1351 | TET2 | EXON | - | chr4:105234201 -105234221 | UCCUCCAUUUUGCAAACACU | 443 |
| 54790_4_1355 | TET2 | EXON | - | chr4:105234245 -105234265 | UGAAGGAGCCCAGAGAGAGA | 444 |
| 54790_4_1367 | TET2 | EXON | - | chr4:105234262 -105234282 | GUUUCAAUUUCUUGAUCUGA | 445 |
| 54790_4_1378 | TET2 | EXON | - | chr4:105234289 -105234309 | GUCUUUCUCCAUUAGCCUUU | 446 |
| 54790_4_1388 | TET2 | EXON | - | chr4:105234328 -105234348 | UUUCACCUGGAUUUCUUUCU | 447 |
| 54790_4_1392 | TET2 | EXON | - | chr4:105234341 -105234361 | UUUGGUUGACUGCUUUCACC | 448 |
| 54790_4_1396 | TET2 | EXON | - | chr4:105234359 -105234379 | UCACUCAAAUCGGAGACAUU | 449 |
| 54790_4_1399 | TET2 | EXON | - | chr4:105234369 -105234389 | UUCUUUCUUAUCACUCAAAU | 450 |
| 54790_4_1410 | TET2 | EXON | - | chr4:105234408 -105234428 | AUCUUUAACUGCAUUUUCUU | 451 |
| 54790_4_1411 | TET2 | EXON | - | chr4:105234409 -105234429 | AAUCUUUAACUGCAUUUUCU | 452 |
| 54790_4_1416 | TET2 | EXON | - | chr4:105234435 -105234455 | GCAGUUAUGUGUUGAAAAAC | 453 |
| 54790_4_1422 | TET2 | EXON | - | chr4:105234464 -105234484 | AUCUGAAGCUCUGGAUUUUC | 454 |
| 54790_4_1423 | TET2 | EXON | - | chr4:105234473 -105234493 | UCAUUCAGAAUCUGAAGCUC | 455 |
| 54790_4_1435 | TET2 | EXON | - | chr4:105234520 -105234540 | GUAAUACAAUGUUCUUGUCA | 456 |
| 54790_4_1441 | TET2 | EXON | - | chr4:105234566 -105234586 | GCAGAAACUGUAGCACCAUU | 457 |
| 54790_4_1444 | TET2 | EXON | - | chr4:105234588 -105234608 | AUGUGUGUGUUCCACGGAAG | 458 |
| 54790_4_1448 | TET2 | EXON | - | chr4:105234594 -105234614 | UUCACCAUGUGUGUGUUCCA | 459 |
| 54790_4_1457 | TET2 | EXON | - | chr4:105234619 -105234639 | AUUGAGACAGUGUUUUUUCC | 460 |
| 54790_4_1461 | TET2 | EXON | - | chr4:105234647 -105234667 | ACCGCAAUGGAAACACAAUC | 461 |
| 54790_4_1466 | TET2 | EXON | - | chr4:105234660 -105234680 | UGUGGUUUUCUGCACCGCAA | 462 |
| 54790_4_1471 | TET2 | EXON | - | chr4:105234678 -105234698 | AAUGGCAUUUAUGUGAGAUG | 463 |
| 54790_4_1474 | TET2 | EXON | - | chr4:105234696 -105234716 | AUUAGUAGCCUGACUGUUAA | 464 |
| 54790_4_1475 | TET2 | EXON | - | chr4:105234726 -105234746 | CGAUGGGUGAGUGAUCUCAC | 465 |
| 54790_4_1479 | TET2 | EXON | - | chr4:105234742 -105234762 | UCUGCCCUGAGGUAUGCGAU | 466 |
| 54790_4_1480 | TET2 | EXON | - | chr4:105234743 -105234763 | AUCUGCCCUGAGGUAUGCGA | 467 |
| 54790_4_1482 | TET2 | EXON | - | chr4:105234753 -105234773 | UGCGGAAUUGAUCUGCCCUG | 468 |
| 54790_4_1485 | TET2 | EXON | - | chr4:105234771 -105234791 | CUCAGAGUUAGAGGUCUGUG | 469 |
| 54790_4_1490 | TET2 | EXON | - | chr4:105234780 -105234800 | UGGAGGCAGCUCAGAGUUAG | 470 |
| 54790_4_1493 | TET2 | EXON | - | chr4:105234797 -105234817 | ACCACUGCAGCUGGCUUUGG | 471 |
| 54790_4_1495 | TET2 | EXON | - | chr4:105234800 -105234820 | CUCACCACUGCAGCUGGCUU | 472 |
| 54790_4_1497 | TET2 | EXON | - | chr4:105234806 -105234826 | GCCUCACUCACCACUGCAGC | 473 |
| 54790_4_1499 | TET2 | EXON | - | chr4:105234828 -105234848 | AUCAGCAUCAUCAGCAUCAC | 474 |
| 54790_4_1505 | TET2 | EXON | - | chr4:105234855 -105234875 | UAGCAUUGCAGCUAGUUUAC | 475 |
| 54790_4_1510 | TET2 | EXON | - | chr4:105234882 -105234902 | UUCUGGUUUCUGAAAGGAAC | 476 |
| 54790_4_1514 | TET2 | EXON | - | chr4:105234888 -105234908 | UAGUUGUUCUGGUUUCUGAA | 477 |
| 54790_4_1521 | TET2 | EXON | - | chr4:105234899 -105234919 | UUUUGUUGUUGUAGUUGUUC | 478 |
| 54790_4_1526 | TET2 | EXON | - | chr4:105234940 -105234960 | UGUUAUUUUCUGCAGGAGAU | 479 |
| 54790_4_1527 | TET2 | EXON | - | chr4:105234941 -105234961 | AUGUUAUUUUCUGCAGGAGA | 480 |
| 54790_4_1531 | TET2 | EXON | - | chr4:105234947 -105234967 | CCCUGGAUGUUAUUUUCUGC | 481 |
| 54790_4_1535 | TET2 | EXON | - | chr4:105234964 -105234984 | ACGCUAGCUUUGUGGUUCCC | 482 |
| 54790_4_1539 | TET2 | EXON | - | chr4:105234972 -105234992 | UUCACCAGACGCUAGCUUUG | 483 |
| 54790_4_1545 | TET2 | EXON | - | chr4:105235011 -105235031 | AGGAGCUUGCAAAUUGCUGC | 484 |
| 54790_4_1551 | TET2 | EXON | - | chr4:105235031 -105235051 | UACCGUUCAGAGCUGCCACC | 485 |
| 54790_4_1569 | TET2 | EXON | - | chr4:105235116 -105235136 | ACCACACCAUCACCCAGAAA | 486 |
| 54790_4_1577 | TET2 | EXON | - | chr4:105235166 -105235186 | ACCUGUGGAAGAGGAGGAGG | 487 |
| 54790_4_1579 | TET2 | EXON | - | chr4:105235167 -105235187 | AACCUGUGGAAGAGGAGGAG | 488 |
| 54790_4_1581 | TET2 | EXON | - | chr4:105235168 -105235188 | GAACCUGUGGAAGAGGAGGA | 489 |
| 54790_4_1582 | TET2 | EXON | - | chr4:105235169 -105235189 | GGAACCUGUGGAAGAGGAGG | 490 |
| 54790_4_1586 | TET2 | EXON | - | chr4:105235172 -105235192 | UGAGGAACCUGUGGAAGAGG | 491 |
| 54790_4_1588 | TET2 | EXON | - | chr4:105235175 -105235195 | AGCUGAGGAACCUGUGGAAG | 492 |
| 54790_4_1593 | TET2 | EXON | - | chr4:105235181 -105235201 | GAAGGAAGCUGAGGAACCUG | 493 |
| 54790_4_1600 | TET2 | EXON | - | chr4:105235190 -105235210 | UUUCCUUCUGAAGGAAGCUG | 494 |
| 54790_4_1606 | TET2 | EXON | - | chr4:105235199 -105235219 | AGAGUGCUUUUUCCUUCUGA | 495 |
| 54790_4_1617 | TET2 | EXON | - | chr4:105235246 -105235266 | UACUUUGGUUGGGGUAGUGG | 496 |
| 54790_4_1618 | TET2 | EXON | - | chr4:105235249 -105235269 | UGUUACUUUGGUUGGGGUAG | 497 |
| 54790_4_1620 | TET2 | EXON | - | chr4:105235255 -105235275 | GUGUUGUGUUACUUUGGUUG | 498 |
| 54790_4_1621 | TET2 | EXON | - | chr4:105235256 -105235276 | AGUGUUGUGUUACUUUGGUU | 499 |
| 54790_4_1623 | TET2 | EXON | - | chr4:105235257 -105235277 | AAGUGUUGUGUUACUUUGGU | 500 |
| 54790_4_1626 | TET2 | EXON | - | chr4:105235261 -105235281 | UUAAAAGUGUUGUGUUACUU | 501 |
| 54790_4_1633 | TET2 | EXON | - | chr4:105235307 -105235327 | CUCUGGGAAGGUGGUGCCUC | 502 |
| 54790_4_1634 | TET2 | EXON | - | chr4:105235316 -105235336 | GGAUUAGGACUCUGGGAAGG | 503 |
| 54790_4_1635 | TET2 | EXON | - | chr4:105235319 -105235339 | GAUGGAUUAGGACUCUGGGA | 504 |
| 54790_4_1636 | TET2 | EXON | - | chr4:105235323 -105235343 | UGUAGAUGGAUUAGGACUCU | 505 |
| 54790_4_1638 | TET2 | EXON | - | chr4:105235324 -105235344 | GUGUAGAUGGAUUAGGACUC | 506 |
| 54790_4_1641 | TET2 | EXON | - | chr4:105235331 -105235351 | CAUACAUGUGUAGAUGGAUU | 507 |
| 54790_4_1643 | TET2 | EXON | - | chr4:105235337 -105235357 | GGGCUGCAUACAUGUGUAGA | 508 |
| 54790_4_1647 | TET2 | EXON | - | chr4:105235357 -105235377 | UUUCAGAAAGCAUCGGAGAA | 509 |
| 54790_4_1648 | TET2 | EXON | - | chr4:105235358 -105235378 | CUUUCAGAAAGCAUCGGAGA | 510 |
| 54790_4_1653 | TET2 | EXON | - | chr4:105235364 -105235384 | UGAGGCCUUUCAGAAAGCAU | 511 |
| 54790_4_1660 | TET2 | EXON | - | chr4:105235382 -105235402 | CUGUUCACACAAUUAUUCUG | 512 |
| 54790_4_1668 | TET2 | EXON | - | chr4:105235439 -105235459 | CUUGUUUUCUCAGAACACAA | 513 |
| 54790_4_1676 | TET2 | EXON | - | chr4:105235463 -105235483 | UGCUUGAGGUGUUCUGACAU | 514 |
| 54790_4_1678 | TET2 | EXON | - | chr4:105235477 -105235497 | AAAUUGGUGGGUUAUGCUUG | 515 |
| 54790_4_1680 | TET2 | EXON | - | chr4:105235489 -105235509 | CACUGCUACCAAAAAUUGGU | 516 |
| 54790_4_1681 | TET2 | EXON | - | chr4:105235490 -105235510 | CCACUGCUACCAAAAAUUGG | 517 |
| 54790_4_1683 | TET2 | EXON | - | chr4:105235493 -105235513 | UCUCCACUGCUACCAAAAAU | 518 |
| 54790_4_1690 | TET2 | EXON | - | chr4:105235531 -105235551 | CUUUGUUUCUCAUCAACUGC | 519 |
| 54790_4_1699 | TET2 | EXON | - | chr4:105235604 -105235624 | UUCAGAUAGUGCUGUGUUGG | 520 |
| 54790_4_1700 | TET2 | EXON | - | chr4:105235605 -105235625 | UUUCAGAUAGUGCUGUGUUG | 521 |
| 54790_4_1702 | TET2 | EXON | - | chr4:105235606 -105235626 | GUUUCAGAUAGUGCUGUGUU | 522 |
| 54790_4_1703 | TET2 | EXON | - | chr4:105235607 -105235627 | GGUUUCAGAUAGUGCUGUGU | 523 |
| 54790_4_1708 | TET2 | EXON | - | chr4:105235628 -105235648 | GCCUUCAAUUCAAUCCAUCC | 524 |
| 54790_4_1711 | TET2 | EXON | - | chr4:105235650 -105235670 | UUCCGCUUGGUGAAAACGAG | 525 |
| 54790_4_1712 | TET2 | EXON | - | chr4:105235651 -105235671 | AUUCCGCUUGGUGAAAACGA | 526 |
| 54790_4_1713 | TET2 | EXON | - | chr4:105235652 -105235672 | GAUUCCGCUUGGUGAAAACG | 527 |
| 54790_4_1722 | TET2 | EXON | - | chr4:105235663 -105235683 | GUUUUAGAUGGGAUUCCGCU | 528 |
| 54790_4_1723 | TET2 | EXON | - | chr4:105235674 -105235694 | UGCCUCAUUACGUUUUAGAU | 529 |
| 54790_4_1724 | TET2 | EXON | - | chr4:105235675 -105235695 | AUGCCUCAUUACGUUUUAGA | 530 |
| 54790_4_1730 | TET2 | EXON | - | chr4:105235703 -105235723 | GGUUGAUACUGAAGAAUUGA | 531 |
| 54790_4_1737 | TET2 | EXON | - | chr4:105235724 -105235744 | GUCAUUUGAUUGGAGAGAUU | 532 |
| 54790_4_1738 | TET2 | EXON | - | chr4:105235725 -105235745 | GGUCAUUUGAUUGGAGAGAU | 533 |
| 54790_4_1743 | TET2 | EXON | - | chr4:105235734 -105235754 | UUGUUUGGAGGUCAUUUGAU | 534 |
| 54790_4_1749 | TET2 | EXON | - | chr4:105235746 -105235766 | AUUUCCAGUGUAUUGUUUGG | 535 |
| 54790_4_1751 | TET2 | EXON | - | chr4:105235749 -105235769 | GGAAUUUCCAGUGUAUUGUU | 536 |
| 54790_4_1756 | TET2 | EXON | - | chr4:105235770 -105235790 | UGGGAGCCCCCCAGGCAUGU | 537 |
| 54790_4_1758 | TET2 | EXON | - | chr4:105235778 -105235798 | GCUUGCCUUGGGAGCCCCCC | 538 |
| 54790_4_1763 | TET2 | EXON | - | chr4:105235789 -105235809 | UCUGGGUGUAAGCUUGCCUU | 539 |
| 54790_4_1766 | TET2 | EXON | - | chr4:105235790 -105235810 | UUCUGGGUGUAAGCUUGCCU | 540 |
| 54790_4_1769 | TET2 | EXON | - | chr4:105235806 -105235826 | CUCCAGCUGUGUUGUUUUCU | 541 |
| 54790_4_1770 | TET2 | EXON | - | chr4:105235807 -105235827 | GCUCCAGCUGUGUUGUUUUC | 542 |
| 54790_4_1779 | TET2 | EXON | - | chr4:105235846 -105235866 | GCCCUUGAUUCAUUUCAACU | 543 |
| 54790_4_1782 | TET2 | EXON | - | chr4:105235872 -105235892 | AUGUUGGUCCACUGUACCUU | 544 |
| 54790_4_1783 | TET2 | EXON | - | chr4:105235873 -105235893 | GAUGUUGGUCCACUGUACCU | 545 |
| 54790_4_1790 | TET2 | EXON | - | chr4:105235888 -105235908 | GUUUUUGGAACUGGAGAUGU | 546 |
| 54790_4_1791 | TET2 | EXON | - | chr4:105235897 -105235917 | GGUGUGAGGGUUUUUGGAAC | 547 |
| 54790_4_1795 | TET2 | EXON | - | chr4:105235903 -105235923 | GCACCUGGUGUGAGGGUUUU | 548 |
| 54790_4_1800 | TET2 | EXON | - | chr4:105235910 -105235930 | GAGAAGUGCACCUGGUGUGA | 549 |
| 54790_4_1801 | TET2 | EXON | - | chr4:105235911 -105235931 | GGAGAAGUGCACCUGGUGUG | 550 |
| 54790_4_1804 | TET2 | EXON | - | chr4:105235918 -105235938 | CUGUUUUGGAGAAGUGCACC | 551 |
| 54790_4_1811 | TET2 | EXON | - | chr4:105235932 -105235952 | UUUUGGUAAAUGGUCUGUUU | 552 |
| 54790_4_1813 | TET2 | EXON | - | chr4:105235942 -105235962 | GCACAUGAGCUUUUGGUAAA | 553 |
| 54790_4_1814 | TET2 | EXON | - | chr4:105235949 -105235969 | AGUGACUGCACAUGAGCUUU | 554 |
| 54790_4_1828 | TET2 | EXON | - | chr4:105236010 -105236030 | GGACAUAAGUUUUUCAGUUU | 555 |
| 54790_4_1829 | TET2 | EXON | - | chr4:105236011 -105236031 | GGGACAUAAGUUUUUCAGUU | 556 |
| 54790_4_1836 | TET2 | EXON | - | chr4:105236031 -105236051 | CAAGUGCUGUUUCAACACUG | 557 |
| 54790_4_1838 | TET2 | EXON | - | chr4:105236032 -105236052 | UCAAGUGCUGUUUCAACACU | 558 |
| 54790_4_1839 | TET2 | EXON | - | chr4:105236033 -105236053 | UUCAAGUGCUGUUUCAACAC | 559 |
| 54790_4_1846 | TET2 | EXON | - | chr4:105236078 -105236098 | AAAAGGUGUGAGUUUGAAAA | 560 |
| 54790_4_1852 | TET2 | EXON | - | chr4:105236095 -105236115 | UAUGAGGCUUAUGUUGCAAA | 561 |
| 54790_4_1856 | TET2 | EXON | - | chr4:105236111 -105236131 | GUUUGUGCUGCCUGUUUAUG | 562 |
| 54790_4_1861 | TET2 | EXON | - | chr4:105236138 -105236158 | GGGAGAUGUGAACUCUGGGA | 563 |
| 54790_4_1862 | TET2 | EXON | - | chr4:105236142 -105236162 | UUGAGGGAGAUGUGAACUCU | 564 |
| 54790_4_1864 | TET2 | EXON | - | chr4:105236143 -105236163 | UUUGAGGGAGAUGUGAACUC | 565 |
| 54790_4_1873 | TET2 | EXON | - | chr4:105236158 -105236178 | GCUGCUGUUGCUGGUUUUGA | 566 |
| 54790_4_1875 | TET2 | EXON | - | chr4:105236159 -105236179 | UGCUGCUGUUGCUGGUUUUG | 567 |
| 54790_4_1880 | TET2 | EXON | - | chr4:105236167 -105236187 | GUAAUUUUUGCUGCUGUUGC | 568 |
| 54790_4_1892 | TET2 | EXON | - | chr4:105236215 -105236235 | UUUGGGGGUGAGGAAAAGUC | 569 |
| 54790_4_1896 | TET2 | EXON | - | chr4:105236225 -105236245 | UCAUUGUUGCUUUGGGGGUG | 570 |
| 54790_4_1901 | TET2 | EXON | - | chr4:105236230 -105236250 | GCUGAUCAUUGUUGCUUUGG | 571 |
| 54790_4_1902 | TET2 | EXON | - | chr4:105236231 -105236251 | UGCUGAUCAUUGUUGCUUUG | 572 |
| 54790_4_1904 | TET2 | EXON | - | chr4:105236232 -105236252 | UUGCUGAUCAUUGUUGCUUU | 573 |
| 54790_4_1906 | TET2 | EXON | - | chr4:105236233 -105236253 | UUUGCUGAUCAUUGUUGCUU | 574 |
| 54790_4_1914 | TET2 | EXON | - | chr4:105236275 -105236295 | AACAUUCUUCCACUUUAGUC | 575 |
| 54790_4_1931 | TET2 | EXON | - | chr4:105236350 -105236370 | GUACUUCCUCCAGUCCCAUU | 576 |
| 54790_4_1941 | TET2 | EXON | - | chr4:105236394 -105236414 | UUUCAUGGUCUGACUAUAAG | 577 |
| 54790_4_1943 | TET2 | EXON | - | chr4:105236395 -105236415 | AUUUCAUGGUCUGACUAUAA | 578 |
| 54790_4_1944 | TET2 | EXON | - | chr4:105236396 -105236416 | GAUUUCAUGGUCUGACUAUA | 579 |
| 54790_4_1950 | TET2 | EXON | - | chr4:105236409 -105236429 | UUUGCAUGCACUUGAUUUCA | 580 |
| 54790_4_1960 | TET2 | EXON | - | chr4:105236461 -105236481 | GUUCUUUAUUCUCUGAAACU | 581 |
| 54790_4_1966 | TET2 | EXON | - | chr4:105236495 -105236515 | UUGUUUCCUGCAAAAAGUUC | 582 |
| 54790_4_1972 | TET2 | EXON | - | chr4:105236520 -105236540 | UUGCAUGUGAUGCAAGUUUU | 583 |
| 54790_4_1973 | TET2 | EXON | - | chr4:105236521 -105236541 | AUUGCAUGUGAUGCAAGUUU | 584 |
| 54790_4_1982 | TET2 | EXON | - | chr4:105236549 -105236569 | UGCUUUGGGAUCACAUUAUU | 585 |
| 54790_4_1984 | TET2 | EXON | - | chr4:105236563 -105236583 | UGUGAAGAAGAUCUUGCUUU | 586 |
| 54790_4_1985 | TET2 | EXON | - | chr4:105236564 -105236584 | CUGUGAAGAAGAUCUUGCUU | 587 |
| 54790_4_2009 | TET2 | EXON | - | chr4:105236653 -105236673 | CUUGUUGACCAGACAUAUCU | 588 |
| 54790_4_2017 | TET2 | EXON | - | chr4:105236713 -105236733 | GCACAGGAAAAACAUUUGCA | 589 |
| 54790_4_2019 | TET2 | EXON | - | chr4:105236729 -105236749 | CUUCCUCCCUGGUCAGGCAC | 590 |
| 54790_4_2022 | TET2 | EXON | - | chr4:105236735 -105236755 | GUGUGACUUCCUCCCUGGUC | 591 |
| 54790_4_2023 | TET2 | EXON | - | chr4:105236740 -105236760 | UCUGAGUGUGACUUCCUCCC | 592 |
| 54790_4_2029 | TET2 | EXON | - | chr4:105236763 -105236783 | UUGAGUGUCCUUCUGGGGAG | 593 |
| 54790_4_2030 | TET2 | EXON | - | chr4:105236764 -105236784 | UUUGAGUGUCCUUCUGGGGA | 594 |
| 54790_4_2031 | TET2 | EXON | - | chr4:105236765 -105236785 | UUUUGAGUGUCCUUCUGGGG | 595 |
| 54790_4_2034 | TET2 | EXON | - | chr4:105236768 -105236788 | UGCUUUUGAGUGUCCUUCUG | 596 |
| 54790_4_2037 | TET2 | EXON | - | chr4:105236769 -105236789 | AUGCUUUUGAGUGUCCUUCU | 597 |
| 54790_4_2039 | TET2 | EXON | - | chr4:105236770 -105236790 | CAUGCUUUUGAGUGUCCUUC | 598 |
| 54790_4_2053 | TET2 | EXON | - | chr4:105236846 -105236866 | CUAUGGCAAGACUCAGUUUG | 599 |
| 54790_4_2054 | TET2 | EXON | - | chr4:105236847 -105236867 | ACUAUGGCAAGACUCAGUUU | 600 |
| 54790_4_2055 | TET2 | EXON | - | chr4:105236848 -105236868 | GACUAUGGCAAGACUCAGUU | 601 |
| 54790_4_2060 | TET2 | EXON | - | chr4:105236863 -105236883 | UUGGCCUGUGCAUCUGACUA | 602 |
| 54790_4_2063 | TET2 | EXON | - | chr4:105236882 -105236902 | CAUCCAGGUUCCACCUUAAU | 603 |
| 54790_4_2064 | TET2 | EXON | - | chr4:105236897 -105236917 | CAGGCAUGUGGCUUGCAUCC | 604 |
| 54790_4_2065 | TET2 | EXON | - | chr4:105236909 -105236929 | GCUGUGUGCAUACAGGCAUG | 605 |
| 54790_4_2069 | TET2 | EXON | - | chr4:105236916 -105236936 | UGGUGGUGCUGUGUGCAUAC | 606 |
| 54790_4_2077 | TET2 | EXON | - | chr4:105236933 -105236953 | UUCCAUGUUUUGUUUUCUGG | 607 |
| 54790_4_2079 | TET2 | EXON | - | chr4:105236936 -105236956 | UUUUUCCAUGUUUUGUUUUC | 608 |
| 54790_4_2085 | TET2 | EXON | - | chr4:105236978 -105236998 | ACAUUAUCACAGCUUGCAGG | 609 |
| 54790_4_2089 | TET2 | EXON | - | chr4:105236981 -105237001 | UGCACAUUAUCACAGCUUGC | 610 |
| 54790_4_2092 | TET2 | EXON | - | chr4:105237024 -105237044 | CUGCUUCAGAUGCUGCUCCA | 611 |
| 54790_4_2096 | TET2 | EXON | - | chr4:105237054 -105237074 | CUUAUGGUCAAAUAACGACU | 612 |
| 54790_4_2099 | TET2 | EXON | - | chr4:105237070 -105237090 | AUUUGAGAGUAAGAGCCUUA | 613 |
| 54790_4_2112 | TET2 | EXON | - | chr4:105237125 -105237145 | UGUCUAGUCAAAACUGUGAC | 614 |
| 54790_4_2114 | TET2 | EXON | - | chr4:105237150 -105237170 | GCUAUCAAGUUCUGCAGCAG | 615 |
| 54790_4_2118 | TET2 | EXON | - | chr4:105237172 -105237192 | GCUGCUCUAAAGCUGGGGUG | 616 |
| 54790_4_2119 | TET2 | EXON | - | chr4:105237177 -105237197 | UGUUUGCUGCUCUAAAGCUG | 617 |
| 54790_4_2120 | TET2 | EXON | - | chr4:105237178 -105237198 | UUGUUUGCUGCUCUAAAGCU | 618 |
| 54790_4_2122 | TET2 | EXON | - | chr4:105237179 -105237199 | GUUGUUUGCUGCUCUAAAGC | 619 |
| 54790_4_2135 | TET2 | EXON | - | chr4:105237218 -105237238 | GAAGCAGCUGUUCUUUUGGU | 620 |
| 54790_4_2137 | TET2 | EXON | - | chr4:105237222 -105237242 | AACAGAAGCAGCUGUUCUUU | 621 |
| 54790_4_2148 | TET2 | EXON | - | chr4:105237266 -105237286 | GGAGUAUCUAGUAAUUUGGA | 622 |
| 54790_4_2153 | TET2 | EXON | - | chr4:105237270 -105237290 | UAUAGGAGUAUCUAGUAAUU | 623 |
| 54790_4_2156 | TET2 | EXON | - | chr4:105237287 -105237307 | GUAUCCAAUAAAUUUUUUAU | 624 |
| 54790_4_2160 | TET2 | EXON | - | chr4:105237311 -105237331 | AAAUCAUAUUGAGUCUUGAC | 625 |
| 54790_4_2163 | TET2 | EXON | - | chr4:105237334 -105237354 | UACCUACACAUCUGCAAGAU | 626 |
| 54790_4_2165 | TET2 | EXON | - | chr4:105237335 -105237355 | UUACCUACACAUCUGCAAGA | 627 |
| 54790_4_2170 | TET2 | EXON | - | chr4:105237361 -105237381 | CAUGUGUCUCAGUACAUUUC | 628 |
| 54790_4_2174 | TET2 | EXON | - | chr4:105237392 -105237412 | GAAGAUAAAUUUGCUAAUUC | 629 |
| 54790_4_2180 | TET2 | EXON | - | chr4:105237429 -105237449 | ACUCAAGAUUUAAAAAAAGA | 630 |
| 54790_4_2197 | TET2 | EXON | - | chr4:105237510 -105237530 | CUUUCACAAGACACAAGCAU | 631 |
| 54790_4_2206 | TET2 | EXON | - | chr4:105237558 -105237578 | GCACGAUUAUUUAAUUCUUU | 632 |
| 54790_4_2213 | TET2 | EXON | - | chr4:105237593 -105237613 | UUUUACAGGAUCUGAAGAGA | 633 |
| 54790_4_2215 | TET2 | EXON | - | chr4:105237594 -105237614 | AUUUUACAGGAUCUGAAGAG | 634 |
| 54790_4_2221 | TET2 | EXON | - | chr4:105237607 -105237627 | CAGAUACAUUCAAAUUUUAC | 635 |
| 54790_4_2225 | TET2 | EXON | - | chr4:105237645 -105237665 | UAAUAUACAAAGAGCUAAAU | 636 |
| 54790_4_2233 | TET2 | EXON | - | chr4:105237671 -105237691 | UGCUGCCUAGCUGUCUCUCC | 637 |
| 54790_4_2247 | TET2 | EXON | - | chr4:105237727 -105237747 | UUCGUACAUUAGACUGCCUA | 638 |
| 54790_4_2270 | TET2 | EXON | - | chr4:105237874 -105237894 | AAUGGAGAAAAGGAAACUUU | 639 |
| 54790_4_2274 | TET2 | EXON | - | chr4:105237884 -105237904 | CAAAUGUAUAAAUGGAGAAA | 640 |
| 54790_4_2277 | TET2 | EXON | - | chr4:105237892 -105237912 | CAACAUUCCAAAUGUAUAAA | 641 |
| 54790_4_2284 | TET2 | EXON | - | chr4:105237936 -105237956 | AGAUGAAAUUUUAGAGAAAA | 642 |
| 54790_4_2287 | TET2 | EXON | - | chr4:105237937 -105237957 | AAGAUGAAAUUUUAGAGAAA | 643 |
| 54790_4_2323 | TET2 | EXON | - | chr4:105240511 -105240531 | AGGGAAAACAUGGCACGGGU | 644 |
| 54790_4_2325 | TET2 | EXON | - | chr4:105240515 -105240535 | CAAGAGGGAAAACAUGGCAC | 645 |
| 54790_4_2326 | TET2 | EXON | - | chr4:105240516 -105240536 | GCAAGAGGGAAAACAUGGCA | 646 |
| 54790_4_2328 | TET2 | EXON | - | chr4:105240521 -105240541 | UCAUUGCAAGAGGGAAAACA | 647 |
| 54790_4_2330 | TET2 | EXON | - | chr4:105240530 -105240550 | UGGGGUAUCUCAUUGCAAGA | 648 |
| 54790_4_2331 | TET2 | EXON | - | chr4:105240531 -105240551 | GUGGGGUAUCUCAUUGCAAG | 649 |
| 54790_4_2336 | TET2 | EXON | - | chr4:105240548 -105240568 | CCAUCCUUCUACACAGUGUG | 650 |
| 54790_4_2337 | TET2 | EXON | - | chr4:105240549 -105240569 | UCCAUCCUUCUACACAGUGU | 651 |
| 54790_4_2338 | TET2 | EXON | - | chr4:105240550 -105240570 | CUCCAUCCUUCUACACAGUG | 652 |
| 54790_4_2342 | TET2 | EXON | - | chr4:105240581 -105240601 | CACACGCAAAGAGGGACAGU | 653 |
| 54790_4_2345 | TET2 | EXON | - | chr4:105240589 -105240609 | UUAAUAACCACACGCAAAGA | 654 |
| 54790_4_2347 | TET2 | EXON | - | chr4:105240590 -105240610 | CUUAAUAACCACACGCAAAG | 655 |
| 54790_4_2353 | TET2 | EXON | - | chr4:105240616 -105240636 | UGUGGUGUUUUAGCCCAGUG | 656 |
| 54790_4_2357 | TET2 | EXON | - | chr4:105240634 -105240654 | AAUAUUAUCUAUGAGAUGUG | 657 |
| 54790_4_2365 | TET2 | EXON | - | chr4:105240693 -105240713 | AAAAGUGGGAAGAUAGGGGU | 658 |
| 54790_4_2366 | TET2 | EXON | - | chr4:105240694 -105240714 | GAAAAGUGGGAAGAUAGGGG | 659 |
| 54790_4_2368 | TET2 | EXON | - | chr4:105240697 -105240717 | AUGGAAAAGUGGGAAGAUAG | 660 |
| 54790_4_2369 | TET2 | EXON | - | chr4:105240698 -105240718 | GAUGGAAAAGUGGGAAGAUA | 661 |
| 54790_4_2370 | TET2 | EXON | - | chr4:105240699 -105240719 | AGAUGGAAAAGUGGGAAGAU | 662 |
| 54790_4_2373 | TET2 | EXON | - | chr4:105240707 -105240727 | ACCAACAAAGAUGGAAAAGU | 663 |
| 54790_4_2377 | TET2 | EXON | - | chr4:105240708 -105240728 | AACCAACAAAGAUGGAAAAG | 664 |
| 54790_4_2380 | TET2 | EXON | - | chr4:105240716 -105240736 | CUGUUGCAAACCAACAAAGA | 665 |
| 54790_4_2382 | TET2 | EXON | - | chr4:105240739 -105240759 | GAGAGUCAGGCAAAAAGAAG | 666 |
| 54790_4_2383 | TET2 | EXON | - | chr4:105240740 -105240760 | GGAGAGUCAGGCAAAAAGAA | 667 |
| 54790_4_2384 | TET2 | EXON | - | chr4:105240741 -105240761 | UGGAGAGUCAGGCAAAAAGA | 668 |
| 54790_4_2389 | TET2 | EXON | - | chr4:105240752 -105240772 | AGAGAAAAUCCUGGAGAGUC | 669 |
| 54790_4_2393 | TET2 | EXON | - | chr4:105240761 -105240781 | UUUAUGAUGAGAGAAAAUCC | 670 |
| 54790_4_2422 | TET2 | EXON | - | chr4:105240882 -105240902 | UAUUGCUUGGUUUAUUGUCA | 671 |
| 54790_4_2424 | TET2 | EXON | - | chr4:105240895 -105240915 | CCCACCCCCAGAAUAUUGCU | 672 |
| 54790_4_2434 | TET2 | EXON | - | chr4:105240954 -105240974 | CUGGAAGCCUACCUAUUACU | 673 |
| 54790_4_2439 | TET2 | EXON | - | chr4:105240973 -105240993 | AAAAAACAUUUAAAGCUAAC | 674 |
| 54790_4_2446 | TET2 | EXON | - | chr4:105241000 -105241020 | UACAAUCCAAUUUUUUGAGC | 675 |
| 54790_4_2454 | TET2 | EXON | - | chr4:105241052 -105241072 | CUAAACAAAGAAUACAGUGA | 676 |
| 54790_4_2456 | TET2 | EXON | - | chr4:105241053 -105241073 | ACUAAACAAAGAAUACAGUG | 677 |
| 54790_4_2468 | TET2 | EXON | - | chr4:105241107 -105241127 | AUAUAUUACAUUUCAGAUAU | 678 |
| 54790_4_2469 | TET2 | EXON | - | chr4:105241108 -105241128 | AAUAUAUUACAUUUCAGAUA | 679 |
| 54790_4_2475 | TET2 | EXON | - | chr4:105241136 -105241156 | UAUAUGUACAUGCUGGUUGU | 680 |
| 54790_4_2477 | TET2 | EXON | - | chr4:105241143 -105241163 | AAUUAAGUAUAUGUACAUGC | 681 |
| 54790_4_2488 | TET2 | EXON | - | chr4:105241193 -105241213 | CUUUAAAAUGAGUAGAUUGA | 682 |
| 54790_4_2498 | TET2 | EXON | - | chr4:105241245 -105241265 | AACCCCCAACUACACAUUAA | 683 |
| 54790_4_2499 | TET2 | EXON | - | chr4:105241246 -105241266 | UAACCCCCAACUACACAUUA | 684 |
| 54790_4_2503 | TET2 | EXON | - | chr4:105241285 -105241305 | CUCAAUUAUACUAAAUAUAA | 685 |
| 54790_4_2519 | TET2 | EXON | - | chr4:105241367 -105241387 | GCUCCUAGAUGGGUAUAAAA | 686 |
| 54790_4_2522 | TET2 | EXON | - | chr4:105241377 -105241397 | AUUAGGACCUGCUCCUAGAU | 687 |
| 54790_4_2523 | TET2 | EXON | - | chr4:105241378 -105241398 | CAUUAGGACCUGCUCCUAGA | 688 |
| 54790_4_2527 | TET2 | EXON | - | chr4:105241394 -105241414 | UCUCUAAUAGCUGCCACAUU | 689 |
| 54790_4_2538 | TET2 | EXON | - | chr4:105241470 -105241490 | AAAAUUCUGACAUAUACAAA | 690 |
| 54790_4_2546 | TET2 | EXON | - | chr4:105241494 -105241514 | ACUGCUUUGUGUGUGAAGGC | 691 |
| 54790_4_2548 | TET2 | EXON | - | chr4:105241498 -105241518 | GUUUACUGCUUUGUGUGUGA | 692 |
| 54790_4_2555 | TET2 | EXON | - | chr4:105241568 -105241588 | AAUAGCACAGUGUGUAGUGU | 693 |
| 54790_4_2558 | TET2 | EXON | - | chr4:105241593 -105241613 | UGUCAAAUAUUGUGACUCUC | 694 |
| 54790_4_2563 | TET2 | EXON | - | chr4:105241647 -105241667 | UCUGGCAUCCAUCGCAAAGU | 695 |
| 54790_4_2564 | TET2 | EXON | - | chr4:105241648 -105241668 | UUCUGGCAUCCAUCGCAAAG | 696 |
| 54790_4_2568 | TET2 | EXON | - | chr4:105241665 -105241685 | CUGUUCAUGCCUGGGUUUUC | 697 |
| 54790_4_2569 | TET2 | EXON | - | chr4:105241673 -105241693 | GCCGAUUCCUGUUCAUGCCU | 698 |
| 54790_4_2570 | TET2 | EXON | - | chr4:105241674 -105241694 | GGCCGAUUCCUGUUCAUGCC | 699 |
| 54790_4_2573 | TET2 | EXON | - | chr4:105241695 -105241715 | CUUGUGGCUGGCAGCCUGGC | 700 |
| 54790_4_2574 | TET2 | EXON | - | chr4:105241699 -105241719 | GUACCUUGUGGCUGGCAGCC | 701 |
| 54790_4_2575 | TET2 | EXON | - | chr4:105241707 -105241727 | CUGUGCCAGUACCUUGUGGC | 702 |
| 54790_4_2577 | TET2 | EXON | - | chr4:105241711 -105241731 | GAGCCUGUGCCAGUACCUUG | 703 |
| 54790_4_2578 | TET2 | EXON | - | chr4:105241733 -105241753 | GCCAGAGUGGGACCUCUCGU | 704 |
| 54790_4_2582 | TET2 | EXON | - | chr4:105241745 -105241765 | UCAGGUGGGAAAGCCAGAGU | 705 |
| 54790_4_2585 | TET2 | EXON | - | chr4:105241746 -105241766 | AUCAGGUGGGAAAGCCAGAG | 706 |
| 54790_4_2591 | TET2 | EXON | - | chr4:105241759 -105241779 | UUGACACUUUAUUAUCAGGU | 707 |
| 54790_4_2595 | TET2 | EXON | - | chr4:105241760 -105241780 | UUUGACACUUUAUUAUCAGG | 708 |
| 54790_4_2598 | TET2 | EXON | - | chr4:105241763 -105241783 | UGCUUUGACACUUUAUUAUC | 709 |
| 54790_4_2609 | TET2 | EXON | - | chr4:105241819 -105241839 | ACUAGGUGAAUUUAAUUCAG | 710 |
| 54790_4_2613 | TET2 | EXON | - | chr4:105241836 -105241856 | AAGUACUCAUUUGCAACACU | 711 |
| 54790_4_2622 | TET2 | EXON | - | chr4:105241878 -105241898 | UCACACUUGCUCUCUUUUUA | 712 |
| 54790_4_2629 | TET2 | EXON | - | chr4:105241939 -105241959 | AUAGCGAAAAAAAAAAAAAA | 713 |
| 54790_4_2633 | TET2 | EXON | - | chr4:105241986 -105242006 | UCUUCUACAUGCAGGAGUAA | 714 |
| 54790_4_2635 | TET2 | EXON | - | chr4:105241994 -105242014 | CAUAAGAGUCUUCUACAUGC | 715 |
| 54790_4_2642 | TET2 | EXON | - | chr4:105242038 -105242058 | GCUGUAUAAAUUUAUAUGAA | 716 |
| 54790_4_2652 | TET2 | EXON | - | chr4:105242086 -105242106 | CUGCCUACCUCUUAAUGAAA | 717 |
| 54790_4_2663 | TET2 | EXON | - | chr4:105242173 -105242193 | AGAAAUGAAUAAUUUGGAAA | 718 |
| 54790_4_2665 | TET2 | EXON | - | chr4:105242179 -105242199 | UAAUUUAGAAAUGAAUAAUU | 719 |
| 54790_4_2679 | TET2 | EXON | - | chr4:105242236 -105242256 | GGAAAUUCACUAUUUCUGCC | 720 |
| 54790_4_2681 | TET2 | EXON | - | chr4:105242257 -105242277 | GUUGUUUUUUUUGGCACUUA | 721 |
| 54790_4_2683 | TET2 | EXON | - | chr4:105242258 -105242278 | UGUUGUUUUUUUUGGCACUU | 722 |
| 54790_4_2685 | TET2 | EXON | - | chr4:105242266 -105242286 | UGUUUUUUUGUUGUUUUUUU | 723 |
| 54790_4_2694 | TET2 | EXON | - | chr4:105242360 -105242380 | AUCCAGCCACAUGGAAAAUA | 724 |
| 54790_4_2697 | TET2 | EXON | - | chr4:105242369 -105242389 | AUAGUUAGUAUCCAGCCACA | 725 |
| 54790_4_2701 | TET2 | EXON | - | chr4:105242395 -105242415 | CACAAUUUAGAAAAGGAGGC | 726 |
| 54790_4_2702 | TET2 | EXON | - | chr4:105242399 -105242419 | GUCUCACAAUUUAGAAAAGG | 727 |
| 54790_4_2703 | TET2 | EXON | - | chr4:105242402 -105242422 | AAUGUCUCACAAUUUAGAAA | 728 |
| 54790_4_2721 | TET2 | EXON | - | chr4:105242462 -105242482 | UUUAGCUAUUUUAAAACUUG | 729 |
| 54790_4_2723 | TET2 | EXON | - | chr4:105242463 -105242483 | AUUUAGCUAUUUUAAAACUU | 730 |
| 54790_4_2726 | TET2 | EXON | - | chr4:105242464 -105242484 | AAUUUAGCUAUUUUAAAACU | 731 |
| 54790_4_2742 | TET2 | EXON | - | chr4:105242539 -105242559 | UUUCACAAAGCACAAAAUUC | 732 |
| 54790_4_2749 | TET2 | EXON | - | chr4:105242583 -105242603 | AAUUACAUGUGGGUGAAAAU | 733 |
| 54790_4_2752 | TET2 | EXON | - | chr4:105242584 -105242604 | AAAUUACAUGUGGGUGAAAA | 734 |
| 54790_4_2755 | TET2 | EXON | - | chr4:105242593 -105242613 | CUAUUUUGUAAAUUACAUGU | 735 |
| 54790_4_2756 | TET2 | EXON | - | chr4:105242594 -105242614 | ACUAUUUUGUAAAUUACAUG | 736 |
| 54790_4_2769 | TET2 | EXON | - | chr4:105242669 -105242689 | ACGCCAAGCAAACUGGAUAA | 737 |
| 54790_4_2772 | TET2 | EXON | - | chr4:105242676 -105242696 | CAGGUCUACGCCAAGCAAAC | 738 |
| 54790_4_2780 | TET2 | EXON | - | chr4:105242695 -105242715 | CGGUUUAUUAUUUUUUAAAC | 739 |
| 54790_4_2781 | TET2 | EXON | - | chr4:105242715 -105242735 | ACCACAUCCUGAGAAAUGAA | 740 |
| 54790_5_3 | TET2 | EXON | + | chr4:105242816 -105242836 | CUGUGGGUUUCUUUAAGGUU | 741 |
| 54790_5_7 | TET2 | EXON | + | chr4:105242824 -105242844 | UUCUUUAAGGUUUGGACAGA | 742 |
| 54790_5_8 | TET2 | EXON | + | chr4:105242825 -105242845 | UCUUUAAGGUUUGGACAGAA | 743 |
| 54790_5_15 | TET2 | EXON | + | chr4:105242838 -105242858 | GACAGAAGGGUAAAGCUAUU | 744 |
| 54790_5_20 | TET2 | EXON | + | chr4:105242861 -105242881 | AUUGAAAGAGUCAUCUAUAC | 745 |
| 54790_5_23 | TET2 | EXON | + | chr4:105242870 -105242890 | GUCAUCUAUACUGGUAAAGA | 746 |
| 54790_5_26 | TET2 | EXON | + | chr4:105242884 -105242904 | UAAAGAAGGCAAAAGUUCUC | 747 |
| 54790_5_27 | TET2 | EXON | + | chr4:105242885 -105242905 | AAAGAAGGCAAAAGUUCUCA | 748 |
| 54790_5_30 | TET2 | EXON | + | chr4:105242904 -105242924 | AGGGAUGUCCUAUUGCUAAG | 749 |
| 54790_5_31 | TET2 | EXON | + | chr4:105242905 -105242925 | GGGAUGUCCUAUUGCUAAGU | 750 |
| 54790_5_51 | TET2 | EXON | - | chr4:105242915 -105242935 | ACACUUACCCACUUAGCAAU | 751 |
| 54790_6_1 | TET2 | EXON | + | chr4:105243550 -105243570 | GGAAUGGUGAUCCACGCAGG | 752 |
| 54790_6_7 | TET2 | EXON | + | chr4:105243589 -105243609 | UGAAGAGAAGCUACUGUGUU | 753 |
| 54790_6_9 | TET2 | EXON | + | chr4:105243594 -105243614 | AGAAGCUACUGUGUUUGGUG | 754 |
| 54790_6_12 | TET2 | EXON | + | chr4:105243595 -105243615 | GAAGCUACUGUGUUUGGUGC | 755 |
| 54790_6_14 | TET2 | EXON | + | chr4:105243605 -105243625 | UGUUUGGUGCGGGAGCGAGC | 756 |
| 54790_6_18 | TET2 | EXON | + | chr4:105243619 -105243639 | GCGAGCUGGCCACACCUGUG | 757 |
| 54790_6_19 | TET2 | EXON | + | chr4:105243646 -105243666 | AGUGAUUGUGAUUCUCAUCC | 758 |
| 54790_6_21 | TET2 | EXON | + | chr4:105243651 -105243671 | UUGUGAUUCUCAUCCUGGUG | 759 |
| 54790_6_24 | TET2 | EXON | + | chr4:105243652 -105243672 | UGUGAUUCUCAUCCUGGUGU | 760 |
| 54790_6_27 | TET2 | EXON | + | chr4:105243656 -105243676 | AUUCUCAUCCUGGUGUGGGA | 761 |
| 54790_6_30 | TET2 | EXON | + | chr4:105243673 -105243693 | GGAAGGAAUCCCGCUGUCUC | 762 |
| 54790_6_32 | TET2 | EXON | + | chr4:105243691 -105243711 | UCUGGCUGACAAACUCUACU | 763 |
| 54790_6_37 | TET2 | EXON | + | chr4:105243711 -105243731 | CGGAGCUUACCGAGACGCUG | 764 |
| 54790_6_39 | TET2 | EXON | + | chr4:105243719 -105243739 | ACCGAGACGCUGAGGAAAUA | 765 |
| 54790_6_41 | TET2 | EXON | + | chr4:105243738 -105243758 | ACGGCACGCUCACCAAUCGC | 766 |
| 54790_6_48 | TET2 | EXON | + | chr4:105243771 -105243791 | AUGAAGAGUAAGUGAAGCCC | 767 |
| 54790_6_49 | TET2 | EXON | + | chr4:105243772 -105243792 | UGAAGAGUAAGUGAAGCCCA | 768 |
| 54790_6_51 | TET2 | EXON | - | chr4:105243564 -105243584 | GCUUCUGCGAACCACCUGCG | 769 |
| 54790_6_56 | TET2 | EXON | - | chr4:105243631 -105243651 | UCACUGCAGCCUCACAGGUG | 770 |
| 54790_6_57 | TET2 | EXON | - | chr4:105243636 -105243656 | CACAAUCACUGCAGCCUCAC | 771 |
| 54790_6_62 | TET2 | EXON | - | chr4:105243667 -105243687 | GCGGGAUUCCUUCCCACACC | 772 |
| 54790_6_66 | TET2 | EXON | - | chr4:105243685 -105243705 | GUUUGUCAGCCAGAGACAGC | 773 |
| 54790_6_67 | TET2 | EXON | - | chr4:105243686 -105243706 | AGUUUGUCAGCCAGAGACAG | 774 |
| 54790_6_75 | TET2 | EXON | - | chr4:105243723 -105243743 | GCCGUAUUUCCUCAGCGUCU | 775 |
| 54790_6_80 | TET2 | EXON | - | chr4:105243753 -105243773 | AUUCAAGGCACACCGGCGAU | 776 |
| 54790_6_82 | TET2 | EXON | - | chr4:105243760 -105243780 | ACUCUUCAUUCAAGGCACAC | 777 |
| 54790_6_84 | TET2 | EXON | - | chr4:105243768 -105243788 | CUUCACUUACUCUUCAUUCA | 778 |
| 54790_7_10 | TET2 | EXON | + | chr4:105259615 -105259635 | CAGGAGAACUUGCGCCUGUC | 779 |
| 54790_7_12 | TET2 | EXON | + | chr4:105259616 -105259636 | AGGAGAACUUGCGCCUGUCA | 780 |
| 54790_7_14 | TET2 | EXON | + | chr4:105259617 -105259637 | GGAGAACUUGCGCCUGUCAG | 781 |
| 54790_7_16 | TET2 | EXON | + | chr4:105259621 -105259641 | AACUUGCGCCUGUCAGGGGC | 782 |
| 54790_7_20 | TET2 | EXON | + | chr4:105259637 -105259657 | GGGCUGGAUCCAGAAACCUG | 783 |
| 54790_7_21 | TET2 | EXON | + | chr4:105259655 -105259675 | UGUGGUGCCUCCUUCUCUUU | 784 |
| 54790_7_23 | TET2 | EXON | + | chr4:105259665 -105259685 | CCUUCUCUUUUGGUUGUUCA | 785 |
| 54790_7_31 | TET2 | EXON | + | chr4:105259682 -105259702 | UCAUGGAGCAUGUACUACAA | 786 |
| 54790_7_35 | TET2 | EXON | + | chr4:105259713 -105259733 | UUGCCAGAAGCAAGAUCCCA | 787 |
| 54790_7_41 | TET2 | EXON | + | chr4:105259730 -105259750 | CCAAGGAAGUUUAAGCUGCU | 788 |
| 54790_7_42 | TET2 | EXON | + | chr4:105259731 -105259751 | CAAGGAAGUUUAAGCUGCUU | 789 |
| 54790_7_44 | TET2 | EXON | + | chr4:105259732 -105259752 | AAGGAAGUUUAAGCUGCUUG | 790 |
| 54790_7_48 | TET2 | EXON | + | chr4:105259747 -105259767 | GCUUGGGGAUGACCCAAAAG | 791 |
| 54790_7_53 | TET2 | EXON | - | chr4:105259632 -105259652 | UUCUGGAUCCAGCCCCUGAC | 792 |
| 54790_7_54 | TET2 | EXON | - | chr4:105259649 -105259669 | AAGGAGGCACCACAGGUUUC | 793 |
| 54790_7_56 | TET2 | EXON | - | chr4:105259656 -105259676 | AAAAGAGAAGGAGGCACCAC | 794 |
| 54790_7_57 | TET2 | EXON | - | chr4:105259665 -105259685 | UGAACAACCAAAAGAGAAGG | 795 |
| 54790_7_58 | TET2 | EXON | - | chr4:105259668 -105259688 | CCAUGAACAACCAAAAGAGA | 796 |
| 54790_7_72 | TET2 | EXON | - | chr4:105259719 -105259739 | CUUCCUUGGGAUCUUGCUUC | 797 |
| 54790_7_73 | TET2 | EXON | - | chr4:105259732 -105259752 | CAAGCAGCUUAAACUUCCUU | 798 |
| 54790_7_74 | TET2 | EXON | - | chr4:105259733 -105259753 | CCAAGCAGCUUAAACUUCCU | 799 |
| 54790_7_80 | TET2 | EXON | - | chr4:105259762 -105259782 | GAAGUAAACAAACCUCUUUU | 800 |
| 54790_7_81 | TET2 | EXON | - | chr4:105259763 -105259783 | GGAAGUAAACAAACCUCUUU | 801 |
| 54790_8_8 | TET2 | EXON | + | chr4:105261748 -105261768 | CUUUAUACAGGAAGAGAAAC | 802 |
| 54790_8_12 | TET2 | EXON | + | chr4:105261781 -105261801 | GCAAAACCUGUCCACUCUUA | 803 |
| 54790_8_18 | TET2 | EXON | + | chr4:105261826 -105261846 | ACCUGAUGCAUAUAAUAAUC | 804 |
| 54790_8_27 | TET2 | EXON | - | chr4:105261790 -105261810 | UUGGUGCCAUAAGAGUGGAC | 805 |
| 54790_8_30 | TET2 | EXON | - | chr4:105261795 -105261815 | AUAUGUUGGUGCCAUAAGAG | 806 |
| 54790_8_34 | TET2 | EXON | - | chr4:105261809 -105261829 | GGUGCAAGUUUCUUAUAUGU | 807 |
| 54790_8_38 | TET2 | EXON | - | chr4:105261830 -105261850 | ACCUGAUUAUUAUAUGCAUC | 808 |
| 54790_9_14 | TET2 | EXON | + | chr4:105269623 -105269643 | CAGAGCACCAGAGUGCCGUC | 809 |
| 54790_9_15 | TET2 | EXON | + | chr4:105269624 -105269644 | AGAGCACCAGAGUGCCGUCU | 810 |
| 54790_9_19 | TET2 | EXON | + | chr4:105269632 -105269652 | AGAGUGCCGUCUGGGUCUGA | 811 |
| 54790_9_20 | TET2 | EXON | + | chr4:105269636 -105269656 | UGCCGUCUGGGUCUGAAGGA | 812 |
| 54790_9_22 | TET2 | EXON | + | chr4:105269651 -105269671 | AAGGAAGGCCGUCCAUUCUC | 813 |
| 54790_9_24 | TET2 | EXON | + | chr4:105269652 -105269672 | AGGAAGGCCGUCCAUUCUCA | 814 |
| 54790_9_25 | TET2 | EXON | + | chr4:105269653 -105269673 | GGAAGGCCGUCCAUUCUCAG | 815 |
| 54790_9_27 | TET2 | EXON | + | chr4:105269668 -105269688 | CUCAGGGGUCACUGCAUGUU | 816 |
| 54790_9_35 | TET2 | EXON | + | chr4:105269714 -105269734 | GACUUGCACAACAUGCAGAA | 817 |
| 54790_9_37 | TET2 | EXON | + | chr4:105269725 -105269745 | CAUGCAGAAUGGCAGCACAU | 818 |
| 54790_9_39 | TET2 | EXON | + | chr4:105269733 -105269753 | AUGGCAGCACAUUGGUAAGU | 819 |
| 54790_9_40 | TET2 | EXON | + | chr4:105269734 -105269754 | UGGCAGCACAUUGGUAAGUU | 820 |
| 54790_9_43 | TET2 | EXON | + | chr4:105269740 -105269760 | CACAUUGGUAAGUUGGGCUG | 821 |
| 54790_9_49 | TET2 | EXON | - | chr4:105269633 -105269653 | UUCAGACCCAGACGGCACUC | 822 |
| 54790_9_50 | TET2 | EXON | - | chr4:105269641 -105269661 | GGCCUUCCUUCAGACCCAGA | 823 |
| 54790_9_51 | TET2 | EXON | - | chr4:105269662 -105269682 | CAGUGACCCCUGAGAAUGGA | 824 |
| 54790_9_52 | TET2 | EXON | - | chr4:105269666 -105269686 | CAUGCAGUGACCCCUGAGAA | 825 |
| 54790_9_61 | TET2 | EXON | - | chr4:105269709 -105269729 | CAUGUUGUGCAAGUCUCUGU | 826 |
| 54790_9_62 | TET2 | EXON | - | chr4:105269710 -105269730 | GCAUGUUGUGCAAGUCUCUG | 827 |
| 54790_10_10 | TET2 | EXON | + | chr4:105272578 -105272598 | AGAGAAGACAAUCGAGAAUU | 828 |
| 54790_10_13 | TET2 | EXON | + | chr4:105272581 -105272601 | GAAGACAAUCGAGAAUUUGG | 829 |
| 54790_10_16 | TET2 | EXON | + | chr4:105272592 -105272612 | AGAAUUUGGAGGAAAACCUG | 830 |
| 54790_10_23 | TET2 | EXON | + | chr4:105272637 -105272657 | UUUAUACAAAGUCUCUGACG | 831 |
| 54790_10_29 | TET2 | EXON | + | chr4:105272647 -105272667 | GUCUCUGACGUGGAUGAGUU | 832 |
| 54790_10_30 | TET2 | EXON | + | chr4:105272648 -105272668 | UCUCUGACGUGGAUGAGUUU | 833 |
| 54790_10_33 | TET2 | EXON | + | chr4:105272655 -105272675 | CGUGGAUGAGUUUGGGAGUG | 834 |
| 54790_10_36 | TET2 | EXON | + | chr4:105272664 -105272684 | GUUUGGGAGUGUGGAAGCUC | 835 |
| 54790_10_40 | TET2 | EXON | + | chr4:105272667 -105272687 | UGGGAGUGUGGAAGCUCAGG | 836 |
| 54790_10_46 | TET2 | EXON | + | chr4:105272678 -105272698 | AAGCUCAGGAGGAGAAAAAA | 837 |
| 54790_10_48 | TET2 | EXON | + | chr4:105272683 -105272703 | CAGGAGGAGAAAAAACGGAG | 838 |
| 54790_10_49 | TET2 | EXON | + | chr4:105272694 -105272714 | AAAACGGAGUGGUGCCAUUC | 839 |
| 54790_10_51 | TET2 | EXON | + | chr4:105272711 -105272731 | UUCAGGUACUGAGUUCUUUU | 840 |
| 54790_10_55 | TET2 | EXON | + | chr4:105272723 -105272743 | GUUCUUUUCGGCGAAAAGUC | 841 |
| 54790_10_64 | TET2 | EXON | + | chr4:105272759 -105272779 | CAGUCAAGACUUGCCGACAA | 842 |
| 54790_10_71 | TET2 | EXON | + | chr4:105272805 -105272825 | AGCUGAAAAGCUUUCCUCCC | 843 |
| 54790_10_78 | TET2 | EXON | + | chr4:105272832 -105272852 | CAGCUCAAAUAAAAAUGAAA | 844 |
| 54790_10_81 | TET2 | EXON | + | chr4:105272880 -105272900 | ACAAACUGAAAACGCAAGCC | 845 |
| 54790_10_82 | TET2 | EXON | + | chr4:105272892 -105272912 | CGCAAGCCAGGCUAAACAGU | 846 |
| 54790_10_83 | TET2 | EXON | + | chr4:105272896 -105272916 | AGCCAGGCUAAACAGUUGGC | 847 |
| 54790_10_85 | TET2 | EXON | - | chr4:105272557 -105272577 | GUGAGAGUGCAUACCUGGUA | 848 |
| 54790_10_87 | TET2 | EXON | - | chr4:105272558 -105272578 | AGUGAGAGUGCAUACCUGGU | 849 |
| 54790_10_91 | TET2 | EXON | - | chr4:105272562 -105272582 | CUCUAGUGAGAGUGCAUACC | 850 |
| 54790_10_99 | TET2 | EXON | - | chr4:105272611 -105272631 | ACGUGAAGCUGCUCAUCCUC | 851 |
| 54790_10_105 | TET2 | EXON | - | chr4:105272638 -105272658 | ACGUCAGAGACUUUGUAUAA | 852 |
| 54790_10_114 | TET2 | EXON | - | chr4:105272711 -105272731 | AAAAGAACUCAGUACCUGAA | 853 |
| 54790_10_127 | TET2 | EXON | - | chr4:105272761 -105272781 | CUUUGUCGGCAAGUCUUGAC | 854 |
| 54790_10_132 | TET2 | EXON | - | chr4:105272775 -105272795 | UGGCUUCUAGUUUCCUUUGU | 855 |
| 54790_10_136 | TET2 | EXON | - | chr4:105272795 -105272815 | CUUUUCAGCUGCAGCUUUCU | 856 |
| 54790_10_145 | TET2 | EXON | - | chr4:105272822 -105272842 | AUUUGAGCUGUUCUCCAGGG | 857 |
| 54790_10_147 | TET2 | EXON | - | chr4:105272825 -105272845 | UUUAUUUGAGCUGUUCUCCA | 858 |
| 54790_10_150 | TET2 | EXON | - | chr4:105272826 -105272846 | UUUUAUUUGAGCUGUUCUCC | 859 |
| 54790_10_167 | TET2 | EXON | - | chr4:105272867 -105272887 | AGUUUGUUUUGUACGUGAUG | 860 |
| 54790_10_168 | TET2 | EXON | - | chr4:105272868 -105272888 | CAGUUUGUUUUGUACGUGAU | 861 |
| 54790_10_169 | TET2 | EXON | - | chr4:105272869 -105272889 | UCAGUUUGUUUUGUACGUGA | 862 |
| 54790_10_177 | TET2 | EXON | - | chr4:105272901 -105272921 | UACCUGCCAACUGUUUAGCC | 863 |
| 54790_11_9 | TET2 | EXON | + | chr4:105275178 -105275198 | GUCAACUCUUAUUCUGCUUC | 864 |
| 54790_11_14 | TET2 | EXON | + | chr4:105275203 -105275223 | CCACCAAUCCAUACAUGAGA | 865 |
| 54790_11_19 | TET2 | EXON | + | chr4:105275256 -105275276 | UCACACACUUCAGAUAUCUA | 866 |
| 54790 11 24 | TET2 | EXON | + | chr4:105275304 -105275324 | UCCACCUCAUCUCAAGCUGC | 867 |
| 54790_11_34 | TET2 | EXON | + | chr4:105275346 -105275366 | AAUCCCAUGAACCCUUACCC | 868 |
| 54790_11_35 | TET2 | EXON | + | chr4:105275347 -105275367 | AUCCCAUGAACCCUUACCCU | 869 |
| 54790_11_44 | TET2 | EXON | + | chr4:105275391 -105275411 | UAUCCAUCAUAUCAAUGCAA | 870 |
| 54790_11_47 | TET2 | EXON | + | chr4:105275405 -105275425 | AUGCAAUGGAAACCUAUCAG | 871 |
| 54790_11_49 | TET2 | EXON | + | chr4:105275426 -105275446 | GGACAACUGCUCCCCAUAUC | 872 |
| 54790_11_50 | TET2 | EXON | + | chr4:105275427 -105275447 | GACAACUGCUCCCCAUAUCU | 873 |
| 54790_11_53 | TET2 | EXON | + | chr4:105275456 -105275476 | UUCUCCCCAGUCUCAGCCGA | 874 |
| 54790_11_55 | TET2 | EXON | + | chr4:105275467 -105275487 | CUCAGCCGAUGGAUCUGUAU | 875 |
| 54790_11_56 | TET2 | EXON | + | chr4:105275533 -105275553 | UCCAUACACUUUACCAGCCA | 876 |
| 54790_11_59 | TET2 | EXON | + | chr4:105275538 -105275558 | ACACUUUACCAGCCAAGGUU | 877 |
| 54790_11_65 | TET2 | EXON | + | chr4:105275571 -105275591 | AGUUUUACAUCUAAAUACUU | 878 |
| 54790_11_68 | TET2 | EXON | + | chr4:105275577 -105275597 | ACAUCUAAAUACUUAGGUUA | 879 |
| 54790_11_74 | TET2 | EXON | + | chr4:105275594 -105275614 | UUAUGGAAACCAAAAUAUGC | 880 |
| 54790_11_77 | TET2 | EXON | + | chr4:105275595 -105275615 | UAUGGAAACCAAAAUAUGCA | 881 |
| 54790_11_79 | TET2 | EXON | + | chr4:105275601 -105275621 | AACCAAAAUAUGCAGGGAGA | 882 |
| 54790_11_85 | TET2 | EXON | + | chr4:105275643 -105275663 | AGACCAAAUGUACAUCAUGU | 883 |
| 54790_11_86 | TET2 | EXON | + | chr4:105275644 -105275664 | GACCAAAUGUACAUCAUGUA | 884 |
| 54790_11_92 | TET2 | EXON | + | chr4:105275675 -105275695 | UCCUUAUCCCACUCAUGAGA | 885 |
| 54790_11_93 | TET2 | EXON | + | chr4:105275679 -105275699 | UAUCCCACUCAUGAGAUGGA | 886 |
| 54790_11_96 | TET2 | EXON | + | chr4:105275690 -105275710 | UGAGAUGGAUGGCCACUUCA | 887 |
| 54790_11_99 | TET2 | EXON | + | chr4:105275691 -105275711 | GAGAUGGAUGGCCACUUCAU | 888 |
| 54790_11_104 | TET2 | EXON | + | chr4:105275735 -105275755 | CAAUCUGAGCAAUCCAAACA | 889 |
| 54790_11_105 | TET2 | EXON | + | chr4:105275748 -105275768 | CCAAACAUGGACUAUAAAAA | 890 |
| 54790_11_110 | TET2 | EXON | + | chr4:105275798 -105275818 | CCAUAACUACAGUGCAGCUC | 891 |
| 54790_11_111 | TET2 | EXON | + | chr4:105275799 -105275819 | CAUAACUACAGUGCAGCUCC | 892 |
| 54790_11_116 | TET2 | EXON | + | chr4:105275843 -105275863 | UGCCCUGCAUCUCCAAAACA | 893 |
| 54790_11_120 | TET2 | EXON | + | chr4:105275874 -105275894 | AUGCUUUCCCACACAGCUAA | 894 |
| 54790_11_121 | TET2 | EXON | + | chr4:105275875 -105275895 | UGCUUUCCCACACAGCUAAU | 895 |
| 54790_11_129 | TET2 | EXON | + | chr4:105275928 -105275948 | GAUAGAACUGCUUGUGUCCA | 896 |
| 54790_11_131 | TET2 | EXON | + | chr4:105275931 -105275951 | AGAACUGCUUGUGUCCAAGG | 897 |
| 54790_11_133 | TET2 | EXON | + | chr4:105275958 -105275978 | CACAAAUUAAGUGAUGCUAA | 898 |
| 54790_11_137 | TET2 | EXON | + | chr4:105275963 -105275983 | AUUAAGUGAUGCUAAUGGUC | 899 |
| 54790_11_139 | TET2 | EXON | + | chr4:105275978 -105275998 | UGGUCAGGAAAAGCAGCCAU | 900 |
| 54790_11_141 | TET2 | EXON | + | chr4:105275990 -105276010 | GCAGCCAUUGGCACUAGUCC | 901 |
| 54790_11_142 | TET2 | EXON | + | chr4:105275991 -105276011 | CAGCCAUUGGCACUAGUCCA | 902 |
| 54790_11_143 | TET2 | EXON | + | chr4:105275996 -105276016 | AUUGGCACUAGUCCAGGGUG | 903 |
| 54790_11_145 | TET2 | EXON | + | chr4:105276003 -105276023 | CUAGUCCAGGGUGUGGCUUC | 904 |
| 54790_11_148 | TET2 | EXON | + | chr4:105276011 -105276031 | GGGUGUGGCUUCUGGUGCAG | 905 |
| 54790_11_150 | TET2 | EXON | + | chr4:105276023 -105276043 | UGGUGCAGAGGACAACGAUG | 906 |
| 54790_11_152 | TET2 | EXON | + | chr4:105276028 -105276048 | CAGAGGACAACGAUGAGGUC | 907 |
| 54790_11_156 | TET2 | EXON | + | chr4:105276053 -105276073 | AGACAGCGAGCAGAGCUUUC | 908 |
| 54790_11_158 | TET2 | EXON | + | chr4:105276066 -105276086 | AGCUUUCUGGAUCCUGACAU | 909 |
| 54790_11_160 | TET2 | EXON | + | chr4:105276067 -105276087 | GCUUUCUGGAUCCUGACAUU | 910 |
| 54790_11_162 | TET2 | EXON | + | chr4:105276068 -105276088 | CUUUCUGGAUCCUGACAUUG | 911 |
| 54790_11_165 | TET2 | EXON | + | chr4:105276069 -105276089 | UUUCUGGAUCCUGACAUUGG | 912 |
| 54790_11_168 | TET2 | EXON | + | chr4:105276074 -105276094 | GGAUCCUGACAUUGGGGGAG | 913 |
| 54790_11_169 | TET2 | EXON | + | chr4:105276080 -105276100 | UGACAUUGGGGGAGUGGCCG | 914 |
| 54790_11_172 | TET2 | EXON | + | chr4:105276093 -105276113 | GUGGCCGUGGCUCCAACUCA | 915 |
| 54790_11_173 | TET2 | EXON | + | chr4:105276094 -105276114 | UGGCCGUGGCUCCAACUCAU | 916 |
| 54790_11_182 | TET2 | EXON | + | chr4:105276160 -105276180 | CCCCUUUAAAGAAUCCCAAU | 917 |
| 54790_11_186 | TET2 | EXON | + | chr4:105276175 -105276195 | CCAAUAGGAAUCACCCCACC | 918 |
| 54790_11_193 | TET2 | EXON | + | chr4:105276225 -105276245 | AGCAUGAAUGAGCCAAAACA | 919 |
| 54790_11_194 | TET2 | EXON | + | chr4:105276230 -105276250 | GAAUGAGCCAAAACAUGGCU | 920 |
| 54790_11_196 | TET2 | EXON | + | chr4:105276238 -105276258 | CAAAACAUGGCUUGGCUCUU | 921 |
| 54790_11_199 | TET2 | EXON | + | chr4:105276239 -105276259 | AAAACAUGGCUUGGCUCUUU | 922 |
| 54790_11_200 | TET2 | EXON | + | chr4:105276251 -105276271 | GGCUCUUUGGGAAGCCAAAA | 923 |
| 54790_11_210 | TET2 | EXON | + | chr4:105276275 -105276295 | UGAAAAAGCCCGUGAGAAAG | 924 |
| 54790_11_214 | TET2 | EXON | + | chr4:105276294 -105276314 | GAGGAAGAGUGUGAAAAGUA | 925 |
| 54790_11_217 | TET2 | EXON | + | chr4:105276324 -105276344 | UAUGUGCCUCAGAAAUCCCA | 926 |
| 54790_11_221 | TET2 | EXON | + | chr4:105276340 -105276360 | CCCAUGGCAAAAAAGUGAAA | 927 |
| 54790_11_223 | TET2 | EXON | + | chr4:105276341 -105276361 | CCAUGGCAAAAAAGUGAAAC | 928 |
| 54790_11_231 | TET2 | EXON | + | chr4:105276409 -105276429 | UCAUCAAGUCUCUUGCCGAA | 929 |
| 54790_11_236 | TET2 | EXON | + | chr4:105276466 -105276486 | CAUCUCCAUAUGCCUUCACU | 930 |
| 54790_11_237 | TET2 | EXON | + | chr4:105276467 -105276487 | AUCUCCAUAUGCCUUCACUC | 931 |
| 54790_11_239 | TET2 | EXON | + | chr4:105276474 -105276494 | UAUGCCUUCACUCGGGUCAC | 932 |
| 54790_11_240 | TET2 | EXON | + | chr4:105276475 -105276495 | AUGCCUUCACUCGGGUCACA | 933 |
| 54790_11_243 | TET2 | EXON | + | chr4:105276515 -105276535 | AUGAUAUCACCCCCUUUUGU | 934 |
| 54790_11_252 | TET2 | EXON | + | chr4:105276573 -105276593 | GUAGUAUAGUUCUCAUGACG | 935 |
| 54790_11_253 | TET2 | EXON | + | chr4:105276574 -105276594 | UAGUAUAGUUCUCAUGACGU | 936 |
| 54790_11_256 | TET2 | EXON | + | chr4:105276580 -105276600 | AGUUCUCAUGACGUGGGCAG | 937 |
| 54790_11_258 | TET2 | EXON | + | chr4:105276581 -105276601 | GUUCUCAUGACGUGGGCAGU | 938 |
| 54790_11_259 | TET2 | EXON | + | chr4:105276582 -105276602 | UUCUCAUGACGUGGGCAGUG | 939 |
| 54790_11_262 | TET2 | EXON | + | chr4:105276587 -105276607 | AUGACGUGGGCAGUGGGGAA | 940 |
| 54790_11_263 | TET2 | EXON | + | chr4:105276611 -105276631 | CACAGUAUUCAUGACAAAUG | 941 |
| 54790_11_265 | TET2 | EXON | + | chr4:105276614 -105276634 | AGUAUUCAUGACAAAUGUGG | 942 |
| 54790_11_267 | TET2 | EXON | + | chr4:105276615 -105276635 | GUAUUCAUGACAAAUGUGGU | 943 |
| 54790_11_271 | TET2 | EXON | + | chr4:105276646 -105276666 | CAGCUCACCAGCAACAAAAG | 944 |
| 54790_11_273 | TET2 | EXON | + | chr4:105276677 -105276697 | CCAUAGCACUUAAUUUUCAC | 945 |
| 54790_11_275 | TET2 | EXON | + | chr4:105276688 -105276708 | AAUUUUCACUGGCUCCCAAG | 946 |
| 54790_11_280 | TET2 | EXON | + | chr4:105276698 -105276718 | GGCUCCCAAGUGGUCACAGA | 947 |
| 54790_11_283 | TET2 | EXON | + | chr4:105276706 -105276726 | AGUGGUCACAGAUGGCAUCU | 948 |
| 54790_11_285 | TET2 | EXON | + | chr4:105276738 -105276758 | AAGCAUUCUAUGCAAAAAGA | 949 |
| 54790_11_288 | TET2 | EXON | + | chr4:105276741 -105276761 | CAUUCUAUGCAAAAAGAAGG | 950 |
| 54790_11_289 | TET2 | EXON | + | chr4:105276742 -105276762 | AUUCUAUGCAAAAAGAAGGU | 951 |
| 54790_11_291 | TET2 | EXON | + | chr4:105276743 -105276763 | UUCUAUGCAAAAAGAAGGUG | 952 |
| 54790_11_297 | TET2 | EXON | + | chr4:105276780 -105276800 | CAAUUUACAUUUUUAAACAC | 953 |
| 54790_11_302 | TET2 | EXON | + | chr4:105276792 -105276812 | UUAAACACUGGUUCUAUUAU | 954 |
| 54790_11_316 | TET2 | EXON | + | chr4:105276885 -105276905 | AUAUCAAGUUUGCAUAGUCA | 955 |
| 54790_11_321 | TET2 | EXON | + | chr4:105276925 -105276945 | UACUGUAGUAUUACAGUGAC | 956 |
| 54790_11_323 | TET2 | EXON | + | chr4:105276945 -105276965 | AGGAAUCUUAAAAUACCAUC | 957 |
| 54790_11_329 | TET2 | EXON | + | chr4:105276975 -105276995 | UAUAUGAUGUACUGAAAUAC | 958 |
| 54790_11_330 | TET2 | EXON | + | chr4:105276983 -105277003 | GUACUGAAAUACUGGAAUUA | 959 |
| 54790_11_344 | TET2 | EXON | + | chr4:105277042 -105277062 | UUAUUUAUCAAAAUAGCUAC | 960 |
| 54790_11_352 | TET2 | EXON | + | chr4:105277058 -105277078 | CUACAGGAAACAUGAAUAGC | 961 |
| 54790_11_356 | TET2 | EXON | + | chr4:105277078 -105277098 | AGGAAAACACUGAAUUUGUU | 962 |
| 54790_11_359 | TET2 | EXON | + | chr4:105277094 -105277114 | UGUUUGGAUGUUCUAAGAAA | 963 |
| 54790_11_367 | TET2 | EXON | + | chr4:105277108 -105277128 | AAGAAAUGGUGCUAAGAAAA | 964 |
| 54790_11_377 | TET2 | EXON | + | chr4:105277187 -105277207 | CUCCAGUGCCCUUGAAUAAU | 965 |
| 54790_11_378 | TET2 | EXON | + | chr4:105277188 -105277208 | UCCAGUGCCCUUGAAUAAUA | 966 |
| 54790_11_379 | TET2 | EXON | + | chr4:105277189 -105277209 | CCAGUGCCCUUGAAUAAUAG | 967 |
| 54790_11_393 | TET2 | EXON | + | chr4:105277255 -105277275 | CAAGCUUAGUUUUUAAAAUG | 968 |
| 54790_11_395 | TET2 | EXON | + | chr4:105277267 -105277287 | UUAAAAUGUGGACAUUUUAA | 969 |
| 54790_11_401 | TET2 | EXON | + | chr4:105277274 -105277294 | GUGGACAUUUUAAAGGCCUC | 970 |
| 54790_11_410 | TET2 | EXON | + | chr4:105277304 -105277324 | UCAUCCAGUGAAGUCCUUGU | 971 |
| 54790_11_419 | TET2 | EXON | + | chr4:105277438 -105277458 | UGACAACUUGAACAAUGCUA | 972 |
| 54790_11_437 | TET2 | EXON | + | chr4:105277501 -105277521 | AUGCAAAGUUGAUUUUUUUA | 973 |
| 54790_11_465 | TET2 | EXON | + | chr4:105277599 -105277619 | ACAGCCAGUUAAAUCCACCA | 974 |
| 54790_11_466 | TET2 | EXON | + | chr4:105277600 -105277620 | CAGCCAGUUAAAUCCACCAU | 975 |
| 54790_11_467 | TET2 | EXON | + | chr4:105277601 -105277621 | AGCCAGUUAAAUCCACCAUG | 976 |
| 54790_11_469 | TET2 | EXON | + | chr4:105277609 -105277629 | AAAUCCACCAUGGGGCUUAC | 977 |
| 54790_11_472 | TET2 | EXON | + | chr4:105277617 -105277637 | CAUGGGGCUUACUGGAUUCA | 978 |
| 54790_11_474 | TET2 | EXON | + | chr4:105277618 -105277638 | AUGGGGCUUACUGGAUUCAA | 979 |
| 54790_11_478 | TET2 | EXON | + | chr4:105277649 -105277669 | AGUCCACAAAACAUGUUUUC | 980 |
| 54790_11_492 | TET2 | EXON | + | chr4:105277753 -105277773 | AAGAAUUUUCUAUUAACUGC | 981 |
| 54790_11_503 | TET2 | EXON | + | chr4:105277818 -105277838 | CUGAAGCCUAUGCUAUUUUA | 982 |
| 54790_11_504 | TET2 | EXON | + | chr4:105277826 -105277846 | UAUGCUAUUUUAUGGAUCAU | 983 |
| 54790_11_511 | TET2 | EXON | + | chr4:105277846 -105277866 | AGGCUCUUCAGAGAACUGAA | 984 |
| 54790_11_524 | TET2 | EXON | + | chr4:105277924 -105277944 | UAAGUGUCCUCUUUAACAAG | 985 |
| 54790_11_532 | TET2 | EXON | + | chr4:105277963 -105277983 | CCUGCAUAAGAUGAAUAAAC | 986 |
| 54790_11_533 | TET2 | EXON | + | chr4:105277964 -105277984 | CUGCAUAAGAUGAAUAAACA | 987 |
| 54790_11_539 | TET2 | EXON | + | chr4:105278008 -105278028 | AGUUAAAAAGAAACAAAAAC | 988 |
| 54790_11_541 | TET2 | EXON | + | chr4:105278015 -105278035 | AAGAAACAAAAACAGGCAGC | 989 |
| 54790_11_542 | TET2 | EXON | + | chr4:105278025 -105278045 | AACAGGCAGCUGGUUUGCUG | 990 |
| 54790_11_543 | TET2 | EXON | + | chr4:105278028 -105278048 | AGGCAGCUGGUUUGCUGUGG | 991 |
| 54790_11_574 | TET2 | EXON | + | chr4:105278210 -105278230 | AAGCAGAAUUCACAUCAUGA | 992 |
| 54790_11_587 | TET2 | EXON | + | chr4:105278310 -105278330 | CAUAUACCUCAACACUAGUU | 993 |
| 54790_11_589 | TET2 | EXON | + | chr4:105278317 -105278337 | CUCAACACUAGUUUGGCAAU | 994 |
| 54790_11_627 | TET2 | EXON | + | chr4:105278467 -105278487 | CCUUUUUGUUCUAAAAAUUC | 995 |
| 54790_11_628 | TET2 | EXON | + | chr4:105278468 -105278488 | CUUUUUGUUCUAAAAAUUCA | 996 |
| 54790_11_637 | TET2 | EXON | + | chr4:105278532 -105278552 | UGUUUAUGUAAAAUUGUUGU | 997 |
| 54790_11_643 | TET2 | EXON | + | chr4:105278556 -105278576 | UAAUAAAUAUAUUCUUUGUC | 998 |
| 54790_11_645 | TET2 | EXON | + | chr4:105278557 -105278577 | AAUAAAUAUAUUCUUUGUCA | 999 |
| 54790_11_664 | TET2 | EXON | + | chr4:105278640 -105278660 | AACUAAUUUUGUAAAUCUGU | 1000 |
| 54790_11_679 | TET2 | EXON | + | chr4:105278680 -105278700 | AAAAGCAUUUUAAAAGUUUG | 1001 |
| 54790_11_686 | TET2 | EXON | + | chr4:105278704 -105278724 | AUCUUUUGACUGUUUCAAGC | 1002 |
| 54790_11_700 | TET2 | EXON | + | chr4:105278748 -105278768 | AGAAUGCACUGAGUUGAUAA | 1003 |
| 54790_11_701 | TET2 | EXON | + | chr4:105278749 -105278769 | GAAUGCACUGAGUUGAUAAA | 1004 |
| 54790_11_703 | TET2 | EXON | + | chr4:105278762 -105278782 | UGAUAAAGGGAAAAAUUGUA | 1005 |
| 54790_11_707 | TET2 | EXON | + | chr4:105278766 -105278786 | AAAGGGAAAAAUUGUAAGGC | 1006 |
| 54790_11_708 | TET2 | EXON | + | chr4:105278773 -105278793 | AAAAUUGUAAGGCAGGAGUU | 1007 |
| 54790_11_710 | TET2 | EXON | + | chr4:105278780 -105278800 | UAAGGCAGGAGUUUGGCAAG | 1008 |
| 54790_11_711 | TET2 | EXON | + | chr4:105278787 -105278807 | GGAGUUUGGCAAGUGGCUGU | 1009 |
| 54790_11_721 | TET2 | EXON | + | chr4:105278846 -105278866 | UUUGAUCCUGUAAUCACUGA | 1010 |
| 54790_11_728 | TET2 | EXON | + | chr4:105278862 -105278882 | CUGAAGGUACAUACUCCAUG | 1011 |
| 54790_11_729 | TET2 | EXON | + | chr4:105278878 -105278898 | CAUGUGGACUUCCCUUAAAC | 1012 |
| 54790_11_731 | TET2 | EXON | + | chr4:105278892 -105278912 | UUAAACAGGCAAACACCUAC | 1013 |
| 54790_11_733 | TET2 | EXON | + | chr4:105278897 -105278917 | CAGGCAAACACCUACAGGUA | 1014 |
| 54790_11_734 | TET2 | EXON | + | chr4:105278927 -105278947 | CAGAUUGUACAAUUACAUUU | 1015 |
| 54790₋11₋748 | TET2 | EXON | + | chr4:105278978 -105278998 | UAAAAUAAAUUCUUAAUCAG | 1016 |
| 54790_11_751 | TET2 | EXON | + | chr4:105278981 -105279001 | AAUAAAUUCUUAAUCAGAGG | 1017 |
| 54790_11_753 | TET2 | EXON | + | chr4:105278988 -105279008 | UCUUAAUCAGAGGAGGCCUU | 1018 |
| 54790_11_754 | TET2 | EXON | + | chr4:105278989 -105279009 | CUUAAUCAGAGGAGGCCUUU | 1019 |
| 54790_11_757 | TET2 | EXON | + | chr4:105278998 -105279018 | AGGAGGCCUUUGGGUUUUAU | 1020 |
| 54790_11_762 | TET2 | EXON | + | chr4:105279017 -105279037 | UUGGUCAAAUCUUUGUAAGC | 1021 |
| 54790_11_772 | TET2 | EXON | + | chr4:105279052 -105279072 | UAAAAAAUUUCUUGAAUUUG | 1022 |
| 54790_11_799 | TET2 | EXON | + | chr4:105279173 -105279193 | UUUGAUUACUACAUGUGCAU | 1023 |
| 54790_11_813 | TET2 | EXON | + | chr4:105279240 -105279260 | ACUGUCAUUUGUUAAACUGC | 1024 |
| 54790_11_818 | TET2 | EXON | + | chr4:105279254 -105279274 | AACUGCUGGCCAACAAGAAC | 1025 |
| 54790_11_822 | TET2 | EXON | + | chr4:105279267 -105279287 | CAAGAACAGGAAGUAUAGUU | 1026 |
| 54790_11_825 | TET2 | EXON | + | chr4:105279268 -105279288 | AAGAACAGGAAGUAUAGUUU | 1027 |
| 54790_11_827 | TET2 | EXON | + | chr4:105279269 -105279289 | AGAACAGGAAGUAUAGUUUG | 1028 |
| 54790_11_828 | TET2 | EXON | + | chr4:105279270 -105279290 | GAACAGGAAGUAUAGUUUGG | 1029 |
| 54790_11_829 | TET2 | EXON | + | chr4:105279271 -105279291 | AACAGGAAGUAUAGUUUGGG | 1030 |
| 54790_11_832 | TET2 | EXON | + | chr4:105279275 -105279295 | GGAAGUAUAGUUUGGGGGGU | 1031 |
| 54790_11_833 | TET2 | EXON | + | chr4:105279276 -105279296 | GAAGUAUAGUUUGGGGGGUU | 1032 |
| 54790_11_836 | TET2 | EXON | + | chr4:105279277 -105279297 | AAGUAUAGUUUGGGGGGUUG | 1033 |
| 54790_11_841 | TET2 | EXON | + | chr4:105279292 -105279312 | GGUUGGGGAGAGUUUACAUA | 1034 |
| 54790_11_851 | TET2 | EXON | + | chr4:105279311 -105279331 | AAGGAAGAGAAGAAAUUGAG | 1035 |
| 54790_11_859 | TET2 | EXON | + | chr4:105279373 -105279393 | CCUGCCUCAGUUAGAAUGAA | 1036 |
| 54790_11_864 | TET2 | EXON | + | chr4:105279402 -105279422 | GAUCUACAAUUUGCUAAUAU | 1037 |
| 54790_11_865 | TET2 | EXON | + | chr4:105279411 -105279431 | UUUGCUAAUAUAGGAAUAUC | 1038 |
| 54790_11_871 | TET2 | EXON | + | chr4:105279449 -105279469 | UACUUGAAAAUGCUUCUGAG | 1039 |
| 54790_11_886 | TET2 | EXON | + | chr4:105279524 -105279544 | CAGUUCACUUCUGAAGCUAG | 1040 |
| 54790_11_890 | TET2 | EXON | + | chr4:105279538 -105279558 | AGCUAGUGGUUAACUUGUGU | 1041 |
| 54790_11_912 | TET2 | EXON | + | chr4:105279632 -105279652 | UUUCAUUUUCAUGAGAUGUU | 1042 |
| 54790_11_920 | TET2 | EXON | + | chr4:105279648 -105279668 | UGUUUGGUUUAUAAGAUCUG | 1043 |
| 54790_11_921 | TET2 | EXON | + | chr4:105279652 -105279672 | UGGUUUAUAAGAUCUGAGGA | 1044 |
| 54790_11_928 | TET2 | EXON | + | chr4:105279691 -105279711 | UAUUGUAAUGUUAUGAAUGC | 1045 |
| 54790_11_954 | TET2 | EXON | - | chr4:105275038 -105275058 | UCGCAAAAGUUCUGUGGACA | 1046 |
| 54790_11_955 | TET2 | EXON | - | chr4:105275039 -105275059 | GUCGCAAAAGUUCUGUGGAC | 1047 |
| 54790_11_957 | TET2 | EXON | - | chr4:105275044 -105275064 | ACAAAGUCGCAAAAGUUCUG | 1048 |
| 54790_11_960 | TET2 | EXON | - | chr4:105275165 -105275185 | AGUUGACAGACUCUGUCUGA | 1049 |
| 54790_11_961 | TET2 | EXON | - | chr4:105275166 -105275186 | GAGUUGACAGACUCUGUCUG | 1050 |
| 54790_11_970 | TET2 | EXON | - | chr4:105275206 -105275226 | CCGUCUCAUGUAUGGAUUGG | 1051 |
| 54790_11_972 | TET2 | EXON | - | chr4:105275209 -105275229 | GGGCCGUCUCAUGUAUGGAU | 1052 |
| 54790_11_973 | TET2 | EXON | - | chr4:105275214 -105275234 | GGAUUGGGCCGUCUCAUGUA | 1053 |
| 54790_11_977 | TET2 | EXON | - | chr4:105275229 -105275249 | GGAUAAGGACUAACUGGAUU | 1054 |
| 54790_11_978 | TET2 | EXON | - | chr4:105275230 -105275250 | UGGAUAAGGACUAACUGGAU | 1055 |
| 54790_11_980 | TET2 | EXON | - | chr4:105275235 -105275255 | GAGUUUGGAUAAGGACUAAC | 1056 |
| 54790_11_982 | TET2 | EXON | - | chr4:105275244 -105275264 | GUGUGUGAAGAGUUUGGAUA | 1057 |
| 54790_11_984 | TET2 | EXON | - | chr4:105275250 -105275270 | UCUGAAGUGUGUGAAGAGUU | 1058 |
| 54790_11_991 | TET2 | EXON | - | chr4:105275287 -105275307 | GGAAUAGAAGUUCAUAGGGC | 1059 |
| 54790_11_992 | TET2 | EXON | - | chr4:105275291 -105275311 | AGGUGGAAUAGAAGUUCAUA | 1060 |
| 54790_11_993 | TET2 | EXON | - | chr4:105275292 -105275312 | GAGGUGGAAUAGAAGUUCAU | 1061 |
| 54790_11_999 | TET2 | EXON | - | chr4:105275308 -105275328 | ACCUGCAGCUUGAGAUGAGG | 1062 |
| 54790_11_1001 | TET2 | EXON | - | chr4:105275311 -105275331 | UGAACCUGCAGCUUGAGAUG | 1063 |
| 54790_11_1012 | TET2 | EXON | - | chr4:105275352 -105275372 | AGCCCAGGGUAAGGGUUCAU | 1064 |
| 54790_11_1013 | TET2 | EXON | - | chr4:105275353 -105275373 | AAGCCCAGGGUAAGGGUUCA | 1065 |
| 54790_11_1017 | TET2 | EXON | - | chr4:105275360 -105275380 | GAUUCAAAAGCCCAGGGUAA | 1066 |
| 54790_11_1018 | TET2 | EXON | - | chr4:105275361 -105275381 | UGAUUCAAAAGCCCAGGGUA | 1067 |
| 54790_11_1021 | TET2 | EXON | - | chr4:105275366 -105275386 | UAUUCUGAUUCAAAAGCCCA | 1068 |
| 54790_11_1022 | TET2 | EXON | - | chr4:105275367 -105275387 | GUAUUCUGAUUCAAAAGCCC | 1069 |
| 54790_11_1026 | TET2 | EXON | - | chr4:105275389 -105275409 | GCAUUGAUAUGAUGGAUAUU | 1070 |
| 54790_11_1027 | TET2 | EXON | - | chr4:105275390 -105275410 | UGCAUUGAUAUGAUGGAUAU | 1071 |
| 54790_11_1031 | TET2 | EXON | - | chr4:105275397 -105275417 | UUUCCAUUGCAUUGAUAUGA | 1072 |
| 54790_11_1034 | TET2 | EXON | - | chr4:105275420 -105275440 | GGGAGCAGUUGUCCACUGAU | 1073 |
| 54790_11_1035 | TET2 | EXON | - | chr4:105275440 -105275460 | AGAAUAGGAACCCAGAUAUG | 1074 |
| 54790_11_1037 | TET2 | EXON | - | chr4:105275441 -105275461 | GAGAAUAGGAACCCAGAUAU | 1075 |
| 54790_11_1040 | TET2 | EXON | - | chr4:105275442 -105275462 | GGAGAAUAGGAACCCAGAUA | 1076 |
| 54790_11_1042 | TET2 | EXON | - | chr4:105275455 -105275475 | CGGCUGAGACUGGGGAGAAU | 1077 |
| 54790_11_1046 | TET2 | EXON | - | chr4:105275463 -105275483 | AGAUCCAUCGGCUGAGACUG | 1078 |
| 54790_11_1049 | TET2 | EXON | - | chr4:105275464 -105275484 | CAGAUCCAUCGGCUGAGACU | 1079 |
| 54790_11_1050 | TET2 | EXON | - | chr4:105275465 -105275485 | ACAGAUCCAUCGGCUGAGAC | 1080 |
| 54790_11_1055 | TET2 | EXON | - | chr4:105275475 -105275495 | GGAUACCUAUACAGAUCCAU | 1081 |
| 54790_11_1058 | TET2 | EXON | - | chr4:105275496 -105275516 | UUAGACAGAGGGUCUUGGCU | 1082 |
| 54790_11_1060 | TET2 | EXON | - | chr4:105275501 -105275521 | UGAGCUUAGACAGAGGGUCU | 1083 |
| 54790_11_1061 | TET2 | EXON | - | chr4:105275507 -105275527 | GUAGACUGAGCUUAGACAGA | 1084 |
| 54790_11_1062 | TET2 | EXON | - | chr4:105275508 -105275528 | GGUAGACUGAGCUUAGACAG | 1085 |
| 54790_11_1067 | TET2 | EXON | - | chr4:105275529 -105275549 | UGGUAAAGUGUAUGGAUGGG | 1086 |
| 54790_11_1068 | TET2 | EXON | - | chr4:105275532 -105275552 | GGCUGGUAAAGUGUAUGGAU | 1087 |
| 54790_11_1069 | TET2 | EXON | - | chr4:105275533 -105275553 | UGGCUGGUAAAGUGUAUGGA | 1088 |
| 54790_11_1072 | TET2 | EXON | - | chr4:105275537 -105275557 | ACCUUGGCUGGUAAAGUGUA | 1089 |
| 54790_11_1075 | TET2 | EXON | - | chr4:105275549 -105275569 | GGCUAUUUCCAAACCUUGGC | 1090 |
| 54790_11_1076 | TET2 | EXON | - | chr4:105275553 -105275573 | CUCUGGCUAUUUCCAAACCU | 1091 |
| 54790_11_1079 | TET2 | EXON | - | chr4:105275570 -105275590 | AGUAUUUAGAUGUAAAACUC | 1092 |
| 54790_11_1085 | TET2 | EXON | - | chr4:105275606 -105275626 | AACCAUCUCCCUGCAUAUUU | 1093 |
| 54790_11_1089 | TET2 | EXON | - | chr4:105275641 -105275661 | AUGAUGUACAUUUGGUCUAA | 1094 |
| 54790_11_1092 | TET2 | EXON | - | chr4:105275649 -105275669 | UUCCCUACAUGAUGUACAUU | 1095 |
| 54790_11_1093 | TET2 | EXON | - | chr4:105275676 -105275696 | AUCUCAUGAGUGGGAUAAGG | 1096 |
| 54790_11_1095 | TET2 | EXON | - | chr4:105275679 -105275699 | UCCAUCUCAUGAGUGGGAUA | 1097 |
| 54790_11_1097 | TET2 | EXON | - | chr4:105275685 -105275705 | UGGCCAUCCAUCUCAUGAGU | 1098 |
| 54790_11_1098 | TET2 | EXON | - | chr4:105275686 -105275706 | GUGGCCAUCCAUCUCAUGAG | 1099 |
| 54790_11_1102 | TET2 | EXON | - | chr4:105275705 -105275725 | UAGAGGUGGCUCCCAUGAAG | 1100 |
| 54790_11_1105 | TET2 | EXON | - | chr4:105275719 -105275739 | AUUGGGUGGUAAUCUAGAGG | 1101 |
| 54790_11_1107 | TET2 | EXON | - | chr4:105275722 -105275742 | CAGAUUGGGUGGUAAUCUAG | 1102 |
| 54790_11_1111 | TET2 | EXON | - | chr4:105275733 -105275753 | UUUGGAUUGCUCAGAUUGGG | 1103 |
| 54790_11_1112 | TET2 | EXON | - | chr4:105275736 -105275756 | AUGUUUGGAUUGCUCAGAUU | 1104 |
| 54790_11_1113 | TET2 | EXON | - | chr4:105275737 -105275757 | CAUGUUUGGAUUGCUCAGAU | 1105 |
| 54790_11_1120 | TET2 | EXON | - | chr4:105275751 -105275771 | CCAUUUUUAUAGUCCAUGUU | 1106 |
| 54790_11_1125 | TET2 | EXON | - | chr4:105275787 -105275807 | UAGUUAUGGAUUAUGUGAGA | 1107 |
| 54790_11_1129 | TET2 | EXON | - | chr4:105275801 -105275821 | CCGGAGCUGCACUGUAGUUA | 1108 |
| 54790_11_1133 | TET2 | EXON | - | chr4:105275820 -105275840 | AGAGAGCUGUUGAACAUGCC | 1109 |
| 54790_11_1144 | TET2 | EXON | - | chr4:105275848 -105275868 | CUCCUUGUUUUGGAGAUGCA | 1110 |
| 54790_11_1145 | TET2 | EXON | - | chr4:105275849 -105275869 | UCUCCUUGUUUUGGAGAUGC | 1111 |
| 54790_11_1148 | TET2 | EXON | - | chr4:105275858 -105275878 | GCAUGUCAUUCUCCUUGUUU | 1112 |
| 54790_11_1154 | TET2 | EXON | - | chr4:105275884 -105275904 | UGAUAACCCAUUAGCUGUGU | 1113 |
| 54790_11_1155 | TET2 | EXON | - | chr4:105275885 -105275905 | UUGAUAACCCAUUAGCUGUG | 1114 |
| 54790_11_1161 | TET2 | EXON | - | chr4:105275916 -105275936 | GUUCUAUCAUGGUUAAGAGC | 1115 |
| 54790_11_1165 | TET2 | EXON | - | chr4:105275927 -105275947 | GGACACAAGCAGUUCUAUCA | 1116 |
| 54790_11_1169 | TET2 | EXON | - | chr4:105275948 -105275968 | UUAAUUUGUGUAAGCCUCCU | 1117 |
| 54790_11_1175 | TET2 | EXON | - | chr4:105275997 -105276017 | ACACCCUGGACUAGUGCCAA | 1118 |
| 54790_11_1176 | TET2 | EXON | - | chr4:105276011 -105276031 | CUGCACCAGAAGCCACACCC | 1119 |
| 54790_11_1182 | TET2 | EXON | - | chr4:105276081 -105276101 | ACGGCCACUCCCCCAAUGUC | 1120 |
| 54790_11_1186 | TET2 | EXON | - | chr4:105276100 -105276120 | UGACCCAUGAGUUGGAGCCA | 1121 |
| 54790_11_1188 | TET2 | EXON | - | chr4:105276108 -105276128 | AUGAGAAUUGACCCAUGAGU | 1122 |
| 54790_11_1200 | TET2 | EXON | - | chr4:105276157 -105276177 | GGGAUUCUUUAAAGGGGUUG | 1123 |
| 54790_11_1202 | TET2 | EXON | - | chr4:105276163 -105276183 | CCUAUUGGGAUUCUUUAAAG | 1124 |
| 54790_11_1203 | TET2 | EXON | - | chr4:105276164 -105276184 | UCCUAUUGGGAUUCUUUAAA | 1125 |
| 54790_11_1205 | TET2 | EXON | - | chr4:105276165 -105276185 | UUCCUAUUGGGAUUCUUUAA | 1126 |
| 54790_11_1207 | TET2 | EXON | - | chr4:105276177 -105276197 | CUGGUGGGGUGAUUCCUAUU | 1127 |
| 54790_11_1209 | TET2 | EXON | - | chr4:105276178 -105276198 | CCUGGUGGGGUGAUUCCUAU | 1128 |
| 54790_11_1211 | TET2 | EXON | - | chr4:105276191 -105276211 | AGACGAGGGAGAUCCUGGUG | 1129 |
| 54790_11_1212 | TET2 | EXON | - | chr4:105276192 -105276212 | AAGACGAGGGAGAUCCUGGU | 1130 |
| 54790_11_1214 | TET2 | EXON | - | chr4:105276193 -105276213 | AAAGACGAGGGAGAUCCUGG | 1131 |
| 54790_11_1216 | TET2 | EXON | - | chr4:105276196 -105276216 | GUAAAAGACGAGGGAGAUCC | 1132 |
| 54790_11_1219 | TET2 | EXON | - | chr4:105276205 -105276225 | CUUAUGCUGGUAAAAGACGA | 1133 |
| 54790_11_1221 | TET2 | EXON | - | chr4:105276206 -105276226 | UCUUAUGCUGGUAAAAGACG | 1134 |
| 54790_11_1228 | TET2 | EXON | - | chr4:105276218 -105276238 | GCUCAUUCAUGCUCUUAUGC | 1135 |
| 54790_11_1230 | TET2 | EXON | - | chr4:105276240 -105276260 | CAAAGAGCCAAGCCAUGUUU | 1136 |
| 54790_11_1241 | TET2 | EXON | - | chr4:105276268 -105276288 | ACGGGCUUUUUCAGCCAUUU | 1137 |
| 54790_11_1246 | TET2 | EXON | - | chr4:105276286 -105276306 | ACACUCUUCCUCUUUCUCAC | 1138 |
| 54790_11_1247 | TET2 | EXON | - | chr4:105276287 -105276307 | CACACUCUUCCUCUUUCUCA | 1139 |
| 54790_11_1251 | TET2 | EXON | - | chr4:105276320 -105276340 | AUUUCUGAGGCACAUAGUCU | 1140 |
| 54790_11_1252 | TET2 | EXON | - | chr4:105276321 -105276341 | GAUUUCUGAGGCACAUAGUC | 1141 |
| 54790_11_1260 | TET2 | EXON | - | chr4:105276333 -105276353 | UUUUUGCCAUGGGAUUUCUG | 1142 |
| 54790_11_1263 | TET2 | EXON | - | chr4:105276343 -105276363 | CCGUUUCACUUUUUUGCCAU | 1143 |
| 54790_11_1265 | TET2 | EXON | - | chr4:105276344 -105276364 | CCCGUUUCACUUUUUUGCCA | 1144 |
| 54790_11_1269 | TET2 | EXON | - | chr4:105276369 -105276389 | GAAGUUUCAUGUGGCUCAGC | 1145 |
| 54790_11_1270 | TET2 | EXON | - | chr4:105276378 -105276398 | GUGGGCUCUGAAGUUUCAUG | 1146 |
| 54790_11_1273 | TET2 | EXON | - | chr4:105276396 -105276416 | UUGAUGAAACGCAGGUAAGU | 1147 |
| 54790_11_1274 | TET2 | EXON | - | chr4:105276397 -105276417 | CUUGAUGAAACGCAGGUAAG | 1148 |
| 54790_11_1277 | TET2 | EXON | - | chr4:105276404 -105276424 | CAAGAGACUUGAUGAAACGC | 1149 |
| 54790_11_1281 | TET2 | EXON | - | chr4:105276427 -105276447 | GGUCACGGACAUGGUCCUUU | 1150 |
| 54790_11_1282 | TET2 | EXON | - | chr4:105276436 -105276456 | GGAGUCUGUGGUCACGGACA | 1151 |
| 54790_11_1284 | TET2 | EXON | - | chr4:105276442 -105276462 | UACUGUGGAGUCUGUGGUCA | 1152 |
| 54790_11_1286 | TET2 | EXON | - | chr4:105276448 -105276468 | UGUAGUUACUGUGGAGUCUG | 1153 |
| 54790_11_1288 | TET2 | EXON | - | chr4:105276457 -105276477 | AUAUGGAGAUGUAGUUACUG | 1154 |
| 54790_11_1290 | TET2 | EXON | - | chr4:105276474 -105276494 | GUGACCCGAGUGAAGGCAUA | 1155 |
| 54790_11_1294 | TET2 | EXON | - | chr4:105276481 -105276501 | AGGCCCUGUGACCCGAGUGA | 1156 |
| 54790_11_1297 | TET2 | EXON | - | chr4:105276501 -105276521 | UAUCAUAUAUAUCUGUUGUA | 1157 |
| 54790_11_1300 | TET2 | EXON | - | chr4:105276527 -105276547 | GUGAGGUAACCAACAAAAGG | 1158 |
| 54790_11_1301 | TET2 | EXON | - | chr4:105276528 -105276548 | AGUGAGGUAACCAACAAAAG | 1159 |
| 54790_11_1303 | TET2 | EXON | - | chr4:105276529 -105276549 | AAGUGAGGUAACCAACAAAA | 1160 |
| 54790_11_1305 | TET2 | EXON | - | chr4:105276530 -105276550 | CAAGUGAGGUAACCAACAAA | 1161 |
| 54790_11_1310 | TET2 | EXON | - | chr4:105276544 -105276564 | GGUUGUGGUCUUUUCAAGUG | 1162 |
| 54790_11_1312 | TET2 | EXON | - | chr4:105276559 -105276579 | UACUACUGACAGGUUGGUUG | 1163 |
| 54790_11_1313 | TET2 | EXON | - | chr4:105276565 -105276585 | GAACUAUACUACUGACAGGU | 1164 |
| 54790_11_1314 | TET2 | EXON | - | chr4:105276569 -105276589 | AUGAGAACUAUACUACUGAC | 1165 |
| 54790_11_1331 | TET2 | EXON | - | chr4:105276646 -105276666 | CUUUUGUUGCUGGUGAGCUG | 1166 |
| 54790_11_1334 | TET2 | EXON | - | chr4:105276656 -105276676 | AAGAUAACCUCUUUUGUUGC | 1167 |
| 54790_11_1336 | TET2 | EXON | - | chr4:105276680 -105276700 | CCAGUGAAAAUUAAGUGCUA | 1168 |
| 54790_11_1339 | TET2 | EXON | - | chr4:105276705 -105276725 | GAUGCCAUCUGUGACCACUU | 1169 |
| 54790_11_1344 | TET2 | EXON | - | chr4:105276706 -105276726 | AGAUGCCAUCUGUGACCACU | 1170 |
| 54790_11_1354 | TET2 | EXON | - | chr4:105276738 -105276758 | UCUUUUUGCAUAGAAUGCUU | 1171 |
| 54790_11_1363 | TET2 | EXON | - | chr4:105276780 -105276800 | GUGUUUAAAAAUGUAAAUUG | 1172 |
| 54790_11_1370 | TET2 | EXON | - | chr4:105276841 -105276861 | AGAGUUGUAAGCGGGGGGGG | 1173 |
| 54790_11_1371 | TET2 | EXON | - | chr4:105276842 -105276862 | UAGAGUUGUAAGCGGGGGGG | 1174 |
| 54790_11_1374 | TET2 | EXON | - | chr4:105276843 -105276863 | GUAGAGUUGUAAGCGGGGGG | 1175 |
| 54790_11_1376 | TET2 | EXON | - | chr4:105276844 -105276864 | UGUAGAGUUGUAAGCGGGGG | 1176 |
| 54790_11_1378 | TET2 | EXON | - | chr4:105276845 -105276865 | GUGUAGAGUUGUAAGCGGGG | 1177 |
| 54790_11_1379 | TET2 | EXON | - | chr4:105276846 -105276866 | UGUGUAGAGUUGUAAGCGGG | 1178 |
| 54790_11_1382 | TET2 | EXON | - | chr4:105276847 -105276867 | AUGUGUAGAGUUGUAAGCGG | 1179 |
| 54790_11_1383 | TET2 | EXON | - | chr4:105276848 -105276868 | GAUGUGUAGAGUUGUAAGCG | 1180 |
| 54790_11_1386 | TET2 | EXON | - | chr4:105276849 -105276869 | AGAUGUGUAGAGUUGUAAGC | 1181 |
| 54790_11_1388 | TET2 | EXON | - | chr4:105276850 -105276870 | CAGAUGUGUAGAGUUGUAAG | 1182 |
| 54790_11_1394 | TET2 | EXON | - | chr4:105276876 -105276896 | AAACUUGAUAUUAUUAAAAG | 1183 |
| 54790_11_1406 | TET2 | EXON | - | chr4:105276963 -105276983 | AUCAUAUAUUCAGCACCAGA | 1184 |
| 54790_11_1440 | TET2 | EXON | - | chr4:105277160 -105277180 | AUAGCAUCUUGAUGAUAUAA | 1185 |
| 54790_11_1444 | TET2 | EXON | - | chr4:105277192 -105277212 | CCCCUAUUAUUCAAGGGCAC | 1186 |
| 54790_11_1446 | TET2 | EXON | - | chr4:105277198 -105277218 | AAGGUACCCCUAUUAUUCAA | 1187 |
| 54790_11_1447 | TET2 | EXON | - | chr4:105277199 -105277219 | AAAGGUACCCCUAUUAUUCA | 1188 |
| 54790_11_1451 | TET2 | EXON | - | chr4:105277217 -105277237 | UGAUAAAAACUUGAAUGAAA | 1189 |
| 54790_11_1457 | TET2 | EXON | - | chr4:105277246 -105277266 | AACUAAGCUUGUGUAAGAAU | 1190 |
| 54790_11_1463 | TET2 | EXON | - | chr4:105277293 -105277313 | ACUGGAUGAGCAAAAUCCAG | 1191 |
| 54790_11_1469 | TET2 | EXON | - | chr4:105277311 -105277331 | UUGUCCUACAAGGACUUCAC | 1192 |
| 54790_11_1471 | TET2 | EXON | - | chr4:105277321 -105277341 | AUAUCGUUUAUUGUCCUACA | 1193 |
| 54790_11_1478 | TET2 | EXON | - | chr4:105277432 -105277452 | UGUUCAAGUUGUCAAAGCUU | 1194 |
| 54790_11_1501 | TET2 | EXON | - | chr4:105277563 -105277583 | AUUGCUCAUCAGCAGAUGCA | 1195 |
| 54790_11_1505 | TET2 | EXON | - | chr4:105277591 -105277611 | UUAACUGGCUGUGUUAAAAA | 1196 |
| 54790_11_1507 | TET2 | EXON | - | chr4:105277606 -105277626 | AGCCCCAUGGUGGAUUUAAC | 1197 |
| 54790_11_1509 | TET2 | EXON | - | chr4:105277616 -105277636 | GAAUCCAGUAAGCCCCAUGG | 1198 |
| 54790_11_1512 | TET2 | EXON | - | chr4:105277619 -105277639 | CUUGAAUCCAGUAAGCCCCA | 1199 |
| 54790_11_1517 | TET2 | EXON | - | chr4:105277655 -105277675 | GCACCAGAAAACAUGUUUUG | 1200 |
| 54790_11_1547 | TET2 | EXON | - | chr4:105277827 -105277847 | UAUGAUCCAUAAAAUAGCAU | 1201 |
| 54790_11_1553 | TET2 | EXON | - | chr4:105277879 -105277899 | GUACAUAAUUAUCAACACAA | 1202 |
| 54790_11_1558 | TET2 | EXON | - | chr4:105277934 -105277954 | GCUCAAUCCUCUUGUUAAAG | 1203 |
| 54790_11_1565 | TET2 | EXON | - | chr4:105277966 -105277986 | CCUGUUUAUUCAUCUUAUGC | 1204 |
| 54790_11_1574 | TET2 | EXON | - | chr4:105277996 -105278016 | UUUUAACUGACAGAUUCACA | 1205 |
| 54790_11_1613 | TET2 | EXON | - | chr4:105278246 -105278266 | CAUUAUGAUAUAUUUGUAGC | 1206 |
| 54790_11_1621 | TET2 | EXON | - | chr4:105278304 -105278324 | UGUUGAGGUAUAUGACAAGU | 1207 |
| 54790_11_1624 | TET2 | EXON | - | chr4:105278319 -105278339 | CUAUUGCCAAACUAGUGUUG | 1208 |
| 54790_11_1630 | TET2 | EXON | - | chr4:105278373 -105278393 | AAGGACUUGGAAAAAAAUGA | 1209 |
| 54790_11_1636 | TET2 | EXON | - | chr4:105278386 -105278406 | UAACAAUAAAAAAAAGGACU | 1210 |
| 54790_11_1643 | TET2 | EXON | - | chr4:105278392 -105278412 | UUUUUUUAACAAUAAAAAAA | 1211 |
| 54790_11_1647 | TET2 | EXON | - | chr4:105278423 -105278443 | AGAAAUCAAGUAUUGAAAAA | 1212 |
| 54790_11_1658 | TET2 | EXON | - | chr4:105278470 -105278490 | CCUGAAUUUUUAGAACAAAA | 1213 |
| 54790_11_1667 | TET2 | EXON | - | chr4:105278513 -105278533 | ACAGGUGACAUGUUGGCAUA | 1214 |
| 54790_11_1669 | TET2 | EXON | - | chr4:105278514 -105278534 | CACAGGUGACAUGUUGGCAU | 1215 |
| 54790_11_1674 | TET2 | EXON | - | chr4:105278520 -105278540 | CAUAAACACAGGUGACAUGU | 1216 |
| 54790_11_1675 | TET2 | EXON | - | chr4:105278531 -105278551 | CAACAAUUUUACAUAAACAC | 1217 |
| 54790_11_1682 | TET2 | EXON | - | chr4:105278589 -105278609 | AGAAGGGAUUCAAAAUAAAA | 1218 |
| 54790_11_1683 | TET2 | EXON | - | chr4:105278590 -105278610 | UAGAAGGGAUUCAAAAUAAA | 1219 |
| 54790_11_1685 | TET2 | EXON | - | chr4:105278605 -105278625 | CAUGUACAAGUAAAAUAGAA | 1220 |
| 54790_11_1687 | TET2 | EXON | - | chr4:105278606 -105278626 | ACAUGUACAAGUAAAAUAGA | 1221 |
| 54790_11_1733 | TET2 | EXON | - | chr4:105278813 -105278833 | GAGAGUUACAAGUAAGUCUC | 1222 |
| 54790_11_1739 | TET2 | EXON | - | chr4:105278855 -105278875 | UAUGUACCUUCAGUGAUUAC | 1223 |
| 54790_11_1746 | TET2 | EXON | - | chr4:105278880 -105278900 | CUGUUUAAGGGAAGUCCACA | 1224 |
| 54790_11_1749 | TET2 | EXON | - | chr4:105278892 -105278912 | GUAGGUGUUUGCCUGUUUAA | 1225 |
| 54790_11_1751 | TET2 | EXON | - | chr4:105278893 -105278913 | UGUAGGUGUUUGCCUGUUUA | 1226 |
| 54790_11_1754 | TET2 | EXON | - | chr4:105278910 -105278930 | CUGUUGCACACCAUACCUGU | 1227 |
| 54790_11_1758 | TET2 | EXON | - | chr4:105278953 -105278973 | UAGUAAGCAAAAAUGUAUUU | 1228 |
| 54790_11_1768 | TET2 | EXON | - | chr4:105279007 -105279027 | AUUUGACCAAUAAAACCCAA | 1229 |
| 54790_11_1784 | TET2 | EXON | - | chr4:105279084 -105279104 | UUUUGGAAAUGUUUGCAAAU | 1230 |
| 54790_11_1789 | TET2 | EXON | - | chr4:105279101 -105279121 | GUAAGCAAAGCAAACAUUUU | 1231 |
| 54790_11_1792 | TET2 | EXON | - | chr4:105279127 -105279147 | CAAAAAACAUUAAAAUCAUG | 1232 |
| 54790_11_1796 | TET2 | EXON | - | chr4:105279154 -105279174 | AUGUUUGGGGCUAGAUAUUA | 1233 |
| 54790_11_1797 | TET2 | EXON | - | chr4:105279167 -105279187 | AUGUAGUAAUCAAAUGUUUG | 1234 |
| 54790_11_1798 | TET2 | EXON | - | chr4:105279168 -105279188 | CAUGUAGUAAUCAAAUGUUU | 1235 |
| 54790_11_1800 | TET2 | EXON | - | chr4:105279169 -105279189 | ACAUGUAGUAAUCAAAUGUU | 1236 |
| 54790_11_1803 | TET2 | EXON | - | chr4:105279212 -105279232 | CAGAAAUCAAAUAUUAAGAA | 1237 |
| 54790_11_1809 | TET2 | EXON | - | chr4:105279240 -105279260 | GCAGUUUAACAAAUGACAGU | 1238 |
| 54790_11_1814 | TET2 | EXON | - | chr4:105279266 -105279286 | ACUAUACUUCCUGUUCUUGU | 1239 |
| 54790_11_1832 | TET2 | EXON | - | chr4:105279376 -105279396 | CCAUUCAUUCUAACUGAGGC | 1240 |
| 54790_11_1833 | TET2 | EXON | - | chr4:105279380 -105279400 | CUUUCCAUUCAUUCUAACUG | 1241 |
| 54790_11_1841 | TET2 | EXON | - | chr4:105279449 -105279469 | CUCAGAAGCAUUUUCAAGUA | 1242 |
| 54790_11_1877 | TET2 | EXON | - | chr4:105279748 -105279768 | AACACUCACAUAGCAUUAUC | 1243 |

### TALEN gene editing systems

TALENs are produced artificially by fusing a TAL effector DNA binding domain to a DNA cleavage domain. Transcription activator-like effects (TALEs) can be engineered to bind any desired DNA sequence, including a portion of the HLA or TCR gene. By combining an engineered TALE with a DNA cleavage domain, a restriction enzyme can be produced which is specific to any desired DNA sequence, including a HLA or TCR sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence.

To produce a TALEN, a TALE protein is fused to a nuclease (N), which is, for example, a wild-type or mutated FokI endonuclease. Several mutations to FokI have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) J. Mol. Biol. 200: 96.

The FokI domain functions as a dimer, requiring two constructs with unique DNA binding domains for sites in the target genome with proper orientation and spacing. Both the number of amino acid residues between the TALE DNA binding domain and the FokI cleavage domain and the number of bases between the two individual TALEN binding sites appear to be important parameters for achieving high levels of activity. Miller et al. (2011) Nature Biotech. 29: 143-8.

A Tet, e.g., Tet1, Tet2 and/or Tet3, e.g., Tet2, TALEN can be used inside a cell to produce a double-stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN, e.g., DNA encoding a CAR, e.g., as described herein; depending on the sequences of the foreign DNA and chromosomal sequence, this process can be used to integrate the DNA encoding the CAR, e.g., as described herein, at or near the site targeted by the TALEN. As shown herein, in the examples, but without being bound by theory, such integration may lead to the expression of the CAR as well as disruption of the Tet, e.g., Tet1, Tet2 and/or Tet3, e.g., Tet2, gene. Such foreign DNA molecule is referred to herein as "template DNA." In embodiments, the template DNA further comprises homology arms 5' to, 3' to, or both 5' and 3' to the nucleic acid of the template DNA which encodes the molecule or molecules of interest (e.g., which encodes a CAR described herein), wherein said homology arms are complementary to genomic DNA sequence flanking the target sequence.

TALENs specific to sequences in Tet, e.g., Tet1, Tet2 and/or Tet3, e.g., Tet2, can be constructed using any method known in the art, including various schemes using modular components. Zhang et al. (2011) Nature Biotech. 29: 149-53; Geibler et al. (2011) PLoS ONE 6: e19509; US 8,420,782 ; US 8,470,973, the contents of which are hereby incorproated by reference in their entirety.

### Zinc finger nuclease to inhibit Tet, e.g., Tet1, Tet2 and/or Tet3, e.g., Tet2

"ZFN" or "Zinc Finger Nuclease" refer to a zinc finger nuclease, an artificial nuclease which can be used to modify, e.g., delete one or more nucleic acids of, a desired nucleic acid sequence, e.g., Tet, e.g., Tet1, Tet2 and/or Tet3, e.g., Tet2,.

Like a TALEN, a ZFN comprises a FokI nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160.

A zinc finger is a small protein structural motif stabilized by one or more zinc ions. A zinc finger can comprise, for example, Cys2His2, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one-hybrid systems, bacterial one-hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5.

Also like a TALEN, a ZFN can create a double-stranded break in the DNA, which can create a frame-shift mutation if improperly repaired, leading to a decrease in the expression and amount of Tet, e.g., Tet1, Tet2 and/or Tet3, e.g., Tet2, in a cell. ZFNs can also be used with homologous recombination to mutate the Tet, e.g., Tet1, Tet2 and/or Tet3, e.g., Tet2, gene, or to introduce nucleic acid encoding a CAR at a site at or near the targeted sequence. As discussed above, the nucleci acid encoding a CAR may be introduced as part of a template DNA. In embodiments, the template DNA further comprises homology arms 5' to, 3' to, or both 5' and 3' to the nucleic acid of the template DNA which encodes the molecule or molecules of interest (e.g., which encodes a CAR described herein), wherein said homology arms are complementary to genomic DNA sequence flanking the target sequence.

ZFNs specific to sequences in the Tet, e.g., Tet1, Tet2 and/or Tet3, e.g., Tet2, gene can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; and Guo et al. (2010) J. Mol. Biol. 400: 96; U.S. Patent Publication 2011/0158957; and U.S. Patent Publication 2012/0060230, the contents of which are hereby incorporated by reference in their entirety. In embodiments, The ZFN gene editing system may also comprise nucleic acid encoding one or more components of the ZFN gene editing system, e.g., a ZFN gene editing system targeted to Tet, e.g., Tet1, Tet2 and/or Tet3, e.g., Tet2.

Without being bound by theory, it is believed that use of gene editing systems (e.g., CRISPR/Cas gene editing systems) which target Tet, e.g., Tet1, Tet2, and/or Tet3, e.g., Tet2, may allow one to inhibit one or more functions of Tet, e.g., Tet1, Tet2, and/or Tet3, e.g., Tet2, by, for example, causing an editing event which results in expression of a truncated Tet, e.g., Tet1, Tet2, and/or Tet3, e.g., Tet2. Again, without being bound by theory, such truncated Tet, e.g., Tet1, Tet2, and/or Tet3, e.g., Tet2 proteins may preserve one or more functions of the Tet, e.g., Tet1, Tet2, and/or Tet3, e.g., Tet2 (e.g., a scaffolding function), while inhibiting onr or more other functions of the Tet, e.g., Tet1, Tet2, and/or Tet3, e.g., Tet2 (e.g., a catalytic function), and as such, may be preferable. Gene editing systems which target a late exon or intron of a Tet gene, e.g., Tet1, Tet2, and/or Tet3 gene, e.g., Tet2 gene, may be particularly preferred in this regard. In an aspect, the gene editing system Tet inhibitor, e.g., Tet1, Tet2, and/or Tet3 inhibitor, e.g., Tet2 inhibitor of the invention targets a late exon or intron of the tet gene. In an aspect, the gene editing system Tet inhibitor, e.g., Tet1, Tet2, and/or Tet3 inhibitor, e.g., Tet2 inhibitor of the invention targets an exon or intron downstream of exon 8. In an aspect, the gene editing system Tet inhibitor, e.g., Tet1, Tet2, and/or Tet3 inhibitor, e.g., Tet2 inhibitor, targets exon 8 or exon 9, e.g., exon 9, of the tet2 gene.

Without being bound by theory, it may also be preferable in other embodiments to target an early exon or intron of Tet gene, e.g., Tet1, Tet2, and/or Tet3 gene, e.g., Tet2 gene, for example, to introduce a premature stop codon in the targeted gene which results in no expression of the gene product, or expression of a completely non-functional gene product. Gene editing systems which target an early exon or intron of a Tet gene, e.g., Tet1, Tet2, and/or Tet3 gene, e.g., Tet2 gene, may be particularly preferred in this regard. In an aspect, the gene editing system Tet inhibitor, e.g., Tet1, Tet2, and/or Tet3 inhibitor, e.g., Tet2 inhibitor of the invention targets an early exon or intron of the tet gene. In an aspect, the gene editing system Tet inhibitor, e.g., Tet1, Tet2, and/or Tet3 inhibitor, e.g., Tet2 inhibitor of the invention targets an exon or intron upstream of exon 4. In embodiments, the gene editing system Tet inhibitor, e.g., Tet1, Tet2, and/or Tet3 inhibitor, e.g., Tet2 inhibitor, targets exon 1, exon 2, or exon 3, e.g., exon 3, of the tet2 gene.

Without being bound by theory, it may also be preferable in other embodiments to target a sequence of a Tet gene, e.g., Tet1, Tet2, and/or Tet3 gene, e.g., Tet2 gene, that is specific to one or more isoforms of the tet (e.g., tet2 gene) but does not affect one or more other isoforms of the tet (e.g., tet2). In embodiments, it may be preferable to specifically target isoforms of the tet (e.g., tet2) which contain a catalytic domain.

### dsRNA, e.g., siRNA or shRNA, inhibitors of Tet, e.g., Tet1, Tet2 and/or Tet3, e.g., Tet2

According to the present invention, double stranded RNA ("dsRNA"), e.g., siRNA or shRNA can be used as Tet, e.g., Tet1, Tet2 and/or Tet3, e.g., Tet2, inhibitors. Also contemplated by the present invention are the uses of nucleic acid encoding said dsRNA Tet, e.g., Tet1, Tet2 and/or Tet3, e.g., Tet2, inhibitors.

In an embodiment, the Tet, e.g., Tet1, Tet2 and/or Tet3, e.g., Tet2, inhibitor is a nucleic acid, e.g., a dsRNA, e.g., a siRNA or shRNA specific for nucleic acid encoding Tet, e.g., Tet1, Tet2 and/or Tet3, e.g., Tet2,, e.g., genomic DNA or mRNA encoding Tet, e.g., Tet1, Tet2 and/or Tet3, e.g., Tet2.

An aspect of the invention provides a composition comprising a dsRNA, e.g., a siRNA or shRNA, comprising at least 15 continguous nucleotides, e.g., 15, 16, 17, 18, 19, 20, 21, 22, 23, 24 or 25 contiguous nucleotides, e.g., 21 contiguous nucleotides, which are complementary (e.g., 100% complementary) to a sequence of a Tet, e.g., Tet1, Tet2 and/or Tet3, e.g., Tet2, nucleic acid sequence (e.g., genomic DNA or mRNA encoding Tet, e.g., Tet1, Tet2 and/or Tet3, e.g., Tet2). In embodiments, the at least 15 continguous nucleotides, e.g., 15, 16, 17, 18, 19, 20, 21, 22, 23, 24 or 25 contiguous nucleotides, e.g., 21 contiguous nucleotides, include contiguous nucleotides of a Target sequence of shRNA or Nucleic Acid encoding Tet2 shRNA listed in table 4. It is understood that some of the target sequences and/or shRNA molecules are presented as DNA, but the dsRNA agents targeting these sequences or comprising these sequences can be RNA, or any nucleotide, modified nucleotide or substitute disclosed herein and/or known in the art, provided that the molecule can still mediate RNA interference.

In an embodiment, a nucleic acid molecule that encodes a dsRNA molecule that inhibits expression of Tet, e.g., Tet1, Tet2 and/or Tet3, e.g., Tet2, is operably linked to a promoter, e.g., a H1- or a U6-derived promoter such that the dsRNA molecule that inhibits expression of Tet, e.g., Tet1, Tet2 and/or Tet3, e.g., Tet2, is expressed within a CAR-expressing cell. See e.g., Tiscornia G., "Development of Lentiviral Vectors Expressing siRNA," Chapter 3, in Gene Transfer: Delivery and Expression of DNA and RNA (eds. Friedmann and Rossi). Cold Spring Harbor Laboratory Press, Cold Spring Harbor, NY, USA, 2007; Brummelkamp TR, et al. (2002) Science 296: 550-553; Miyagishi M, et al. (2002) Nat. Biotechnol. 19: 497-500. In an embodiment the nucleic acid molecule that encodes a dsRNA molecule that inhibits Tet, e.g., Tet1, Tet2 and/or Tet3, e.g., Tet2, is present on the same vector, e.g., a lentiviral vector, that comprises a nucleic acid molecule that encodes a component, e.g., all of the components, of the CAR. In such an embodiment, the nucleic acid molecule that encodes a dsRNA molecule that inhibits Tet, e.g., Tet1, Tet2 and/or Tet3, e.g., Tet2, is located on the vector, e.g., the lentiviral vector, 5'- or 3'- to the nucleic acid that encodes a component, e.g., all of the components, of the CAR. The nucleic acid molecule that encodes a dsRNA molecule that inhibits expression of Tet, e.g., Tet1, Tet2 and/or Tet3, e.g., Tet2, can be transcribed in the same or different direction as the nucleic acid that encodes a component, e.g., all of the components, of the CAR. In an embodiment the nucleic acid molecule that encodes a dsRNA molecule that inhibits expression of Tet, e.g., Tet1, Tet2 and/or Tet3, e.g., Tet2, is present on a vector other than the vector that comprises a nucleic acid molecule that encodes a component, e.g., all of the components, of the CAR. In an embodiment, the nucleic acid molecule that encodes a dsRNA molecule that inhibits expression of Tet, e.g., Tet1, Tet2 and/or Tet3, e.g., Tet2, is transiently expressed within a CAR-expressing cell. In an embodiment, the nucleic acid molecule that encodes a dsRNA molecule that inhibits expression of Tet, e.g., Tet1, Tet2 and/or Tet3, e.g., Tet2, is stably integrated into the genome of a CAR-expressing cell.

Examples of nucleic acid sequences that encode shRNA sequences are provided below. The Target Sequence refers to the sequence within the Tet2 genomic DNA (or surrounding DNA). The nucleic acid encoding Tet2 shRNA encodes shRNA molecules useful in the present invention. In embodiments, the Tet2 inhibitor is an siRNA or shRNA specific for a Target sequence listed below, or specific for its mRNA complement. In embodiments, the Tet2 inhibitor is a shRNA encoded by the Nucleic Acid encoding Tet2 shRNA of the table 4 below. In embodiments, the Tet2 inhibitor is nucleic acid comprising by the Nucleic Acid encoding Tet2 shRNA of the table 4 below, e.g., which is under the control of a U6 or H1 promoter such that a Tet2 shRNA is produced. In embodiments, the invention provides a siRNA or shRNA comprising sequence which is the RNA analog (i.e., all T nucleic acid residues replaced with U nucleic acid residues) of the Target sequence of shRNA, e.g., the Target sequenc of shRNA of any of the shRNAs of Table 4.

**Table 4**

| | **SHRNA_NAME** | **Target sequence of shRNA** | **Nucleic Acid encoding Tet2 shRNA** |
|---|---|---|---|
| TET2 | TET2-3838_76472_insert (TET2 shRNA #1) | CACATGGCGTTTA TCCAGAAT (SEQ ID NO: 1244) | CACATGGCGTTTATCCAGAAT CTCGAGATTCTGGATAAACGCCATGT GTTTTTGAATTCGCACCAGCACGCT ACGCACACACAGTACACACACTGACG TTTCGCCGTCTTC (SEQ ID NO: 1253) |
| TET2 | TET2_NM_017628.4 _25616_concept (TET2 shRNA #2) | CAGATGCACAGGC CAATTAAG (SEQ ID NO: 1245) | GAAGACGCACCGGCAGATGTACAGG CTAATTAAGGTTAATATTCATAGCCTT AATTGGCCTGTGCATCTGTTTTTGAA TTCGCACCAGCACGCTACGCAACACG TCAACCAGTGTCAGTGTTTCGCCGT (SEQ ID NO: 1254) |
| TET2 | TET2_NM_017628.4 _25625_concept (TET2 shRNA #3) | GAGCTGCTGAATT CAACTAGA (SEQ ID NO: 1246) | GAAGACGCACCGGGAGCTGCTGAAT TCAATTAGAGTTAATATTCATAGCTCT AGTTGAATTCAGCAGCTCTTTTTTGA ATTCGCACCAGCACGCTACGCATGCA GTCAACCAGTGTCAACCATTCGCCGT (SEQ ID NO: 1255) |
| TET2 | TET2-6571_76471_target (TET2 shRNA #4) | CAGATCGCCATAA CATAAATA (SEQ ID NO: 1247) | CAGATCGCCATAACATAAATACTCGA GTATTTATGTTATGGCGATCTGTTTTT TGAATTCGCACCAGCACGCTACGCAT GACCAGTACACACACTGCATGTTCGC CGTCTTC (SEQ ID NO: 1256) |
| TET2 | TET2_NM_017628.4 _25619_target (TET2 shRNA #5) | GACCATGGAGCAG CATCTGAA (SEQ ID NO: 1248) | GAAGACGCACCGGGACCATGGAGTA GCATTTGAAGTTAATATTCATAGCTTC AGATGCTGCTCCATGGTCTTTTTTGA ATTCGCACCAGCACGCTACGCATGGT GTCAACCAGTGTCAGTTGTTCGCCGT (SEQ ID NO: 1257) |
| TET2 | TET2 shRNA #6 | GCCAAGTCATTATT TGACCAT (SEQ ID NO: 1249) | GCCAAGTCATTATTTGACCATCTCGA GATGGTCAAATAATGACTTGGCTTTT TTGA (SEQ ID NO: 1258) |
| TET2 | TET2 shRNA #7 | CCTCAGAGATATT GTGGGTTT (SEQ ID NO: 1250) | CCTCAGAGATATTGTGGGTTTCTCGA GAAACCCACAATATCTCTGAGGTTTT TTGA (SEQ ID NO: 1259) |
| TET2 | TET2 shRNA #8 | GGGTAAGCCAAGA AAGAAA (SEQ ID NO: 1251) | GGGTAAGCCAAGAAAGAAACTCGAG TTTCTTTCTTGGCTTACCCTTTTTTGA (SEQ ID NO: 1260) |
| TET2 | TET2 8 long (TET2 shRNA #9) | GGGTAAGCCAAGA AAGAAA (SEQ ID NO: 1252) | GAAGACGCACCGGGGGTAAGCCAAG AAAGAAAGTTAATATTCATAGCTTTC TTTCTTGGCTTACCCTTTTTTGAATTC GCACCAGCACGCTACGCAACACGTCA ACCAGTGTCAGTGTTTCGCCGT (SEQ ID NO: 1261) |

Additional dsRNA inhibitor of Tet2, e.g., shRNA and siRNA molecules can be designed and tested using methods known in the art and as described herein. In embodiments, the dsRNA Tet2 inhibitor, e.g., shRNA or siRNA, targets a sequence of SEQ ID NO: 1358. In embodiments, the dsRNA Tet2 inhibitor, e.g., shRNA or siRNA, targets a sequence of SEQ ID NO: 1359. In embodiments, the dsRNA Tet2 inhibitor, e.g., shRNA or siRNA, targets a sequence of SEQ ID NO: 1360. In embodiments, the dsRNA Tet2 inhibitor, e.g., shRNA or siRNA, targets a sequence of SEQ ID NO: 1361. In embodiments, the dsRNA Tet2 inhibitor, e.g., shRNA or siRNA, targets a sequence of SEQ ID NO: 1362. In embodiments, the dsRNA Tet2 inhibitor, e.g., shRNA or siRNA, targets a sequence of SEQ ID NO: 1363. In embodiments, the dsRNA Tet2 inhibitor, e.g., shRNA or siRNA, targets a sequence of an mRNA encoding Tet2.

In embodiments, the inhibitor is a nucleic acid, e.g., DNA, encoding a dsRNA Tet2 inhibitor, e.g., shRNA or siRNA, of any of the above embodiments. In embodiments, the nucleic acid, e.g., DNA, is disposed on a vector, e.g., any conventional expression system, e.g., as described herein, e.g., a lentiviral vector.

Without being bound by theory, a dsRNA TET inhibitor (e.g., siRNA or shRNA) which targets a sequence of a Tet mRNA, e.g., Tet1, Tet2, and/or Tet3 gene, e.g., Tet2 mRNA, that is specific to one or more isoforms of tet (e.g., tet2) but does not affect one or more other isoforms of tet (e.g., tet2) (for example, due to targeting a unique splice junction, or targeting a domain which is present in one or more isoforms of tet, e.g., tet2, but is not present in one or more other isoforms of tet, e.g., tet2). In embodiments, it may be preferable to specifically target isoforms of the tet (e.g., tet2) which contain a catalytic domain.

### Small molecules

### Tet inhibitors

In embodiments, a Tet inhibitor is a small molecule that inhibits expression and/or a function of Tet, e.g., Tet1, Tet2 and/or Tet3, e.g., Tet2.

### Tet2 inhibitors

In embodiments, a Tet2 inhibitor is a small molecule that inhibits Tet2 expression and/or function. For example, a Tet2 inhibitor according to the present invention is 2-hydroxyglutarate (CAS #2889-31-8).

In another example, a Tet2 inhibitor according to the present invention has the following structure:

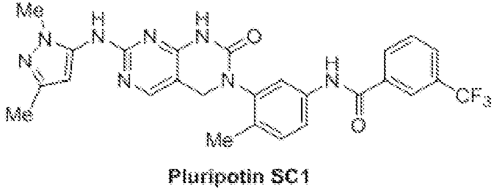

In another example, a Tet2 inhibitor according to the present invention is N-[3-[7-(2,5-Dimethyl-2H-pyrazol-3-ylamino)-1-methyl-2-oxo-1,4-dihydro-2H-pyrimido[4,5-d]pyrimidin-3-yl]-4-methylphenyl]-3-trifluoromethyl-benzamide (CAS #839707-37-8), and has the following structure:

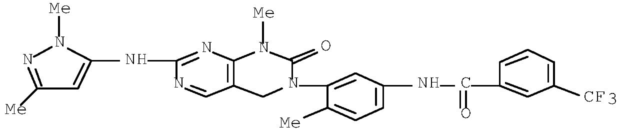

In another example, a Tet2 inhibitor according to the present invention is 2-[(2,6-dichloro-3-methylphenyl)amino]benzoic acid (CAS # 644-62-2), and has the following structure:

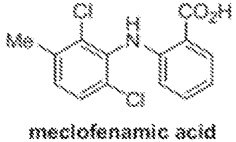

In embodiments, the Tet2 inhibitor of the present invention is a pharmaceutically acceptable salt of any of the foregoing.

### HDAC inhibitors

Any known HDAC inhibitors can be used according to the present invention. Nonlimiting examples of HDAC inhibitors include Voninostat (Zolinza^{®}); Romidepsin (Istodax^{®}); Treichostatin A (TSA); Oxamflatin; Vorinostat (Zolinza^{®}, Suberoylanilide hydroxamic acid); Pyroxamide (syberoyl-3-aminopyridineamide hydroxamic acid); Trapoxin A (RF-1023A); Trapoxin B (RF-10238); Cyclo[(αS,2S)-α-amino-η-oxo-2-oxiraneoctanoyl-O-methyl-D-tyrosyl-L-isoleucyl-L-prolyl] (Cyl-1); Cyclo[(αS,2S)-α-amino-η-oxo-2-oxiraneoctanoyl-O-methyl-D-tyrosyl-L-isoleucyl-(2S)-2-piperidinecarbonyl] (Cyl-2); Cyclic[L-alanyl-D-alanyl-(2S)-η-oxo-L-α-aminooxiraneoctanoyl-D-prolyl] (HC-toxin); Cyclo[(αS,2S)-α-amino-η-oxo-2-oxiraneoctanoyl-D-phenylalanyl-L-leucyl-(2S)-2-piperidinecarbonyl] (WF-3161); Chlamydocin ((S)-Cyclic(2-methylalanyl-L-phenylalanyl-D-prolyl-η-oxo-L-α-aminooxiraneoctanoyl); Apicidin (Cyclo(8-oxo-L-2-aminodecanoyl-1-methoxy-L-tryptophyl-L-isoleucyl-D-2-piperidinecarbonyl); Romidepsin (Istodax^{®}, FR-901228); 4-Phenylbutyrate; Spiruchostatin A; Mylproin (Valproic acid); Entinostat (MS-275, N-(2-Aminophenyl)-4-[N-(pyridine-3-yl-methoxycarbonyl)-amino-methyl]-benzamide); Depudecin (4,5:8,9-dianhydro-1,2,6,7,11-pentadeoxy- D-threo-D-ido-Undeca-1,6-dienitol); 4-(Acetylamino)-N-(2-aminophenyl)-benzamide (also known as CI-994); N1-(2-Aminophenyl)-N8-phenyl-octanediamide (also known as BML-210); 4-(Dimethylamino)-N-(7-(hydroxyamino)-7-oxoheptyl)benzamide (also known as M344); (E)-3-(4-(((2-(1H-indol-3-yl)ethyl)(2-hydroxyethyl)amino)-methyl)phenyl)-N-hydroxyacrylamide (NVP-LAQ824); Panobinostat (Farydak^{®}); Mocetinostat, and Belinostat.

### Proteins

### Dominant Negative Tet2

According to the present invention, dominant negative Tet2 isoforms, and nucleic acid encoding said dominant negative Tet2, can be used as Tet2 inhibitors. In embodiments, the dominant negative Tet2 lacks catalytic function of Tet2. An example of a dominant negative Tet2 is a protein comprising or consisting of SEQ ID NO: 1357 with the mutation R1261G, according to the numbering of SEQ ID NO: 1357. An example of a dominant negative Tet2 is a protein comprising or consisting of SEQ ID NO: 1357 with the mutation R1262A, according to the numbering of SEQ ID NO: 1357. An example of a dominant negative Tet2 is a protein comprising or consisting of SEQ ID NO: 1357 with the mutation S1290A, according to the numbering of SEQ ID NO: 1357. An example of a dominant negative Tet2 is a protein comprising or consisting of SEQ ID NO: 1357 with the mutation WSMYYN (amino acids 1291-1296 of SEQ ID NO: 1357) to GGSGGS (SEQ ID NNO: 67), according to the numbering of SEQ ID NO: 1357. An example of a dominant negative Tet2 is a protein comprising or consisting of SEQ ID NO: 1357 with the mutation M1293A and Y1294A, according to the numbering of SEQ ID NO: 1357. An example of a dominant negative Tet2 is a protein comprising or consisting of SEQ ID NO: 1357 with the mutation Y1295A, according to the numbering of SEQ ID NO: 1357. An example of a dominant negative Tet2 is a protein comprising or consisting of SEQ ID NO: 1357 with the mutation S1303N, according to the numbering of SEQ ID NO: 1357. An example of a dominant negative Tet2 is a protein comprising or consisting of SEQ ID NO: 1357 with the mutation H1382Y, according to the numbering of SEQ ID NO: 1357. An example of a dominant negative Tet2 is a protein comprising or consisting of SEQ ID NO: 1357 with the mutation D1384A, according to the numbering of SEQ ID NO: 1357. An example of a dominant negative Tet2 is a protein comprising or consisting of SEQ ID NO: 1357 with the mutation D1384V, according to the numbering of SEQ ID NO: 1357. In embodiments, the dominant negative Tet2 may include combinations of any of the aforementioned mutations. Such mutations are additionally described in, for example, Chen et al., Nature, 493:561-564 (2013); Hu et al, Cell, 155:1545-1555 (2013), the contents of which are hereby incorporated by reference in their entirety.

### Dominant Negative Tet2 binding partners

Without being bound by theory, it is believed that Tet2 interacts, e.g., binds, with one or more HDAC, e.g., one or more HDAC expressed in immune effector cells, e.g., in T cells, and that such Tet2:HDAC complexes may contribute to Tet2 activity in the cell. In embodiments, a Tet2 inhibitor of the invention is a dominant negative Tet2 binding partner, e.g., a dominant negative Tet2-binding HDAC. In other embodiments, a Tet2 inhibitor of the invention comprises nucleic acid encoding a dominant negative Tet2 binding partner, e.g., a dominant negative Tet2-binding HDAC.

### Vectors encoding Tet2 inhibitors

As described herein, the invention provides vectors, e.g., as described herein, which encode Tet, e.g., Tet1, Tet2 and/or Tet3, e.g., Tet2, inhibitors, such as the gene editing systems, shRNA or siRNA inhibitors or dominant negative inhibitors of Tet, e.g., Tet1, Tet2 and/or Tet3, e.g., Tet2 (e.g., as described herein).

In embodiments further comprising, for example, a CAR, the nucleic acid may further comprise sequence encoding a CAR, e.g., as described herein. In some embodiments, the invention provides a vector comprising a nucleic acid sequence encoding a Tet, e.g., Tet1, Tet2 and/or Tet3, e.g., Tet2 inhibitor described herein and comprising a nucleic acid sequence encoding a CAR molecule described herein. In embodiments, nucleic acid sequences are disposed on separate vectors. In other embodiments, the two or more nucleic acid sequences are encoded by a single nucleic molecule in the same frame and as a single polypeptide chain. In this aspect, the two or more CARs can, e.g., be separated by one or more peptide cleavage sites. (e.g., an auto-cleavage site or a substrate for an intracellular protease). Examples of peptide cleavage sites include the following, wherein the GSG residues are optional:
T2A: (GSG) E G R G S L LTC G D V E E N P G P (SEQ ID NO: 68)
P2A: (GSG) A T N F S L L K Q A G D V E E N P G P (SEQ ID NO: 69)
E2A: (GSG) Q C T N Y A L L K L A G D V E S N P G P (SEQ ID NO: 70)
F2A: (GSG) V K Q T L N F D L L K L A G D V E S N P G P (SEQ ID NO: 71).

These peptide cleavage sites are referred to collectively herein as "2A sites." In embodiments, the vector comprises nucleic acid sequence encoding a CAR described herein and nucleic acid sequence encoding a shRNA or siRNA Tet, e.g., Tet1, Tet2 and/or Tet3, e.g., Tet2, Inhibitor described herein. In embodiments, the vector comprises nucleic acid sequence encoding a CAR described herein and nucleic acid sequence encoding a genome editing system (e.g., a CRISPR/Cas system) Tet e.g., Tet1, Tet2 and/or Tet3, e.g., Tet2, Inhibitor described herein.

### Methods of Use of Tet, e.g., Tet1, Tet2 and/or Tet3, e.g., Tet2, Inhibitors

The invention provides methods of increasing the therapeutic efficacy of a CAR-expressing cell, e.g., a cell expressing a CAR as described herein, e.g., a CAR19-expressing cell (e.g., CTL019), comprising a step of decreasing the level of 5-hydroxymethylcytosine in said cell. In embodiments, the method comprises reducing or eliminating the function or expression of Tet, e.g., Tet1, Tet2 and/or Tet3, e.g., Tet2,. In embodiments, the method comprises contacting said cells with a Tet, e.g., Tet1, Tet2 and/or Tet3, e.g., Tet2, inhibitor as described herein.

The invention further provides methods of manufacturing a CAR-expressing cell, e.g., a CAR-expressing cell having improved function (e.g., having improved efficacy, e.g., tumor targeting, or proliferation) comprising the step of reducing or eliminating the expression or function of Tet, e.g., Tet1, Tet2 and/or Tet3, e.g., Tet2, in said cell. In embodiments, the method comprises contacting said cells with a Tet, e.g., Tet1, Tet2 and/or Tet3, e.g., Tet2, inhibitor as described herein. In embodiments, the contacting is done *ex vivo.* In embodiments, the contacting is done *in vivo.* In embodiments, the contacting is done prior to, simultaneously with, or after said cells are modified to express a CAR, e.g., a CAR as described herein.

In embodiments, the invention provides a method for inhibiting a function or expression of Tet, e.g., Tet1, Tet2 and/or Tet3, e.g., Tet2,, in a CAR-expressing cell, e.g., a cell expressing a CAR as described herein, e.g., a CAR19-expressing cell (e.g., CTL019-expressing cell), the method comprising a step of decreasing the level of 5-hydroxymethylcytosine in said cell. In embodiments, the method comprises reducing or eliminating the function or expression of Tet, e.g., Tet1, Tet2 and/or Tet3, e.g., Tet2,. In embodiments, the method comprises contacting said cells with a Tet, e.g., Tet1, Tet2 and/or Tet3, e.g., Tet2, inhibitor as described herein.

In one embodiment, the invention provides a method, e.g., a method described above, comprises introducing nucleic acid encoding a CAR into a cell, e.g., an immune effector cell, e.g., a T cell, at a site within the Tet gene, or its regulatory elements, such that expression of Tet, e.g., Tet1, Tet2 and/or Tet3, e.g., Tet2, is disrupted. Integration at a site within the Tet, e.g., Tet1, Tet2 and/or Tet3, e.g., Tet2, gene may be accomplished, for example, using a Tet, e.g., Tet1, Tet2 and/or Tet3, e.g., Tet2,-targeting gene editing system as described above.

In one embodiment, the invention provides a method, e.g., a method described above, comprising a step of introducing into the cell a gene editing system, e.g., a CRISPR/Cas gene editing system which targets Tet, e.g., Tet1, Tet2 and/or Tet3, e.g., Tet2,, e.g., a CRISPR/Cas system comprising a gRNA which has a targeting sequence complementary to a target sequence of the Tet, e.g., Tet1, Tet2 and/or Tet3, e.g., Tet2, gene. In embodiments, the CRISPR/Cas system is introduced into said cell as a ribonuclear protein complex of gRNA and Cas enzyme, e.g., is introduced via electroporation. In one embodiment, the method comprises introducing nucleic acid encoding one or more of the components of the CRISPR/Cas system into said cell. In one embodiment, said nucleic acid is disposed on the vector encoding a CAR, e.g., a CAR as described herein.

In one embodiment, the invention provides a method, e.g., a method described above, comprising a step of introducing into the cell an inhibitory dsRNA, e.g., a shRNA or siRNA, which targets Tet, e.g., Tet1, Tet2 and/or Tet3, e.g., Tet2. In one embodiment, the method comprises introducing into said cell nucleic acid encoding an inhibitory dsRNA, e.g., a shRNA or siRNA, which targets Tet, e.g., Tet1, Tet2 and/or Tet3, e.g., Tet2,. In one embodiment, said nucleic acid is disposed on the vector encoding a CAR, e.g., a CAR as described herein.

Additional componentents of CARs and CAR T cells, and methods pertaining to the invention are described below.

Provided herein are compositions of matter and methods of use for the treatment of a disease such as cancer using immune effector cells (e.g., T cells, NK cells) engineered with CARs of the invention.

In one aspect, the invention provides a number of chimeric antigen receptors (CAR) comprising an antigen binding domain (e.g., antibody or antibody fragment, TCR or TCR fragment) engineered for specific binding to a tumor antigen, e.g., a tumor antigen described herein. In one aspect, the invention provides an immune effector cell (e.g., T cell, NK cell) engineered to express a CAR, wherein the engineered immune effector cell exhibits an anticancer property. In one aspect, a cell is transformed with the CAR and the CAR is expressed on the cell surface. In some embodiments, the cell (e.g., T cell, NK cell) is transduced with a viral vector encoding a CAR. In some embodiments, the viral vector is a retroviral vector. In some embodiments, the viral vector is a lentiviral vector. In some such embodiments, the cell may stably express the CAR. In another embodiment, the cell (e.g., T cell, NK cell) is transfected with a nucleic acid, e.g., mRNA, cDNA, DNA, encoding a CAR. In some such embodiments, the cell may transiently express the CAR.

In one aspect, the antigen binding domain of a CAR described herein is a scFv antibody fragment. In one aspect, such antibody fragments are functional in that they retain the equivalent binding affinity, e.g., they bind the same antigen with comparable affinity, as the IgG antibody from which it is derived. In other embodiments, the antibody fragment has a lower binding affinity, e.g., it binds the same antigen with a lower binding affinity than the antibody from which it is derived, but is functional in that it provides a biological response described herein. In one embodiment, the CAR molecule comprises an antibody fragment that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the antibody fragment has a binding affinity that is at least five-fold, 10-fold, 20-fold, 30-fold, 50-fold, 100-fold or 1,000-fold less than a reference antibody, e.g., an antibody described herein.

In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan.

In one aspect, the antigen binding domain of the CAR is a scFv antibody fragment that is humanized compared to the murine sequence of the scFv from which it is derived.

In one aspect, the antigen binding domain of a CAR of the invention (e.g., a scFv) is encoded by a nucleic acid molecule whose sequence has been codon optimized for expression in a mammalian cell. In one aspect, entire CAR construct of the invention is encoded by a nucleic acid molecule whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

In one aspect, the CARs of the invention combine an antigen binding domain of a specific antibody with an intracellular signaling molecule. For example, in some aspects, the intracellular signaling molecule includes, but is not limited to, CD3-zeta chain, 4-1BB and CD28 signaling modules and combinations thereof. In one aspect, the antigen binding domain binds to a tumor antigen as described herein.

Furthermore, the present invention provides CARs and CAR-expressing cells and their use in medicaments or methods for treating, among other diseases, cancer or any malignancy or autoimmune diseases involving cells or tissues which express a tumor antigen as described herein.

In one aspect, the CAR of the invention can be used to eradicate a normal cell that express a tumor antigen as described herein, thereby applicable for use as a cellular conditioning therapy prior to cell transplantation. In one aspect, the normal cell that expresses a tumor antigen as described herein is a normal stem cell and the cell transplantation is a stem cell transplantation.

In one aspect, the invention provides an immune effector cell (e.g., T cell, NK cell) engineered to express a chimeric antigen receptor (CAR), wherein the engineered immune effector cell exhibits an antitumor property. A preferred antigen is a cancer associated antigen (i.e., tumor antigen) described herein. In one aspect, the antigen binding domain of the CAR comprises a partially humanized antibody fragment. In one aspect, the antigen binding domain of the CAR comprises a partially humanized scFv. Accordingly, the invention provides CARs that comprises a humanized antigen binding domain and is engineered into a cell, e.g., a T cell or a NK cell, and methods of their use for adoptive therapy.

In one aspect, the CARs of the invention comprise at least one intracellular domain selected from the group of a CD137 (4-1BB) signaling domain, a CD28 signaling domain, a CD27 signal domain, a CD3zeta signal domain, and any combination thereof. In one aspect, the CARs of the invention comprise at least one intracellular signaling domain is from one or more costimulatory molecule(s) other than a CD137 (4-1BB) or CD28.

Sequences of some examples of various components of CARs of the instant invention is listed in Table 1, where aa stands for amino acids, and na stands for nucleic acids that encode the corresponding peptide.

**Table 1. Sequences of various components of CAR (aa - amino acids, na - nucleic acids that encodes the corresponding protein)**

| SEQ ID NO | description | Sequence | Corresp. To huCD19 |
|---|---|---|---|
| 1 | EF-1 promoter | CGTGAGGCTCCGGTGCCCGTCAGTGGGCAGAGCGCACATCGCCCAC AGTCCCCGAGAAGTTGGGGGGAGGGGTCGGCAATTGAACCGGTGC CTAGAGAAGGTGGCGCGGGGTAAACTGGGAAAGTGATGTCGTGTA CTGGCTCCGCCTTTTTCCCGAGGGTGGGGGAGAACCGTATATAAGT GCAGTAGTCGCCGTGAACGTTCTTTTTCGCAACGGGTTTGCCGCCAG AACACAGGTAAGTGCCGTGTGTGGTTCCCGCGGGCCTGGCCTCTTTA CGGGTTATGGCCCTTGCGTGCCTTGAATTACTTCCACCTGGCTGCAG TACGTGATTCTTGATCCCGAGCTTCGGGTTGGAAGTGGGTGGGAGA GTTCGAGGCCTTGCGCTTAAGGAGCCCCTTCGCCTCGTGCTTGAGTT GAGGCCTGGCCTGGGCGCTGGGGCCGCCGCGTGCGAATCTGGTGG CACCTTCGCGCCTGTCTCGCTGCTTTCGATAAGTCTCTAGCCATTTAA AATTTTTGATGACCTGCTGCGACGCTTTTTTTCTGGCAAGATAGTCTT GTAAATGCGGGCCAAGATCTGCACACTGGTATTTCGGTTTTTGGGGC CGCGGGCGGCGACGGGGCCCGTGCGTCCCAGCGCACATGTTCGGC GAGGCGGGGCCTGCGAGCGCGGCCACCGAGAATCGGACGGGGGTA GTCTCAAGCTGGCCGGCCTGCTCTGGTGCCTGGCCTCGCGCCGCCGT GTATCGCCCCGCCCTGGGCGGCAAGGCTGGCCCGGTCGGCACCAGT TGCGTGAGCGGAAAGATGGCCGCTTCCCGGCCCTGCTGCAGGGAGC TCAAAATGGAGGACGCGGCGCTCGGGAGAGCGGGCGGGTGAGTCA CCCACACAAAGGAAAAGGGCCTTTCCGTCCTCAGCCGTCGCTTCATG TGACTCCACGGAGTACCGGGCGCCGTCCAGGCACCTCGATTAGTTCT CGAGCTTTTGGAGTACGTCGTCTTTAGGTTGGGGGGAGGGGTTTTAT GCGATGGAGTTTCCCCACACTGAGTGGGTGGAGACTGAAGTTAGGC CAGCTTGGCACTTGATGTAATTCTCCTTGGAATTTGCCCTTTTTGAGT TTGGATCTTGGTTCATTCTCAAGCCTCAGACAGTGGTTCAAAGTTTTT TTCTTCCATTTCAGGTGTCGTGA | 100 |
| 2 | Leader (aa) | MALPVTALLLPLALLLHAARP | 13 |
| 3 | Leader (na) | ATGGCCCTGCCTGTGACAGCCCTGCTGCTGCCTCTGGCTCTGCTGCT GCATGCCGCTAGACCC | 54 |
| 4 | CD 8 hinge (aa) | TTTPAPRPPTPAPTIASQPLSLRPEACRPAAGGAVHTRGLDFACD | 14 |
| 5 | CD8 hinge (na) | ACCACGACGCCAGCGCCGCGACCACCAACACCGGCGCCCACCATCG CGTCGCAGCCCCTGTCCCTGCGCCCAGAGGCGTGCCGGCCAGCGGC GGGGGGCGCAGTGCACACGAGGGGGCTGGACTTCGCCTGTGAT | 55 |
| 6 | Ig4 hinge (aa) | ESKYGPPCPPCPAPEFLGGPSVFLFPPKPKDTLMISRTPEVTCVVVDVSQ EDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDKS RWQEGNVFSCSVMHEALHNHYTQKSLSLSLGKM | 102 |
| 7 | Ig4 hinge (na) | GAGAGCAAGTACGGCCCTCCCTGCCCCCCTTGCCCTGCCCCCGAGTT CCTGGGCGGACCCAGCGTGTTCCTGTTCCCCCCCAAGCCCAAGGACA | 103 |
| | | CCCTGATGATCAGCCGGACCCCCGAGGTGACCTGTGTGGTGGTGGA CGTGTCCCAGGAGGACCCCGAGGTCCAGTTCAACTGGTACGTGGAC GGCGTGGAGGTGCACAACGCCAAGACCAAGCCCCGGGAGGAGCAG TTCAATAGCACCTACCGGGTGGTGTCCGTGCTGACCGTGCTGCACCA GGACTGGCTGAACGGCAAGGAATACAAGTGTAAGGTGTCCAACAAG GGCCTGCCCAGCAGCATCGAGAAAACCATCAGCAAGGCCAAGGGCC AGCCTCGGGAGCCCCAGGTGTACACCCTGCCCCCTAGCCAAGAGGA GATGACCAAGAACCAGGTGTCCCTGACCTGCCTGGTGAAGGGCTTCT ACCCCAGCGACATCGCCGTGGAGTGGGAGAGCAACGGCCAGCCCG AGAACAACTACAAGACCACCCCCCCTGTGCTGGACAGCGACGGCAG CTTCTTCCTGTACAGCCGGCTGACCGTGGACAAGAGCCGGTGGCAG GAGGGCAACGTCTTTAGCTGCTCCGTGATGCACGAGGCCCTGCACA ACCACTACACCCAGAAGAGCCTGAGCCTGTCCCTGGGCAAGATG | |
| 8 | IgD hinge (aa) | RWPESPKAQASSVPTAQPQAEGSLAKATTAPATTRNTGRGGEEKKKEK EKEEQEERETKTPECPSHTQPLGVYLLTPAVQDLWLRDKATFTCFVVGS DLKDAHLTWEVAGKVPTGGVEEGLLERHSNGSQSQHSRLTLPRSLWN AGTSVTCTLNHPSLPPQRLMALREPAAQAPVKLSLNLLASSDPPEAASW LLCEVSGFSPPNILLMWLEDQREVNTSGFAPARPPPQPGSTTFWAWSV LRVPAPPSPQPATYTCVVSHEDSRTLLNASRSLEVSYVTDH | 47 |
| 9 | IgD hinge (na) | AGGTGGCCCGAAAGTCCCAAGGCCCAGGCATCTAGTGTTCCTACTGC ACAGCCCCAGGCAGAAGGCAGCCTAGCCAAAGCTACTACTGCACCT GCCACTACGCGCAATACTGGCCGTGGCGGGGAGGAGAAGAAAAAG GAGAAAGAGAAAGAAGAACAGGAAGAGAGGGAGACCAAGACCCCT GAATGTCCATCCCATACCCAGCCGCTGGGCGTCTATCTCTTGACTCCC GCAGTACAGGACTTGTGGCTTAGAGATAAGGCCACCTTTACATGTTT CGTCGTGGGCTCTGACCTGAAGGATGCCCATTTGACTTGGGAGGTT GCCGGAAAGGTACCCACAGGGGGGGTTGAGGAAGGGTTGCTGGAG CGCCATTCCAATGGCTCTCAGAGCCAGCACTCAAGACTCACCCTTCC GAGATCCCTGTGGAACGCCGGGACCTCTGTCACATGTACTCTAAATC ATCCTAGCCTGCCCCCACAGCGTCTGATGGCCCTTAGAGAGCCAGCC GCCCAGGCACCAGTTAAGCTTAGCCTGAATCTGCTCGCCAGTAGTGA TCCCCCAGAGGCCGCCAGCTGGCTCTTATGCGAAGTGTCCGGCTTTA GCCCGCCCAACATCTTGCTCATGTGGCTGGAGGACCAGCGAGAAGT GAACACCAGCGGCTTCGCTCCAGCCCGGCCCCCACCCCAGCCGGGTT CTACCACATTCTGGGCCTGGAGTGTCTTAAGGGTCCCAGCACCACCT AGCCCCCAGCCAGCCACATACACCTGTGTTGTGTCCCATGAAGATAG CAGGACCCTGCTAAATGCTTCTAGGAGTCTGGAGGTTTCCTACGTGA CTGACCATT | 48 |
| 10 | GS hinge/linker (aa) | GGGGSGGGGS | 49 |
| 11 | GS hinge/linker (na) | GGTGGCGGAGGTTCTGGAGGTGGAGGTTCC | 50 |
| 12 | CD8TM (aa) | IYIWAPLAGTCGVLLLSLVITLYC | 15 |
| 13 | CD8 TM (na) | ATCTACATCTGGGCGCCCTTGGCCGGGACTTGTGGGGTCCTTCTCCT GTCACTGGTTATCACCCTTTACTGC | 56 |
| 14 | 4-1BB intracellular domain (aa) | KRGRKKLLYIFKQPFMRPVQTTQEEDGCSCRFPEEEEGGCEL | 16 |
| 15 | 4-1BB intracellular domain (na) | AAACGGGGCAGAAAGAAACTCCTGTATATATTCAAACAACCATTTAT GAGACCAGTACAAACTACTCAAGAGGAAGATGGCTGTAGCTGCCGA TTTCCAGAAGAAGAAGAAGGAGGATGTGAACTG | 60 |
| 16 | CD27 (aa) | QRRKYRSNKGESPVEPAEPCRYSCPREEEGSTIPIQEDYRKPEPACSP | 51 |
| 17 | CD27 (na) | AGGAGTAAGAGGAGCAGGCTCCTGCACAGTGACTACATGAACATGA CTCCCCGCCGCCCCGGGCCCACCCGCAAGCATTACCAGCCCTATGCC CCACCACGCGACTTCGCAGCCTATCGCTCC | 52 |
| 18 | CD3-zeta (aa) | RVKFSRSADAPAYKQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR | 17 |
| 19 | CD3-zeta (na) | AGAGTGAAGTTCAGCAGGAGCGCAGACGCCCCCGCGTACAAGCAG GGCCAGAACCAGCTCTATAACGAGCTCAATCTAGGACGAAGAGAGG AGTACGATGTTTTGGACAAGAGACGTGGCCGGGACCCTGAGATGGG GGGAAAGCCGAGAAGGAAGAACCCTCAGGAAGGCCTGTACAATGA ACTGCAGAAAGATAAGATGGCGGAGGCCTACAGTGAGATTGGGAT GAAAGGCGAGCGCCGGAGGGGCAAGGGGCACGATGGCCTTTACCA GGGTCTCAGTACAGCCACCAAGGACACCTACGACGCCCTTCACATGC AGGCCCTGCCCCCTCGC | 101 |
| 20 | CD3-zeta (aa) | RVKFSRSADAPAYQQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR | 43 |
| 21 | CD3-zeta (na) | AGAGTGAAGTTCAGCAGGAGCGCAGACGCCCCCGCGTACCAGCAG GGCCAG AACCAGCTCTATAACGAGCTCAATCTAGGACGAAGAGAGGAGTACG ATGTTT TGGACAAGAGACGTGGCCGGGACCCTGAGATGGGGGGAAAGCCGA GAAGGA AGAACCCTCAGGAAGGCCTGTACAATGAACTGCAGAAAGATAAGAT GGCGG AGGCCTACAGTGAGATTGGGATGAAAGGCGAGCGCCGGAGGGGCA AGGGGC ACGATGGCCTTTACCAGGGTCTCAGTACAGCCACCAAGGACACCTAC GACGC CCTTCACATGCAGGCCCTGCCCCCTCGC | 44 |
| 22 | linker | GGGGS | 18 |
| 23 | linker | GGTGGCGGAGGTTCTGGAGGTGGAGGTTCC | 50 |
| 24 | PD-1 extracellular domain (aa) | | |
| 25 | PD-1 extracellular domain (na) | Cccggatggtttctggactctccggatcgcccgtggaatcccccaaccttctcaccggcact cttggttgtgactgagggcgataatgcgaccttcacgtgctcgttctccaacacctccgaat cattcgtgctgaactggtaccgcatgagcccgtcaaaccagaccgacaagctcgccgcgtt tccggaagatcggtcgcaaccgggacaggattgtcggttccgcgtgactcaactgccgaat ggcagagacttccacatgagcgtggtccgcgctaggcgaaacgactccgggacctacctg | |
| | | tgcggagccatctcgctggcgcctaaggcccaaatcaaagagagcttgagggccgaactg agagtgaccgagcgcagagctgaggtgccaactgcacatccatccccatcgcctcggcct gcggggcagtttcagaccctggtc | |
| 26 | PD-1 CAR (aa) with signal | Malpvtalllplalllhaarppgwfldspdrpwnpptfspallvvtegdnatftcsfsntse sfvlnwyrmspsnqtdklaafpedrsqpgqdcrfrvtqlpngrdfhmsvvrarrndsgt ylcgaislapkaqikeslraelrvterraevptahpspsprpagqfqtlvtttpaprpptpa ptiasqplslrpeacrpaaggavhtrgldfacdiyiwaplagtcgvlllslvitlyckrgrkklly ifkqpfmrpvqttqeedgcscrfpeeeeggcelrvkfsrsadapaykqgqnqlynelnl grreeydvldkrrgrdpemggkprrknpqeglynelqkdkmaeayseigmkgerrrg kghdglyqglstatkdtydalhmqalppr | |
| 27 | PD-1 CAR (na) | Atggccctccctgtcactgccctgcttctcccctcgcactcctgctccacgccgctagacca cccggatggtttctggactctccggatcgcccgtggaatcccccaaccttctcaccggcact cttggttgtgactgagggcgataatgcgaccttcacgtgctcgttctccaacacctccgaat cattcgtgctgaactggtaccgcatgagcccgtcaaaccagaccgacaagctcgccgcgtt tccggaagatcggtcgcaaccgggacaggattgtcggttccgcgtgactcaactgccgaat ggcagagacttccacatgagcgtggtccgcgctaggcgaaacgactccgggacctacctg tgcggagccatctcgctggcgcctaaggcccaaatcaaagagagcttgagggccgaactg agagtgaccgagcgcagagctgaggtgccaactgcacatccatccccatcgcctcggcct gcggggcagtttcagaccctggtcacgaccactccggcgccgcgcccaccgactccggcc ccaactatcgcgagccagcccctgtcgctgaggccggaagcatgccgccctgccgccgga ggtgctgtgcatacccggggattggacttcgcatgcgacatctacatttgggctcctctcgc cggaacttgtggcgtgctccttctgtccctggtcatcaccctgtactgcaagcggggtcgga aaaagcttctgtacattttcaagcagcccttcatgaggcccgtgcaaaccacccaggagga ggacggttgctcctgccggttccccgaagaggaagaaggaggttgcgagctgcgcgtgaa gttctcccggagcgccgacgcccccgcctataagcagggccagaaccagctgtacaacga actgaacctgggacggcgggaagagtacgatgtgctggacaagcggcgcggccgggacc ccgaaatgggcgggaagcctagaagaaagaaccctcaggaaggcctgtataacgagctg cagaaggacaagatggccgaggcctactccgaaattgggatgaagggagagcggcgga ggggaaaggggcacgacggcctgtaccaaggactgtccaccgccaccaaggacacatac gatgccctgcacatgcaggcccttccccctcgc | |
| 28 | linker | (Gly-Gly-Gly-Ser)n, where n = 1-10 | 105 |
| 29 | linker | (Gly4 Ser)4 | 106 |
| 30 | linker | (Gly4 Ser)3 | 107 |
| 31 | linker | (Gly3Ser) | 108 |
| 32 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 118 |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| 33 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 104 |
| 34 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 109 |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| 35 | polyA | tttttttttt tttttttttt tttttttttt tttttttttt tttttttttt tttttttttt tttttttttt tttttttttt tttttttttt tttttttttt | 110 |
| 36 | polyA | tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt | 111 |
| | | tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt | |
| | | tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt | |
| 37 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 112 |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| 38 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa  aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa  aaaaaaaaaa aaaaaaaaaa aaaaaaaaaa aaaaaaaaaa | 113 |
| 39 | PD1 CAR (aa) | Pgwfldspdrpwnpptfspallvvtegdnatftcsfsntsesfvlnwyrmspsnqtdkl aafpedrsqpgqdcrfrvtqlpngrdfhmsvvrarrndsgtylcgaislapkaqikeslra elrvterraevptahpspsprpagqfqtlvtttpaprpptpaptiasqplslrpeacrpaa ggavhtrgldfacdiyiwaplagtcgvlllslvitlyckrgrkkllyifkqpfmrpvqttqeed gcscrfpeeeeggcelrvkfsrsadapaykqgqnqlynelnlgrreeydvldkrrgrdpe mggkprrknpqeglynelqkdkmaeayseigmkgerrrgkghdglyqglstatkdty dalhmqalppr | |

### Cancer Associated Antigens

The present invention provides immune effector cells (e.g., T cells, NK cells) that are engineered to contain one or more CARs that direct the immune effector cells to cancer. This is achieved through an antigen binding domain on the CAR that is specific for a cancer associated antigen. There are two classes of cancer associated antigens (tumor antigens) that can be targeted by the CARs of the instant invention: (1) cancer associated antigens that are expressed on the surface of cancer cells; and (2) cancer associated antigens that itself is intracellar, however, a fragment of such antigen (peptide) is presented on the surface of the cancer cells by MHC (major histocompatibility complex).

Accordingly, the present invention provides CARs that target the following cancer associated antigens (tumor antigens): CD19, CD123, CD22, CD30, CD171, CS-1, CLL-1 (CLECL1), CD33, EGFRvIII , GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, PRSS21, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

### Tumor-supporting antigens

A CAR described herein can comprise an antigen binding domain (e.g., antibody or antibody fragment, TCR or TCR fragment) that binds to a tumor-supporting antigen (e.g., a tumor-supporting antigen as described herein). In some embodiments, the tumor-supporting antigen is an antigen present on a stromal cell or a myeloid-derived suppressor cell (MDSC). Stromal cells can secrete growth factors to promote cell division in the microenvironment. MDSC cells can inhibit T cell proliferation and activation. Without wishing to be bound by theory, in some embodiments, the CAR-expressing cells destroy the tumor-supporting cells, thereby indirectly inhibiting tumor growth or survival.

In embodiments, the stromal cell antigen is chosen from one or more of: bone marrow stromal cell antigen 2 (BST2), fibroblast activation protein (FAP) and tenascin. In an embodiment, the FAP-specific antibody is, competes for binding with, or has the same CDRs as, sibrotuzumab. In embodiments, the MDSC antigen is chosen from one or more of: CD33, CD11b, C14, CD15, and CD66b. Accordingly, in some embodiments, the tumor-supporting antigen is chosen from one or more of: bone marrow stromal cell antigen 2 (BST2), fibroblast activation protein (FAP) or tenascin, CD33, CD11b, C14, CD15, and CD66b.

### Chimeric Antigen Receptor (CAR)

The present invention encompasses a recombinant DNA construct comprising sequences encoding a CAR, wherein the CAR comprises an antigen binding domain (e.g., antibody or antibody fragment, TCR or TCR fragment) that binds specifically to a cancer associated antigen described herein, wherein the sequence of the antigen binding domain is contiguous with and in the same reading frame as a nucleic acid sequence encoding an intracellular signaling domain. The intracellular signaling domain can comprise a costimulatory signaling domain and/or a primary signaling domain, e.g., a zeta chain. The costimulatory signaling domain refers to a portion of the CAR comprising at least a portion of the intracellular domain of a costimulatory molecule.

In specific aspects, a CAR construct of the invention comprises a scFv domain, wherein the scFv may be preceded by an optional leader sequence such as provided in SEQ ID NO: 2, and followed by an optional hinge sequence such as provided in SEQ ID NO:4 or SEQ ID NO:6 or SEQ ID NO:8 or SEQ ID NO:10, a transmembrane region such as provided in SEQ ID NO:12, an intracellular signalling domain that includes SEQ ID NO:14 or SEQ ID NO:16 and a CD3 zeta sequence that includes SEQ ID NO:18 or SEQ ID NO:20, e.g., wherein the domains are contiguous with and in the same reading frame to form a single fusion protein.

In one aspect, an exemplary CAR constructs comprise an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), and an intracellular stimulatory domain (e.g., an intracellular stimulatory domain decribed herein). In one aspect, an exemplary CAR construct comprises an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), an intracellular costimulatory signaling domain (e.g., a costimulatory signaling domain described herein) and/or an intracellular primary signaling domain (e.g., a primary signaling domain described herein).

An exemplary leader sequence is provided as SEQ ID NO: 2. An exemplary hinge/spacer sequence is provided as SEQ ID NO: 4 or SEQ ID NO:6 or SEQ ID NO:8 or SEQ ID NO:10. An exemplary transmembrane domain sequence is provided as SEQ ID NO:12. An exemplary sequence of the intracellular signaling domain of the 4-1BB protein is provided as SEQ ID NO: 14. An exemplary sequence of the intracellular signaling domain of CD27 is provided as SEQ ID NO:16. An exemplary CD3zeta domain sequence is provided as SEQ ID NO: 18 or SEQ ID NO:20.

In one aspect, the present invention encompasses a recombinant nucleic acid construct comprising a nucleic acid molecule encoding a CAR, wherein the nucleic acid molecule comprises the nucleic acid sequence encoding an antigen binding domain, e.g., described herein, that is contiguous with and in the same reading frame as a nucleic acid sequence encoding an intracellular signaling domain.

In one aspect, the present invention encompasses a recombinant nucleic acid construct comprising a nucleic acid molecule encoding a CAR, wherein the nucleic acid molecule comprises a nucleic acid sequence encoding an antigen binding domain, wherein the sequence is contiguous with and in the same reading frame as the nucleic acid sequence encoding an intracellular signaling domain. An exemplary intracellular signaling domain that can be used in the CAR includes, but is not limited to, one or more intracellular signaling domains of, e.g., CD3-zeta, CD28, CD27, 4-1BB, and the like. In some instances, the CAR can comprise any combination of CD3-zeta, CD28, 4-1BB, and the like.

The nucleic acid sequences coding for the desired molecules can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned.

The present invention includes retroviral and lentiviral vector constructs expressing a CAR that can be directly transduced into a cell.

The present invention also includes an RNA construct that can be directly transfected into a cell. A method for generating mRNA for use in transfection involves *in vitro* transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-2000 bases in length (SEQ ID NO:32). RNA so produced can efficiently transfect different kinds of cells. In one embodiment, the template includes sequences for the CAR. In an embodiment, an RNA CAR vector is transduced into a cell, e.g., a T cell or a NK cell, by electroporation.

### Antigen binding domain

In one aspect, the CAR of the invention comprises a target-specific binding element otherwise referred to as an antigen binding domain. The choice of moiety depends upon the type and number of ligands that define the surface of a target cell. For example, the antigen binding domain may be chosen to recognize a ligand that acts as a cell surface marker on target cells associated with a particular disease state. Thus, examples of cell surface markers that may act as ligands for the antigen binding domain in a CAR of the invention include those associated with viral, bacterial and parasitic infections, autoimmune disease and cancer cells.

In one aspect, the CAR-mediated T-cell response can be directed to an antigen of interest by way of engineering an antigen binding domain that specifically binds a desired antigen into the CAR.

In one aspect, the portion of the CAR comprising the antigen binding domain comprises an antigen binding domain that targets a tumor antigen, e.g., a tumor antigen described herein.

The antigen binding domain can be any domain that binds to the antigen including but not limited to a monoclonal antibody, a polyclonal antibody, a recombinant antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, and to an alternative scaffold known in the art to function as antigen binding domain, such as a recombinant fibronectin domain, a T cell receptor (TCR), or a fragment there of, e.g., single chain TCR, and the like. In some instances, it is beneficial for the antigen binding domain to be derived from the same species in which the CAR will ultimately be used in. For example, for use in humans, it may be beneficial for the antigen binding domain of the CAR to comprise human or humanized residues for the antigen binding domain of an antibody or antibody fragment.

In one embodiment, the CD19 CAR is a CD19 CAR described in US Pat. No. 8,399,645; US Pat. No. 7,446,190; Xu et al., Leuk Lymphoma. 2013 54(2):255-260(2012); Cruz et al., Blood 122(17):2965-2973 (2013); Brentjens et al., Blood, 118(18):4817-4828 (2011); Kochenderfer et al., Blood 116(20):4099-102 (2010); Kochenderfer et al., Blood 122 (25):4129-39(2013); or 16th Annu Meet Am Soc Gen Cell Ther (ASGCT) (May 15-18, Salt Lake City) 2013, Abst 10 (each of which is herein incorporated by reference in their entirety). In one embodiment, an antigen binding domain against CD19 is an antigen binding portion, e.g., CDRs, of a CAR, antibody or antigen-binding fragment thereof described in, e.g., PCT publication WO2012/079000 (incorporated herein by reference in its entirety). In one embodiment, an antigen binding domain against CD19 is an antigen binding portion, e.g., CDRs, of a CAR, antibody or antigen-binding fragment thereof described in, e.g., PCT publication WO2014/153270; Kochenderfer, J.N. et al., J. Immunother. 32 (7), 689-702 (2009); Kochenderfer, J.N., et al., Blood, 116 (20), 4099-4102 (2010); PCT publication WO2014/031687; Bejcek, Cancer Research, 55, 2346-2351, 1995; or U.S. Patent No. 7,446,190 (each of which is herein incorporated by reference in their entirety).

In one embodiment, the antigen binding domain against mesothelin is or may be derived from an antigen binding domain, e.g., CDRs, scFv, or VH and VL, of an antibody, antigen-binding fragment or CAR described in, e.g., PCT publication WO2015/090230 (In one embodiment the CAR is a CAR described in WO2015/090230, the contents of which are incorporated herein in their entirety). In embodiments, the antigen binding domain against mesothelin is or is derived from an antigen binding portion, e.g., CDRs, scFv, or VH and VL, of an antibody, antigen-binding fragment, or CAR described in, e.g., PCT publication WO1997/025068, WO1999/028471, WO2005/014652, WO2006/099141, WO2009/045957, WO2009/068204, WO2013/142034, WO2013/040557, or WO2013/063419 (each of which is herein incorporated by reference in their entirety).

In one embodiment, an antigen binding domain against CD123 is or is derived from an antigen binding portion, e.g., CDRs, scFv or VH and VL, of an antibody, antigen-binding fragment or CAR described in, e.g., PCT publication WO2014/130635 (incorporated herein by referenc in its entirety). In one embodiment, an antigen binding domain against CD123 is or is derived from an antigen binding portion, e.g., CDRs, scFv or VH and VL, of an antibody, antigen-binding fragment or CAR described in, e.g., PCT publication WO2016/028896 (incorporated herein by referenc in its entirety); in embodiments, the CAR is a CAR described in WO2016/028896. In one embodiment, an antigen binding domain against CD123 is or is derived from an antigen binding portion, e.g., CDRs, scFv, or VL and VH, of an antibody, antigen-binding fragment, or CAR described in, e.g., PCT publication WO1997/024373, WO2008/127735 (e.g., a CD123 binding domain of 26292, 32701, 37716 or 32703), WO2014/138805 (e.g., a CD123 binding domain of CSL362), WO2014/138819, WO2013/173820, WO2014/144622, WO2001/66139, WO2010/126066 (e.g., the CD123 binding domain of any of Old4, Old5, Old17, Old19, New102, or Old6), WO2014/144622, or US2009/0252742 (each of which is incorporated herein by referenc in its entirety).

In one embodiment, an antigen binding domain against CD22 is an antigen binding portion, e.g., CDRs, of an antibody described in, e.g., Haso et al., Blood, 121(7): 1165-1174 (2013); Wayne et al., Clin Cancer Res 16(6): 1894-1903 (2010); Kato et al., Leuk Res 37(1):83-88 (2013); Creative BioMart (creativebiomart.net): MOM-18047-S(P).

In one embodiment, an antigen binding domain against CS-1 is an antigen binding portion, e.g., CDRs, of Elotuzumab (BMS), see e.g., Tai et al., 2008, Blood 112(4):1329-37; Tai et al., 2007, Blood. 110(5):1656-63.

In one embodiment, an antigen binding domain against CLL-1 is an antigen binding portion, e.g., CDRs or VH and VL, of an antibody, antigen-binding fragment or CAR described in, e.g., PCT publication WO2016/014535, the contents of which are incorporated herein in their entirety. In one embodiment, an antigen binding domain against CLL-1 is an antigen binding portion, e.g., CDRs, of an antibody available from R&D, ebiosciences, Abcam, for example, PE-CLL1-hu Cat# 353604 (BioLegend); and PE-CLL1 (CLEC12A) Cat# 562566 (BD).

In one embodiment, an antigen binding domain against CD33 is an antigen binding portion, e.g., CDRs, of an antibody described in, e.g., Bross et al., Clin Cancer Res 7(6):1490-1496 (2001) (Gemtuzumab Ozogamicin, hP67.6),Caron et al., Cancer Res 52(24):6761-6767 (1992) (Lintuzumab, HuM195), Lapusan et al., Invest New Drugs 30(3):1121-1131 (2012) (AVE9633), Aigner et al., Leukemia 27(5): 1107-1115 (2013) (AMG330, CD33 BiTE), Dutour et al., Adv hematol 2012:683065 (2012), and Pizzitola et al., Leukemia doi:10.1038/Lue.2014.62 (2014). Exemplary CAR molecules that target CD33 are described herein, and are provided in WO2016/014576, e.g., in Table 2 of WO2016/014576 (incorporated by reference in its entirety).

In one embodiment, an antigen binding domain against GD2 is an antigen binding portion, e.g., CDRs, of an antibody described in, e.g., Mujoo et al., Cancer Res. 47(4):1098-1104 (1987); Cheung et al., Cancer Res 45(6):2642-2649 (1985), Cheung et al., J Clin Oncol 5(9):1430-1440 (1987), Cheung et al., J Clin Oncol 16(9):3053-3060 (1998), Handgretinger et al., Cancer Immunol Immunother 35(3):199-204 (1992). In some embodiments, an antigen binding domain against GD2 is an antigen binding portion of an antibody selected from mAb 14.18, 14G2a, ch14.18, hu14.18, 3F8, hu3F8, 3G6, 8B6, 60C3, 10B8, ME36.1, and 8H9, see e.g., WO2012033885, WO2013040371, WO2013192294, WO2013061273, WO2013123061, WO2013074916, and WO201385552. In some embodiments, an antigen binding domain against GD2 is an antigen binding portion of an antibody described in US Publication No.: 20100150910 or PCT Publication No.: WO 2011160119.

In one embodiment, an antigen binding domain against BCMA is an antigen binding portion, e.g., CDRs, of an antibody described in, e.g., WO2012163805, WO200112812, and WO2003062401. In embodiments, additional exemplary BCMA CAR constructs are generated using an antigen binding domain, e.g., CDRs, scFv, or VH and VL sequences from PCT Publication WO2012/0163805 (the contents of which are hereby incorporated by reference in its entirety). In embodiments, additional exemplary BCMA CAR constructs are generated using an antigen binding domain, e.g., CDRs, scFv, or VH and VL sequences from PCT Publication WO2016/014565 (the contents of which are hereby incorporated by reference in its entirety). In embodiments, additional exemplary BCMA CAR constructs are generated using an antigen binding domain, e.g., CDRs, scFv, or VH and VL sequences from PCT Publication WO2014/122144 (the contents of which are hereby incorporated by reference in its entirety). In embodiments, additional exemplary BCMA CAR constructs are generated using the CAR molecules, and/or the BCMA binding domains (e.g., CDRs, scFv, or VH and VL sequences) from PCT Publication WO2016/014789 (the contents of which are hereby incorporated by reference in its entirety). In embodiments, additional exemplary BCMA CAR constructs are generated using the CAR molecules, and/or the BCMA binding domains (e.g., CDRs, scFv, or VH and VL sequences) from PCT Publication WO2014/089335 (the contents of which are hereby incorporated by reference in its entirety). In embodiments, additional exemplary BCMA CAR constructs are generated using the CAR molecules, and/or the BCMA binding domains (e.g., CDRs, scFv, or VH and VL sequences) from PCT Publication WO2014/140248 (the contents of which are hereby incorporated by reference in its entirety).

In one embodiment, an antigen binding domain against Tn antigen is an antigen binding portion, e.g., CDRs, of an antibody described in, e.g., US 2014/0178365, US8,440,798, Brooks et al., PNAS 107(22):10056-10061 (2010), and Stone et al., OncoImmunology 1(6):863-873(2012).

In one embodiment, an antigen binding domain against PSMA is an antigen binding portion, e.g., CDRs, of an antibody described in, e.g., Parker et al., Protein Expr Purif 89(2):136-145 (2013), US 20110268656 (J591 ScFv); Frigerio et al, European J Cancer 49(9):2223-2232 (2013) (scFvD2B); WO 2006125481 (mAbs 3/A12, 3/E7 and 3/F11) and single chain antibody fragments (scFv A5 and D7).

In one embodiment, an antigen binding domain against ROR1 is an antigen binding portion, e.g., CDRs, of an antibody described in, e.g., Hudecek et al., Clin Cancer Res 19(12):3153-3164 (2013); WO 2011159847; and US20130101607.

In one embodiment, an antigen binding domain against FLT3 is an antigen binding portion, e.g., CDRs, of an antibody described in, e.g., WO2011076922, US5777084, EP0754230, US20090297529, and several commercial catalog antibodies (R&D, ebiosciences, Abcam).

In one embodiment, an antigen binding domain against TAG72 is an antigen binding portion, e.g., CDRs, of an antibody described in, e.g., Hombach et al., Gastroenterology 113(4):1163-1170 (1997); and Abcam ab691.

In one embodiment, an antigen binding domain against FAP is an antigen binding portion, e.g., CDRs, of an antibody described in, e.g., Ostermann et al., Clinical Cancer Research 14:4584-4592 (2008) (FAP5), US Pat. Publication No. 2009/0304718; sibrotuzumab (see e.g., Hofheinz et al., Oncology Research and Treatment 26(1), 2003); and Tran et al., J Exp Med 210(6):1125-1135 (2013).

In one embodiment, an antigen binding domain against CD38 is an antigen binding portion, e.g., CDRs, of daratumumab (see, e.g., Groen et al., Blood 116(21):1261-1262 (2010); MOR202 (see, e.g., US8,263,746); or antibodies described in US8,362,211.

In one embodiment, an antigen binding domain against CD44v6 is an antigen binding portion, e.g., CDRs, of an antibody described in, e.g., Casucci et al., Blood 122(20):3461-3472 (2013).

In one embodiment, an antigen binding domain against CEA is an antigen binding portion, e.g., CDRs, of an antibody described in, e.g., Chmielewski et al., Gastoenterology 143(4):1095-1107 (2012).

In one embodiment, an antigen binding domain against EPCAM is an antigen binding portion, e.g., CDRS, of an antibody selected from MT110, EpCAM-CD3 bispecific Ab (see, e.g., clinicaltrials.gov/ct2/show/NCT00635596); Edrecolomab; 3622W94; ING-1; and adecatumumab (MT201).

In one embodiment, an antigen binding domain against PRSS21 is an antigen binding portion, e.g., CDRs, of an antibody described in US Patent No.: 8,080,650.

In one embodiment, an antigen binding domain against B7H3 is an antigen binding portion, e.g., CDRs, of an antibody MGA271 (Macrogenics).

In one embodiment, an antigen binding domain against KIT is an antigen binding portion, e.g., CDRs, of an antibody described in, e.g., US7915391, US20120288506 , and several commercial catalog antibodies.

In one embodiment, an antigen binding domain against IL-13Ra2 is an antigen binding portion, e.g., CDRs, of an antibody described in, e.g., WO2008/146911, WO2004087758, several commercial catalog antibodies, and WO2004087758.

In one embodiment, an antigen binding domain against CD30 is an antigen binding portion, e.g., CDRs, of an antibody described in, e.g., US7090843 B1, and EP0805871.

In one embodiment, an antigen binding domain against GD3 is an antigen binding portion, e.g., CDRs, of an antibody described in, e.g., US7253263; US 8,207,308; US 20120276046; EP1013761; WO2005035577; and US6437098.

In one embodiment, an antigen binding domain against CD171 is an antigen binding portion, e.g., CDRs, of an antibody described in, e.g., Hong et al., J Immunother 37(2):93-104 (2014).

In one embodiment, an antigen binding domain against IL-11Ra is an antigen binding portion, e.g., CDRs, of an antibody available from Abcam (cat# ab55262) or Novus Biologicals (cat# EPR5446). In another embodiment, an antigen binding domain again IL-11Ra is a peptide, see, e.g., Huang et al., Cancer Res 72(1):271-281 (2012).

In one embodiment, an antigen binding domain against PSCA is an antigen binding portion, e.g., CDRs, of an antibody described in, e.g., Morgenroth et al., Prostate 67(10):1121-1131 (2007) (scFv 7F5); Nejatollahi et al., J of Oncology 2013(2013), article ID 839831 (scFv C5-II); and US Pat Publication No. 20090311181.

In one embodiment, an antigen binding domain against VEGFR2 is an antigen binding portion, e.g., CDRs, of an antibody described in, e.g., Chinnasamy et al., J Clin Invest 120(11):3953-3968 (2010).

In one embodiment, an antigen binding domain against LewisY is an antigen binding portion, e.g., CDRs, of an antibody described in, e.g., Kelly et al., Cancer Biother Radiopharm 23(4):411-423 (2008) (hu3S193 Ab (scFvs)); Dolezal et al., Protein Engineering 16(1):47-56 (2003) (NC10 scFv).

In one embodiment, an antigen binding domain against CD24 is an antigen binding portion, e.g., CDRs, of an antibody described in, e.g., Maliar et al., Gastroenterology 143(5):1375-1384 (2012).

In one embodiment, an antigen binding domain against PDGFR-beta is an antigen binding portion, e.g., CDRs, of an antibody Abcam ab32570.

In one embodiment, an antigen binding domain against SSEA-4 is an antigen binding portion, e.g., CDRs, of antibody MC813 (Cell Signaling), or other commercially available antibodies.

In one embodiment, an antigen binding domain against CD20 is an antigen binding portion, e.g., CDRs, of the antibody Rituximab, Ofatumumab, Ocrelizumab, Veltuzumab, or GA101.

In one embodiment, an antigen binding domain against Folate receptor alpha is an antigen binding portion, e.g., CDRs, of the antibody IMGN853, or an antibody described in US20120009181; US4851332, LK26: US5952484.

In one embodiment, an antigen binding domain against ERBB2 (Her2/neu) is an antigen binding portion, e.g., CDRs, of the antibody trastuzumab, or pertuzumab.

In one embodiment, an antigen binding domain against MUC1 is an antigen binding portion, e.g., CDRs, of the antibody SAR566658.

In one embodiment, the antigen binding domain against EGFR is antigen binding portion, e.g., CDRs, of the antibody cetuximab, panitumumab, zalutumumab, nimotuzumab, or matuzumab. In one embodiment, the antigen binding domain against EGFRvIII is or may be derived from an antigen binding domain, e.g., CDRs, scFv, or VH and VL, of an antibody, antigen-binding fragment or CAR described in, e.g., PCT publication WO2014/130657 (In one embodiment the CAR is a CAR described in WO2014/130657, the contents of which are incorporated herein in their entirety).

In one embodiment, an antigen binding domain against NCAM is an antigen binding portion, e.g., CDRs, of the antibody clone 2-2B: MAB5324 (EMD Millipore)
In one embodiment, an antigen binding domain against Ephrin B2 is an antigen binding portion, e.g., CDRs, of an antibody described in, e.g., Abengozar et al., Blood 119(19):4565-4576 (2012).

In one embodiment, an antigen binding domain against IGF-I receptor is an antigen binding portion, e.g., CDRs, of an antibody described in, e.g., US8344112 B2; EP2322550 A1; WO 2006/138315, or PCT/US2006/022995.

In one embodiment, an antigen binding domain against CAIX is an antigen binding portion, e.g., CDRs, of the antibody clone 303123 (R&D Systems).

In one embodiment, an antigen binding domain against LMP2 is an antigen binding portion, e.g., CDRs, of an antibody described in, e.g., US7,410,640, or US20050129701.

In one embodiment, an antigen binding domain against gp100 is an antigen binding portion, e.g., CDRs, of the antibody HMB45, NKIbetaB, or an antibody described in WO2013165940, or US20130295007

In one embodiment, an antigen binding domain against tyrosinase is an antigen binding portion, e.g., CDRs, of an antibody described in, e.g., US5843674; or US 19950504048.

In one embodiment, an antigen binding domain against EphA2 is an antigen binding portion, e.g., CDRs, of an antibody described in, e.g., Yu et al., Mol Ther 22(1):102-111 (2014).

In one embodiment, an antigen binding domain against GD3 is an antigen binding portion, e.g., CDRs, of an antibody described in, e.g., US7253263; US 8,207,308; US 20120276046; EP1013761 A3; 20120276046; WO2005035577; or US6437098.

In one embodiment, an antigen binding domain against fucosyl GM1 is an antigen binding portion, e.g., CDRs, of an antibody described in, e.g., US20100297138; or WO2007/067992.

In one embodiment, an antigen binding domain against sLe is an antigen binding portion, e.g., CDRs, of the antibody G193 (for lewis Y), see Scott AM et al, Cancer Res 60: 3254-61 (2000), also as described in Neeson et al, J Immunol May 2013 190 (Meeting Abstract Supplement) 177.10.

In one embodiment, an antigen binding domain against GM3 is an antigen binding portion, e.g., CDRs, of the antibody CA 2523449 (mAb 14F7).

In one embodiment, an antigen binding domain against HMWMAA is an antigen binding portion, e.g., CDRs, of an antibody described in, e.g., Kmiecik et al., Oncoimmunology 3(1):e27185 (2014) (PMID: 24575382) (mAb9.2.27); US6528481; WO2010033866; or US 20140004124.

In one embodiment, an antigen binding domain against o-acetyl-GD2 is an antigen binding portion, e.g., CDRs, of the antibody 8B6.

In one embodiment, an antigen binding domain against TEM1/CD248 is an antigen binding portion, e.g., CDRs, of an antibody described in, e.g., Marty et al., Cancer Lett 235(2):298-308 (2006); Zhao et al., J Immunol Methods 363(2):221-232 (2011).

In one embodiment, an antigen binding domain against CLDN6 is an antigen binding portion, e.g., CDRs, of the antibody IMAB027 (Ganymed Pharmaceuticals), see e.g., clinicaltrial.gov/show/NCT02054351.

In one embodiment, an antigen binding domain against TSHR is an antigen binding portion, e.g., CDRs, of an antibody described in, e.g., US8,603,466; US8,501,415; or US8,309,693.

In one embodiment, an antigen binding domain against GPRC5D is an antigen binding portion, e.g., CDRs, of the antibody FAB6300A (R&D Systems); or LS-A4180 (Lifespan Biosciences).

In one embodiment, an antigen binding domain against CD97 is an antigen binding portion, e.g., CDRs, of an antibody described in, e.g., US6,846,911;de Groot et al., J Immunol 183(6):4127-4134 (2009); or an antibody from R&D:MAB3734.

In one embodiment, an antigen binding domain against ALK is an antigen binding portion, e.g., CDRs, of an antibody described in, e.g., Mino-Kenudson et al., Clin Cancer Res 16(5):1561-1571 (2010).

In one embodiment, an antigen binding domain against polysialic acid is an antigen binding portion, e.g., CDRs, of an antibody described in, e.g., Nagae et al., J Biol Chem 288(47):33784-33796 (2013).

In one embodiment, an antigen binding domain against PLAC1 is an antigen binding portion, e.g., CDRs, of an antibody described in, e.g., Ghods et al., Biotechnol Appl Biochem 2013 doi:10.1002/bab.1177.

In one embodiment, an antigen binding domain against GloboH is an antigen binding portion of the antibody VK9; or an antibody described in, e.g., Kudryashov V et al, Glycoconj J.15(3):243-9 ( 1998), Lou et al., Proc Natl Acad Sci USA 111(7):2482-2487 (2014) ; MBr1: Bremer E-G et al. J Biol Chem 259:14773-14777 (1984).

In one embodiment, an antigen binding domain against NY-BR-1 is an antigen binding portion, e.g., CDRs of an antibody described in, e.g., Jager et al., Appl Immunohistochem Mol Morphol 15(1):77-83 (2007).

In one embodiment, an antigen binding domain against WT-1 is an antigen binding portion, e.g., CDRs, of an antibody described in, e.g., Dao et al., Sci Transl Med 5(176):176ra33 (2013); or WO2012/135854.

In one embodiment, an antigen binding domain against MAGE-A1 is an antigen binding portion, e.g., CDRs, of an antibody described in, e.g., Willemsen et al., J Immunol 174(12):7853-7858 (2005) (TCR-like scFv).

In one embodiment, an antigen binding domain against sperm protein 17 is an antigen binding portion, e.g., CDRs, of an antibody described in, e.g., Song et al., Target Oncol 2013 Aug 14 (PMID: 23943313); Song et al., Med Oncol 29(4):2923-2931 (2012).

In one embodiment, an antigen binding domain against Tie 2 is an antigen binding portion, e.g., CDRs, of the antibody AB33 (Cell Signaling Technology).

In one embodiment, an antigen binding domain against MAD-CT-2 is an antigen binding portion, e.g., CDRs, of an antibody described in, e.g., PMID: 2450952; US7635753.

In one embodiment, an antigen binding domain against Fos-related antigen 1 is an antigen binding portion, e.g., CDRs, of the antibody 12F9 (Novus Biologicals).

In one embodiment, an antigen binding domain against MelanA/MART1 is an antigen binding portion, e.g., CDRs, of an antibody described in, EP2514766 A2; or US 7,749,719.

In one embodiment, an antigen binding domain against sarcoma translocation breakpoints is an antigen binding portion, e.g., CDRs, of an antibody described in, e.g., Luo et al, EMBO Mol. Med. 4(6):453-461 (2012).

In one embodiment, an antigen binding domain against TRP-2 is an antigen binding portion, e.g., CDRs, of an antibody described in, e.g., Wang et al, J Exp Med. 184(6):2207-16 (1996).

In one embodiment, an antigen binding domain against CYP1B1 is an antigen binding portion, e.g., CDRs, of an antibody described in, e.g., Maecker et al, Blood 102 (9): 3287-3294 (2003).

In one embodiment, an antigen binding domain against RAGE-1 is an antigen binding portion, e.g., CDRs, of the antibody MAB5328 (EMD Millipore).

In one embodiment, an antigen binding domain against human telomerase reverse transcriptase is an antigen binding portion, e.g., CDRs, of the antibody cat no: LS-B95-100

### (Lifespan Biosciences)

In one embodiment, an antigen binding domain against intestinal carboxyl esterase is an antigen binding portion, e.g., CDRs, of the antibody 4F12: cat no: LS-B6190-50 (Lifespan Biosciences).

In one embodiment, an antigen binding domain against mut hsp70-2 is an antigen binding portion, e.g., CDRs, of the antibody Lifespan Biosciences: monoclonal: cat no: LS-C133261-100 (Lifespan Biosciences).

In one embodiment, an antigen binding domain against CD79a is an antigen binding portion, e.g., CDRs, of the antibody Anti-CD79a antibody [HM47/A9] (ab3121), available from Abcam; antibody CD79A Antibody #3351 available from Cell Signalling Technology; or antibody HPA017748 - Anti-CD79A antibody produced in rabbit, available from Sigma Aldrich.

In one embodiment, an antigen binding domain against CD79b is an antigen binding portion, e.g., CDRs, of the antibody polatuzumab vedotin, anti-CD79b described in Dornan et al., "Therapeutic potential of an anti-CD79b antibody-drug conjugate, anti-CD79b-vc-MMAE, for the treatment of non-Hodgkin lymphoma" Blood. 2009 Sep 24;114(13):2721-9. doi: 10.1182/blood-2009-02-205500. Epub 2009 Jul 24, or the bispecific antibody Anti-CD79b/CD3 described in "4507 Pre-Clinical Characterization of T Cell-Dependent Bispecific Antibody Anti-CD79b/CD3 As a Potential Therapy for B Cell Malignancies" Abstracts of 56th ASH Annual Meeting and Exposition, San Francisco, CA December 6-9 2014.

In one embodiment, an antigen binding domain against CD72 is an antigen binding portion, e.g., CDRs, of the antibody J3-109 described in Myers, and Uckun, "An anti-CD72 immunotoxin against therapy-refractory B-lineage acute lymphoblastic leukemia." Leuk Lymphoma. 1995 Jun;18(1-2):119-22, or anti-CD72 (10D6.8.1, mIgG1) described in Polson et al., "Antibody-Drug Conjugates for the Treatment of Non-Hodgkin's Lymphoma: Target and Linker-Drug Selection" Cancer Res March 15, 2009 69; 2358.

In one embodiment, an antigen binding domain against LAIR1 is an antigen binding portion, e.g., CDRs, of the antibody ANT-301 LAIR1 antibody, available from ProSpec; or antihuman CD305 (LAIR1) Antibody, available from BioLegend.

In one embodiment, an antigen binding domain against FCAR is an antigen binding portion, e.g., CDRs, of the antibody CD89/FCARAntibody (Catalog#10414-H08H), available from Sino Biological Inc.

In one embodiment, an antigen binding domain against LILRA2 is an antigen binding portion, e.g., CDRs, of the antibody LILRA2 monoclonal antibody (M17), clone 3C7, available from Abnova, or Mouse Anti-LILRA2 antibody, Monoclonal (2D7), available from Lifespan Biosciences..

In one embodiment, an antigen binding domain against CD300LF is an antigen binding portion, e.g., CDRs, of the antibody Mouse Anti-CMRF35-like molecule 1 antibody, Monoclonal[UP-D2], available from BioLegend, or Rat Anti-CMRF35-like molecule 1 antibody, Monoclonal[234903], available from R&D Systems..

In one embodiment, an antigen binding domain against CLEC12A is an antigen binding portion, e.g., CDRs, of the antibody Bispecific T cell Engager (BiTE) scFv-antibody and ADC described in Noordhuis et al., "Targeting of CLEC12A In Acute Myeloid Leukemia by Antibody-Drug-Conjugates and Bispecific CLL-1xCD3 BiTE Antibody" 53rd ASH Annual Meeting and Exposition, December 10-13, 2011, and MCLA-117 (Merus).

In one embodiment, an antigen binding domain against BST2 (also called CD317) is an antigen binding portion, e.g., CDRs, of the antibody Mouse Anti-CD317 antibody, Monoclonal[3H4], available from Antibodies-Online or Mouse Anti-CD317 antibody, Monoclonal[696739], available from R&D Systems.

In one embodiment, an antigen binding domain against EMR2 (also called CD312) is an antigen binding portion, e.g., CDRs, of the antibody Mouse Anti-CD312 antibody, Monoclonal[LS-B8033] available from Lifespan Biosciences, or Mouse Anti-CD312 antibody, Monoclonal[494025] available from R&D Systems.

In one embodiment, an antigen binding domain against LY75 is an antigen binding portion, e.g., CDRs, of the antibody Mouse Anti-Lymphocyte antigen 75 antibody, Monoclonal[HD30] available from EMD Millipore or Mouse Anti-Lymphocyte antigen 75 antibody, Monoclonal[A15797] available from Life Technologies.

In one embodiment, an antigen binding domain against GPC3 is an antigen binding portion, e.g., CDRs, of the antibody hGC33 described in Nakano K, Ishiguro T, Konishi H, et al. Generation of a humanized anti-glypican 3 antibody by CDR grafting and stability optimization. Anticancer Drugs. 2010 Nov;21(10):907-916, or MDX-1414, HN3, or YP7, all three of which are described in Feng et al., "Glypican-3 antibodies: a new therapeutic target for liver cancer." FEBS Lett. 2014 Jan 21;588(2):377-82.

In one embodiment, an antigen binding domain against FCRL5 is an antigen binding portion, e.g., CDRs, of the anti-FcRL5 antibody described in Elkins et al., "FcRL5 as a target of antibody-drug conjugates for the treatment of multiple myeloma" Mol Cancer Ther. 2012 Oct;11(10):2222-32..

In one embodiment, an antigen binding domain against IGLL1 is an antigen binding portion, e.g., CDRs, of the antibody Mouse Anti-Immunoglobulin lambda-like polypeptide 1 antibody, Monoclonal[AT1G4] available from Lifespan Biosciences, Mouse Anti-Immunoglobulin lambda-like polypeptide 1 antibody, Monoclonal[HSL11] available from BioLegend.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed above.

In another aspect, the antigen binding domain comprises a humanized antibody or an antibody fragment. In some aspects, a non-human antibody is humanized, where specific sequences or regions of the antibody are modified to increase similarity to an antibody naturally produced in a human or fragment thereof. In one aspect, the antigen binding domain is humanized.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089, each of which is incorporated herein in its entirety by reference), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973, each of which is incorporated herein by its entirety by reference), chain shuffling (see, e.g., U.S. Pat. No. 5,565,332, which is incorporated herein in its entirety by reference), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16):10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu J S, Gene, 150(2):409-10 (1994), and Pedersen et al., J. Mol. Biol., 235(3):959-73 (1994), each of which is incorporated herein in its entirety by reference. Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323, which are incorporated herein by reference in their entireties.)

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments comprise one or more CDRs from nonhuman immunoglobulin molecules and framework regions wherein the amino acid residues comprising the framework are derived completely or mostly from human germline. Multiple techniques for humanization of antibodies or antibody fragments are well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640, the contents of which are incorporated herein by reference herein in their entirety). In such humanized antibodies and antibody fragments, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies. Humanization of antibodies and antibody fragments can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332), the contents of which are incorporated herein by reference herein in their entirety.

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987), the contents of which are incorporated herein by reference herein in their entirety). Another method uses a particular framework derived from the consensus sequence of all human antibodies of a particular subgroup of light or heavy chains. The same framework may be used for several different humanized antibodies (see, e.g., Nicholson et al. Mol. Immun. 34 (16-17): 1157-1165 (1997); Carter et al., Proc. Natl. Acad. Sci. USA, 89:4285 (1992); Presta et al., J. Immunol., 151:2623 (1993), the contents of which are incorporated herein by reference herein in their entirety). In some embodiments, the framework region, e.g., all four framework regions, of the heavy chain variable region are derived from a VH4_4-59 germline sequence. In one embodiment, the framework region can comprise, one, two, three, four or five modifications, e.g., substitutions, e.g., from the amino acid at the corresponding murine sequence. In one embodiment, the framework region, e.g., all four framework regions of the light chain variable region are derived from a VK3_1.25 germline sequence. In one embodiment, the framework region can comprise, one, two, three, four or five modifications, e.g., substitutions, e.g., from the amino acid at the corresponding murine sequence.

In some aspects, the portion of a CAR composition of the invention that comprises an antibody fragment is humanized with retention of high affinity for the target antigen and other favorable biological properties. According to one aspect of the invention, humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

A humanized antibody or antibody fragment may retain a similar antigenic specificity as the original antibody, e.g., in the present invention, the ability to bind human a cancer associated antigen as described herein. In some embodiments, a humanized antibody or antibody fragment may have improved affinity and/or specificity of binding to human a cancer associated antigen as described herein.

In one aspect, the antigen binding domain of the invention is characterized by particular functional features or properties of an antibody or antibody fragment. For example, in one aspect, the portion of a CAR composition of the invention that comprises an antigen binding domain specifically binds a tumor antigen as described herein.

In one aspect, the anti-cancer associated antigen as described herein binding domain is a fragment, e.g., a single chain variable fragment (scFv). In one aspect, the anti- cancer associated antigen as described herein binding domain is a Fv, a Fab, a (Fab')2, or a bi-functional (e.g. bi-specific) hybrid antibody (e.g., Lanzavecchia et al., Eur. J. Immunol. 17, 105 (1987)). In one aspect, the antibodies and fragments thereof of the invention binds a cancer associated antigen as described herein protein with wild-type or enhanced affinity.

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715, is incorporated herein by reference.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:22). In one embodiment, the linker can be (Gly₄Ser)₄ (SEQ ID NO:29) or (Gly₄Ser)₃(SEQ ID NO:30). Variation in the linker length may retain or enhance activity, giving rise to superior efficacy in activity studies.

In another aspect, the antigen binding domain is a T cell receptor ("TCR"), or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012) (references are incorporated herein by its entirety). For example, scTCR can be engineered that contains the Vα and Vβ genes from a T cell clone linked by a linker (e.g., a flexible peptide). This approach is very useful to cancer associated target that itself is intracellar, however, a fragment of such antigen (peptide) is presented on the surface of the cancer cells by MHC.

### Bispecific CARs

In an embodiment a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an embodiment the first and second epitopes are on the same antigen, e.g., the same protein (or subunit of a multimeric protein). In an embodiment the first and second epitopes overlap. In an embodiment the first and second epitopes do not overlap. In an embodiment the first and second epitopes are on different antigens, e.g., different proteins (or different subunits of a multimeric protein). In an embodiment a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an embodiment a bispecific antibody molecule comprises a half antibody having binding specificity for a first epitope and a half antibody having binding specificity for a second epitope. In an embodiment a bispecific antibody molecule comprises a half antibody, or fragment thereof, having binding specificity for a first epitope and a half antibody, or fragment thereof, having binding specificity for a second epitope. In an embodiment a bispecific antibody molecule comprises a scFv, or fragment thereof, have binding specificity for a first epitope and a scFv, or fragment thereof, have binding specificity for a second epitope.

In certain embodiments, the antibody molecule is a multi-specific (e.g., a bispecific or a trispecific) antibody molecule. Protocols for generating bispecific or heterodimeric antibody molecules are known in the art; including but not limited to, for example, the "knob in a hole" approach described in, *e.g.,* US 5731168; the electrostatic steering Fc pairing as described in, *e.g.,* WO 09/089004, WO 06/106905 and WO 2010/129304; Strand Exchange Engineered Domains (SEED) heterodimer formation as described in, *e.g.,* WO 07/110205; Fab arm exchange as described in, *e.g.,* WO 08/119353, WO 2011/131746, and WO 2013/060867; double antibody conjugate, *e.g.,* by antibody cross-linking to generate a bi-specific structure using a heterobifunctional reagent having an amine-reactive group and a sulfhydryl reactive group as described in, *e.g.,* US 4433059; bispecific antibody determinants generated by recombining half antibodies (heavy-light chain pairs or Fabs) from different antibodies through cycle of reduction and oxidation of disulfide bonds between the two heavy chains, as described in, *e.g.,* US 4444878; trifunctional antibodies, *e.g.,* three Fab' fragments cross-linked through sulfhdryl reactive groups, as described in, *e.g.,* US5273743; biosynthetic binding proteins, *e.g.,* pair of scFvs cross-linked through C-terminal tails preferably through disulfide or amine-reactive chemical cross-linking, as described in, *e.g.,* US5534254; bifunctional antibodies, *e.g.,* Fab fragments with different binding specificities dimerized through leucine zippers (*e.g.*, c-fos and c-jun) that have replaced the constant domain, as described in, *e.g*., US5582996; bispecific and oligospecific mono-and oligovalent receptors, *e.g*., VH-CH1 regions of two antibodies (two Fab fragments) linked through a polypeptide spacer between the CH1 region of one antibody and the VH region of the other antibody typically with associated light chains, as described in, *e.g.,* US5591828; bispecific DNA-antibody conjugates, *e.g*., crosslinking of antibodies or Fab fragments through a double stranded piece of DNA, as described in, *e.g*., US5635602; bispecific fusion proteins, *e.g*., an expression construct containing two scFvs with a hydrophilic helical peptide linker between them and a full constant region, as described in, *e.g*., US5637481; multivalent and multispecific binding proteins, *e.g*., dimer of polypeptides having first domain with binding region of Ig heavy chain variable region, and second domain with binding region of Ig light chain variable region, generally termed diabodies (higher order structures are also encompassed creating for bispecifc, trispecific, or tetraspecific molecules, as described in, *e.g.,* US5837242; minibody constructs with linked VL and VH chains further connected with peptide spacers to an antibody hinge region and CH3 region, which can be dimerized to form bispecific/multivalent molecules, as described in, *e.g*., US5837821; VH and VL domains linked with a short peptide linker (*e.g.,* 5 or 10 amino acids) or no linker at all in either orientation, which can form dimers to form bispecific diabodies; trimers and tetramers, as described in, *e.g.,* US5844094; String of VH domains (or VL domains in family members) connected by peptide linkages with crosslinkable groups at the C-terminus futher associated with VL domains to form a series of FVs (or scFvs), as described in, e.g., US5864019; and single chain binding polypeptides with both a VH and a VL domain linked through a peptide linker are combined into multivalent structures through non-covalent or chemical crosslinking to form, *e.g.,* homobivalent, heterobivalent, trivalent, and tetravalent structures using both scFV or diabody type format, as described in, e.g., US5869620. Additional exemplary multispecific and bispecific molecules and methods of making the same are found, for example, in US5910573, US5932448, US5959083, US5989830, US6005079, US6239259, US6294353, US6333396, US6476198, US6511663, US6670453, US6743896, US6809185, US6833441, US7129330, US7183076, US7521056, US7527787, US7534866, US7612181, US2002004587A1, US2002076406A1, US2002103345A1, US2003207346A1, US2003211078A1, US2004219643A1, US2004220388A1, US2004242847A1, US2005003403A1, US2005004352A1, US2005069552A1, US2005079170A1, US2005100543A1, US2005136049A1, US2005136051A1, US2005163782A1, US2005266425A1, US2006083747A1, US2006120960A1, US2006204493A1, US2006263367A1, US2007004909A1, US2007087381A1, US2007128150A1, US2007141049A1, US2007154901A1, US2007274985A1, US2008050370A1, US2008069820A1, US2008152645A1, US2008171855A1, US2008241884A1, US2008254512A1, US2008260738A1, US2009130106A1, US2009148905A1, US2009155275A1, US2009162359A1, US2009162360A1, US2009175851A1, US2009175867A1, US2009232811A1, US2009234105A1, US2009263392A1, US2009274649A1, EP346087A2, WO0006605A2, WO02072635A2, WO04081051A1, WO06020258A2, WO2007044887A2, WO2007095338A2, WO2007137760A2, WO2008119353A1, WO2009021754A2, WO2009068630A1, WO9103493A1, WO9323537A1, WO9409131A1, WO9412625A2, WO9509917A1, WO9637621A2, WO9964460A1. The contents of the above-referenced applications are incorporated herein by reference in their entireties.

Within each antibody or antibody fragment (e.g., scFv) of a bispecific antibody molecule, the VH can be upstream or downstream of the VL. In some embodiments, the upstream antibody or antibody fragment (e.g., scFv) is arranged with its VH (VH₁) upstream of its VL (VL₁) and the downstream antibody or antibody fragment (e.g., scFv) is arranged with its VL (VL₂) upstream of its VH (VH₂), such that the overall bispecific antibody molecule has the arrangement VH₁-VL₁-VL₂-VH₂. In other embodiments, the upstream antibody or antibody fragment (e.g., scFv) is arranged with its VL (VL₁) upstream of its VH (VH₁) and the downstream antibody or antibody fragment (e.g., scFv) is arranged with its VH (VH₂) upstream of its VL (VL₂), such that the overall bispecific antibody molecule has the arrangement VL₁-VH₁-VH₂-VL₂. Optionally, a linker is disposed between the two antibodies or antibody fragments (e.g., scFvs), e.g., between VL₁ and VL₂ if the construct is arranged as VH₁-VL₁-VL₂-VH₂, or between VH₁ and VH₂ if the construct is arranged as VL₁-VH₁-VH₂-VL₂. The linker may be a linker as described herein, e.g., a (Gly₄-Ser)n linker, wherein n is 1, 2, 3, 4, 5, or 6, preferably 4 (SEQ ID NO: 72). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some embodiments, a bispecific CAR comprises VLs, VHs, and optionally one or more linkers in an arrangement as described herein.

### Stability and Mutations

The stability of an antigen binding domain to a cancer associated antigen as described herein, e.g., scFv molecules (e.g., soluble scFv), can be evaluated in reference to the biophysical properties (e.g., thermal stability) of a conventional control scFv molecule or a full length antibody. In one embodiment, the humanized scFv has a thermal stability that is greater than about 0.1, about 0.25, about 0.5, about 0.75, about 1, about 1.25, about 1.5, about 1.75, about 2, about 2.5, about 3, about 3.5, about 4, about 4.5, about 5, about 5.5, about 6, about 6.5, about 7, about 7.5, about 8, about 8.5, about 9, about 9.5, about 10 degrees, about 11 degrees, about 12 degrees, about 13 degrees, about 14 degrees, or about 15 degrees Celsius than a control binding molecule (e.g. a conventional scFv molecule) in the described assays.

The improved thermal stability of the antigen binding domain to a cancer associated antigen described herein, e.g., scFv is subsequently conferred to the entire CAR construct, leading to improved therapeutic properties of the CAR construct. The thermal stability of the antigen binding domain of -a cancer associated antigen described herein, e.g., scFv, can be improved by at least about 2°C or 3°C as compared to a conventional antibody. In one embodiment, the antigen binding domain of-a cancer associated antigen described herein, e.g., scFv, has a 1°C improved thermal stability as compared to a conventional antibody. In another embodiment, the antigen binding domain of a cancer associated antigen described herein, e.g., scFv, has a 2°C improved thermal stability as compared to a conventional antibody. In another embodiment, the scFv has a 4, 5, 6, 7, 8, 9, 10, 11, 12, 13, 14, 15°C improved thermal stability as compared to a conventional antibody. Comparisons can be made, for example, between the scFv molecules disclosed herein and scFv molecules or Fab fragments of an antibody from which the scFv VH and VL were derived. Thermal stability can be measured using methods known in the art. For example, in one embodiment, Tm can be measured. Methods for measuring Tm and other methods of determining protein stability are described in more detail below.

Mutations in scFv (arising through humanization or direct mutagenesis of the soluble scFv) can alter the stability of the scFv and improve the overall stability of the scFv and the CAR construct. Stability of the humanized scFv is compared against the murine scFv using measurements such as Tm, temperature denaturation and temperature aggregation.

The binding capacity of the mutant scFvs can be determined using assays know in the art and described herein.

In one embodiment, the antigen binding domain of -a cancer associated antigen described herein, e.g., scFv, comprises at least one mutation arising from the humanization process such that the mutated scFv confers improved stability to the CAR construct. In another embodiment, the antigen binding domain of -a cancer associated antigen described herein, e.g., scFv, comprises at least 1, 2, 3, 4, 5, 6, 7, 8, 9, 10 mutations arising from the humanization process such that the mutated scFv confers improved stability to the CAR construct.

### Methods of Evaluating Protein Stability

The stability of an antigen binding domain may be assessed using, e.g., the methods described below. Such methods allow for the determination of multiple thermal unfolding transitions where the least stable domain either unfolds first or limits the overall stability threshold of a multidomain unit that unfolds cooperatively (e.g., a multidomain protein which exhibits a single unfolding transition). The least stable domain can be identified in a number of additional ways. Mutagenesis can be performed to probe which domain limits the overall stability. Additionally, protease resistance of a multidomain protein can be performed under conditions where the least stable domain is known to be intrinsically unfolded via DSC or other spectroscopic methods (Fontana, et al., (1997) Fold. Des., 2: R17-26; Dimasi et al. (2009) J. Mol. Biol. 393: 672-692). Once the least stable domain is identified, the sequence encoding this domain (or a portion thereof) may be employed as a test sequence in the methods.

### a) Thermal Stability

The thermal stability of the compositions may be analyzed using a number of non-limiting biophysical or biochemical techniques known in the art. In certain embodiments, thermal stability is evaluated by analytical spectroscopy.

An exemplary analytical spectroscopy method is Differential Scanning Calorimetry (DSC). DSC employs a calorimeter which is sensitive to the heat absorbances that accompany the unfolding of most proteins or protein domains (see, e.g. Sanchez-Ruiz, et al., Biochemistry, 27: 1648-52, 1988). To determine the thermal stability of a protein, a sample of the protein is inserted into the calorimeter and the temperature is raised until the Fab or scFv unfolds. The temperature at which the protein unfolds is indicative of overall protein stability.

Another exemplary analytical spectroscopy method is Circular Dichroism (CD) spectroscopy. CD spectrometry measures the optical activity of a composition as a function of increasing temperature. Circular dichroism (CD) spectroscopy measures differences in the absorption of left-handed polarized light versus right-handed polarized light which arise due to structural asymmetry. A disordered or unfolded structure results in a CD spectrum very different from that of an ordered or folded structure. The CD spectrum reflects the sensitivity of the proteins to the denaturing effects of increasing temperature and is therefore indicative of a protein's thermal stability (see van Mierlo and Steemsma, J. Biotechnol., 79(3):281-98, 2000).

Another exemplary analytical spectroscopy method for measuring thermal stability is Fluorescence Emission Spectroscopy (see van Mierlo and Steemsma, supra). Yet another exemplary analytical spectroscopy method for measuring thermal stability is Nuclear Magnetic Resonance (NMR) spectroscopy (see, e.g. van Mierlo and Steemsma, supra).

The thermal stability of a composition can be measured biochemically. An exemplary biochemical method for assessing thermal stability is a thermal challenge assay. In a "thermal challenge assay", a composition is subjected to a range of elevated temperatures for a set period of time. For example, in one embodiment, test scFv molecules or molecules comprising scFv molecules are subject to a range of increasing temperatures, e.g., for 1-1.5 hours. The activity of the protein is then assayed by a relevant biochemical assay. For example, if the protein is a binding protein (e.g. an scFv or scFv-containing polypeptide) the binding activity of the binding protein may be determined by a functional or quantitative ELISA.

Such an assay may be done in a high-throughput format and those disclosed in the Examples using *E. coli* and high throughput screening. A library of antigen binding domains, e.g., that includes an antigen binding domain to -a cancer associated antigen described herein, e.g., scFv variants, may be created using methods known in the art. Antigen binding domain, e.g., to -a cancer associated antigen described herein, e.g., scFv, expression may be induced and the antigen binding domain, e.g., to -a cancer associated antigen described herein, e.g., scFv, may be subjected to thermal challenge. The challenged test samples may be assayed for binding and those antigen binding domains to -a cancer associated antigen described herein, e.g., scFvs, which are stable may be scaled up and further characterized.

Thermal stability is evaluated by measuring the melting temperature (Tm) of a composition using any of the above techniques (e.g. analytical spectroscopy techniques). The melting temperature is the temperature at the midpoint of a thermal transition curve wherein 50% of molecules of a composition are in a folded state (See e.g., Dimasi et al. (2009) J. Mol Biol. 393: 672-692). In one embodiment, Tm values for an antigen binding domain to -a cancer associated antigen described herein, e.g., scFv, are about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, 100°C. In one embodiment, Tm values for an IgG is about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, 100°C. In one embodiment, Tm values for an multivalent antibody is about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, 100°C.

Thermal stability is also evaluated by measuring the specific heat or heat capacity (Cp) of a composition using an analytical calorimetric technique (e.g. DSC). The specific heat of a composition is the energy (e.g. in kcal/mol) is required to rise by 1°C, the temperature of 1 mol of water. As large Cp is a hallmark of a denatured or inactive protein composition. The change in heat capacity (ΔCp) of a composition is measured by determining the specific heat of a composition before and after its thermal transition. Thermal stability may also be evaluated by measuring or determining other parameters of thermodynamic stability including Gibbs free energy of unfolding (ΔG), enthalpy of unfolding (ΔH), or entropy of unfolding (ΔS). One or more of the above biochemical assays (e.g. a thermal challenge assay) are used to determine the temperature (i.e. the T_{C} value) at which 50% of the composition retains its activity (e.g. binding activity).

In addition, mutations to the antigen binding domain of a cancer associated antigen described herein, e.g., scFv, can be made to alter the thermal stability of the antigen binding domain of a cancer associated antigen described herein, e.g., scFv, as compared with the unmutated antigen binding domain of a cancer associated antigen described herein, e.g., scFv. When the humanized antigen binding domain of a cancer associated antigen described herein, e.g., scFv, is incorporated into a CAR construct, the antigen binding domain of the cancer associated antigen described herein, e.g., humanized scFv, confers thermal stability to the overall CARs of the present invention. In one embodiment, the antigen binding domain to a cancer associated antigen described herein, e.g., scFv, comprises a single mutation that confers thermal stability to the antigen binding domain of the cancer associated antigen described herein, e.g., scFv. In another embodiment, the antigen binding domain to a cancer associated antigen described herein, e.g., scFv, comprises multiple mutations that confer thermal stability to the antigen binding domain to the cancer associated antigen described herein, e.g., scFv. In one embodiment, the multiple mutations in the antigen binding domain to a cancer associated antigen described herein, e.g., scFv, have an additive effect on thermal stability of the antigen binding domain to the cancer associated antigen described herein binding domain, e.g., scFv.

### b) % Aggregation

The stability of a composition can be determined by measuring its propensity to aggregate. Aggregation can be measured by a number of non-limiting biochemical or biophysical techniques. For example, the aggregation of a composition may be evaluated using chromatography, e.g. Size-Exclusion Chromatography (SEC). SEC separates molecules on the basis of size. A column is filled with semi-solid beads of a polymeric gel that will admit ions and small molecules into their interior but not large ones. When a protein composition is applied to the top of the column, the compact folded proteins (i.e. non-aggregated proteins) are distributed through a larger volume of solvent than is available to the large protein aggregates. Consequently, the large aggregates move more rapidly through the column, and in this way the mixture can be separated or fractionated into its components. Each fraction can be separately quantified (e.g. by light scattering) as it elutes from the gel. Accordingly, the % aggregation of a composition can be determined by comparing the concentration of a fraction with the total concentration of protein applied to the gel. Stable compositions elute from the column as essentially a single fraction and appear as essentially a single peak in the elution profile or chromatogram.

### c) Binding Affinity

The stability of a composition can be assessed by determining its target binding affinity. A wide variety of methods for determining binding affinity are known in the art. An exemplary method for determining binding affinity employs surface plasmon resonance. Surface plasmon resonance is an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, N.J.). For further descriptions, see Jonsson, U., et al. (1993) Ann. Biol. Clin. 51:19-26; Jonsson, U., i (1991) Biotechniques 11:620-627; Johnsson, B., et al. (1995) J. Mol. Recognit. 8:125-131; and Johnnson, B., et al. (1991) Anal. Biochem. 198:268-277.

In one aspect, the antigen binding domain of the CAR comprises an amino acid sequence that is homologous to an antigen binding domain amino acid sequence described herein, and the antigen binding domain retains the desired functional properties of the antigen binding domain described herein.

In one specific aspect, the CAR composition of the invention comprises an antibody fragment. In a further aspect, the antibody fragment comprises an scFv.

In various aspects, the antigen binding domain of the CAR is engineered by modifying one or more amino acids within one or both variable regions (e.g., VH and/or VL), for example within one or more CDR regions and/or within one or more framework regions. In one specific aspect, the CAR composition of the invention comprises an antibody fragment. In a further aspect, the antibody fragment comprises an scFv.

It will be understood by one of ordinary skill in the art that the antibody or antibody fragment of the invention may further be modified such that they vary in amino acid sequence (e.g., from wild-type), but not in desired activity. For example, additional nucleotide substitutions leading to amino acid substitutions at "non-essential" amino acid residues may be made to the protein For example, a nonessential amino acid residue in a molecule may be replaced with another amino acid residue from the same side chain family. In another embodiment, a string of amino acids can be replaced with a structurally similar string that differs in order and/or composition of side chain family members, e.g., a conservative substitution, in which an amino acid residue is replaced with an amino acid residue having a similar side chain, may be made.

Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Percent identity in the context of two or more nucleic acids or polypeptide sequences, refers to two or more sequences that are the same. Two sequences are "substantially identical" if two sequences have a specified percentage of amino acid residues or nucleotides that are the same (e.g., 60% identity, optionally 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

In one aspect, the present invention contemplates modifications of the starting antibody or fragment (e.g., scFv) amino acid sequence that generate functionally equivalent molecules. For example, the VH or VL of an antigen binding domain to -a cancer associated antigen described herein, e.g., scFv, comprised in the CAR can be modified to retain at least about 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity of the starting VH or VL framework region of the antigen binding domain to the cancer associated antigen described herein, e.g., scFv. The present invention contemplates modifications of the entire CAR construct, e.g., modifications in one or more amino acid sequences of the various domains of the CAR construct in order to generate functionally equivalent molecules. The CAR construct can be modified to retain at least about 70%, 71%. 72%. 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity of the starting CAR construct.

### Transmembrane domain

With respect to the transmembrane domain, in various embodiments, a CAR can be designed to comprise a transmembrane domain that is attached to the extracellular domain of the CAR. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR e.g., in one embodiment, the transmembrane domain may be from the same protein that the signaling domain, costimulatory domain or the hinge domain is derived from. In another aspect, the transmembrane domain is not derived from the same protein that any other domain of the CAR is derived from. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the cell surface of a CAR-expressing cell. In a different aspect, the amino acid sequence of the transmembrane domain may be modified or substituted so as to minimize interactions with the binding domains of the native binding partner present in the same CAR-expressing cell.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. A transmembrane domain of particular use in this invention may include at least the transmembrane region(s) of e.g., the alpha, beta or zeta chain of the T-cell receptor, CD28, CD27, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C.

In some instances, the transmembrane domain can be attached to the extracellular region of the CAR, e.g., the antigen binding domain of the CAR, via a hinge, e.g., a hinge from a human protein. For example, in one embodiment, the hinge can be a human Ig (immunoglobulin) hinge (e.g., an IgG4 hinge, an IgD hinge), a GS linker (e.g., a GS linker described herein), a KIR2DS2 hinge or a CD8a hinge. In one embodiment, the hinge or spacer comprises (e.g., consists of) the amino acid sequence of SEQ ID NO:4. In one aspect, the transmembrane domain comprises (e.g., consists of) a transmembrane domain of SEQ ID NO: 12.

In one aspect, the hinge or spacer comprises an IgG4 hinge. For example, in one embodiment, the hinge or spacer comprises a hinge of the amino acid sequence ESKYGPPCPPCPAPEFLGGPSVFLFPPKPKDTLMISRTPEVTCVVVDVSQEDPEVQFNWY VDGVEVHNAKTKPREEQFNSTYRVVSVLTVLHQDWLNGKEYKCKVSNKGLPSSIEKTIS KAKGQPREPQVYTLPPSQEEMTKNQVSLTCLVKGFYPSDIAVEWESNGQPENNYKTTPP VLDSDGSFFLYSRLTVDKSRWQEGNVFSCSVMHEALHNHYTQKSLSLSLGKM (SEQ ID NO:6). In some embodiments, the hinge or spacer comprises a hinge encoded by a nucleotide sequence of

GAGAGCAAGTACGGCCCTCCCTGCCCCCCTTGCCCTGCCCCCGAGTTCCTGGGCGGA
CCCAGCGTGTTCCTGTTCCCCCCCAAGCCCAAGGACACCCTGATGATCAGCCGGACC
CCCGAGGTGACCTGTGTGGTGGTGGACGTGTCCCAGGAGGACCCCGAGGTCCAGTT
CAACTGGTACGTGGACGGCGTGGAGGTGCACAACGCCAAGACCAAGCCCCGGGAG
GAGCAGTTCAATAGCACCTACCGGGTGGTGTCCGTGCTGACCGTGCTGCACCAGGA
CTGGCTGAACGGCAAGGAATACAAGTGTAAGGTGTCCAACAAGGGCCTGCCCAGCA
GCATCGAGAAAACCATCAGCAAGGCCAAGGGCCAGCCTCGGGAGCCCCAGGTGTAC
ACCCTGCCCCCTAGCCAAGAGGAGATGACCAAGAACCAGGTGTCCCTGACCTGCCT
GGTGAAGGGCTTCTACCCCAGCGACATCGCCGTGGAGTGGGAGAGCAACGGCCAGC
CCGAGAACAACTACAAGACCACCCCCCCTGTGCTGGACAGCGACGGCAGCTTCTTC
CTGTACAGCCGGCTGACCGTGGACAAGAGCCGGTGGCAGGAGGGCAACGTCTTTAG
CTGCTCCGTGATGCACGAGGCCCTGCACAACCACTACACCCAGAAGAGCCTGAGCC
TGTCCCTGGGCAAGATG (SEQ ID NO:7).

In one aspect, the hinge or spacer comprises an IgD hinge. For example, in one embodiment, the hinge or spacer comprises a hinge of the amino acid sequence 
RWPESPKAQASSVPTAQPQAEGSLAKATTAPATTRNTGRGGEEKKKEKEKEEQEERETK
TPECPSHTQPLGVYLLTPAVQDLWLRDKATFTCFVVGSDLKDAHLTWEVAGKVPTGGV
EEGLLERHSNGSQSQHSRLTLPRSLWNAGTSVTCTLNHPSLPPQRLMALREPAAQAPVK
LSLNLLASSDPPEAASWLLCEVSGFSPPNILLMWLEDQREVNTSGFAPARPPPQPGSTTF
WAWSVLRVPAPPSPQPATYTCVVSHEDSRTLLNASRSLEVSYVTDH (SEQ ID NO:8). In
 some embodiments, the hinge or spacer comprises a hinge encoded by a nucleotide sequence of

AGGTGGCCCGAAAGTCCCAAGGCCCAGGCATCTAGTGTTCCTACTGCACAGCCCCA
GGCAGAAGGCAGCCTAGCCAAAGCTACTACTGCACCTGCCACTACGCGCAATACTG
GCCGTGGCGGGGAGGAGAAGAAAAAGGAGAAAGAGAAAGAAGAACAGGAAGAGA
GGGAGACCAAGACCCCTGAATGTCCATCCCATACCCAGCCGCTGGGCGTCTATCTCT
TGACTCCCGCAGTACAGGACTTGTGGCTTAGAGATAAGGCCACCTTTACATGTTTCG
TCGTGGGCTCTGACCTGAAGGATGCCCATTTGACTTGGGAGGTTGCCGGAAAGGTAC
CCACAGGGGGGGTTGAGGAAGGGTTGCTGGAGCGCCATTCCAATGGCTCTCAGAGC

CAGCACTCAAGACTCACCCTTCCGAGATCCCTGTGGAACGCCGGGACCTCTGTCACA
TGTACTCTAAATCATCCTAGCCTGCCCCCACAGCGTCTGATGGCCCTTAGAGAGCCA
GCCGCCCAGGCACCAGTTAAGCTTAGCCTGAATCTGCTCGCCAGTAGTGATCCCCCA
GAGGCCGCCAGCTGGCTCTTATGCGAAGTGTCCGGCTTTAGCCCGCCCAACATCTTG
CTCATGTGGCTGGAGGACCAGCGAGAAGTGAACACCAGCGGCTTCGCTCCAGCCCG
GCCCCCACCCCAGCCGGGTTCTACCACATTCTGGGCCTGGAGTGTCTTAAGGGTCCC
AGCACCACCTAGCCCCCAGCCAGCCACATACACCTGTGTTGTGTCCCATGAAGATAG
CAGGACCCTGCTAAATGCTTCTAGGAGTCTGGAGGTTTCCTACGTGACTGACCATT
(SEQ ID NO:9).

In one aspect, the transmembrane domain may be recombinant, in which case it will comprise predominantly hydrophobic residues such as leucine and valine. In one aspect a triplet of phenylalanine, tryptophan and valine can be found at each end of a recombinant transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO: 10). In some embodiments, the linker is encoded by a nucleotide sequence of GGTGGCGGAGGTTCTGGAGGTGGAGGTTCC (SEQ ID NO: 11).

In one aspect, the hinge or spacer comprises a KIR2DS2 hinge.

### Cytoplasmic domain

The cytoplasmic domain or region of the CAR includes an intracellular signaling domain. An intracellular signaling domain is generally responsible for activation of at least one of the normal effector functions of the immune cell in which the CAR has been introduced. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

Examples of intracellular signaling domains for use in the CAR of the invention include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are insufficient for full activation of the T cell and that a secondary and/or costimulatory signal is also required. Thus, T cell activation can be said to be mediated by two distinct classes of cytoplasmic signaling sequences: those that initiate antigen-dependent primary activation through the TCR (primary intracellular signaling domains) and those that act in an antigen-independent manner to provide a secondary or costimulatory signal (secondary cytoplasmic domain, e.g., a costimulatory domain).

A primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs.

Examples of ITAM containing primary intracellular signaling domains that are of particular use in the invention include those of CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12. In one embodiment, a CAR of the invention comprises an intracellular signaling domain, e.g., a primary signaling domain of CD3-zeta.

In one embodiment, a primary signaling domain comprises a modified ITAM domain, e.g., a mutated ITAM domain which has altered (e.g., increased or decreased) activity as compared to the native ITAM domain. In one embodiment, a primary signaling domain comprises a modified ITAM-containing primary intracellular signaling domain, e.g., an optimized and/or truncated ITAM-containing primary intracellular signaling domain. In an embodiment, a primary signaling domain comprises one, two, three, four or more ITAM motifs.

The intracellular signalling domain of the CAR can comprise the CD3-zeta signaling domain by itself or it can be combined with any other desired intracellular signaling domain(s) useful in the context of a CAR of the invention. For example, the intracellular signaling domain of the CAR can comprise a CD3 zeta chain portion and a costimulatory signaling domain. The costimulatory signaling domain refers to a portion of the CAR comprising the intracellular domain of a costimulatory molecule. A costimulatory molecule is a cell surface molecule other than an antigen receptor or its ligands that is required for an efficient response of lymphocytes to an antigen. Examples of such molecules include CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83, and the like. For example, CD27 costimulation has been demonstrated to enhance expansion, effector function, and survival of human CART cells in vitro and augments human T cell persistence and antitumor activity in vivo (Song et al. Blood. 2012; 119(3):696-706). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), NKG2D, CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, and CD19a.

The intracellular signaling sequences within the cytoplasmic portion of the CAR of the invention may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequence. In one embodiment, a glycine-serine doublet can be used as a suitable linker. In one embodiment, a single amino acid, e.g., an alanine, a glycine, can be used as a suitable linker.

In one aspect, the intracellular signaling domain is designed to comprise two or more, e.g., 2, 3, 4, 5, or more, costimulatory signaling domains. In an embodiment, the two or more, e.g., 2, 3, 4, 5, or more, costimulatory signaling domains, are separated by a linker molecule, e.g., a linker molecule described herein. In one embodiment, the intracellular signaling domain comprises two costimulatory signaling domains. In some embodiments, the linker molecule is a glycine residue. In some embodiments, the linker is an alanine residue.

In one aspect, the intracellular signaling domain is designed to comprise the signaling domain of CD3-zeta and the signaling domain of CD28. In one aspect, the intracellular signaling domain is designed to comprise the signaling domain of CD3-zeta and the signaling domain of 4-1BB. In one aspect, the signaling domain of 4-1BB is a signaling domain of SEQ ID NO: 14. In one aspect, the signaling domain of CD3-zeta is a signaling domain of SEQ ID NO: 18.

In one aspect, the intracellular signaling domain is designed to comprise the signaling domain of CD3-zeta and the signaling domain of CD27. In one aspect, the signaling domain of CD27 comprises an amino acid sequence of QRRKYRSNKGESPVEPAEPCRYSCPREEEGSTIPIQEDYRKPEPACSP (SEQ ID NO: 16). In one aspect, the signalling domain of CD27 is encoded by a nucleic acid sequence of

AGGAGTAAGAGGAGCAGGCTCCTGCACAGTGACTACATGAACATGACTCCCCGCCG
CCCCGGGCCCACCCGCAAGCATTACCAGCCCTATGCCCCACCACGCGACTTCGCAGC
CTATCGCTCC (SEQ ID NO: 17).

In one aspect, the CAR-expressing cell described herein can further comprise a second CAR, e.g., a second CAR that includes a different antigen binding domain, e.g., to the same target or a different target (e.g., a target other than a cancer associated antigen described herein or a different cancer associated antigen described herein). In one embodiment, the second CAR includes an antigen binding domain to a target expressed the same cancer cell type as the cancer associated antigen. In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. While not wishing to be bound by theory, placement of a costimulatory signaling domain, e.g., 4-1BB, CD28, CD27 or OX-40, onto the first CAR, and the primary signaling domain, e.g., CD3 zeta, on the second CAR can limit the CAR activity to cells where both targets are expressed. In one embodiment, the CAR expressing cell comprises a first cancer associated antigen CAR that includes an antigen binding domain that binds a target antigen described herein, a transmembrane domain and a costimulatory domain and a second CAR that targets a different target antigen (e.g., an antigen expressed on that same cancer cell type as the first target antigen) and includes an antigen binding domain, a transmembrane domain and a primary signaling domain. In another embodiment, the CAR expressing cell comprises a first CAR that includes an antigen binding domain that binds a target antigen described herein, a transmembrane domain and a primary signaling domain and a second CAR that targets an antigen other than the first target antigen (e.g., an antigen expressed on the same cancer cell type as the first target antigen) and includes an antigen binding domain to the antigen, a transmembrane domain and a costimulatory signaling domain.

In one embodiment, the CAR-expressing cell comprises an XCAR described herein and an inhibitory CAR. In one embodiment, the inhibitory CAR comprises an antigen binding domain that binds an antigen found on normal cells but not cancer cells, e.g., normal cells that also express CLL. In one embodiment, the inhibitory CAR comprises the antigen binding domain, a transmembrane domain and an intracellular domain of an inhibitory molecule. For example, the intracellular domain of the inhibitory CAR can be an intracellular domain of PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGF beta.

In one embodiment, when the CAR-expressing cell comprises two or more different CARs, the antigen binding domains of the different CARs can be such that the antigen binding domains do not interact with one another. For example, a cell expressing a first and second CAR can have an antigen binding domain of the first CAR, e.g., as a fragment, e.g., an scFv, that does not form an association with the antigen binding domain of the second CAR, e.g., the antigen binding domain of the second CAR is a VHH.

In some embodiments, the antigen binding domain comprises a single domain antigen binding (SDAB) molecules include molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies. SDAB molecules may be any of the art, or any future single domain molecules. SDAB molecules may be derived from any species including, but not limited to mouse, human, camel, llama, lamprey, fish, shark, goat, rabbit, and bovine. This term also includes naturally occurring single domain antibody molecules from species other than Camelidae and sharks.

In one aspect, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from Camelidae species, for example in camel, llama, dromedary, alpaca and guanaco. Other species besides Camelidae may produce heavy chain molecules naturally devoid of light chain; such VHHs are within the scope of the invention.

The SDAB molecules can be recombinant, CDR-grafted, humanized, camelized, de-immunized and/or in vitro generated (e.g., selected by phage display).

It has also been discovered, that cells having a plurality of chimeric membrane embedded receptors comprising an antigen binding domain that interactions between the antigen binding domain of the receptors can be undesirable, e.g., because it inhibits the ability of one or more of the antigen binding domains to bind its cognate antigen. Accordingly, disclosed herein are cells having a first and a second non-naturally occurring chimeric membrane embedded receptor comprising antigen binding domains that minimize such interactions. Also disclosed herein are nucleic acids encoding a first and a second non-naturally occurring chimeric membrane embedded receptor comprising a antigen binding domains that minimize such interactions, as well as methods of making and using such cells and nucleic acids. In an embodiment the antigen binding domain of one of said first said second non-naturally occurring chimeric membrane embedded receptor, comprises an scFv, and the other comprises a single VH domain, e.g., a camelid, shark, or lamprey single VH domain, or a single VH domain derived from a human or mouse sequence.

In some embodiments, the claimed invention comprises a first and second CAR, wherein the antigen binding domain of one of said first CAR said second CAR does not comprise a variable light domain and a variable heavy domain. In some embodiments, the antigen binding domain of one of said first CAR said second CAR is an scFv, and the other is not an scFv. In some embodiments, the antigen binding domain of one of said first CAR said second CAR comprises a single VH domain, e.g., a camelid, shark, or lamprey single VH domain, or a single VH domain derived from a human or mouse sequence. In some embodiments, the antigen binding domain of one of said first CAR said second CAR comprises a nanobody. In some embodiments, the antigen binding domain of one of said first CAR said second CAR comprises a camelid VHH domain.

In some embodiments, the antigen binding domain of one of said first CAR said second CAR comprises an scFv, and the other comprises a single VH domain, e.g., a camelid, shark, or lamprey single VH domain, or a single VH domain derived from a human or mouse sequence. In some embodiments, the antigen binding domain of one of said first CAR said second CAR comprises an scFv, and the other comprises a nanobody. In some embodiments, the antigen binding domain of one of said first CAR said second CAR comprises comprises an scFv, and the other comprises a camelid VHH domain.

In some embodiments, when present on the surface of a cell, binding of the antigen binding domain of said first CAR to its cognate antigen is not substantially reduced by the presence of said second CAR. In some embodiments, binding of the antigen binding domain of said first CAR to its cognate antigen in the presence of said second CAR is 85%, 90%, 95%, 96%, 97%, 98% or 99% of binding of the antigen binding domain of said first CAR to its cognate antigen in the absence of said second CAR.

In some embodiments, when present on the surface of a cell, the antigen binding domains of said first CAR said second CAR, associate with one another less than if both were scFv antigen binding domains. In some embodiments, the antigen binding domains of said first CAR said second CAR, associate with one another 85%, 90%, 95%, 96%, 97%, 98% or 99% less than if both were scFv antigen binding domains.

In another aspect, the CAR-expressing cell described herein can further express another agent, e.g., an agent which enhances the activity of a CAR-expressing cell. For example, in one embodiment, the agent can be an agent which inhibits an inhibitory molecule. Inhibitory molecules, e.g., PD1, can, in some embodiments, decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGF beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGF beta, or a fragment of any of these (e.g., at least a portion of an extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of an extracellular domain of PD1), and a second polypeptide of an intracellular signaling domain described herein (e.g., a CD28 signaling domain described herein and/or a CD3 zeta signaling domain described herein). PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

In one embodiment, the agent comprises the extracellular domain (ECD) of an inhibitory molecule, e.g., Programmed Death 1 (PD1), fused to a transmembrane domain and intracellular signaling domains such as 41BB and CD3 zeta (also referred to herein as a PD1 CAR). In one embodiment, the PD1 CAR, when used incombinations with a XCAR described herein, improves the persistence of the T cell. In one embodiment, the CAR is a PD1 CAR comprising the extracellular domain of PD1 indicated as underlined in SEQ ID NO: 26. In one embodiment, the PD1 CAR comprises the amino acid sequence of SEQ ID NO: 26.

Malpvtalllplalllhaarppgwfldspdrpwnpptfspallvvtegdnatftcsfsntsesfvlnwyrmspsnqtdklaafpedrsqpg
qdcrfrvtqlpngrdfhmsvvrarrndsgtylcgaislapkaqikeslraelrvterraevptahpspsprpagqfqtlvtttpaprpptpap
tiasqplslrpeacrpaaggavhtrgldfacdiyiwaplagtcgvlllslvitlyckrgrkkllyifkqpfmrpvqttqeedgcscrfpeeee
ggcelrvkfsrsadapaykqgqnqlynelnlgrreeydvldkrrgrdpemggkprrknpqeglynelqkdkmaeayseigmkgerr
rgkghdglyqglstatkdtydalhmqalppr (SEQ ID NO:26).

In one embodiment, the PD1 CAR comprises the amino acid sequence provided below (SEQ ID NO: 39).

pgwfldspdrpwnpptfspallvvtegdnatftcsfsntsesfvlnwyrmspsnqtdklaafpedrsqpgqdcrfrvtqlpngrdfhms
vvrarrndsgtylcgaislapkaqikeslraelrvterraevptahpspsprpagqfqtlvtttpaprpptpaptiasqplslrpeacrpaagg
avhtrgldfacdiyiwaplagtcgvlllslvitlyckrgrkkllyifkqpfmrpvqttqeedgcscrfpeeeeggcelrvkfsrsadapayk
qgqnqlynelnlgrreeydvldkrrgrdpemggkprrknpqeglynelqkdkmaeayseigmkgerrrgkghdglyqglstatkdt
ydalhmqalppr (SEQ ID NO:39).

In one embodiment, the agent comprises a nucleic acid sequence encoding the PD1 CAR, e.g., the PD1 CAR described herein. In one embodiment, the nucleic acid sequence for the PD 1 CAR is shown below, with the PD1 ECD underlined below in SEQ ID NO: 27.

atggccctccctgtcactgccctgcttctccccctcgcactcctgctccacgccgctagaccacccggatggtttctggactctccggatcgc
ccgtggaatcccccaaccttctcaccggcactcttggttgtgactgagggcgataatgcgaccttcacgtgctcgttctccaacacctccgaa
tcattcgtgctgaactggtaccgcatgagcccgtcaaaccagaccgacaagctcgccgcgtttccggaagatcggtcgcaaccgggacag
gattgtcggttccgcgtgactcaactgccgaatggcagagacttccacatgagcgtggtccgcgctaggcgaaacgactccgggacctac
ctgtgcggagccatctcgctggcgcctaaggcccaaatcaaagagagcttgagggccgaactgagagtgaccgagcgcagagctgagg
tgccaactgcacatccatccccatcgcctcggcctgcggggcagtttcagaccctggtcacgaccactccggcgccgcgcccaccgactc
cggccccaactatcgcgagccagcccctgtcgctgaggccggaagcatgccgccctgccgccggaggtgctgtgcatacccggggatt
ggacttcgcatgcgacatctacatttgggctcctctcgccggaacttgtggcgtgctccttctgtccctggtcatcaccctgtactgcaagcgg
ggtcggaaaaagcttctgtacattttcaagcagcccttcatgaggcccgtgcaaaccacccaggaggaggacggttgctcctgccggttcc
ccgaagaggaagaaggaggttgcgagctgcgcgtgaagttctcccggagcgccgacgcccccgcctataagcagggccagaaccagc
tgtacaacgaactgaacctgggacggcgggaagagtacgatgtgctggacaagcggcgcggccgggaccccgaaatgggcgggaag
cctagaagaaagaaccctcaggaaggcctgtataacgagctgcagaaggacaagatggccgaggcctactccgaaattgggatgaagg
gagagcggcggaggggaaaggggcacgacggcctgtaccaaggactgtccaccgccaccaaggacacatacgatgccctgcacatgc
aggcccttccccctcgc (SEQ ID NO: 27).

In another aspect, the present invention provides a population of CAR-expressing cells, e.g., CART cells. In some embodiments, the population of CAR-expressing cells comprises a mixture of cells expressing different CARs. For example, in one embodiment, the population of CART cells can include a first cell expressing a CAR having an antigen binding domain to a cancer associated antigen described herein, and a second cell expressing a CAR having a different antigen binding domain, e.g., an antigen binding domain to a different a cancer associated antigen described herein, e.g., an antigen binding domain to a cancer associated antigen described herein that differs from the cancer associated antigen bound by the antigen binding domain of the CAR expressed by the first cell. As another example, the population of CAR-expressing cells can include a first cell expressing a CAR that includes an antigen binding domain to a cancer associated antigen described herein, and a second cell expressing a CAR that includes an antigen binding domain to a target other than a cancer associated antigen as described herein. In one embodiment, the population of CAR-expressing cells includes, e.g., a first cell expressing a CAR that includes a primary intracellular signaling domain, and a second cell expressing a CAR that includes a secondary signaling domain.

In another aspect, the present invention provides a population of cells wherein at least one cell in the population expresses a CAR having an antigen binding domain to a cancer associated antigen described herein, and a second cell expressing another agent, e.g., an agent which enhances the activity of a CAR-expressing cell. For example, in one embodiment, the agent can be an agent which inhibits an inhibitory molecule. Inhibitory molecules, e.g., PD-1, can, in some embodiments, decrease the ability of a CAR-expressing cell to mount an immune effector response. Examples of inhibitory molecules include PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGF beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGF beta, or a fragment of any of these, and a second polypeptide which is an intracellular signaling domain described herein (e.g., comprising a costimulatory domain (e.g., 41BB, CD27, OX40 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28 signaling domain described herein and/or a CD3 zeta signaling domain described herein).

In one aspect, the present invention provides methods comprising administering a population of CAR-expressing cells, e.g., CART cells, e.g., a mixture of cells expressing different CARs, in combination with another agent, e.g., a kinase inhibitor, such as a kinase inhibitor described herein. In another aspect, the present invention provides methods comprising administering a population of cells wherein at least one cell in the population expresses a CAR having an antigen binding domain of a cancer associated antigen described herein, and a second cell expressing another agent, e.g., an agent which enhances the activity of a CAR-expressing cell, in combination with another agent, e.g., a kinase inhibitor, such as a kinase inhibitor described herein.

### Regulatable Chimeric Antigen Receptors

In some embodiments, a regulatable CAR (RCAR) where the CAR activity can be controlled is desirable to optimize the safety and efficacy of a CAR therapy. There are many ways CAR activities can be regulated. For example, inducible apoptosis using, e.g., a caspase fused to a dimerization domain (see, e.g., Di et al., N Egnl. J. Med. 2011 Nov. 3; 365(18):1673-1683), can be used as a safety switch in the CAR therapy of the instant invention. In an aspect, a RCAR comprises a set of polypeptides, typically two in the simplest embodiments, in which the components of a standard CAR described herein, e.g., an antigen binding domain and an intracellular signaling domain, are partitioned on separate polypeptides or members. In some embodiments, the set of polypeptides include a dimerization switch that, upon the presence of a dimerization molecule, can couple the polypeptides to one another, e.g., can couple an antigen binding domain to an intracellular signaling domain.

In an aspect, an RCAR comprises two polypeptides or members: 1) an intracellular signaling member comprising an intracellular signaling domain, e.g., a primary intracellular signaling domain described herein, and a first switch domain; 2) an antigen binding member comprising an antigen binding domain, e.g., that targets a tumor antigen described herein, as described herein and a second switch domain. Optionally, the RCAR comprises a transmembrane domain described herein. In an embodiment, a transmembrane domain can be disposed on the intracellular signaling member, on the antigen binding member, or on both. (Unless otherwise indicated, when members or elements of an RCAR are described herein, the order can be as provided, but other orders are included as well. In other words, in an embodiment, the order is as set out in the text, but in other embodiments, the order can be different. E.g., the order of elements on one side of a transmembrane region can be different from the example, e.g., the placement of a switch domain relative to a intracellular signaling domain can be different, e.g., reversed).

In an embodiment, the first and second switch domains can form an intracellular or an extracellular dimerization switch. In an embodiment, the dimerization switch can be a homodimerization switch, e.g., where the first and second switch domain are the same, or a heterodimerization switch, e.g., where the first and second switch domain are different from one another.

In embodiments, an RCAR can comprise a "multi switch." A multi switch can comprise heterodimerization switch domains or homodimerization switch domains. A multi switch comprises a plurality of, e.g., 2, 3, 4, 5, 6, 7, 8, 9, or 10, switch domains, independently, on a first member, e.g., an antigen binding member, and a second member, e.g., an intracellular signaling member. In an embodiment, the first member can comprise a plurality of first switch domains, e.g., FKBP-based switch domains, and the second member can comprise a plurality of second switch domains, e.g., FRB-based switch domains. In an embodiment, the first member can comprise a first and a second switch domain, e.g., a FKBP-based switch domain and a FRB-based switch domain, and the second member can comprise a first and a second switch domain, e.g., a FKBP-based switch domain and a FRB-based switch domain.

In an embodiment, the intracellular signaling member comprises one or more intracellular signaling domains, e.g., a primary intracellular signaling domain and one or more costimulatory signaling domains.

In an embodiment, the antigen binding member may comprise one or more intracellular signaling domains, e.g., one or more costimulatory signaling domains. In an embodiment, the antigen binding member comprises a plurality, e.g., 2 or 3 costimulatory signaling domains described herein, e.g., selected from 41BB, CD28, CD27, ICOS, and OX40, and in embodiments, no primary intracellular signaling domain. In an embodiment, the antigen binding member comprises the following costimulatory signaling domains, from the extracellular to intracellular direction: 41BB-CD27; 41BB-CD27; CD27-41BB; 41BB-CD28; CD28-41BB; OX40-CD28; CD28-OX40; CD28-41BB; or 41BB-CD28. In such embodiments, the intracellular binding member comprises a CD3zeta domain. In one such embodiment the RCAR comprises (1) an antigen binding member comprising, an antigen binding domain, a transmembrane domain, and two costimulatory domains and a first switch domain; and (2) an intracellular signaling domain comprising a transmembrane domain or membrane tethering domain and at least one primary intracellular signaling domain, and a second switch domain.

An embodiment provides RCARs wherein the antigen binding member is not tethered to the surface of the CAR cell. This allows a cell having an intracellular signaling member to be conveniently paired with one or more antigen binding domains, without transforming the cell with a sequence that encodes the antigen binding member. In such embodiments, the RCAR comprises: 1) an intracellular signaling member comprising: a first switch domain, a transmembrane domain, an intracellular signaling domain, e.g., a primary intracellular signaling domain, and a first switch domain; and 2) an antigen binding member comprising: an antigen binding domain, and a second switch domain, wherein the antigen binding member does not comprise a transmembrane domain or membrane tethering domain, and, optionally, does not comprise an intracellular signaling domain. In some embodiments, the RCAR may further comprise 3) a second antigen binding member comprising: a second antigen binding domain, e.g., a second antigen binding domain that binds a different antigen than is bound by the antigen binding domain; and a second switch domain.

Also provided herein are RCARs wherein the antigen binding member comprises bispecific activation and targeting capacity. In this embodiment, the antigen binding member can comprise a plurality, e.g., 2, 3, 4, or 5 antigen binding domains, e.g., scFvs, wherein each antigen binding domain binds to a target antigen, e.g. different antigens or the same antigen, e.g., the same or different epitopes on the same antigen. In an embodiment, the plurality of antigen binding domains are in tandem, and optionally, a linker or hinge region is disposed between each of the antigen binding domains. Suitable linkers and hinge regions are described herein.

An embodiment provides RCARs having a configuration that allows switching of proliferation. In this embodiment, the RCAR comprises: 1) an intracellular signaling member comprising: optionally, a transmembrane domain or membrane tethering domain; one or more co-stimulatory signaling domain, e.g., selected from 41BB, CD28, CD27, ICOS, and OX40, and a switch domain; and 2) an antigen binding member comprising: an antigen binding domain, a transmembrane domain, and a primary intracellular signaling domain, e.g., a CD3zeta domain, wherein the antigen binding member does not comprise a switch domain, or does not comprise a switch domain that dimerizes with a switch domain on the intracellular signaling member. In an embodiment, the antigen binding member does not comprise a co-stimulatory signaling domain. In an embodiment, the intracellular signaling member comprises a switch domain from a homodimerization switch. In an embodiment, the intracellular signaling member comprises a first switch domain of a heterodimerization switch and the RCAR comprises a second intracellular signaling member which comprises a second switch domain of the heterodimerization switch. In such embodiments, the second intracellular signaling member comprises the same intracellular signaling domains as the intracellular signaling member. In an embodiment, the dimerization switch is intracellular. In an embodiment, the dimerization switch is extracellular.

In any of the RCAR configurations described here, the first and second switch domains comprise a FKBP-FRB based switch as described herein.

Also provided herein are cells comprising an RCAR described herein. Any cell that is engineered to express a RCAR can be used as a RCARX cell. In an embodiment the RCARX cell is a T cell, and is referred to as a RCART cell. In an embodiment the RCARX cell is an NK cell, and is referred to as a RCARN cell.

Also provided herein are nucleic acids and vectors comprising RCAR encoding sequences. Sequence encoding various elements of an RCAR can be disposed on the same nucleic acid molecule, e.g., the same plasmid or vector, e.g., viral vector, e.g., lentiviral vector. In an embodiment, (i) sequence encoding an antigen binding member and (ii) sequence encoding an intracellular signaling member, can be present on the same nucleic acid, e.g., vector. Production of the corresponding proteins can be achieved, e.g., by the use of separate promoters, or by the use of a bicistronic transcription product (which can result in the production of two proteins by cleavage of a single translation product or by the translation of two separate protein products). In an embodiment, a sequence encoding a cleavable peptide, e.g., a P2A or F2A sequence, is disposed between (i) and (ii). Examples of peptide cleavage sites include the following, wherein the GSG residues are optional:

| | |
|---|---|
| T2A: | (GSG)EGRGSLLTCGDVEENPGP (SEQ ID NO: 68) |
| P2A: | (GSG)ATNFSLLKQAGDVEENPGP (SEQ ID NO: 69) |
| E2A: | (GSG)QCTNYALLKLAGDVESNPGP (SEQ ID NO: 70) |
| F2A: | (GSG)VKQTLNFDLLKLAGDVESNPGP(SEQ ID NO: 71) |

In an embodiment, a sequence encoding an IRES, e.g., an EMCV or EV71 IRES, is disposed between (i) and (ii). In these embodiments, (i) and (ii) are transcribed as a single RNA. In an embodiment, a first promoter is operably linked to (i) and a second promoter is operably linked to (ii), such that (i) and (ii) are transcribed as separate mRNAs.

Alternatively, the sequence encoding various elements of an RCAR can be disposed on the different nucleic acid molecules, e.g., different plasmids or vectors, e.g., viral vector, e.g., lentiviral vector. E.g., the (i) sequence encoding an antigen binding member can be present on a first nucleic acid, e.g., a first vector, and the (ii) sequence encoding an intracellular signaling member can be present on the second nucleic acid, e.g., the second vector.

### Dimerization switches

Dimerization switches can be non-covalent or covalent. In a non-covalent dimerization switch, the dimerization molecule promotes a non-covalent interaction between the switch domains. In a covalent dimerization switch, the dimerization molecule promotes a covalent interaction between the switch domains.

In an embodiment, the RCAR comprises a FKBP/FRAP, or FKBP/FRB,-based dimerization switch. FKBP12 (FKBP, or FK506 binding protein) is an abundant cytoplasmic protein that serves as the initial intracellular target for the natural product immunosuppressive drug, rapamycin. Rapamycin binds to FKBP and to the large PI3K homolog FRAP (RAFT, mTOR). FRB is a 93 amino acid portion of FRAP, that is sufficient for binding the FKBP-rapamycin complex (Chen, J., Zheng, X. F., Brown, E. J. & Schreiber, S. L. (1995) Identification of an 11-kDa FKBP12-rapamycin-binding domain within the 289-kDa FKBP12-rapamycin-associated protein and characterization of a critical serine residue. Proc Natl Acad Sci U S A 92: 4947-51.)

In embodiments, an FKBP/FRAP, e.g., an FKBP/FRB, based switch can use a dimerization molecule, e.g., rapamycin or a rapamycin analog.

The amino acid sequence of FKBP is as follows:

D V P D Y A S L G G P S S P K K K R K V S R G V Q V E T I S P G D G R T F P K R G Q T
C V V H Y T G M L E D G K K F D S S R D R N K P F K F M L G K Q E V I R G W E E G V
A Q M S V G Q R A K L T I S P D Y A Y G A T G H P G I I P P H A T L V F D V E L L K L
E T S Y (SEQ ID NO: 54)

In embodiments, an FKBP switch domain can comprise a fragment of FKBP having the ability to bind with FRB, or a fragment or analog thereof, in the presence of rapamycin or a rapalog, e.g., the underlined portion of SEQ ID NO: 54, which is:

V Q V E T I S P G D G R T F P K R G Q T C V V H Y T G M L E D G K K F D S S R D R N
K P F K F M L G K Q E V I R G W E E G V A Q M S V G Q R A K L T I S P D Y A Y G A T
G H P G I I P P H A T L V F D V E L L K L E T S (SEQ ID NO:55)

The amino acid sequence of FRB is as follows:


 (SEQ ID NO: 56)

"FKBP/FRAP, e.g., an FKBP/FRB, based switch" as that term is used herein, refers to a dimerization switch comprising: a first switch domain, which comprises an FKBP fragment or analog thereof having the ability to bind with FRB, or a fragment or analog thereof, in the presence of rapamycin or a rapalog, e.g., RAD001, and has at least 70, 75, 80, 85, 90, 95, 96, 97, 98, or 99% identity with, or differs by no more than 30, 25, 20, 15, 10, 5, 4, 3, 2, or 1 amino acid residues from, the FKBP sequence of SEQ ID NO: 54 or 55; and a second switch domain, which comprises an FRB fragment or analog thereof having the ability to bind with FRB, or a fragment or analog thereof, in the presence of rapamycin or a rapalog, and has at least 70, 75, 80, 85, 90, 95, 96, 97, 98, or 99% identity with, or differs by no more than 30, 25, 20, 15, 10, 5, 4, 3, 2, or 1 amino acid residues from, the FRB sequence of SEQ ID NO: 56. In an embodiment, a RCAR described herein comprises one switch domain comprises amino acid residues disclosed in SEQ ID NO: 54 (or SEQ ID NO: 55), and one switch domain comprises amino acid residues disclosed in SEQ ID NO: 56.

In embodiments, the FKBP/FRB dimerization switch comprises a modified FRB switch domain that exhibits altered, e.g., enhanced, complex formation between an FRB-based switch domain, e.g., the modified FRB switch domain, a FKBP-based switch domain, and the dimerization molecule, e.g., rapamycin or a rapalogue, e.g., RAD001. In an embodiment, the modified FRB switch domain comprises one or more mutations, e.g., 2, 3, 4, 5, 6, 7, 8, 9, 10 or more, selected from mutations at amino acid position(s) L2031, E2032, S2035, R2036, F2039, G2040, T2098, W2101, D2102, Y2105, and F2108, where the wild-type amino acid is mutated to any other naturally-occurring amino acid. In an embodiment, a mutant FRB comprises a mutation at E2032, where E2032 is mutated to phenylalanine (E2032F), methionine (E2032M), arginine (E2032R), valine (E2032V), tyrosine (E2032Y), isoleucine (E2032I), e.g., SEQ ID NO: 57, or leucine (E2032L), e.g., SEQ ID NO: 58. In an embodiment, a mutant FRB comprises a mutation at T2098, where T2098 is mutated to phenylalanine (T2098F) or leucine (T2098L), e.g., SEQ ID NO: 59. In an embodiment, a mutant FRB comprises a mutation at E2032 and at T2098, where E2032 is mutated to any amino acid, and where T2098 is mutated to any amino acid, e.g., SEQ ID NO: 60. In an embodiment, a mutant FRB comprises an E2032I and a T2098L mutation, e.g., SEQ ID NO: 61. In an embodiment, a mutant FRB comprises an E2032L and a T2098L mutation, e.g., SEQ ID NO: 62.

**Table 10. Exemplary mutant FRB having increased affinity for a dimerization molecule**

| **FRB mutant** | **Amino Acid Sequence** | **SEQ ID NO:** |
|---|---|---|
| E2032I mutant | ILWHEMWHEGLIEASRLYFGERNVKGMFEVLEPLHAMMERGPQTLK ETSFNQAYGRDLMEAQEWCRKYMKSGNVKDLTQAWDLYYHVFRRIS KTS | 57 |
| E2032L mutant | ILWHEMWHEGLLEASRLYFGERNVKGMFEVLEPLHAMMERGPQTLK ETSFNQAYGRDLMEAQEWCRKYMKSGNVKDLTQAWDLYYHVFRRIS KTS | 58 |
| T2098L mutant | ILWHEMWHEGLEEASRLYFGERNVKGMFEVLEPLHAMMERGPQTLK ETSFNQAYGRDLMEAQEWCRKYMKSGNVKDLLQAWDLYYHVFRRIS KTS | 59 |
| E2032, T2098 mutant | ILWHEMWHEGLXEASRLYFGERNVKGMFEVLEPLHAMMERGPQTLK ETSFNQAYGRDLMEAQEWCRKYMKSGNVKDLXQAWDLYYHVFRRIS KTS | 60 |
| E2032I, T2098L mutant | ILWHEMWHEGLIEASRLYFGERNVKGMFEVLEPLHAMMERGPQTLK ETSFNQAYGRDLMEAQEWCRKYMKSGNVKDLLQAWDLYYHVFRRIS KTS | 61 |
| E2032L, T2098L mutant | ILWHEMWHEGLLEASRLYFGERNVKGMFEVLEPLHAMMERGPQTLK ETSFNQAYGRDLMEAQEWCRKYMKSGNVKDLLQAWDLYYHVFRRIS KTS | 62 |

Other suitable dimerization switches include a GyrB-GyrB based dimerization switch, a Gibberellin-based dimerization switch, a tag/binder dimerization switch, and a halo-tag/snap-tag dimerization switch. Following the guidance provided herein, such switches and relevant dimerization molecules will be apparent to one of ordinary skill.

### Dimerization molecule

Association between the switch domains is promoted by the dimerization molecule. In the presence of dimerization molecule interaction or association between switch domains allows for signal transduction between a polypeptide associated with, e.g., fused to, a first switch domain, and a polypeptide associated with, e.g., fused to, a second switch domain. In the presence of non-limiting levels of dimerization molecule signal transduction is increased by 1.1, 1.2, 1.3, 1.4, 1.5, 1.6, 1.7, 1.8, 1.9, 2, 5, 10, 50, 100 fold, e.g., as measured in a system described herein.

Rapamycin and rapamycin analogs (sometimes referred to as rapalogues), e.g., RAD001, can be used as dimerization molecules in a FKBP/FRB-based dimerization switch described herein. In an embodiment the dimerization molecule can be selected from rapamycin (sirolimus), RAD001 (everolimus), zotarolimus, temsirolimus, AP-23573 (ridaforolimus), biolimus and AP21967. Additional rapamycin analogs suitable for use with FKBP/FRB-based dimerization switches are further described in the section entitled "Combination Therapies", or in the subsection entitled "Exemplary mTOR inhibitors."

### Split CAR

In some embodiments, the CAR-expressing cell uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 41BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens.

### RNA Transfection

Disclosed herein are methods for producing an in vitro transcribed RNA CAR. The present invention also includes a CAR encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO:32). RNA so produced can efficiently transfect different kinds of cells. In one aspect, the template includes sequences for the CAR.

In one aspect, a CAR of the present invention is encoded by a messenger RNA (mRNA). In one aspect, the mRNA encoding a CAR described herein is introduced into an immune effector cell, e.g., a T cell or a NK cell, for production of a CAR-expressing cell, e.g., a CART cell or a CAR NK cell.

In one embodiment, the *in vitro* transcribed RNA CAR can be introduced to a cell as a form of transient transfection. The RNA is produced by *in vitro* transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired temple for *in vitro* transcription is a CAR described herein. For example, the template for the RNA CAR comprises an extracellular region comprising a single chain variable domain of an antibody to a tumor associated antigen described herein; a hinge region (e.g., a hinge region described herein), a transmembrane domain (e.g., a transmembrane domain described herein such as a transmembrane domain of CD8a); and a cytoplasmic region that includes an intracellular signaling domain, e.g., an intracellular signaling domain described herein, e.g., comprising the signaling domain of CD3-zeta and the signaling domain of 4-1BB.

In one embodiment, the DNA to be used for PCR contains an open reading frame. The DNA can be from a naturally occurring DNA sequence from the genome of an organism. In one embodiment, the nucleic acid can include some or all of the 5' and/or 3' untranslated regions (UTRs). The nucleic acid can include exons and introns. In one embodiment, the DNA to be used for PCR is a human nucleic acid sequence. In another embodiment, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one embodiment, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be 5'UTR of an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (SEQ ID NO: 35) (size can be 50-5000 T (SEQ ID NO: 36)), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines (SEQ ID NO: 37).

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides (SEQ ID NO: 38) results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

### Non-viral delivery methods

In some aspects, non-viral methods can be used to deliver a nucleic acid encoding a CAR described herein into a cell or tissue or a subject.

In some embodiments, the non-viral method includes the use of a transposon (also called a transposable element). In some embodiments, a transposon is a piece of DNA that can insert itself at a location in a genome, for example, a piece of DNA that is capable of self-replicating and inserting its copy into a genome, or a piece of DNA that can be spliced out of a longer nucleic acid and inserted into another place in a genome. For example, a transposon comprises a DNA sequence made up of inverted repeats flanking genes for transposition.

Exemplary methods of nucleic acid delivery using a transposon include a Sleeping Beauty transposon system (SBTS) and a piggyBac (PB) transposon system. See, e.g., Aronovich et al. Hum. Mol. Genet. 20.R1(2011):R14-20; Singh et al. Cancer Res. 15(2008):2961-2971; Huang et al. Mol. Ther. 16(2008):580-589; Grabundzija et al. Mol. Ther. 18(2010):1200-1209; Kebriaei et al. Blood. 122.21(2013):166; Williams. Molecular Therapy 16.9(2008):1515-16; Bell et al. Nat. Protoc. 2.12(2007):3153-65; and Ding et al. Cell. 122.3(2005):473-83, all of which are incorporated herein by reference.

The SBTS includes two components: 1) a transposon containing a transgene and 2) a source of transposase enzyme. The transposase can transpose the transposon from a carrier plasmid (or other donor DNA) to a target DNA, such as a host cell chromosome/genome. For example, the transposase binds to the carrier plasmid/donor DNA, cuts the transposon (including transgene(s)) out of the plasmid, and inserts it into the genome of the host cell. See, e.g., Aronovich et al. *supra.*

Exemplary transposons include a pT2-based transposon. See, e.g., Grabundzija et al. Nucleic Acids Res. 41.3(2013):1829-47; and Singh et al. Cancer Res. 68.8(2008): 2961-2971, all of which are incorporated herein by reference. Exemplary transposases include a Tc1/mariner-type transposase, e.g., the SB10 transposase or the SB11 transposase (a hyperactive transposase which can be expressed, e.g., from a cytomegalovirus promoter). See, e.g., Aronovich et al.; Kebriaei et al.; and Grabundzija et al., all of which are incorporated herein by reference.

Use of the SBTS permits efficient integration and expression of a transgene, e.g., a nucleic acid encoding a CAR described herein. Provided herein are methods of generating a cell, e.g., T cell or NK cell, that stably expresses a CAR described herein, e.g., using a transposon system such as SBTS.

In accordance with methods described herein, in some embodiments, one or more nucleic acids, e.g., plasmids, containing the SBTS components are delivered to a cell (e.g., T or NK cell). For example, the nucleic acid(s) are delivered by standard methods of nucleic acid (e.g., plasmid DNA) delivery, e.g., methods described herein, e.g., electroporation, transfection, or lipofection. In some embodiments, the nucleic acid contains a transposon comprising a transgene, e.g., a nucleic acid encoding a CAR described herein. In some embodiments, the nucleic acid contains a transposon comprising a transgene (e.g., a nucleic acid encoding a CAR described herein) as well as a nucleic acid sequence encoding a transposase enzyme. In other embodiments, a system with two nucleic acids is provided, e.g., a dual-plasmid system, e.g., where a first plasmid contains a transposon comprising a transgene, and a second plasmid contains a nucleic acid sequence encoding a transposase enzyme. For example, the first and the second nucleic acids are co-delivered into a host cell.

In some embodiments, cells, e.g., T or NK cells, are generated that express a CAR described herein by using a combination of gene insertion using the SBTS and genetic editing using a nuclease (e.g., Zinc finger nucleases (ZFNs), Transcription Activator-Like Effector Nucleases (TALENs), the CRISPR/Cas system, or engineered meganuclease re-engineered homing endonucleases).

In some embodiments, use of a non-viral method of delivery permits reprogramming of cells, e.g., T or NK cells, and direct infusion of the cells into a subject. Advantages of non-viral vectors include but are not limited to the ease and relatively low cost of producing sufficient amounts required to meet a patient population, stability during storage, and lack of immunogenicity.

### Nucleic Acid Constructs Encoding a CAR

The present invention also provides nucleic acid molecules encoding one or more CAR constructs described herein. In one aspect, the nucleic acid molecule is provided as a messenger RNA transcript. In one aspect, the nucleic acid molecule is provided as a DNA construct.

Accordingly, in one aspect, the invention pertains to a nucleic acid molecule encoding a chimeric antigen receptor (CAR), wherein the CAR comprises an antigen binding domain that binds to a tumor antigen described herein, a transmembrane domain (e.g., a transmembrane domain described herein), and an intracellular signaling domain (e.g., an intracellular signaling domain described herein) comprising a stimulatory domain, e.g., a costimulatory signaling domain (e.g., a costimulatory signaling domain described herein) and/or a primary signaling domain (e.g., a primary signaling domain described herein, e.g., a zeta chain described herein). In one embodiment, the transmembrane domain is transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp.

In one embodiment, the transmembrane domain comprises a sequence of SEQ ID NO: 12, or a sequence with 95-99% identity thereof. In one embodiment, the antigen binding domain is connected to the transmembrane domain by a hinge region, e.g., a hinge described herein. In one embodiment, the hinge region comprises SEQ ID NO:4 or SEQ ID NO:6 or SEQ ID NO:8 or SEQ ID NO:10, or a sequence with 95-99% identity thereof. In one embodiment, the isolated nucleic acid molecule further comprises a sequence encoding a costimulatory domain. In one embodiment, the costimulatory domain is a functional signaling domain of a protein selected from the group consisting of OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, and PAG/Cbp. In one embodiment, the costimulatory domain comprises a sequence of SEQ ID NO:16, or a sequence with 95-99% identity thereof. In one embodiment, the intracellular signaling domain comprises a functional signaling domain of 4-1BB and a functional signaling domain of CD3 zeta. In one embodiment, the intracellular signaling domain comprises the sequence of SEQ ID NO: 14 or SEQ ID NO:16, or a sequence with 95-99% identity thereof, and the sequence of SEQ ID NO: 18 or SEQ ID NO:20, or a sequence with 95-99% identity thereof, wherein the sequences comprising the intracellular signaling domain are expressed in the same frame and as a single polypeptide chain.

In another aspect, the invention pertains to an isolated nucleic acid molecule encoding a CAR construct comprising a leader sequence of SEQ ID NO: 2, a scFv domain as described herein, a hinge region of SEQ ID NO:4 or SEQ ID NO:6 or SEQ ID NO:8 or SEQ ID NO:10 (or a sequence with 95-99% identity thereof), a transmembrane domain having a sequence of SEQ ID NO: 12 (or a sequence with 95-99% identity thereof), a 4-1BB costimulatory domain having a sequence of SEQ ID NO:14 or a CD27 costimulatory domain having a sequence of SEQ ID NO:16 (or a sequence with 95-99% identity thereof), and a CD3 zeta stimulatory domain having a sequence of SEQ ID NO:18 or SEQ ID NO:20 (or a sequence with 95-99% identity thereof).

In another aspect, the invention pertains to a nucleic acid molecule encoding a chimeric antigen receptor (CAR) molecule that comprises an antigen binding domain, a transmembrane domain, and an intracellular signaling domain comprising a stimulatory domain, and wherein said antigen binding domain binds to a tumor antigen selected from a group consisting of: CD19, CD123, CD22, CD30, CD171, CS-1, CLL-1 (CLECL1), CD33, EGFRvIII , GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, SSEA-4, CD20, Folate receptor alpha, ERBB2 (Her2/neu), MUC1, EGFR, NCAM, Prostase, PRSS21, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

In one embodiment, the encoded CAR molecule further comprises a sequence encoding a costimulatory domain. In one embodiment, the costimulatory domain is a functional signaling domain of a protein selected from the group consisting of OX40, CD27, CD28, CDS, ICAM-1, LFA-1 (CD11a/CD18) and 4-1BB (CD137). In one embodiment, the costimulatory domain comprises a sequence of SEQ ID NO: 14. In one embodiment, the transmembrane domain is a transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In one embodiment, the transmembrane domain comprises a sequence of SEQ ID NO:12. In one embodiment, the intracellular signaling domain comprises a functional signaling domain of 4-1BB and a functional signaling domain of zeta. In one embodiment, the intracellular signaling domain comprises the sequence of SEQ ID NO: 14 and the sequence of SEQ ID NO: 18, wherein the sequences comprising the intracellular signaling domain are expressed in the same frame and as a single polypeptide chain. In one embodiment, the anti-a cancer associated antigen as described herein binding domain is connected to the transmembrane domain by a hinge region. In one embodiment, the hinge region comprises SEQ ID NO:4. In one embodiment, the hinge region comprises SEQ ID NO:6 or SEQ ID NO:8 or SEQ ID NO:10.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present invention also provides vectors in which a DNA of the present invention is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. A retroviral vector may also be, e.g., a gammaretroviral vector. A gammaretroviral vector may include, e.g., a promoter, a packaging signal (ψ), a primer binding site (PBS), one or more (e.g., two) long terminal repeats (LTR), and a transgene of interest, e.g., a gene encoding a CAR. A gammaretroviral vector may lack viral structural gens such as gag, pol, and env. Exemplary gammaretroviral vectors include Murine Leukemia Virus (MLV), Spleen-Focus Forming Virus (SFFV), and Myeloproliferative Sarcoma Virus (MPSV), and vectors derived therefrom. Other gammaretroviral vectors are described, e.g., in Tobias Maetzig et al., "Gammaretroviral Vectors: Biology, Technology and Application" Viruses. 2011 Jun; 3(6): 677-713.

In another embodiment, the vector comprising the nucleic acid encoding the desired CAR of the invention is an adenoviral vector (A5/35). In another embodiment, the expression of nucleic acids encoding CARs can be accomplished using of transposons such as sleeping beauty, crisper, CAS9, and zinc finger nucleases. See below June et al. 2009Nature Reviews Immunology 9.10: 704-716, is incorporated herein by reference.

In brief summary, the expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

The expression constructs of the present invention may also be used for nucleic acid immunization and gene therapy, using standard gene delivery protocols. Methods for gene delivery are known in the art. See, e.g., U.S. Pat. Nos. 5,399,346, 5,580,859, 5,589,466, incorporated by reference herein in their entireties. In another embodiment, the invention provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals. Viruses, which are useful as vectors include, but are not limited to, retroviruses, adenoviruses, adeno- associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription. Exemplary promoters include the CMV IE gene, EF-1α, ubiquitin C, or phosphoglycerokinase (PGK) promoters.

An example of a promoter that is capable of expressing a CAR encoding nucleic acid molecule in a mammalian T cell is the EFla promoter. The native EFla promoter drives expression of the alpha subunit of the elongation factor-1 complex, which is responsible for the enzymatic delivery of aminoacyl tRNAs to the ribosome. The EFla promoter has been extensively used in mammalian expression plasmids and has been shown to be effective in driving CAR expression from nucleic acid molecules cloned into a lentiviral vector. See, e.g., Milone et al., Mol. Ther. 17(8): 1453-1464 (2009). In one aspect, the EFla promoter comprises the sequence provided as SEQ ID NO: 1.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1α promoter, the hemoglobin promoter, and the creatine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1 -4, Cold Spring Harbor Press, NY). A preferred method for the introduction of a polynucleotide into a host cell is calcium phosphate transfection

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g. , an artificial membrane vesicle). Other methods of state-of-the-art targeted delivery of nucleic acids are available, such as delivery of polynucleotides with targeted nanoparticles or other suitable sub-micron sized delivery system.

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present invention, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

The present invention further provides a vector comprising a CAR encoding nucleic acid molecule. In one aspect, a CAR vector can be directly transduced into a cell, e.g., a T cell or a NK cell. In one aspect, the vector is a cloning or expression vector, e.g., a vector including, but not limited to, one or more plasmids (*e.g*., expression plasmids, cloning vectors, minicircles, minivectors, double minute chromosomes), retroviral and lentiviral vector constructs. In one aspect, the vector is capable of expressing the CAR construct in mammalian immune effector cells (e.g., T cells, NK cells). In one aspect, the mammalian T cell is a human T cell. In one aspect, the mammalian NK cell is a human NK cell.

### Sources of Cells

Prior to expansion and genetic modification or other modification, a source of cells, e.g., T cells or natural killer (NK) cells, can be obtained from a subject. The term "subject" is intended to include living organisms in which an immune response can be elicited (e.g., mammals). Examples of subjects include humans, monkeys, chimpanzees, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors.

In certain aspects of the present disclosure, immune effector cells, e.g., T cells, can be obtained from a unit of blood collected from a subject using any number of techniques known to the skilled artisan, such as Ficoll^{™} separation. In one preferred aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one embodiment, the cells are washed with phosphate buffered saline (PBS). In an alternative embodiment, the wash solution lacks calcium and may lack magnesium or may lack many if not all divalent cations.

Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi:10.1038/cti.2014.31.

In one aspect, T cells are isolated from peripheral blood lymphocytes by lysing the red blood cells and depleting the monocytes, for example, by centrifugation through a PERCOLL^{™} gradient or by counterflow centrifugal elutriation.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells.

In one embodiment, T regulatory cells, e.g., CD25+ T cells, are removed from the population using an anti-CD25 antibody, or fragment thereof, or a CD25-binding ligand, IL-2. In one embodiment, the anti-CD25 antibody, or fragment thereof, or CD25-binding ligand is conjugated to a substrate, e.g., a bead, or is otherwise coated on a substrate, e.g., a bead. In one embodiment, the anti-CD25 antibody, or fragment thereof, is conjugated to a substrate as described herein.

In one embodiment, the T regulatory cells, e.g., CD25+ T cells, are removed from the population using CD25 depletion reagent from Miltenyi^{™}. In one embodiment, the ratio of cells to CD25 depletion reagent is 1e7 cells to 20 uL, or 1e7 cells to15 uL, or 1e7 cells to 10 uL, or 1e7 cells to 5 uL, or 1e7 cells to 2.5 uL, or 1e7 cells to 1.25 uL. In one embodiment, e.g., for T regulatory cells, e.g., CD25+ depletion, greater than 500 million cells/ml is used. In a further aspect, a concentration of cells of 600, 700, 800, or 900 million cells/ml is used.

In one embodiment, the population of immune effector cells to be depleted includes about 6 x 10⁹ CD25+ T cells. In other aspects, the population of immune effector cells to be depleted include about 1 x 10⁹ to 1x 10¹⁰ CD25+ T cell, and any integer value in between. In one embodiment, the resulting population T regulatory depleted cells has 2 x 10⁹ T regulatory cells, e.g., CD25+ cells, or less (e.g., 1 x 10⁹, 5 x 10⁸, 1 x 10⁸, 5 x 10⁷, 1 x 10⁷, or less CD25+ cells).

In one embodiment, the T regulatory cells, e.g., CD25+ cells, are removed from the population using the CliniMAC system with a depletion tubing set, such as, e.g., tubing 162-01. In one embodiment, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product can reduce the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, and combinations thereof.

In some embodiments, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In an embodiment, a subject is pre-treated with one or more therapies that reduce T_{REG} cells prior to collection of cells for CAR-expressing cell product manufacturing, thereby reducing the risk of subject relapse to CAR-expressing cell treatment. In an embodiment, methods of decreasing T_{REG} cells include, but are not limited to, administration to the subject of one or more of cyclophosphamide, anti-GITR antibody, CD25-depletion, or a combination thereof. Administration of one or more of cyclophosphamide, anti-GITR antibody, CD25-depletion, or a combination thereof, can occur before, during or after an infusion of the CAR-expressing cell product.

In an embodiment, a subject is pre-treated with cyclophosphamide prior to collection of cells for CAR-expressing cell product manufacturing, thereby reducing the risk of subject relapse to CAR-expressing cell treatment. In an embodiment, a subject is pre-treated with an anti-GITR antibody prior to collection of cells for CAR-expressing cell product manufacturing, thereby reducing the risk of subject relapse to CAR-expressing cell treatment.

In one embodiment, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one embodiment, such cells are envisioned to be removed concurrently with regulatory T cells and/or tumor cells, or following said depletion, or in another order.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a CAR, e.g., a CAR described herein. In one embodiment, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other embodiments, the removal of T regulatory cells, e.g., CD25+ cells, and the removal of the tumor antigen expressing cells is sequential, and can occur, e.g., in either order.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary check point inhibitors include B7-H1, B7-1, CD160, P1H, 2B4, PD1, TIM3, CEACAM (e.g., CEACAM-1, CEACAM-3 and/or CEACAM-5), LAG3, TIGIT, CTLA-4, BTLA and LAIR1. In one embodiment, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other embodiments, the removal of T regulatory cells, e.g., CD25+ cells, and the removal of the check point inhibitor expressing cells is sequential, and can occur, e.g., in either order.

Methods described herein can include a positive selection step. For example, T cells can isolated by incubation with anti-CD3/anti-CD28 (e.g., 3x28)-conjugated beads, such as DYNABEADS^{®} M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another embodiment, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points.

In one embodiment, a T cell population can be selected that expresses one or more of IFN-γ, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 10 billion cells/ml, 9 billion/ml, 8 billion/ml, 7 billion/ml, 6 billion/ml, or 5 billion/ml is used. In one aspect, a concentration of 1 billion cells/ml is used. In yet one aspect, a concentration of cells from 75, 80, 85, 90, 95, or 100 million cells/ml is used. In further aspects, concentrations of 125 or 150 million cells/ml can be used.

Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 x 10⁶/ml. In other aspects, the concentration used can be from about 1 x 10⁵/ml to 1 x 10⁶/ml, and any integer value in between.

In other aspects, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present invention.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in immune effector cell therapy for any number of diseases or conditions that would benefit from immune effector cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present invention, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present invention to collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one embodiment, the immune effector cells expressing a CAR molecule, e.g., a CAR molecule described herein, are obtained from a subject that has received a low, immune enhancing dose of an mTOR inhibitor. In an embodiment, the population of immune effector cells, e.g., T cells, to be engineered to express a CAR, are harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other embodiments, population of immune effector cells, e.g., T cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In one embodiment, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one embodiment, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In embodiments, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an embodiment, the NK cells are obtained from the subject. In another embodiment, the NK cells are an NK cell line, e.g., NK-92 cell line (Conkwest).

### Allogeneic CAR

In embodiments described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

A T cell lacking a functional TCR can be, e.g., engineered such that it does not express any functional TCR on its surface, engineered such that it does not express one or more subunits that comprise a functional TCR or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, is downregulated.

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not express or expresses at low levels an inhibitory molecule, e.g. by any mehod described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGF beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some embodiments, TCR expression and/or HLA expression can be inhibited using siRNA or shRNA that targets a nucleic acid encoding a TCR and/or HLA in a T cell.

Expression of siRNA and shRNAs in T cells can be achieved using any conventional expression system, e.g., such as a lentiviral expression system.

Exemplary shRNAs that downregulate expression of components of the TCR are described, e.g., in US Publication No.: 2012/0321667. Exemplary siRNA and shRNA that downregulate expression of HLA class I and/or HLA class II genes are described, e.g., in U.S. publication No.: US 2007/0036773.

### CRISPR to inhibit TCR or HLA

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene.

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845.

The CRISPR/Cas system has been modified for use in gene editing (silencing, enhancing or changing specific genes) in eukaryotes such as mice or primates. Wiedenheft et al. (2012) Nature 482: 331-8. This is accomplished by introducing into the eukaryotic cell a plasmid containing a specifically designed CRISPR and one or more appropriate Cas.

The CRISPR sequence, sometimes called a CRISPR locus, comprises alternating repeats and spacers. In a naturally-occurring CRISPR, the spacers usually comprise sequences foreign to the bacterium such as a plasmid or phage sequence; in the TCR and/or HLA CRISPR/Cas system, the spacers are derived from the TCR or HLA gene sequence.

RNA from the CRISPR locus is constitutively expressed and processed by Cas proteins into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836.

As these naturally occur in many different types of bacteria, the exact arrangements of the CRISPR and structure, function and number of Cas genes and their product differ somewhat from species to species. Haft et al. (2005) PLoS Comput. Biol. 1: e60; Kunin et al. (2007) Genome Biol. 8: R61; Mojica et al. (2005) J. Mol. Evol. 60: 174-182; Bolotin et al. (2005) Microbiol. 151: 2551-2561; Pourcel et al. (2005) Microbiol. 151: 653-663; and Stern et al. (2010) Trends. Genet. 28: 335-340. For example, the Cse (Cas subtype, E. coli) proteins (e.g., CasA) form a functional complex, Cascade, that processes CRISPR RNA transcripts into spacer-repeat units that Cascade retains. Brouns et al. (2008) Science 321: 960-964. In other prokaryotes, Cas6 processes the CRISPR transcript. The CRISPR-based phage inactivation in E. coli requires Cascade and Cas3, but not Cas1 or Cas2. The Cmr (Cas RAMP module) proteins in Pyrococcus furiosus and other prokaryotes form a functional complex with small CRISPR RNAs that recognizes and cleaves complementary target RNAs. A simpler CRISPR system relies on the protein Cas9, which is a nuclease with two active cutting sites, one for each strand of the double helix. Combining Cas9 and modified CRISPR locus RNA can be used in a system for gene editing. Pennisi (2013) Science 341: 833-836.

The CRISPR/Cas system can thus be used to edit a TCR and/or HLA gene (adding or deleting a basepair), or introducing a premature stop which thus decreases expression of a TCR and/or HLA. The CRISPR/Cas system can alternatively be used like RNA interference, turning off TCR and/or HLA gene in a reversible fashion. In a mammalian cell, for example, the RNA can guide the Cas protein to a TCR and/or HLA promoter, sterically blocking RNA polymerases.

Artificial CRISPR/Cas systems can be generated which inhibit TCR and/or HLA, using technology known in the art, e.g., that described in U.S. Publication No. 20140068797, and Cong (2013) Science 339: 819-823. Other artificial CRISPR/Cas systems that are known in the art may also be generated which inhibit TCR and/or HLA, e.g., that described in Tsai (2014) Nature Biotechnol., 32:6 569-576, U.S. Patent No.: 8,871,445; 8,865,406; 8,795,965; 8,771,945; and 8,697,359.

### TALEN to inhibit TCR and/or HLA

"TALEN" or "TALEN to HLA and/or TCR" or "TALEN to inhibit HLA and/or TCR" refers to a transcription activator-like effector nuclease, an artificial nuclease which can be used to edit the HLA and/or TCR gene.

TALENs are produced artificially by fusing a TAL effector DNA binding domain to a DNA cleavage domain. Transcription activator-like effects (TALEs) can be engineered to bind any desired DNA sequence, including a portion of the HLA or TCR gene. By combining an engineered TALE with a DNA cleavage domain, a restriction enzyme can be produced which is specific to any desired DNA sequence, including a HLA or TCR sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence.

To produce a TALEN, a TALE protein is fused to a nuclease (N), which is a wild-type or mutated FokI endonuclease. Several mutations to FokI have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) J. Mol. Biol. 200: 96.

The FokI domain functions as a dimer, requiring two constructs with unique DNA binding domains for sites in the target genome with proper orientation and spacing. Both the number of amino acid residues between the TALE DNA binding domain and the FokI cleavage domain and the number of bases between the two individual TALEN binding sites appear to be important parameters for achieving high levels of activity. Miller et al. (2011) Nature Biotech. 29: 143-8.

A HLA or TCR TALEN can be used inside a cell to produce a double-stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN; depending on the sequences of the foreign DNA and chromosomal sequence, this process can be used to correct a defect in the HLA or TCR gene or introduce such a defect into a wt HLA or TCR gene, thus decreasing expression of HLA or TCR.

TALENs specific to sequences in HLA or TCR can be constructed using any method known in the art, including various schemes using modular components. Zhang et al. (2011) Nature Biotech. 29: 149-53; Geibler et al. (2011) PLoS ONE 6: e19509.

### Zinc finger nuclease to inhibit HLA and/or TCR

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR gene.

Like a TALEN, a ZFN comprises a FokI nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160.

A zinc finger is a small protein structural motif stabilized by one or more zinc ions. A zinc finger can comprise, for example, Cys2His2, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one-hybrid systems, bacterial one-hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5.

Also like a TALEN, a ZFN can create a double-stranded break in the DNA, which can create a frame-shift mutation if improperly repaired, leading to a decrease in the expression and amount of HLA and/or TCR in a cell. ZFNs can also be used with homologous recombination to mutate in the HLA or TCR gene.

ZFNs specific to sequences in HLA AND/OR TCR can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; Guo et al. (2010) J. Mol. Biol. 400: 96; U.S. Patent Publication 2011/0158957; and U.S. Patent Publication 2012/0060230.

### Telomerase expression

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an embodiment, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

In one aspect, the disclosure features a method of making a population of immune effector cells (e.g., T cells, NK cells). In an embodiment, the method comprises: providing a population of immune effector cells (e.g., T cells or NK cells), contacting the population of immune effector cells with a nucleic acid encoding a CAR; and contacting the population of immune effector cells with a nucleic acid encoding a telomerase subunit, e.g., hTERT, under conditions that allow for CAR and telomerase expression.

In an embodiment, the nucleic acid encoding the telomerase subunit is DNA. In an embodiment, the nucleic acid encoding the telomerase subunit comprises a promoter capable of driving expression of the telomerase subunit.

In an embodiment, hTERT has the amino acid sequence of GenBank Protein ID AAC51724.1 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795) as follows:





















 (SEQ ID NO: 63)

In an embodiment, the hTERT has a sequence at least 80%, 85%, 90%, 95%, 96^, 97%, 98%, or 99% identical to the sequence of SEQ ID NO: 63. In an embodiment, the hTERT has a sequence of SEQ ID NO: 63. In an embodiment, the hTERT comprises a deletion (e.g., of no more than 5, 10, 15, 20, or 30 amino acids) at the N-terminus, the C-terminus, or both. In an embodiment, the hTERT comprises a transgenic amino acid sequence (e.g., of no more than 5, 10, 15, 20, or 30 amino acids) at the N-terminus, the C-terminus, or both.

In an embodiment, the hTERT is encoded by the nucleic acid sequence of GenBank Accession No. AF018167 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795):

1 caggcagcgt ggtcctgctg cgcacgtggg aagccctggc cccggccacc cccgcgatgc
61 cgcgcgctcc ccgctgccga gccgtgcgct ccctgctgcg cagccactac cgcgaggtgc
121 tgccgctggc cacgttcgtg cggcgcctgg ggccccaggg ctggcggctg gtgcagcgcg
181 gggacccggc ggctttccgc gcgctggtgg cccagtgcct ggtgtgcgtg ccctgggacg
241 cacggccgcc ccccgccgcc ccctccttcc gccaggtgtc ctgcctgaag gagctggtgg
301 cccgagtgct gcagaggctg tgcgagcgcg gcgcgaagaa cgtgctggcc ttcggcttcg
361 cgctgctgga cggggcccgc gggggccccc ccgaggcctt caccaccagc gtgcgcagct
421 acctgcccaa cacggtgacc gacgcactgc gggggagcgg ggcgtggggg ctgctgttgc
481 gccgcgtggg cgacgacgtg ctggttcacc tgctggcacg ctgcgcgctc tttgtgctgg
541 tggctcccag ctgcgcctac caggtgtgcg ggccgccgct gtaccagctc ggcgctgcca
601 ctcaggcccg gcccccgcca cacgctagtg gaccccgaag gcgtctggga tgcgaacggg
661 cctggaacca tagcgtcagg gaggccgggg tccccctggg cctgccagcc ccgggtgcga
721 ggaggcgcgg gggcagtgcc agccgaagtc tgccgttgcc caagaggccc aggcgtggcg

781 ctgcccctga gccggagcgg acgcccgttg ggcaggggtc ctgggcccac ccgggcagga
841 cgcgtggacc gagtgaccgt ggtttctgtg tggtgtcacc tgccagaccc gccgaagaag
901 ccacctcttt ggagggtgcg ctctctggca cgcgccactc ccacccatcc gtgggccgcc
961 agcaccacgc gggcccccca tccacatcgc ggccaccacg tccctgggac acgccttgtc
1021 ccccggtgta cgccgagacc aagcacttcc tctactcctc aggcgacaag gagcagctgc
1081 ggccctcctt cctactcagc tctctgaggc ccagcctgac tggcgctcgg aggctcgtgg
1141 agaccatctt tctgggttcc aggccctgga tgccagggac tccccgcagg ttgccccgcc
1201 tgccccagcg ctactggcaa atgcggcccc tgtttctgga gctgcttggg aaccacgcgc
1261 agtgccccta cggggtgctc ctcaagacgc actgcccgct gcgagctgcg gtcaccccag
1321 cagccggtgt ctgtgcccgg gagaagcccc agggctctgt ggcggccccc gaggaggagg
1381 acacagaccc ccgtcgcctg gtgcagctgc tccgccagca cagcagcccc tggcaggtgt
1441 acggcttcgt gcgggcctgc ctgcgccggc tggtgccccc aggcctctgg ggctccaggc
1501 acaacgaacg ccgcttcctc aggaacacca agaagttcat ctccctgggg aagcatgcca
1561 agctctcgct gcaggagctg acgtggaaga tgagcgtgcg gggctgcgct tggctgcgca
1621 ggagcccagg ggttggctgt gttccggccg cagagcaccg tctgcgtgag gagatcctgg
1681 ccaagttcct gcactggctg atgagtgtgt acgtcgtcga gctgctcagg tctttctttt
1741 atgtcacgga gaccacgttt caaaagaaca ggctcttttt ctaccggaag agtgtctgga
1801 gcaagttgca aagcattgga atcagacagc acttgaagag ggtgcagctg cgggagctgt
1861 cggaagcaga ggLcaggcag caLcgggaag ccaggcccgc ccLgcLgacg Lccagaclcc
1921 gcttcatccc caagcctgac gggctgcggc cgattgtgaa catggactac gtcgtgggag
1981 ccagaacgtt ccgcagagaa aagagggccg agcgtctcac ctcgagggtg aaggcactgt
2041 tcagcgtgct caactacgag cgggcgcggc gccccggcct cctgggcgcc tctgtgctgg
2101 gcctggacga tatccacagg gcctggcgca ccttcgtgct gcgtgtgcgg gcccaggacc
2161 cgccgcctga gctgtacttt gtcaaggtgg atgtgacggg cgcgtacgac accatccccc
2221 aggacaggct cacggaggtc atcgccagca tcatcaaacc ccagaacacg tactgcgtgc
2281 gtcggtatgc cgtggtccag aaggccgccc atgggcacgt ccgcaaggcc ttcaagagcc
2341 acgtctctac cttgacagac ctccagccgt acatgcgaca gttcgtggct cacctgcagg
2401 agaccagccc gctgagggat gccgtcgtca tcgagcagag ctcctccctg aatgaggcca
2461 gcagtggcct cttcgacgtc ttcctacgct tcatgtgcca ccacgccgtg cgcatcaggg
2521 gcaagtccta cgtccagtgc caggggatcc cgcagggctc catcctctcc acgctgctct
2581 gcagcctgtg ctacggcgac atggagaaca agctgtttgc ggggattcgg cgggacgggc
2641 tgctcctgcg tttggtggat gatttcttgt tggtgacacc tcacctcacc cacgcgaaaa
2701 ccttcctcag gaccctggtc cgaggtgtcc ctgagtatgg ctgcgtggtg aacttgcgga
2761 agacagtggt gaacttccct gtagaagacg aggccctggg tggcacggct tttgttcaga
2821 tgccggccca cggcctattc ccctggtgcg gcctgctgct ggatacccgg accctggagg
2881 tgcagagcga ctactccagc tatgcccgga cctccatcag agccagtctc accttcaacc
2941 gcggcttcaa ggctgggagg aacatgcgtc gcaaactctt tggggtcttg cggctgaagt
3001 gtcacagcct gtttctggat ttgcaggtga acagcctcca gacggtgtgc accaacatct
3061 acaagatcct cctgctgcag gcgtacaggt ttcacgcatg tgtgctgcag ctcccatttc
3121 atcagcaagt ttggaagaac cccacatttt tcctgcgcgt catctctgac acggcctccc
3181 tctgctactc catcctgaaa gccaagaacg cagggatgtc gctgggggcc aagggcgccg
3241 ccggccctct gccctccgag gccgtgcagt ggctgtgcca ccaagcattc ctgctcaagc
3301 tgactcgaca ccgtgtcacc tacgtgccac tcctggggtc actcaggaca gcccagacgc
3361 agctgagtcg gaagctcccg gggacgacgc tgactgccct ggaggccgca gccaacccgg
3421 cactgccctc agacttcaag accatcctgg actgatggcc acccgcccac agccaggccg
3481 agagcagaca ccagcagccc tgtcacgccg ggctctacgt cccagggagg gaggggcggc
3541 ccacacccag gcccgcaccg ctgggagtct gaggcctgag tgagtgtttg gccgaggcct
3601 gcatgtccgg ctgaaggctg agtgtccggc tgaggcctga gcgagtgtcc agccaagggc
3661 tgagtgtcca gcacacctgc cgtcttcact tccccacagg ctggcgctcg gctccacccc
3721 agggccagct tttcctcacc aggagcccgg cttccactcc ccacatagga atagtccatc
3781 cccagattcg ccattgttca cccctcgccc tgccctcctt tgccttccac ccccaccatc
3841 caggtggaga ccctgagaag gaccctggga gctctgggaa tttggagtga ccaaaggtgt
3901 gccctgtaca caggcgagga ccctgcacct ggatgggggt ccctgtgggt caaattgggg
3961 ggaggtgctg tgggagtaaa atactgaata tatgagtttt tcagttttga aaaaaaaaaa
4021 aaaaaaa (SEQ ID NO: 64)

In an embodiment, the hTERT is encoded by a nucleic acid having a sequence at least 80%, 85%, 90%, 95%, 96, 97%, 98%, or 99% identical to the sequence of SEQ ID NO: 64. In an embodiment, the hTERT is encoded by a nucleic acid of SEQ ID NO: 64.

### Activation and Expansion of Immune Effector Cells (e.g., T Cells)

Immune effector cells such as T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, a population of immune effector cells e.g., T regulatory cell depleted cells, may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody can be used. Examples of an anti-CD28 antibody include 9.3, B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then crosslinked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present invention.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3:CD28 antibody bound to the beads ranges from 100:1 to 1:100 and all integer values there between. In one aspect, more anti-CD28 antibody is bound to the particles than anti-CD3 antibody, i.e., the ratio of CD3:CD28 is less than one. In certain aspects, the ratio of anti CD28 antibody to anti CD3 antibody bound to the beads is greater than 2:1. In one particular aspect, a 1:100 CD3:CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one preferred aspect, a 1:10 CD3:CD28 ratio of antibody bound to beads is used. In one aspect, a 1:3 CD3:CD28 ratio of antibody bound to the beads is used. In yet one aspect, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one aspect, a ratio of particles to cells of 1:1 or less is used. In one particular aspect, a preferred particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type. In one aspect, the most typical ratios for use are in the neighborhood of 1:1, 2:1 and 3:1 on the first day.

In further aspects, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS^{®} M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present invention. In certain aspects, it may be desirable to significantly decrease the volume in which particles and cells are mixed together (i.e., increase the concentration of cells), to ensure maximum contact of cells and particles. For example, in one aspect, a concentration of about 10 billion cells/ml, 9 billion/ml, 8 billion/ml, 7 billion/ml, 6 billion/ml, 5 billion/ml, or 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+ T cells that normally have weaker CD28 expression.

In one embodiment, cells transduced with a nucleic acid encoding a CAR, e.g., a CAR described herein, are expanded, e.g., by a method described herein. In one embodiment, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one embodiment, the cells are expanded for a period of 4 to 9 days. In one embodiment, the cells are expanded for a period of 8 days or less, e.g., 7, 6 or 5 days. In one embodiment, the cells, e.g., a CD19 CAR cell described herein, are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one embodiment, the cells, e.g., a CD19 CAR cell described herein, expanded for 5 days show at least a one, two, three or four fold increase in cells doublings upon antigen stimulation as compared to the same cells expanded in culture for 9 days under the same culture conditions. In one embodiment, the cells, e.g., the cells expressing a CD19 CAR described herein, are expanded in culture for 5 days, and the resulting cells exhibit higher proinflammatory cytokine production, e.g., IFN-γ and/or GM-CSF levels, as compared to the same cells expanded in culture for 9 days under the same culture conditions. In one embodiment, the cells, e.g., a CD19 CAR cell described herein, expanded for 5 days show at least a one, two, three, four, five, ten fold or more increase in pg/ml of proinflammatory cytokine production, e.g., IFN-γ and/or GM-CSF levels, as compared to the same cells expanded in culture for 9 days under the same culture conditions.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one embodiment, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one embodiment, the cells are expanded in the presence of IL-15 and/or IL-7 (e.g., IL-15 and IL-7).

In embodiments, methods described herein, e.g., CAR-expressing cell manufacturing methods, comprise removing T regulatory cells, e.g., CD25+ T cells, from a cell population, e.g., using an anti-CD25 antibody, or fragment thereof, or a CD25-binding ligand, IL-2. Methods of removing T regulatory cells, e.g., CD25+ T cells, from a cell population are described herein. In embodiments, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with IL-15 and/or IL-7. For example, the cell population (e.g., that has previously contacted an anti-CD25 antibody, fragment thereof, or CD25-binding ligand) is expanded in the presence of IL-15 and/or IL-7.

In some embodiments a CAR-expressing cell described herein is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15, during the manufacturing of the CAR-expressing cell, e.g., ex vivo. In embodiments, a CAR-expressing cell described herein is contacted with a composition comprising a IL-15 polypeptide during the manufacturing of the CAR-expressing cell, e.g., ex vivo. In embodiments, a CAR-expressing cell described herein is contacted with a composition comprising a combination of both a IL-15 polypeptide and a IL-15 Ra polypeptide during the manufacturing of the CAR-expressing cell, e.g., ex vivo. In embodiments, a CAR-expressing cell described herein is contacted with a composition comprising hetIL-15 during the manufacturing of the CAR-expressing cell, e.g., ex vivo.

In one embodiment the CAR-expressing cell described herein is contacted with a composition comprising hetIL-15 during ex vivo expansion. In an embodiment, the CAR-expressing cell described herein is contacted with a composition comprising an IL-15 polypeptide during ex vivo expansion. In an embodiment, the CAR-expressing cell described herein is contacted with a composition comprising both an IL-15 polypeptide and an IL-15Ra polypeptide during ex vivo expansion. In one embodiment the contacting results in the survival and proliferation of a lymphocyte subpopulation, e.g., CD8+ T cells.

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

Once a CAR described herein is constructed, various assays can be used to evaluate the activity of the molecule, such as but not limited to, the ability to expand T cells following antigen stimulation, sustain T cell expansion in the absence of re-stimulation, and anti-cancer activities in appropriate in vitro and animal models. Assays to evaluate the effects of a cars of the present invention are described in further detail below

Western blot analysis of CAR expression in primary T cells can be used to detect the presence of monomers and dimers. See, *e.g.,* Milone et al., Molecular Therapy 17(8): 1453-1464 (2009). Very briefly, T cells (1:1 mixture of CD4⁺ and CD8⁺ T cells) expressing the CARs are expanded *in vitro* for more than 10 days followed by lysis and SDS-PAGE under reducing conditions. CARs containing the full length TCR-ζ cytoplasmic domain and the endogenous TCR-ζ chain are detected by western blotting using an antibody to the TCR-ζ chain. The same T cell subsets are used for SDS-PAGE analysis under non-reducing conditions to permit evaluation of covalent dimer formation.

*In vitro* expansion of CAR⁺ T cells following antigen stimulation can be measured by flow cytometry. For example, a mixture of CD4⁺ and CD8⁺ T cells are stimulated with αCD3/αCD28 aAPCs followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, *e.g.,* Milone et al., Molecular Therapy 17(8): 1453-1464 (2009). Alternatively, a mixture of CD4⁺ and CD8⁺ T cells are stimulated with αCD3/αCD28 coated magnetic beads on day 0, and transduced with CAR on day 1 using a bicistronic lentiviral vector expressing CAR along with eGFP using a 2A ribosomal skipping sequence. Cultures are re-stimulated with either a cancer associated antigen as described herein⁺ K562 cells (K562 expressing a cancer associated antigen as described herein), wild-type K562 cells (K562 wild type) or K562 cells expressing hCD32 and 4-1BBL in the presence of antiCD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, *e.g.,* Milone et al., Molecular Therapy 17(8): 1453-1464 (2009).

Sustained CAR⁺ T cell expansion in the absence of re-stimulation can also be measured. See, e.g., Milone et al., Molecular Therapy 17(8): 1453-1464 (2009). Briefly, mean T cell volume (fl) is measured on day 8 of culture using a Coulter Multisizer III particle counter, a Nexcelom Cellometer Vision or Millipore Scepter, following stimulation with αCD3/αCD28 coated magnetic beads on day 0, and transduction with the indicated CAR on day 1.

Animal models can also be used to measure a CART activity. For example, xenograft model using human a cancer associated antigen described herein-specific CAR⁺ T cells to treat a primary human pre-B ALL in immunodeficient mice can be used. See, e.g., Milone et al., Molecular Therapy 17(8): 1453-1464 (2009). Very briefly, after establishment of ALL, mice are randomized as to treatment groups. Different numbers of a cancer associated antigen -specific CARengineered T cells are coinjected at a 1:1 ratio into NOD-SCID-γ^{-/-} mice bearing B-ALL. The number of copies of a cancer associated antigen -specific CAR vector in spleen DNA from mice is evaluated at various times following T cell injection. Animals are assessed for leukemia at weekly intervals. Peripheral blood a cancer associate antigen as described herein⁺ B-ALL blast cell counts are measured in mice that are injected with a cancer associated antigen described herein-ζ CAR⁺ T cells or mock-transduced T cells. Survival curves for the groups are compared using the log-rank test. In addition, absolute peripheral blood CD4⁺ and CD8⁺ T cell counts 4 weeks following T cell injection in NOD-SCID-γ^{-/-} mice can also be analyzed. Mice are injected with leukemic cells and 3 weeks later are injected with T cells engineered to express CAR by a bicistronic lentiviral vector that encodes the CAR linked to eGFP. T cells are normalized to 45-50% input GFP⁺ T cells by mixing with mock-transduced cells prior to injection, and confirmed by flow cytometry. Animals are assessed for leukemia at 1-week intervals. Survival curves for the CAR⁺ T cell groups are compared using the log-rank test.

Dose dependent CAR treatment response can be evaluated. See, *e.g.,* Milone et al., Molecular Therapy 17(8): 1453-1464 (2009). For example, peripheral blood is obtained 35-70 days after establishing leukemia in mice injected on day 21 with CAR T cells, an equivalent number of mock-transduced T cells, or no T cells. Mice from each group are randomly bled for determination of peripheral blood a cancer associate antigen as described herein⁺ ALL blast counts and then killed on days 35 and 49. The remaining animals are evaluated on days 57 and 70.

Assessment of cell proliferation and cytokine production has been previously described, *e.g.,* at Milone et al., Molecular Therapy 17(8): 1453-1464 (2009). Briefly, assessment of CAR-mediated proliferation is performed in microtiter plates by mixing washed T cells with K562 cells expressing a cancer associated antigen described herein (K19) or CD32 and CD137 (KT32-BBL) for a final T-cell: K562 ratio of 2:1. K562 cells are irradiated with gamma-radiation prior to use. Anti-CD3 (clone OKT3) and anti- CD28 (clone 9.3) monoclonal antibodies are added to cultures with KT32-BBL cells to serve as a positive control for stimulating T-cell proliferation since these signals support long-term CD8⁺ T cell expansion *ex vivo.* T cells are enumerated in cultures using CountBright^{™} fluorescent beads (Invitrogen, Carlsbad, CA) and flow cytometry as described by the manufacturer. CAR⁺ T cells are identified by GFP expression using T cells that are engineered with eGFP-2A linked CAR-expressing lentiviral vectors. For CAR+ T cells not expressing GFP, the CAR+ T cells are detected with biotinylated recombinant a cancer associate antigen as described herein protein and a secondary avidin-PE conjugate. CD4+ and CD8⁺ expression on T cells are also simultaneously detected with specific monoclonal antibodies (BD Biosciences). Cytokine measurements are performed on supernatants collected 24 hours following re-stimulation using the human TH1/TH2 cytokine cytometric bead array kit (BD Biosciences, San Diego, CA) according the manufacturer's instructions. Fluorescence is assessed using a FACScalibur flow cytometer, and data is analyzed according to the manufacturer's instructions.

Cytotoxicity can be assessed by a standard 51Cr-release assay. See, e.g., Milone et al., Molecular Therapy 17(8): 1453-1464 (2009). Briefly, target cells (K562 lines and primary pro-B-ALL cells) are loaded with 51Cr (as NaCrO4, New England Nuclear, Boston, MA) at 37°C for 2 hours with frequent agitation, washed twice in complete RPMI and plated into microtiter plates. Effector T cells are mixed with target cells in the wells in complete RPMI at varying ratios of effector cell:target cell (E:T). Additional wells containing media only (spontaneous release, SR) or a 1% solution of triton-X 100 detergent (total release, TR) are also prepared. After 4 hours of incubation at 37°C, supernatant from each well is harvested. Released 51Cr is then measured using a gamma particle counter (Packard Instrument Co., Waltham, MA). Each condition is performed in at least triplicate, and the percentage of lysis is calculated using the formula: % Lysis = (ER- SR) / (TR - SR), where ER represents the average 51Cr released for each experimental condition.

Imaging technologies can be used to evaluate specific trafficking and proliferation of CARs in tumor-bearing animal models. Such assays have been described, for example, in Barrett et al., Human Gene Therapy 22:1575-1586 (2011). Briefly, NOD/SCID/γc^{-/-} (NSG) mice are injected IV with Nalm-6 cells followed 7 days later with T cells 4 hour after electroporation with the CAR constructs. The T cells are stably transfected with a lentiviral construct to express firefly luciferase, and mice are imaged for bioluminescence. Alternatively, therapeutic efficacy and specificity of a single injection of CAR⁺ T cells in Nalm-6 xenograft model can be measured as the following: NSG mice are injected with Nalm-6 transduced to stably express firefly luciferase, followed by a single tail-vein injection of T cells electroporated with cars of the present invention 7 days later. Animals are imaged at various time points post injection. For example, photon-density heat maps of firefly luciferasepositive leukemia in representative mice at day 5 (2 days before treatment) and day 8 (24 hr post CAR⁺ PBLs) can be generated.

Other assays, including those described in the Example section herein as well as those that are known in the art can also be used to evaluate the CARs described herein.

### Therapeutic Application

In one aspect, the invention provides methods for treating a disease associated with expression of a cancer associated antigen described herein.

In one aspect, the present invention provides methods of treating cancer by providing to the subject in need thereof immune effector cells (e.g., T cells, NK cells) that are engineered to express an XCAR, wherein X represents a tumor antigen as described herein, and wherein the cancer cells express said X tumor antigen.

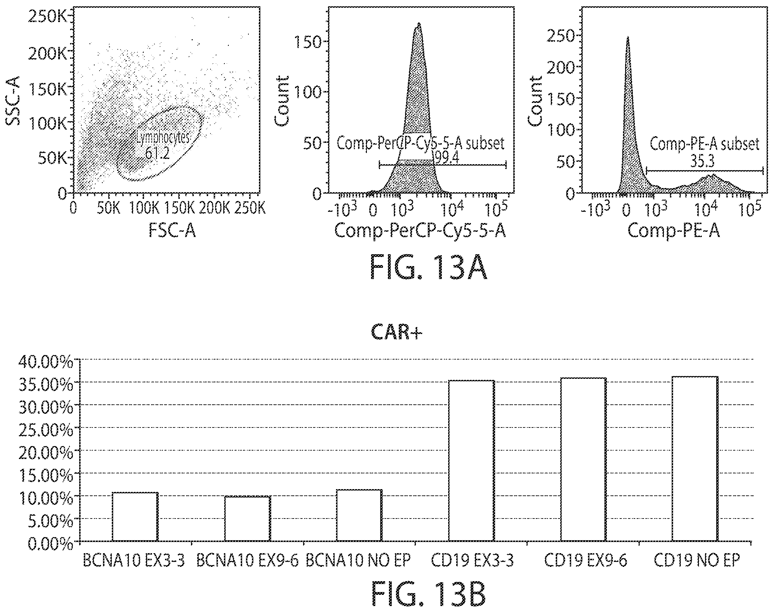

In one aspect, the present invention provides methods of treating cancer by providing to the subject in need thereof immune effector cells (e.g., T cells, NK cells) that are engineered to express a XCAR described herein, wherein the cancer cells express X. In one embodiment, X is expressed on both normal cells and cancers cells, but is expressed at lower levels on normal cells. In one embodiment, the method further comprises selecting a CAR that binds X with an affinity that allows the XCAR to bind and kill the cancer cells expressing X but less than 30%, 25%, 20%, 15%, 10%, 5% or less of the normal cells expressing X are killed, e.g., as determined by an assay described herein. For example, the assay described in FIGS. 13A and 13B can be used or a killing assay such as flow cytometry based on Cr51 CTL. In one embodiment, the selected CAR has an antigen binding domain that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the selected antigen binding domain has a binding affinity that is at least five-fold, 10-fold, 20-fold, 30-fold, 50-fold, 100-fold or 1,000-fold less than a reference antibody, e.g., an antibody described herein.

In one embodiment, the present invention provides methods of treating cancer by providing to the subject in need thereof immune effector cells (e.g., T cells, NK cells) that are engineered to express CD19 CAR, wherein the cancer cells express CD19. In one embodiment, the cancer to be treated is ALL (acute lymphoblastic leukemia), CLL (chronic lymphocytic leukemia), DLBCL (diffuse large B-cell lymphoma), MCL (Mantle cell lymphoma, or MM (multiple myeloma).

In one aspect, the present invention provides methods of treating cancer by providing to the subject in need thereof immune effector cells (e.g., T cells, NK cells) that are engineered to express an EGFRvIIICAR, wherein the cancer cells express EGFRvIII. In one embodiment, the cancer to be treated is glioblastoma.

In one aspect, the present invention provides methods of treating cancer by providing to the subject in need thereof immune effector cells (e.g., T cells, NK cells) that are engineered to express a mesothelinCAR, wherein the cancer cells express mesothelin. In one embodiment, the cancer to be treated is mesothelioma, pancreatic cancer, or ovarian cancer.

In one aspect, the present invention provides methods of treating cancer by providing to the subject in need thereof immune effector cells (e.g., T cells, NK cells) that are engineered to express a CD123CAR, wherein the cancer cells express CD123. In one embodiment, the cancer to be treated is AML.

In one aspect, the present invention provides methods of treating cancer by providing to the subject in need thereof immune effector cells (e.g., T cells, NK cells) that are engineered to express a CD22CAR, wherein the cancer cells express CD22. In one embodiment, the cancer to be treated is B cell malignancies.

In one aspect, the present invention provides methods of treating cancer by providing to the subject in need thereof immune effector cells (e.g., T cells, NK cells) that are engineered to express a CS-1CAR, wherein the cancer cells express CS-1. In one embodiment, the cancer to be treated is multiple myeloma.

In one aspect, the present invention provides methods of treating cancer by providing to the subject in need thereof immune effector cells (e.g., T cells, NK cells) that are engineered to express a CLL-1CAR, wherein the cancer cells express CLL-1. In one embodiment, the cancer to be treated is AML.

In one aspect, the present invention provides methods of treating cancer by providing to the subject in need thereof immune effector cells (e.g., T cells, NK cells) that are engineered to express a CD33CAR, wherein the cancer cells express CD33. In one embodiment, the cancer to be treated is AML.

In one aspect, the present invention provides methods of treating cancer by providing to the subject in need thereof immune effector cells (e.g., T cells, NK cells) that are engineered to express a GD2CAR, wherein the cancer cells express GD2. In one embodiment, the cancer to be treated is neuroblastoma.

In one aspect, the present invention provides methods of treating cancer by providing to the subject in need thereof immune effector cells (e.g., T cells, NK cells) that are engineered to express a BCMACAR, wherein the cancer cells express BCMA. In one embodiment, the cancer to be treated is multiple myeloma.

In one aspect, the present invention provides methods of treating cancer by providing to the subject in need thereof immune effector cells (e.g., T cells, NK cells) that are engineered to express a TnCAR, wherein the cancer cells express Tn antigen. In one embodiment, the cancer to be treated is ovarian cancer.

In one aspect, the present invention provides methods of treating cancer by providing to the subject in need thereof immune effector cells (e.g., T cells, NK cells) that are engineered to express a PSMACAR, wherein the cancer cells express PSMA. In one embodiment, the cancer to be treated is prostate cancer.

In one aspect, the present invention provides methods of treating cancer by providing to the subject in need thereof immune effector cells (e.g., T cells, NK cells) that are engineered to express a ROR1CAR, wherein the cancer cells express ROR1. In one embodiment, the cancer to be treated is B cell malignancies.

In one aspect, the present invention provides methods of treating cancer by providing to the subject in need thereof immune effector cells (e.g., T cells, NK cells) that are engineered to express a FLT3 CAR, wherein the cancer cells express FLT3. In one embodiment, the cancer to be treated is AML.

In one aspect, the present invention provides methods of treating cancer by providing to the subject in need thereof immune effector cells (e.g., T cells, NK cells) that are engineered to express a TAG72CAR, wherein the cancer cells express TAG72. In one embodiment, the cancer to be treated is gastrointestinal cancer.

In one aspect, the present invention provides methods of treating cancer by providing to the subject in need thereof immune effector cells (e.g., T cells, NK cells) that are engineered to express a CD38CAR, wherein the cancer cells express CD38. In one embodiment, the cancer to be treated is multiple myeloma.

In one aspect, the present invention provides methods of treating cancer by providing to the subject in need thereof immune effector cells (e.g., T cells, NK cells) that are engineered to express a CD44v6CAR, wherein the cancer cells express CD44v6. In one embodiment, the cancer to be treated is cervical cancer, AML, or MM.

In one aspect, the present invention provides methods of treating cancer by providing to the subject in need thereof immune effector cells (e.g., T cells, NK cells) that are engineered to express a CEACAR, wherein the cancer cells express CEA. In one embodiment, the cancer to be treated is pastrointestinal cancer, or pancreatic cancer.

In one aspect, the present invention provides methods of treating cancer by providing to the subject in need thereof immune effector cells (e.g., T cells, NK cells) that are engineered to express an EPCAMCAR, wherein the cancer cells express EPCAM. In one embodiment, the cancer to be treated is gastrointestinal cancer.

In one aspect, the present invention provides methods of treating cancer by providing to the subject in need thereof immune effector cells (e.g., T cells, NK cells) that are engineered to express a B7H3CAR, wherein the cancer cells express B7H3.

In one aspect, the present invention provides methods of treating cancer by providing to the subject in need thereof immune effector cells (e.g., T cells, NK cells) that are engineered to express a KITCAR, wherein the cancer cells express KIT. In one embodiment, the cancer to be treated is gastrointestinal cancer.

In one aspect, the present invention provides methods of treating cancer by providing to the subject in need thereof immune effector cells (e.g., T cells, NK cells) that are engineered to express an IL-13Ra2CAR, wherein the cancer cells express IL-13Ra2. In one embodiment, the cancer to be treated is glioblastoma.

In one aspect, the present invention provides methods of treating cancer by providing to the subject in need thereof immune effector cells (e.g., T cells, NK cells) that are engineered to express a PRSS21CAR, wherein the cancer cells express PRSS21. In one embodiment, the cancer to be treated is selected from ovarian, pancreatic, lung and breast cancer.

In one aspect, the present invention provides methods of treating cancer by providing to the subject in need thereof immune effector cells (e.g., T cells, NK cells) that are engineered to express a CD30CAR, wherein the cancer cells express CD30. In one embodiment, the cancer to be treated is lymphomas, or leukemias.

In one aspect, the present invention provides methods of treating cancer by providing to the subject in need thereof immune effector cells (e.g., T cells, NK cells) that are engineered to express a GD3CAR, wherein the cancer cells express GD3. In one embodiment, the cancer to be treated is melanoma.

In one aspect, the present invention provides methods of treating cancer by providing to the subject in need thereof immune effector cells (e.g., T cells, NK cells) that are engineered to express a CD171CAR, wherein the cancer cells express CD171. In one embodiment, the cancer to be treated is neuroblastoma, ovarian cancer, melanoma, breast cancer, pancreatic cancer, colon cancers, or NSCLC (non-small cell lung cancer).

In one aspect, the present invention provides methods of treating cancer by providing to the subject in need thereof immune effector cells (e.g., T cells, NK cells) that are engineered to express an IL-11RaCAR, wherein the cancer cells express IL-11Ra. In one embodiment, the cancer to be treated is osteosarcoma.

In one aspect, the present invention provides methods of treating cancer by providing to the subject in need thereof immune effector cells (e.g., T cells, NK cells) that are engineered to express a PSCACAR, wherein the cancer cells express PSCA. In one embodiment, the cancer to be treated is prostate cancer.

In one aspect, the present invention provides methods of treating cancer by providing to the subject in need thereof immune effector cells (e.g., T cells, NK cells) that are engineered to express a VEGFR2CAR, wherein the cancer cells express VEGFR2. In one embodiment, the cancer to be treated is a solid tumor.

In one aspect, the present invention provides methods of treating cancer by providing to the subject in need thereof immune effector cells (e.g., T cells, NK cells) that are engineered to express a LewisYCAR, wherein the cancer cells express LewisY. In one embodiment, the cancer to be treated is ovarian cancer, or AML.

In one aspect, the present invention provides methods of treating cancer by providing to the subject in need thereof immune effector cells (e.g., T cells, NK cells) that are engineered to express a CD24CAR, wherein the cancer cells express CD24. In one embodiment, the cancer to be treated is pancreatic cancer.

In one aspect, the present invention provides methods of treating cancer by providing to the subject in need thereof immune effector cells (e.g., T cells, NK cells) that are engineered to express a PDGFR-betaCAR, wherein the cancer cells express PDGFR-beta. In one embodiment, the cancer to be treated is breast cancer, prostate cancer, GIST (gastrointestinal stromal tumor), CML, DFSP (dermatofibrosarcoma protuberans), or glioma.

In one aspect, the present invention provides methods of treating cancer by providing to the subject in need thereof immune effector cells (e.g., T cells, NK cells) that are engineered to express a SSEA-4CAR, wherein the cancer cells express SSEA-4. In one embodiment, the cancer to be treated is glioblastoma, breast cancer, lung cancer, or stem cell cancer.

In one aspect, the present invention provides methods of treating cancer by providing to the subject in need thereof immune effector cells (e.g., T cells, NK cells) that are engineered to express a CD20CAR, wherein the cancer cells express CD20. In one embodiment, the cancer to be treated is B cell malignancies.

In one aspect, the present invention provides methods of treating cancer by providing to the subject in need thereof immune effector cells (e.g., T cells, NK cells) that are engineered to express a Folate receptor alphaCAR, wherein the cancer cells express folate receptor alpha. In one embodiment, the cancer to be treated is ovarian cancer, NSCLC, endometrial cancer, renal cancer, or other solid tumors.

In one aspect, the present invention provides methods of treating cancer by providing to the subject in need thereof immune effector cells (e.g., T cells, NK cells) that are engineered to express an ERBB2CAR, wherein the cancer cells express ERBB2 (Her2/neu). In one embodiment, the cancer to be treated is breast cancer, gastric cancer, colorectal cancer, lung cancer, or other solid tumors.

In one aspect, the present invention provides methods of treating cancer by providing to the subject in need thereof immune effector cells (e.g., T cells, NK cells) that are engineered to express a MUC1CAR, wherein the cancer cells express MUC1. In one embodiment, the cancer to be treated is breast cancer, lung cancer, or other solid tumors.

In one aspect, the present invention provides methods of treating cancer by providing to the subject in need thereof immune effector cells (e.g., T cells, NK cells) that are engineered to express an EGFRCAR, wherein the cancer cells express EGFR. In one embodiment, the cancer to be treated is glioblastoma, SCLC (small cell lung cancer), SCCHN (squamous cell carcinoma of the head and neck), NSCLC, or other solid tumors.

In one aspect, the present invention provides methods of treating cancer by providing to the subject in need thereof immune effector cells (e.g., T cells, NK cells) that are engineered to express a NCAMCAR, wherein the cancer cells express NCAM. In one embodiment, the cancer to be treated is neuroblastoma, or other solid tumors.

In one aspect, the present invention provides methods of treating cancer by providing to the subject in need thereof immune effector cells (e.g., T cells, NK cells) that are engineered to express a CAIXCAR, wherein the cancer cells express CAIX. In one embodiment, the cancer to be treated is renal cancer, CRC, cervical cancer, or other solid tumors.

In one aspect, the present invention provides methods of treating cancer by providing to the subject in need thereof immune effector cells (e.g., T cells, NK cells) that are engineered to express an EphA2CAR, wherein the cancer cells express EphA2. In one embodiment, the cancer to be treated is GBM.

In one aspect, the present invention provides methods of treating cancer by providing to the subject in need thereof immune effector cells (e.g., T cells, NK cells) that are engineered to express a GD3CAR, wherein the cancer cells express GD3. In one embodiment, the cancer to be treated is melanoma.

In one aspect, the present invention provides methods of treating cancer by providing to the subject in need thereof immune effector cells (e.g., T cells, NK cells) that are engineered to express a Fucosyl GM1CAR, wherein the cancer cells express Fucosyl GM

In one aspect, the present invention provides methods of treating cancer by providing to the subject in need thereof immune effector cells (e.g., T cells, NK cells) that are engineered to express a sLeCAR, wherein the cancer cells express sLe. In one embodiment, the cancer to be treated is NSCLC, or AML.

In one aspect, the present invention provides methods of treating cancer by providing to the subject in need thereof immune effector cells (e.g., T cells, NK cells) that are engineered to express a GM3CAR, wherein the cancer cells express GM3.

In one aspect, the present invention provides methods of treating cancer by providing to the subject in need thereof immune effector cells (e.g., T cells, NK cells) that are engineered to express a TGS5CAR, wherein the cancer cells express TGS5.

In one aspect, the present invention provides methods of treating cancer by providing to the subject in need thereof immune effector cells (e.g., T cells, NK cells) that are engineered to express a HMWMAACAR, wherein the cancer cells express HMWMAA. In one embodiment, the cancer to be treated is melanoma, glioblastoma, or breast cancer.

In one aspect, the present invention provides methods of treating cancer by providing to the subject in need thereof immune effector cells (e.g., T cells, NK cells) that are engineered to express an o-acetyl-GD2CAR, wherein the cancer cells express o-acetyl-GD2. In one embodiment, the cancer to be treated is neuroblastoma, or melanoma.

In one aspect, the present invention provides methods of treating cancer by providing to the subject in need thereof immune effector cells (e.g., T cells, NK cells) that are engineered to express a CD19CAR, wherein the cancer cells express CD19. In one embodiment, the cancer to be treated isFolate receptor beta AML, myeloma

In one aspect, the present invention provides methods of treating cancer by providing to the subject in need thereof immune effector cells (e.g., T cells, NK cells) that are engineered to express a TEM1/CD248CAR, wherein the cancer cells express TEM1/CD248. In one embodiment, the cancer to be treated is a solid tumor.

In one aspect, the present invention provides methods of treating cancer by providing to the subject in need thereof immune effector cells (e.g., T cells, NK cells) that are engineered to express a TEM7RCAR, wherein the cancer cells express TEM7R. In one embodiment, the cancer to be treated is solid tumor.

In one aspect, the present invention provides methods of treating cancer by providing to the subject in need thereof immune effector cells (e.g., T cells, NK cells) that are engineered to express a CLDN6CAR, wherein the cancer cells express CLDN6. In one embodiment, the cancer to be treated is ovarian cancer, lung cancer, or breast cancer.

In one aspect, the present invention provides methods of treating cancer by providing to the subject in need thereof immune effector cells (e.g., T cells, NK cells) that are engineered to express a TSHRCAR, wherein the cancer cells express TSHR. In one embodiment, the cancer to be treated is thyroid cancer, or multiple myeloma.

In one aspect, the present invention provides methods of treating cancer by providing to the subject in need thereof immune effector cells (e.g., T cells, NK cells) that are engineered to express a GPRC5DCAR, wherein the cancer cells express GPRC5D. In one embodiment, the cancer to be treated is multiple myeloma.

In one aspect, the present invention provides methods of treating cancer by providing to the subject in need thereof immune effector cells (e.g., T cells, NK cells) that are engineered to express a CXORF61CAR, wherein the cancer cells express CXORF61.

In one aspect, the present invention provides methods of treating cancer by providing to the subject in need thereof immune effector cells (e.g., T cells, NK cells) that are engineered to express a CD97CAR, wherein the cancer cells express CD97. In one embodiment, the cancer to be treated is B cell malignancies, gastric cancer, pancreatic cancer, esophageal cancer, glioblastoma, breast cancer, or colorectal cancer.

In one aspect, the present invention provides methods of treating cancer by providing to the subject in need thereof immune effector cells (e.g., T cells, NK cells) that are engineered to express a CD179aCAR, wherein the cancer cells express CD179a. In one embodiment, the cancer to be treated is B cell malignancies.

In one aspect, the present invention provides methods of treating cancer by providing to the subject in need thereof immune effector cells (e.g., T cells, NK cells) that are engineered to express an ALK CAR, wherein the cancer cells express ALK. In one embodiment, the cancer to be treated is NSCLC, ALCL (anaplastic large cell lymphoma), IMT (inflammatory myofibroblastic tumor), or neuroblastoma.

In one aspect, the present invention provides methods of treating cancer by providing to the subject in need thereof immune effector cells (e.g., T cells, NK cells) that are engineered to express a Polysialic acid CAR, wherein the cancer cells express Polysialic acid. In one embodiment, the cancer to be treated is small cell lung cancer.

In one aspect, the present invention provides methods of treating cancer by providing to the subject in need thereof immune effector cells (e.g., T cells, NK cells) that are engineered to express a PLAC1CAR, wherein the cancer cells express PLAC1. In one embodiment, the cancer to be treated is HCC (hepatocellular carcinoma).

In one aspect, the present invention provides methods of treating cancer by providing to the subject in need thereof immune effector cells (e.g., T cells, NK cells) that are engineered to express a GloboHCAR, wherein the cancer cells express GloboH. In one embodiment, the cancer to be treated is ovarian cancer, gastric cancer, prostate cancer, lung cancer, breast cancer, or pancreatic cancer.

In one aspect, the present invention provides methods of treating cancer by providing to the subject in need thereof immune effector cells (e.g., T cells, NK cells) that are engineered to express a NY-BR-1CAR, wherein the cancer cells express NY-BR-1. In one embodiment, the cancer to be treated is breast cancer.

In one aspect, the present invention provides methods of treating cancer by providing to the subject in need thereof immune effector cells (e.g., T cells, NK cells) that are engineered to express a UPK2CAR, wherein the cancer cells express UPK2. In one embodiment, the cancer to be treated is bladder cancer.

In one aspect, the present invention provides methods of treating cancer by providing to the subject in need thereof immune effector cells (e.g., T cells, NK cells) that are engineered to express a HAVCR1CAR, wherein the cancer cells express HAVCR1. In one embodiment, the cancer to be treated is renal cancer.

In one aspect, the present invention provides methods of treating cancer by providing to the subject in need thereof immune effector cells (e.g., T cells, NK cells) that are engineered to express a ADRB3CAR, wherein the cancer cells express ADRB3. In one embodiment, the cancer to be treated is Ewing sarcoma.

In one aspect, the present invention provides methods of treating cancer by providing to the subject in need thereof immune effector cells (e.g., T cells, NK cells) that are engineered to express a PANX3CAR, wherein the cancer cells express PANX3. In one embodiment, the cancer to be treated is osteosarcoma.

In one aspect, the present invention provides methods of treating cancer by providing to the subject in need thereof immune effector cells (e.g., T cells, NK cells) that are engineered to express a GPR20CAR, wherein the cancer cells express GPR20. In one embodiment, the cancer to be treated is GIST.

In one aspect, the present invention provides methods of treating cancer by providing to the subject in need thereof immune effector cells (e.g., T cells, NK cells) that are engineered to express a LY6KCAR, wherein the cancer cells express LY6K. In one embodiment, the cancer to be treated is breast cancer, lung cancer, ovary caner, or cervix cancer.

In one aspect, the present invention provides methods of treating cancer by providing to the subject in need thereof immune effector cells (e.g., T cells, NK cells) that are engineered to express a OR51E2CAR, wherein the cancer cells express OR51E2. In one embodiment, the cancer to be treated is prostate cancer.

In one aspect, the present invention provides methods of treating cancer by providing to the subject in need thereof immune effector cells (e.g., T cells, NK cells) that are engineered to express a TARPCAR, wherein the cancer cells express TARP. In one embodiment, the cancer to be treated is prostate cancer.

In one aspect, the present invention provides methods of treating cancer by providing to the subject in need thereof immune effector cells (e.g., T cells, NK cells) that are engineered to express a WT1CAR, wherein the cancer cells express WT1.

In one aspect, the present invention provides methods of treating cancer by providing to the subject in need thereof immune effector cells (e.g., T cells, NK cells) that are engineered to express a NY-ESO-1CAR, wherein the cancer cells express NY-ESO-1.

In one aspect, the present invention provides methods of treating cancer by providing to the subject in need thereof immune effector cells (e.g., T cells, NK cells) that are engineered to express a LAGE-1a CAR, wherein the cancer cells express LAGE-1a.

In one aspect, the present invention provides methods of treating cancer by providing to the subject in need thereof immune effector cells (e.g., T cells, NK cells) that are engineered to express a MAGE-A1CAR, wherein the cancer cells express MAGE-A1. In one embodiment, the cancer to be treated is melanoma.

In one aspect, the present invention provides methods of treating cancer by providing to the subject in need thereof immune effector cells (e.g., T cells, NK cells) that are engineered to express a MAGE A1CAR, wherein the cancer cells express MAGE A1.

In one aspect, the present invention provides methods of treating cancer by providing to the subject in need thereof immune effector cells (e.g., T cells, NK cells) that are engineered to express a ETV6-AML CAR, wherein the cancer cells express ETV6-AML.

In one aspect, the present invention provides methods of treating cancer by providing to the subject in need thereof immune effector cells (e.g., T cells, NK cells) that are engineered to express a sperm protein 17 CAR, wherein the cancer cells express sperm protein 17. In one embodiment, the cancer to be treated is ovarian cancer, HCC, or NSCLC.

In one aspect, the present invention provides methods of treating cancer by providing to the subject in need thereof immune effector cells (e.g., T cells, NK cells) that are engineered to express a XAGE1CAR, wherein the cancer cells express XAGE1. In one embodiment, the cancer to be treated is Ewings, or rhabdo cancer.

In one aspect, the present invention provides methods of treating cancer by providing to the subject in need thereof immune effector cells (e.g., T cells, NK cells) that are engineered to express a Tie 2 CAR, wherein the cancer cells express Tie 2. In one embodiment, the cancer to be treated is a solid tumor.

In one aspect, the present invention provides methods of treating cancer by providing to the subject in need thereof immune effector cells (e.g., T cells, NK cells) that are engineered to express a MAD-CT-1CAR, wherein the cancer cells express MAD-CT-1. In one embodiment, the cancer to be treated is prostate cancer, or melanoma.

In one aspect, the present invention provides methods of treating cancer by providing to the subject in need thereof immune effector cells (e.g., T cells, NK cells) that are engineered to express a MAD-CT-2CAR, wherein the cancer cells express MAD-CT-2. In one embodiment, the cancer to be treated is prostate cancer, melanoma.

In one aspect, the present invention provides methods of treating cancer by providing to the subject in need thereof immune effector cells (e.g., T cells, NK cells) that are engineered to express a Fos-related antigen 1 CAR, wherein the cancer cells express Fos-related antigen 1. In one embodiment, the cancer to be treated is glioma, squamous cell cancer, or pancreatic cancer.

In one aspect, the present invention provides methods of treating cancer by providing to the subject in need thereof immune effector cells (e.g., T cells, NK cells) that are engineered to express a p53CAR, wherein the cancer cells express p53.

In one aspect, the present invention provides methods of treating cancer by providing to the subject in need thereof immune effector cells (e.g., T cells, NK cells) that are engineered to express a prostein CAR, wherein the cancer cells express prostein.

In one aspect, the present invention provides methods of treating cancer by providing to the subject in need thereof immune effector cells (e.g., T cells, NK cells) that are engineered to express a survivin and telomerase CAR, wherein the cancer cells express survivin and telomerase.

In one aspect, the present invention provides methods of treating cancer by providing to the subject in need thereof immune effector cells (e.g., T cells, NK cells) that are engineered to express a PCTA-1/Galectin 8 CAR, wherein the cancer cells express PCTA-1/Galectin 8.

In one aspect, the present invention provides methods of treating cancer by providing to the subject in need thereof immune effector cells (e.g., T cells, NK cells) that are engineered to express a MelanA/MART1CAR, wherein the cancer cells express MelanA/MART1.

In one aspect, the present invention provides methods of treating cancer by providing to the subject in need thereof immune effector cells (e.g., T cells, NK cells) that are engineered to express a Ras mutant CAR, wherein the cancer cells express Ras mutant.

In one aspect, the present invention provides methods of treating cancer by providing to the subject in need thereof immune effector cells (e.g., T cells, NK cells) that are engineered to express a p53 mutant CAR, wherein the cancer cells express p53 mutant.

In one aspect, the present invention provides methods of treating cancer by providing to the subject in need thereof immune effector cells (e.g., T cells, NK cells) that are engineered to express a hTERT CAR, wherein the cancer cells express hTERT.

In one aspect, the present invention provides methods of treating cancer by providing to the subject in need thereof immune effector cells (e.g., T cells, NK cells) that are engineered to express a sarcoma translocation breakpoints CAR, wherein the cancer cells express sarcoma translocation breakpoints. In one embodiment, the cancer to be treated is sarcoma.

In one aspect, the present invention provides methods of treating cancer by providing to the subject in need thereof immune effector cells (e.g., T cells, NK cells) that are engineered to express a ML-IAP CAR, wherein the cancer cells express ML-IAP. In one embodiment, the cancer to be treated is melanoma.

In one aspect, the present invention provides methods of treating cancer by providing to the subject in need thereof immune effector cells (e.g., T cells, NK cells) that are engineered to express an ERGCAR, wherein the cancer cells express ERG (TMPRSS2 ETS fusion gene).

In one aspect, the present invention provides methods of treating cancer by providing to the subject in need thereof immune effector cells (e.g., T cells, NK cells) that are engineered to express a NA17CAR, wherein the cancer cells express NA17. In one embodiment, the cancer to be treated is melanoma.

In one aspect, the present invention provides methods of treating cancer by providing to the subject in need thereof immune effector cells (e.g., T cells, NK cells) that are engineered to express a PAX3CAR, wherein the cancer cells express PAX3. In one embodiment, the cancer to be treated is alveolar rhabdomyosarcoma.

In one aspect, the present invention provides methods of treating cancer by providing to the subject in need thereof immune effector cells (e.g., T cells, NK cells) that are engineered to express an androgen receptor CAR, wherein the cancer cells express androgen receptor. In one embodiment, the cancer to be treated is metastatic prostate cancer.

In one aspect, the present invention provides methods of treating cancer by providing to the subject in need thereof immune effector cells (e.g., T cells, NK cells) that are engineered to express a Cyclin B1CAR, wherein the cancer cells express Cyclin B1.

In one aspect, the present invention provides methods of treating cancer by providing to the subject in need thereof immune effector cells (e.g., T cells, NK cells) that are engineered to express a MYCNCAR, wherein the cancer cells express MYCN.

In one aspect, the present invention provides methods of treating cancer by providing to the subject in need thereof immune effector cells (e.g., T cells, NK cells) that are engineered to express a RhoC CAR, wherein the cancer cells express RhoC.

In one aspect, the present invention provides methods of treating cancer by providing to the subject in need thereof immune effector cells (e.g., T cells, NK cells) that are engineered to express a TRP-2CAR, wherein the cancer cells express TRP-2. In one embodiment, the cancer to be treated is melanoma.

In one aspect, the present invention provides methods of treating cancer by providing to the subject in need thereof immune effector cells (e.g., T cells, NK cells) that are engineered to express a CYP1B1CAR, wherein the cancer cells express CYP1B1. In one embodiment, the cancer to be treated is breast cancer, colon cancer, lung cancer, esophagus cancer, skin cancer, lymph node cancer, brain cancer, or testis cancer.

In one aspect, the present invention provides methods of treating cancer by providing to the subject in need thereof immune effector cells (e.g., T cells, NK cells) that are engineered to express a BORIS CAR, wherein the cancer cells express BORIS. In one embodiment, the cancer to be treated is lung cancer.

In one aspect, the present invention provides methods of treating cancer by providing to the subject in need thereof immune effector cells (e.g., T cells, NK cells) that are engineered to express a SART3CAR, wherein the cancer cells express SART3

In one aspect, the present invention provides methods of treating cancer by providing to the subject in need thereof immune effector cells (e.g., T cells, NK cells) that are engineered to express a PAX5CAR, wherein the cancer cells express PAX5.

In one aspect, the present invention provides methods of treating cancer by providing to the subject in need thereof immune effector cells (e.g., T cells, NK cells) that are engineered to express a OY-TES1CAR, wherein the cancer cells express OY-TES1.

In one aspect, the present invention provides methods of treating cancer by providing to the subject in need thereof immune effector cells (e.g., T cells, NK cells) that are engineered to express a LCK CAR, wherein the cancer cells express LCK.

In one aspect, the present invention provides methods of treating cancer by providing to the subject in need thereof immune effector cells (e.g., T cells, NK cells) that are engineered to express a AKAP-4CAR, wherein the cancer cells express AKAP-4.

In one aspect, the present invention provides methods of treating cancer by providing to the subject in need thereof immune effector cells (e.g., T cells, NK cells) that are engineered to express a SSX2CAR, wherein the cancer cells express SSX2.

In one aspect, the present invention provides methods of treating cancer by providing to the subject in need thereof immune effector cells (e.g., T cells, NK cells) that are engineered to express a RAGE-1CAR, wherein the cancer cells express RAGE-1. In one embodiment, the cancer to be treated is RCC (renal cell cancer), or other solid tumors

In one aspect, the present invention provides methods of treating cancer by providing to the subject in need thereof immune effector cells (e.g., T cells, NK cells) that are engineered to express a human telomerase reverse transcriptase CAR, wherein the cancer cells express human telomerase reverse transcriptase. In one embodiment, the cancer to be treated is solid tumors.

In one aspect, the present invention provides methods of treating cancer by providing to the subject in need thereof immune effector cells (e.g., T cells, NK cells) that are engineered to express a RU1CAR, wherein the cancer cells express RU1.

In one aspect, the present invention provides methods of treating cancer by providing to the subject in need thereof immune effector cells (e.g., T cells, NK cells) that are engineered to express a RU2CAR, wherein the cancer cells express RU2.

In one aspect, the present invention provides methods of treating cancer by providing to the subject in need thereof immune effector cells (e.g., T cells, NK cells) that are engineered to express an intestinal carboxyl esterase CAR, wherein the cancer cells express intestinal carboxyl esterase. In one embodiment, the cancer to be treated is thyroid cancer, RCC, CRC (colorectal cancer), breast cancer, or other solid tumors.

In one aspect, the present invention provides methods of treating cancer by providing to the subject in need thereof immune effector cells (e.g., T cells, NK cells) that are engineered to express a Prostase CAR, wherein the cancer cells express Prostase.

In one aspect, the present invention provides methods of treating cancer by providing to the subject in need thereof immune effector cells (e.g., T cells, NK cells) that are engineered to express a PAPCAR, wherein the cancer cells express PAP.

In one aspect, the present invention provides methods of treating cancer by providing to the subject in need thereof immune effector cells (e.g., T cells, NK cells) that are engineered to express an IGF-I receptor CAR, wherein the cancer cells express IGF-I receptor.

In one aspect, the present invention provides methods of treating cancer by providing to the subject in need thereof immune effector cells (e.g., T cells, NK cells) that are engineered to express a gp100 CAR, wherein the cancer cells express gp100.

In one aspect, the present invention provides methods of treating cancer by providing to the subject in need thereof immune effector cells (e.g., T cells, NK cells) that are engineered to express a bcr-abl CAR, wherein the cancer cells express bcr-abl.

In one aspect, the present invention provides methods of treating cancer by providing to the subject in need thereof immune effector cells (e.g., T cells, NK cells) that are engineered to express a tyrosinase CAR, wherein the cancer cells express tyrosinase.

In one aspect, the present invention provides methods of treating cancer by providing to the subject in need thereof immune effector cells (e.g., T cells, NK cells) that are engineered to express a Fucosyl GM1CAR, wherein the cancer cells express Fucosyl GM1.

In one aspect, the present invention provides methods of treating cancer by providing to the subject in need thereof immune effector cells (e.g., T cells, NK cells) that are engineered to express a mut hsp70-2CAR, wherein the cancer cells express mut hsp70-2. In one embodiment, the cancer to be treated is melanoma.

In one aspect, the present invention provides methods of treating cancer by providing to the subject in need thereof immune effector cells (e.g., T cells, NK cells) that are engineered to express a CD79a CAR, wherein the cancer cells express CD79a.

In one aspect, the present invention provides methods of treating cancer by providing to the subject in need thereof immune effector cells (e.g., T cells, NK cells) that are engineered to express a CD79b CAR, wherein the cancer cells express CD79b.

In one aspect, the present invention provides methods of treating cancer by providing to the subject in need thereof immune effector cells (e.g., T cells, NK cells) that are engineered to express a CD72 CAR, wherein the cancer cells express CD72.

In one aspect, the present invention provides methods of treating cancer by providing to the subject in need thereof immune effector cells (e.g., T cells, NK cells) that are engineered to express a LAIR1 CAR, wherein the cancer cells express LAIR1.

In one aspect, the present invention provides methods of treating cancer by providing to the subject in need thereof immune effector cells (e.g., T cells, NK cells) that are engineered to express a FCAR CAR, wherein the cancer cells express FCAR.

In one aspect, the present invention provides methods of treating cancer by providing to the subject in need thereof immune effector cells (e.g., T cells, NK cells) that are engineered to express a LILRA2 CAR, wherein the cancer cells express LILRA2.

In one aspect, the present invention provides methods of treating cancer by providing to the subject in need thereof immune effector cells (e.g., T cells, NK cells) that are engineered to express a CD300LF CAR, wherein the cancer cells express CD300LF.

In one aspect, the present invention provides methods of treating cancer by providing to the subject in need thereof immune effector cells (e.g., T cells, NK cells) that are engineered to express a CLEC12A CAR, wherein the cancer cells express CLEC12A.

In one aspect, the present invention provides methods of treating cancer by providing to the subject in need thereof immune effector cells (e.g., T cells, NK cells) that are engineered to express a BST2 CAR, wherein the cancer cells express BST2.

In one aspect, the present invention provides methods of treating cancer by providing to the subject in need thereof immune effector cells (e.g., T cells, NK cells) that are engineered to express an EMR2 CAR, wherein the cancer cells express EMR2.

In one aspect, the present invention provides methods of treating cancer by providing to the subject in need thereof immune effector cells (e.g., T cells, NK cells) that are engineered to express a LY75 CAR, wherein the cancer cells express LY75.

In one aspect, the present invention provides methods of treating cancer by providing to the subject in need thereof immune effector cells (e.g., T cells, NK cells) that are engineered to express a GPC3 CAR, wherein the cancer cells express GPC3.

In one aspect, the present invention provides methods of treating cancer by providing to the subject in need thereof immune effector cells (e.g., T cells, NK cells) that are engineered to express a FCRL5 CAR, wherein the cancer cells express FCRL5.

In one aspect, the present invention provides methods of treating cancer by providing to the subject in need thereof immune effector cells (e.g., T cells, NK cells) that are engineered to express an IGLL1 CAR, wherein the cancer cells express IGLL1.

In one aspect, the present invention relates to treatment of a subject in vivo using an PD1 CAR such that growth of cancerous tumors is inhibited. A PD1 CAR may be used alone to inhibit the growth of cancerous tumors. Alternatively, PD1 CAR may be used in conjunction with other CARs, immunogenic agents, standard cancer treatments, or other antibodies. In one embodiment, the subject is treated with a PD1 CAR and an XCAR described herein. In an embodiment, a PD1 CAR is used in conjunction with another CAR, e.g., a CAR described herein, and a kinase inhibitor, e.g., a kinase inhibitor described herein.

In another aspect, a method of treating a subject, e.g., reducing or ameliorating, a hyperproliferative condition or disorder (e.g., a cancer), e.g., solid tumor, a soft tissue tumor, or a metastatic lesion, in a subject is provided. As used herein, the term "cancer" is meant to include all types of cancerous growths or oncogenic processes, metastatic tissues or malignantly transformed cells, tissues, or organs, irrespective of histopathologic type or stage of invasiveness. Examples of solid tumors include malignancies, e.g., sarcomas, adenocarcinomas, and carcinomas, of the various organ systems, such as those affecting liver, lung, breast, lymphoid, gastrointestinal (e.g., colon), genitourinary tract (e.g., renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, e.g., an advanced stage melanoma. Metastatic lesions of the aforementioned cancers can also be treated or prevented using the methods and compositions of the invention. Examples of other cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. Treatment of metastatic cancers, e.g., metastatic cancers that express PD-L1 (Iwai et al. (2005) Int. Immunol. 17:133-144) can be effected using the antibody molecules described herein.

Exemplary cancers whose growth can be inhibited include cancers typically responsive to immunotherapy. Non-limiting examples of cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer). Additionally, refractory or recurrent malignancies can be treated using the molecules described herein.

In one aspect, the invention pertains to a vector comprising a CAR operably linked to promoter for expression in mammalian immune effector cells (e.g., T cells, NK cells). In one aspect, the invention provides a recombinant immune effector cell expressing a CAR of the present invention for use in treating cancer expressing a cancer associate antigen as described herein. In one aspect, CAR-expressing cells of the invention is capable of contacting a tumor cell with at least one cancer associated antigen expressed on its surface such that the CAR-expressing cell targets the cancer cell and growth of the cancer is inhibited.

In one aspect, the invention pertains to a method of inhibiting growth of a cancer, comprising contacting the cancer cell with a CAR-expressing cell of the present invention such that the CART is activated in response to the antigen and targets the cancer cell, wherein the growth of the tumor is inhibited.

In one aspect, the invention pertains to a method of treating cancer in a subject. The method comprises administering to the subject CAR-expressing cell of the present invention such that the cancer is treated in the subject. In one aspect, the cancer associated with expression of a cancer associate antigen as described herein is a hematological cancer. In one aspect, the hematological cancer is a leukemia or a lymphoma. In one aspect, a cancer associated with expression of a cancer associate antigen as described herein includes cancers and malignancies including, but not limited to, e.g., one or more acute leukemias including but not limited to, e.g., B-cell acute Lymphoid Leukemia ("BALL"), T-cell acute Lymphoid Leukemia ("TALL"), acute lymphoid leukemia (ALL); one or more chronic leukemias including but not limited to, e.g., chronic myelogenous leukemia (CML), Chronic Lymphoid Leukemia (CLL). Additional cancers or hematologic conditions associated with expression of a cancer associate antigen as described herein include, but are not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further a disease associated with a cancer associate antigen as described herein expression include, but not limited to, e.g., atypical and/or non-classical cancers, malignancies, precancerous conditions or proliferative diseases associated with expression of a cancer associate antigen as described herein.

In some embodiments, a cancer that can be treated with CAR-expressing cell of the present invention is multiple myeloma. Multiple myeloma is a cancer of the blood, characterized by accumulation of a plasma cell clone in the bone marrow. Current therapies for multiple myeloma include, but are not limited to, treatment with lenalidomide, which is an analog of thalidomide. Lenalidomide has activities which include anti-tumor activity, angiogenesis inhibition, and immunomodulation. Generally, myeloma cells are thought to be negative for a cancer associate antigen as described herein expression by flow cytometry. Thus, in some embodiments, a CD19 CAR, e.g., as described herein, may be used to target myeloma cells. In some embodiments, cars of the present invention therapy can be used in combination with one or more additional therapies, e.g., lenalidomide treatment.

The invention includes a type of cellular therapy where immune effector cells (e.g., T cells, NK cells) are genetically modified to express a chimeric antigen receptor (CAR) and the CAR-expressing T cell or NK cell is infused to a recipient in need thereof. The infused cell is able to kill tumor cells in the recipient. Unlike antibody therapies, CAR-modified immune effector cells (e.g., T cells, NK cells) are able to replicate in vivo resulting in long-term persistence that can lead to sustained tumor control. In various aspects, the immune effector cells (e.g., T cells, NK cells) administered to the patient, or their progeny, persist in the patient for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years after administration of the T cell or NK cell to the patient.

The invention also includes a type of cellular therapy where immune effector cells (e.g., T cells, NK cells) are modified, e.g., by in vitro transcribed RNA, to transiently express a chimeric antigen receptor (CAR) and the CAR T cell or NK cell is infused to a recipient in need thereof. The infused cell is able to kill tumor cells in the recipient. Thus, in various aspects, the immune effector cells (e.g., T cells, NK cells) administered to the patient, is present for less than one month, e.g., three weeks, two weeks, one week, after administration of the T cell or NK cell to the patient.

Without wishing to be bound by any particular theory, the anti-tumor immunity response elicited by the CAR-modified immune effector cells (e.g., T cells, NK cells) may be an active or a passive immune response, or alternatively may be due to a direct vs indirect immune response. In one aspect, the CAR transduced immune effector cells (e.g., T cells, NK cells) exhibit specific proinflammatory cytokine secretion and potent cytolytic activity in response to human cancer cells expressing the a cancer associate antigen as described herein, resist soluble a cancer associate antigen as described herein inhibition, mediate bystander killing and mediate regression of an established human tumor. For example, antigen-less tumor cells within a heterogeneous field of a cancer associate antigen as described herein-expressing tumor may be susceptible to indirect destruction by a cancer associate antigen as described herein-redirected immune effector cells (e.g., T cells, NK cells) that has previously reacted against adjacent antigen-positive cancer cells.

In one aspect, the fully-human CAR-modified immune effector cells (e.g., T cells, NK cells) of the invention may be a type of vaccine for ex vivo immunization and/or in vivo therapy in a mammal. In one aspect, the mammal is a human.

With respect to ex vivo immunization, at least one of the following occurs in vitro prior to administering the cell into a mammal: i) expansion of the cells, ii) introducing a nucleic acid encoding a CAR to the cells or iii) cryopreservation of the cells.

Ex vivo procedures are well known in the art and are discussed more fully below. Briefly, cells are isolated from a mammal (e.g., a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

The procedure for ex vivo expansion of hematopoietic stem and progenitor cells is described in U.S. Pat. No. 5,199,942, incorporated herein by reference, can be applied to the cells of the present invention. Other suitable methods are known in the art, therefore the present invention is not limited to any particular method of ex vivo expansion of the cells. Briefly, ex vivo culture and expansion of immune effector cells (e.g., T cells, NK cells) comprises: (1) collecting CD34+ hematopoietic stem and progenitor cells from a mammal from peripheral blood harvest or bone marrow explants; and (2) expanding such cells ex vivo. In addition to the cellular growth factors described in U.S. Pat. No. 5,199,942, other factors such as flt3-L, IL-1, IL-3 and c-kit ligand, can be used for culturing and expansion of the cells.

In addition to using a cell-based vaccine in terms of ex vivo immunization, the present invention also provides compositions and methods for in vivo immunization to elicit an immune response directed against an antigen in a patient.

Generally, the cells activated and expanded as described herein may be utilized in the treatment and prevention of diseases that arise in individuals who are immunocompromised. In particular, the CAR-modified immune effector cells (e.g., T cells, NK cells) of the invention are used in the treatment of diseases, disorders and conditions associated with expression of a cancer associate antigen as described herein. In certain aspects, the cells of the invention are used in the treatment of patients at risk for developing diseases, disorders and conditions associated with expression of a cancer associate antigen as described herein. Thus, the present invention provides methods for the treatment or prevention of diseases, disorders and conditions associated with expression of a cancer associate antigen as described herein comprising administering to a subject in need thereof, a therapeutically effective amount of the CAR-modified immune effector cells (e.g., T cells, NK cells) of the invention.

In one aspect the CAR-expressing cells of the inventions may be used to treat a proliferative disease such as a cancer or malignancy or is a precancerous condition such as a myelodysplasia, a myelodysplastic syndrome or a preleukemia. Further a disease associated with a cancer associate antigen as described herein expression include, but not limited to, e.g., atypical and/or non-classical cancers, malignancies, precancerous conditions or proliferative diseases expressing a cancer associated antigen as described herein. Non-cancer related indications associated with expression of a cancer associate antigen as described herein include, but are not limited to, e.g., autoimmune disease, (e.g., lupus), inflammatory disorders (allergy and asthma) and transplantation.

The CAR-modified immune effector cells (e.g., T cells, NK cells) of the present invention may be administered either alone, or as a pharmaceutical composition in combination with diluents and/or with other components such as IL-2 or other cytokines or cell populations.

### Hematologic Cancer

Hematological cancer conditions are the types of cancer such as leukemia, lymphoma, and malignant lymphoproliferative conditions that affect blood, bone marrow and the lymphatic system.

Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

Lymphoma is a group of blood cell tumors that develop from lymphocytes. Exemplary lymphomas include non-Hodgkin lymphoma and Hodgkin lymphoma.

The present invention provides for compositions and methods for treating cancer. In one aspect, the cancer is a hematologic cancer including but is not limited to hematolical cancer is a leukemia or a lymphoma. In one aspect, the CAR-expressing cells of the invention may be used to treat cancers and malignancies such as, but not limited to, e.g., acute leukemias including but not limited to, e.g., B-cell acute lymphoid leukemia ("BALL"), T-cell acute lymphoid leukemia ("TALL"), acute lymphoid leukemia (ALL); one or more chronic leukemias including but not limited to, e.g., chronic myelogenous leukemia (CML), chronic lymphocytic leukemia (CLL); additional hematologic cancers or hematologic conditions including, but not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further a disease associated with a cancer associate antigen as described herein expression includes, but not limited to, e.g., atypical and/or non-classical cancers, malignancies, precancerous conditions or proliferative diseases expressing a cancer associate antigen as described herein.

The present invention also provides methods for inhibiting the proliferation or reducing a cancer associated antigen as described herein-expressing cell population, the methods comprising contacting a population of cells comprising a cancer associated antigen as described herein-expressing cell with a CAR-expressing T cell or NK cell of the invention that binds to the a cancer associate antigen as described herein-expressing cell. In a specific aspect, the present invention provides methods for inhibiting the proliferation or reducing the population of cancer cells expressing a cancer associated antigen as described herein, the methods comprising contacting a cancer associate antigen as described herein-expressing cancer cell population with a CAR-expressing T cell or NK cell of the invention that binds to a cancer associated antigen as described herein-expressing cell. In one aspect, the present invention provides methods for inhibiting the proliferation or reducing the population of cancer cells expressing a cancer associated antigen as described herein, the methods comprising contacting a cancer associated antigen as described herein-expressing cancer cell population with a CAR-expressing T cell or NK cell of the invention that binds to a cancer associated antigen as described herein-expressing cell. In certain aspects, a CAR-expressing T cell or NK cell of the invention reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for myeloid leukemia or another cancer associated with a cancer associated antigen as described herein-expressing cells relative to a negative control. In one aspect, the subject is a human.

The present invention also provides methods for preventing, treating and/or managing a disease associated with a cancer associated antigen as described herein-expressing cells (e.g., a hematologic cancer or atypical cancer expessing a cancer associated antigen as described herein), the methods comprising administering to a subject in need a CAR T cell or NK cell of the invention that binds to a cancer associated antigen as described herein-expressing cell. In one aspect, the subject is a human. Non-limiting examples of disorders associated with a cancer associated antigen as described herein-expressing cells include autoimmune disorders (such as lupus), inflammatory disorders (such as allergies and asthma) and cancers (such as hematological cancers or atypical cancers expessing a cancer associated antigen as described herein).

The present invention also provides methods for preventing, treating and/or managing a disease associated with a cancer associated antigen as described herein-expressing cells, the methods comprising administering to a subject in need a CAR T cell or NK cell of the invention that binds to a cancer associated antigen as described herein-expressing cell. In one aspect, the subject is a human.

The present invention provides methods for preventing relapse of cancer associated with a cancer associated antigen as described herein-expressing cells, the methods comprising administering to a subject in need thereof aCAR T cell or NK cell of the invention that binds to a cancer associated antigen as described herein-expressing cell. In one aspect, the methods comprise administering to the subject in need thereof an effective amount of a CAR-expressingT cell or NK cell described herein that binds to a cancer associated antigen as described herein-expressing cell in combination with an effective amount of another therapy.

### Combination Therapies

A CAR-expressing cell described herein may be used in combination with other known agents and therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

A CAR-expressing cell described herein and the at least one additional therapeutic agent can be administered simultaneously, in the same or in separate compositions, or sequentially. For sequential administration, the CAR-expressing cell described herein can be administered first, and the additional agent can be administered second, or the order of administration can be reversed.

The CAR therapy and/or other therapeutic agents, procedures or modalities can be administered during periods of active disorder, or during a period of remission or less active disease. The CAR therapy can be administered before the other treatment, concurrently with the treatment, post-treatment, or during remission of the disorder.

When administered in combination, the CAR therapy and the additional agent (e.g., second or third agent), or all, can be administered in an amount or dose that is higher, lower or the same than the amount or dosage of each agent used individually, e.g., as a monotherapy. In certain embodiments, the administered amount or dosage of the CAR therapy, the additional agent (e.g., second or third agent), or all, is lower (e.g., at least 20%, at least 30%, at least 40%, or at least 50%) than the amount or dosage of each agent used individually, e.g., as a monotherapy. In other embodiments, the amount or dosage of the CAR therapy, the additional agent (e.g., second or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

In further aspects, a CAR-expressing cell described herein may be used in a treatment regimen in combination with surgery, chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation. peptide vaccine, such as that described in Izumoto et al. 2008 J Neurosurg 108:963-971.

In one embodiment, a CAR-expressing cell described herein can be used in combination with a chemotherapeutic agent. Exemplary chemotherapeutic agents include an anthracycline (e.g., doxorubicin (e.g., liposomal doxorubicin)). a vinca alkaloid (e.g., vinblastine, vincristine, vindesine, vinorelbine), an alkylating agent (e.g., cyclophosphamide, decarbazine, melphalan, ifosfamide, temozolomide), an immune cell antibody (e.g., alemtuzamab, gemtuzumab, rituximab, ofatumumab, tositumomab, brentuximab), an antimetabolite (including, e.g., folic acid antagonists, pyrimidine analogs, purine analogs and adenosine deaminase inhibitors (e.g., fludarabine)), an mTOR inhibitor, a TNFR glucocorticoid induced TNFR related protein (GITR) agonist, a proteasome inhibitor (e.g., aclacinomycin A, gliotoxin or bortezomib), an immunomodulator such as thalidomide or a thalidomide derivative (e.g., lenalidomide).

General Chemotherapeutic agents considered for use in combination therapies include anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin@), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}).

Exemplary alkylating agents include, without limitation, nitrogen mustards, ethylenimine derivatives, alkyl sulfonates, nitrosoureas and triazenes): uracil mustard (Aminouracil Mustard^{®}, Chlorethaminacil^{®}, Demethyldopan^{®}, Desmethyldopan^{®}, Haemanthamine^{®}, Nordopan^{®}, Uracil nitrogen mustard^{®}, Uracillost^{®}, Uracilmostaza^{®}, Uramustin^{®}, Uramustine^{®}), chlormethine (Mustargen^{®}), cyclophosphamide (Cytoxan^{®}, Neosar^{®}, Clafen^{®}, Endoxan^{®}, Procytox^{®}, Revimmune^{™}), ifosfamide (Mitoxana@), melphalan (Alkeran^{®}), Chlorambucil (Leukeran^{®}), pipobroman (Amedel^{®}, Vercyte^{®}), triethylenemelamine (Hemel^{®}, Hexalen^{®}, Hexastat^{®}), triethylenethiophosphoramine, Temozolomide (Temodar^{®}), thiotepa (Thioplex^{®}), busulfan (Busilvex^{®}, Myleran^{®}), carmustine (BiCNU^{®}), lomustine (CeeNU^{®}), streptozocin (Zanosar^{®}), and Dacarbazine (DTIC-Dome^{®}). Additional exemplary alkylating agents include, without limitation, Oxaliplatin (Eloxatin^{®}); Temozolomide (Temodar^{®} and Temodal^{®}); Dactinomycin (also known as actinomycin-D, Cosmegen^{®}); Melphalan (also known as L-PAM, L-sarcolysin, and phenylalanine mustard, Alkeran^{®}); Altretamine (also known as hexamethylmelamine (HMM), Hexalen^{®}); Carmustine (BiCNU^{®}); Bendamustine (Treanda^{®}); Busulfan (Busulfex^{®} and Myleran^{®}); Carboplatin (Paraplatin^{®}); Lomustine (also known as CCNU, CeeNU^{®}); Cisplatin (also known as CDDP, Platinol^{®} and Platinol^{®}-AQ); Chlorambucil (Leukeran^{®}); Cyclophosphamide (Cytoxan^{®} and Neosar^{®}); Dacarbazine (also known as DTIC, DIC and imidazole carboxamide, DTIC-Dome^{®}); Altretamine (also known as hexamethylmelamine (HMM), Hexalen^{®}); Ifosfamide (Ifex^{®}); Prednumustine; Procarbazine (Matulane^{®}); Mechlorethamine (also known as nitrogen mustard, mustine and mechloroethamine hydrochloride, Mustargen^{®}); Streptozocin (Zanosar^{®}); Thiotepa (also known as thiophosphoamide, TESPA and TSPA, Thioplex^{®}); Cyclophosphamide (Endoxan^{®}, Cytoxan^{®}, Neosar^{®}, Procytox^{®}, Revimmune^{®}); and Bendamustine HCl (Treanda^{®}).

In embodiments, a CAR-expressing cell described herein is administered to a subject in combination with fludarabine, cyclophosphamide, and/or rituximab. In embodiments, a CAR-expressing cell described herein is administered to a subject in combination with fludarabine, cyclophosphamide, and rituximab (FCR). In embodiments, the subject has CLL. For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region (*I_{G}V_{H}*) gene. In other embodiments, the subject does not comprise a leukemic cell comprising a mutation in the immunoglobulin heavy-chain variable-region (*I_{G}V_{H}*) gene. In embodiments, the fludarabine is administered at a dosage of about 10-50 mg/m² (e.g., about 10-15, 15-20, 20-25, 25-30, 30-35, 35-40, 40-45, or 45-50 mg/m²), e.g., intravenously. In embodiments, the cyclophosphamide is administered at a dosage of about 200-300 mg/m² (e.g., about 200-225, 225-250, 250-275, or 275-300 mg/m²), e.g., intravenously. In embodiments, the rituximab is administered at a dosage of about 400-600 mg/m2 (e.g., 400-450, 450-500, 500-550, or 550-600 mg/m²), e.g., intravenously.

In embodiments, a CAR-expressing cell described herein is administered to a subject in combination with bendamustine and rituximab. In embodiments, the subject has CLL. For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region (*I_{G}V_{H}*) gene. In other embodiments, the subject does not comprise a leukemic cell comprising a mutation in the immunoglobulin heavy-chain variable-region (*I_{G}V_{H}*) gene. In embodiments, the bendamustine is administered at a dosage of about 70-110 mg/m2 (e.g., 70-80, 80-90, 90-100, or 100-110 mg/m2), e.g., intravenously. In embodiments, the rituximab is administered at a dosage of about 400-600 mg/m2 (e.g., 400-450, 450-500, 500-550, or 550-600 mg/m²), e.g., intravenously.

In embodiments, a CAR-expressing cell described herein is administered to a subject in combination with rituximab, cyclophosphamide, doxorubicine, vincristine, and/or a corticosteroid (e.g., prednisone). In embodiments, a CAR-expressing cell described herein is administered to a subject in combination with rituximab, cyclophosphamide, doxorubicine, vincristine, and prednisone (R-CHOP). In embodiments, the subject has diffuse large B-cell lymphoma (DLBCL). In embodiments, the subject has nonbulky limited-stage DLBCL (e.g., comprises a tumor having a size/diameter of less than 7 cm). In embodiments, the subject is treated with radiation in combination with the R-CHOP. For example, the subject is administered R-CHOP (e.g., 1-6 cycles, e.g., 1, 2, 3, 4, 5, or 6 cycles of R-CHOP), followed by radiation. In some cases, the subject is administered R-CHOP (e.g., 1-6 cycles, e.g., 1, 2, 3, 4, 5, or 6 cycles of R-CHOP) following radiation.

In embodiments, a CAR-expressing cell described herein is administered to a subject in combination with etoposide, prednisone, vincristine, cyclophosphamide, doxorubicin, and/or rituximab. In embodiments, a CAR-expressing cell described herein is administered to a subject in combination with etoposide, prednisone, vincristine, cyclophosphamide, doxorubicin, and rituximab (EPOCH-R). In embodiments, a CAR-expressing cell described herein is administered to a subject in combination with dose-adjusted EPOCH-R (DA-EPOCH-R). In embodiments, the subject has a B cell lymphoma, e.g., a Myc-rearranged aggressive B cell lymphoma.

In embodiments, a CAR-expressing cell described herein is administered to a subject in combination with rituximab and/or lenalidomide. Lenalidomide ((*RS*)-3-(4-Amino-1-oxo 1,3-dihydro-2*H*-isoindol- 2-yl)piperidine-2,6-dione) is an immunomodulator. In embodiments, a CAR-expressing cell described herein is administered to a subject in combination with rituximab and lenalidomide. In embodiments, the subject has follicular lymphoma (FL) or mantle cell lymphoma (MCL). In embodiments, the subject has FL and has not previously been treated with a cancer therapy. In embodiments, lenalidomide is administered at a dosage of about 10-20 mg (e.g., 10-15 or 15-20 mg), e.g., daily. In embodiments, rituximab is administered at a dosage of about 350-550 mg/m² (e.g., 350-375, 375-400, 400-425, 425-450, 450-475, or 475-500 mg/m²), e.g., intravenously.

Exemplary mTOR inhibitors include, e.g., temsirolimus; ridaforolimus (formally known as deferolimus, (1*R*,2*R*,4*S*)-4-[(2*R*)-2[(1*R*,9*S*,12*S,*15*R*,16*E,*18*R*,19*R*,21*R,* 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); everolimus (Afinitor^{®} or RAD001); rapamycin (AY22989, Sirolimus@); simapimod (CAS 164301-51-3); emsirolimus, (5-{2,4-Bis[(3S)-3-methylmorpholin-4-yl]pyrido[2,3-*d*]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502, CAS 1013101-36-4); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126, CAS 936487-67-1) (SEQ ID NO: 1262), and XL765.

Exemplary immunomodulators include, e.g., afutuzumab (available from Roche^{®}); pegfilgrastim (Neulasta^{®}); lenalidomide (CC-5013, Revlimid@); thalidomide (Thalomid^{®}), actimid (CC4047); and IRX-2 (mixture of human cytokines including interleukin 1, interleukin 2, and interferon γ, CAS 951209-71-5, available from IRX Therapeutics).

Exemplary anthracyclines include, e.g., doxorubicin (Adriamycin^{®} and Rubex^{®}); bleomycin (lenoxane^{®}); daunorubicin (dauorubicin hydrochloride, daunomycin, and rubidomycin hydrochloride, Cerubidine^{®}); daunorubicin liposomal (daunorubicin citrate liposome, DaunoXome^{®}); mitoxantrone (DHAD, Novantrone^{®}); epirubicin (Ellence^{™}); idarubicin (Idamycin^{®}, Idamycin PFS^{®}); mitomycin C (Mutamycin^{®}); geldanamycin; herbimycin; ravidomycin; and desacetylravidomycin.

Exemplary vinca alkaloids include, e.g., vinorelbine tartrate (Navelbine^{®}), Vincristine (Oncovin^{®}), and Vindesine (Eldisine^{®})); vinblastine (also known as vinblastine sulfate, vincaleukoblastine and VLB, Alkaban-AQ^{®} and Velban^{®}); and vinorelbine (Navelbine^{®}).

Exemplary proteosome inhibitors include bortezomib (Velcade^{®}); carfilzomib (PX-171-007, (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)-pentanamide); marizomib (NPI-0052); ixazomib citrate (MLN-9708); delanzomib (CEP-18770); and *O*-Methyl-*N*-[(2-methyl-5-thiazolyl)carbonyl]-L-seryl-*O*-methyl-N-[(1*S*)-2-[(2*R*)-2-methyl-2-oxiranyl]-2-oxo-1-(phenylmethyl)ethyl]- L-serinamide (ONX-0912).

In embodiments, a CAR-expressing cell described herein is administered to a subject in combination with brentuximab. Brentuximab is an antibody-drug conjugate of anti-CD30 antibody and monomethyl auristatin E. In embodiments, the subject has Hodgkin's lymphoma (HL), e.g., relapsed or refractory HL. In embodiments, the subject comprises CD30+ HL. In embodiments, the subject has undergone an autologous stem cell transplant (ASCT). In embodiments, the subject has not undergone an ASCT. In embodiments, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In embodiments, a CAR-expressing cell described herein is administered to a subject in combination with brentuximab and dacarbazine or in combination with brentuximab and bendamustine. Dacarbazine is an alkylating agent with a chemical name of 5-(3,3-Dimethyl-1-triazenyl)imidazole-4-carboxamide. Bendamustine is an alkylating agent with a chemical name of 4-[5-[Bis(2-chloroethyl)amino]-1-methylbenzimidazol-2-yl]butanoic acid. In embodiments, the subject has Hodgkin's lymphoma (HL). In embodiments, the subject has not previously been treated with a cancer therapy. In embodiments, the subject is at least 60 years of age, e.g., 60, 65, 70, 75, 80, 85, or older. In embodiments, dacarbazine is administered at a dosage of about 300-450 mg/m² (e.g., about 300-325, 325-350, 350-375, 375-400, 400-425, or 425-450 mg/m²), e.g., intravenously. In embodiments, bendamustine is administered at a dosage of about 75-125 mg/m2 (e.g., 75-100 or 100-125 mg/m², e.g., about 90 mg/m²), e.g., intravenously. In embodiments, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In some embodiments, a CAR-expressing cell described herein is administered to a subject in combination with a CD20 inhibitor, e.g., an anti-CD20 antibody (e.g., an anti-CD20 mono- or bispecific antibody) or a fragment thereof. Exemplary anti-CD20 antibodies include but are not limited to rituximab, ofatumumab, ocrelizumab, veltuzumab, obinutuzumab, TRU-015 (Trubion Pharmaceuticals), ocaratuzumab, and Pro131921 (Genentech). See, e.g., Lim et al. Haematologica. 95.1(2010):135-43.

In some embodiments, the anti-CD20 antibody comprises rituximab. Rituximab is a chimeric mouse/human monoclonal antibody IgG1 kappa that binds to CD20 and causes cytolysis of a CD20 expressing cell, e.g., as described in www.accessdata.fda.gov/drugsatfda_docs/label/2010/103705s5311lbl.pdf. In embodiments, a CAR-expressing cell described herein is administered to a subject in combination with rituximab. In embodiments, the subject has CLL or SLL.

In some embodiments, rituximab is administered intravenously, e.g., as an intravenous infusion. For example, each infusion provides about 500-2000 mg (e.g., about 500-550, 550-600, 600-650, 650-700, 700-750, 750-800, 800-850, 850-900, 900-950, 950-1000, 1000-1100, 1100-1200, 1200-1300, 1300-1400, 1400-1500, 1500-1600, 1600-1700, 1700-1800, 1800-1900, or 1900-2000 mg) of rituximab. In some embodiments, rituximab is administered at a dose of 150 mg/m² to 750 mg/m², e.g., about 150-175 mg/m², 175-200 mg/m², 200-225 mg/m², 225-250 mg/m², 250-300 mg/m², 300-325 mg/m², 325-350 mg/m², 350-375 mg/m², 375-400 mg/m², 400-425 mg/m², 425-450 mg/m², 450-475 mg/m², 475-500 mg/m², 500-525 mg/m², 525-550 mg/m², 550-575 mg/m², 575-600 mg/m², 600-625 mg/m², 625-650 mg/m², 650-675 mg/m², or 675-700 mg/m², where m² indicates the body surface area of the subject. In some embodiments, rituximab is administered at a dosing interval of at least 4 days, e.g., 4, 7, 14, 21, 28, 35 days, or more. For example, rituximab is administered at a dosing interval of at least 0.5 weeks, e.g., 0.5, 1, 2, 3, 4, 5, 6, 7, 8 weeks, or more. In some embodiments, rituximab is administered at a dose and dosing interval described herein for a period of time, e.g., at least 2 weeks, e.g., at least 2, 3, 4, 5, 6, 7, 8, 9, 10, 11, 12, 13, 14, 15, 16, 17, 18, 19, 20 weeks, or greater. For example, rituximab is administered at a dose and dosing interval described herein for a total of at least 4 doses per treatment cycle (e.g., at least 4, 5, 6, 7, 8, 9, 10, 11, 12, 13, 14, 15, 16, or more doses per treatment cycle).

In some embodiments, the anti-CD20 antibody comprises ofatumumab. Ofatumumab is an anti-CD20 IgG1κ human monoclonal antibody with a molecular weight of approximately 149 kDa. For example, ofatumumab is generated using transgenic mouse and hybridoma technology and is expressed and purified from a recombinant murine cell line (NS0). See, e.g., www.accessdata.fda.gov/drugsatfda_docs/label/2009/125326lbl.pdf; and Clinical Trial Identifier number NCT01363128, NCT01515176, NCT01626352, and NCT01397591. In embodiments, a CAR-expressing cell described herein is administered to a subject in combination with ofatumumab. In embodiments, the subject has CLL or SLL.

In some embodiments, ofatumumab is administered as an intravenous infusion. For example, each infusion provides about 150-3000 mg (e.g., about 150-200, 200-250, 250-300, 300-350, 350-400, 400-450, 450-500, 500-550, 550-600, 600-650, 650-700, 700-750, 750-800, 800-850, 850-900, 900-950, 950-1000, 1000-1200, 1200-1400, 1400-1600, 1600-1800, 1800-2000, 2000-2200, 2200-2400, 2400-2600, 2600-2800, or 2800-3000 mg) of ofatumumab. In embodiments, ofatumumab is administered at a starting dosage of about 300 mg, followed by 2000 mg, e.g., for about 11 doses, e.g., for 24 weeks. In some embodiments, ofatumumab is administered at a dosing interval of at least 4 days, e.g., 4, 7, 14, 21, 28, 35 days, or more. For example, ofatumumab is administered at a dosing interval of at least 1 week, e.g., 1, 2, 3, 4, 5, 6, 7, 8, 9, 10, 11, 12, 24, 26, 28, 20, 22, 24, 26, 28, 30 weeks, or more. In some embodiments, ofatumumab is administered at a dose and dosing interval described herein for a period of time, e.g., at least 1 week, e.g., 1, 2, 3, 4, 5, 6, 7, 8, 9, 10, 11, 12, 13, 14, 15, 16, 17, 18, 19, 20, 22, 24, 26, 28, 30, 40, 50, 60 weeks or greater, or 1, 2, 3, 4, 5, 6, 7, 8, 9, 10, 11, 12 months or greater, or 1, 2, 3, 4, 5 years or greater. For example, ofatumumab is administered at a dose and dosing interval described herein for a total of at least 2 doses per treatment cycle (e.g., at least 2, 3, 4, 5, 6, 7, 8, 9, 10, 11, 12, 13, 14, 15, 16, 18, 20, or more doses per treatment cycle).

In some cases, the anti-CD20 antibody comprises ocrelizumab. Ocrelizumab is a humanized anti-CD20 monoclonal antibody, e.g., as described in Clinical Trials Identifier Nos. NCT00077870, NCT01412333, NCT00779220, NCT00673920, NCT01194570, and Kappos et al. Lancet. 19.378(2011):1779-87.

In some cases, the anti-CD20 antibody comprises veltuzumab. Veltuzumab is a humanized monoclonal antibody against CD20. See, e.g., Clinical Trial Identifier No. NCT00547066, NCT00546793, NCT01101581, and Goldenberg et al. Leuk Lymphoma. 51(5)(2010):747-55.

In some cases, the anti-CD20 antibody comprises GA101. GA101 (also called obinutuzumab or RO5072759) is a humanized and glyco-engineered anti-CD20 monoclonal antibody. See, e.g., Robak. Curr. Opin. Investig. Drugs. 10.6(2009):588-96; Clinical Trial Identifier Numbers: NCT01995669, NCT01889797, NCT02229422, and NCT01414205; and www.accessdata.fda.gov/drugsatfda_docs/label/2013/125486s000lbl.pdf.

In some cases, the anti-CD20 antibody comprises AME-133v. AME-133v (also called LY2469298 or ocaratuzumab) is a humanized IgG1 monoclonal antibody against CD20 with increased affinity for the FcγRIIIa receptor and an enhanced antibody dependent cellular cytotoxicity (ADCC) activity compared with rituximab. See, e.g., Robak et al. BioDrugs 25.1(2011):13-25; and Forero-Torres et al. Clin Cancer Res. 18.5(2012):1395-403.

In some cases, the anti-CD20 antibody comprises PRO131921. PRO131921 is a humanized anti-CD20 monoclonal antibody engineered to have better binding to FcγRIIIa and enhanced ADCC compared with rituximab. See, e.g., Robak et al. BioDrugs 25.1(2011):13-25; and Casulo et al. Clin Immunol. 154.1(2014):37-46; and Clinical Trial Identifier No. NCT00452127.

In some cases, the anti-CD20 antibody comprises TRU-015. TRU-015 is an anti-CD20 fusion protein derived from domains of an antibody against CD20. TRU-015 is smaller than monoclonal antibodies, but retains Fc-mediated effector functions. See, e.g., Robak et al. BioDrugs 25.1(2011):13-25. TRU-015 contains an anti-CD20 single-chain variable fragment (scFv) linked to human IgG1 hinge, CH2, and CH3 domains but lacks CH1 and CL domains.

In some embodiments, an anti-CD20 antibody described herein is conjugated or otherwise bound to a therapeutic agent, e.g., a chemotherapeutic agent (e.g., cytoxan, fludarabine, histone deacetylase inhibitor, demethylating agent, peptide vaccine, anti-tumor antibiotic, tyrosine kinase inhibitor, alkylating agent, anti-microtubule or anti-mitotic agent), anti-allergic agent, anti-nausea agent (or anti-emetic), pain reliever, or cytoprotective agent described herein.

In embodiments, a CAR-expressing cell described herein is administered to a subject in combination with a B-cell lymphoma 2 (BCL-2) inhibitor (e.g., venetoclax, also called ABT-199 or GDC-0199;) and/or rituximab. In embodiments, a CAR-expressing cell described herein is administered to a subject in combination with venetoclax and rituximab. Venetoclax is a small molecule that inhibits the anti-apoptotic protein, BCL-2. The structure of venetoclax (4-(4-{[2-(4-chlorophenyl)-4,4-dimethylcyclohex-1-en-1-yl]methyl]piperazin-1-yl)-*N*-([3-nitro-4-[(tetrahydro-2*H*-pyran-4-ylmethyl)amino]phenyl}sulfonyl)-2-(1*H*-pyrrolo[2,3-*b*]pyridin-5-yloxy)benzamide) is shown below.

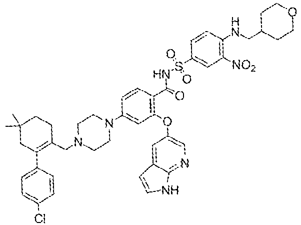

In embodiments, the subject has CLL. In embodiments, the subject has relapsed CLL, e.g., the subject has previously been administered a cancer therapy. In embodiments, venetoclax is administered at a dosage of about 15-600 mg (e.g., 15-20, 20-50, 50-75, 75-100, 100-200, 200-300, 300-400, 400-500, or 500-600 mg), e.g., daily. In embodiments, rituximab is administered at a dosage of about 350-550 mg/m2 (e.g., 350-375, 375-400, 400-425, 425-450, 450-475, or 475-500 mg/m2), e.g., intravenously, e.g., monthly
In an embodiment, cells expressing a CAR described herein are administered to a subject in combination with a molecule that decreases the Treg cell population. Methods that decrease the number of (e.g., deplete) Treg cells are known in the art and include, e.g., CD25 depletion, cyclophosphamide administration, modulating GITR function. Without wishing to be bound by theory, it is believed that reducing the number of Treg cells in a subject prior to apheresis or prior to administration of a CAR-expressing cell described herein reduces the number of unwanted immune cells (e.g., Tregs) in the tumor microenvironment and reduces the subject's risk of relapse. In one embodiment, cells expressing a CAR described herein are administered to a subject in combination with a molecule targeting GITR and/or modulating GITR functions, such as a GITR agonist and/or a GITR antibody that depletes regulatory T cells (Tregs). In embodiments, cells expressing a CAR described herein are administered to a subject in combination with cyclophosphamide. In one embodiment, the GITR binding molecules and/or molecules modulating GITR functions (e.g., GITR agonist and/or Treg depleting GITR antibodies) are administered prior to administration of the CAR-expressing cell. For example, in one embodiment, the GITR agonist can be administered prior to apheresis of the cells. In embodiments, cyclophosphamide is administered to the subject prior to administration (e.g., infusion or re-infusion) of the CAR-expressing cell or prior to aphersis of the cells. In embodiments, cyclophosphamide and an anti-GITR antibody are administered to the subject prior to administration (e.g., infusion or re-infusion) of the CAR-expressing cell or prior to apheresis of the cells. In one embodiment, the subject has cancer (e.g., a solid cancer or a hematological cancer such as ALL or CLL). In an embodiment, the subject has CLL. In embodiments, the subject has ALL. In embodiments, the subject has a solid cancer, e.g., a solid cancer described herein. Exemplary GITR agonists include, e.g., GITR fusion proteins and anti-GITR antibodies (e.g., bivalent anti-GITR antibodies) such as, e.g., a GITR fusion protein described in U.S. Patent No.: 6,111,090, European Patent No.: 090505B1, U.S Patent No.: 8,586,023, PCT Publication Nos.: WO 2010/003118 and 2011/090754, or an anti-GITR antibody described, e.g., in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, PCT Publication No.:WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Patent No.: 7,618,632, and PCT Publication No.: WO 2011/051726.

In one embodiment, a CAR expressing cell described herein is administered to a subject in combination with an mTOR inhibitor, e.g., an mTOR inhibitor described herein, e.g., a rapalog such as everolimus. In one embodiment, the mTOR inhibitor is administered prior to the CAR-expressing cell. For example, in one embodiment, the mTOR inhibitor can be administered prior to apheresis of the cells. In one embodiment, the subject has CLL.

In one embodiment, a CAR expressing cell described herein is administered to a subject in combination with a GITR agonist, e.g., a GITR agonist described herein. In one embodiment, the GITR agonist is administered prior to the CAR-expressing cell. For example, in one embodiment, the GITR agonist can be administered prior to apheresis of the cells. In one embodiment, the subject has CLL.

In one embodiment, a CAR-expressing cell described herein can be used in combination with a kinase inhibitor. In one embodiment, the kinase inhibitor is a CDK4 inhibitor, e.g., a CDK4 inhibitor described herein, e.g., a CD4/6 inhibitor, such as, e.g., 6-Acetyl-8-cyclopentyl-5-methyl-2-(5-piperazin-1-yl-pyridin-2-ylamino)-8H-pyrido[2,3-d]pyrimidin-7-one, hydrochloride (also referred to as palbociclib or PD0332991). In one embodiment, the kinase inhibitor is a BTK inhibitor, e.g., a BTK inhibitor described herein, such as, e.g., ibrutinib. In one embodiment, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one embodiment, the kinase inhibitor is a MNK inhibitor, e.g., a MNK inhibitor described herein, such as, e.g., 4-amino-5-(4-fluoroanilino)-pyrazolo [3,4*-d*] pyrimidine. The MNK inhibitor can be, e.g., a MNK1a, MNK1b, MNK2a and/or MNK2b inhibitor. In one embodiment, the kinase inhibitor is a dual PI3K/mTOR inhibitor described herein, such as, e.g., PF-04695102.

In one embodiment, the kinase inhibitor is a CDK4 inhibitor selected from aloisine A; flavopiridol or HMR-1275, 2-(2-chlorophenyl)-5,7-dihydroxy-8-[(3*S*,4*R*)-3-hydroxy-1-methyl-4-piperidinyl]-4-chromenone; crizotinib (PF-02341066; 2-(2-Chlorophenyl)-5,7-dihydroxy-8-[(2*R*,3*S*)-2-(hydroxymethyl)-1-methyl-3-pyrrolidinyl]- 4*H*-1-benzopyran-4-one, hydrochloride (P276-00); 1-methyl-5-[[2-[5-(trifluoromethyl)-1*H*-imidazol-2-yl]-4-pyridinyl]oxy]-*N*-[4-(trifluoromethyl)phenyl]-1*H*-benzimidazol-2-amine (RAF265); indisulam (E7070); roscovitine (CYC202); palbociclib (PD0332991); dinaciclib (SCH727965); *N*-[5-[[(5-*tert-*butyloxazol-2-yl)methyl]thio]thiazol-2-yl]piperidine-4-carboxamide (BMS 387032); 4-[[9-chloro-7-(2,6-difluorophenyl)-5*H*-pyrimido[5,4-*d*][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1*H*-imidazol-5-yl]-*N*-[4-(methylsulfonyl)phenyl]- 2-pyrimidinamine (AZD5438); and XL281 (BMS908662).

In one embodiment, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one embodiment, 1, 2, 3, 4, 5, 6, 7, 8, 9, 10, 11, 12 or more cycles of palbociclib are administered.

In embodiments, a CAR-expressing cell described herein is administered to a subject in combination with a cyclin-dependent kinase (CDK) 4 or 6 inhibitor, e.g., a CDK4 inhibitor or a CDK6 inhibitor described herein. In embodiments, a CAR-expressing cell described herein is administered to a subject in combination with a CDK4/6 inhibitor (e.g., an inhibitor that targets both CDK4 and CDK6), e.g., a CDK4/6 inhibitor described herein. In an embodiment, the subject has MCL. MCL is an aggressive cancer that is poorly responsive to currently available therapies, i.e., essentially incurable. In many cases of MCL, cyclin D1 (a regulator of CDK4/6) is expressed (e.g., due to chromosomal translocation involving immunoglobulin and Cyclin D1 genes) in MCL cells. Thus, without being bound by theory, it is thought that MCL cells are highly sensitive to CDK4/6 inhibition with high specificity (i.e., minimal effect on normal immune cells). CDK4/6 inhibitors alone have had some efficacy in treating MCL, but have only achieved partial remission with a high relapse rate. An exemplary CDK4/6 inhibitor is LEE011 (also called ribociclib), the structure of which is shown below.

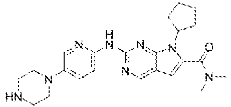

Without being bound by theory, it is believed that administration of a CAR-expressing cell described herein with a CDK4/6 inhibitor (e.g., LEE011 or other CDK4/6 inhibitor described herein) can achieve higher responsiveness, e.g., with higher remission rates and/or lower relapse rates, e.g., compared to a CDK4/6 inhibitor alone.

In one embodiment, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In a preferred embodiment, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In one embodiment, the kinase inhibitor is a BTK inhibitor, e.g., ibrutinib (PCI-32765). In embodiments, a CAR-expressing cell described herein is administered to a subject in combination with a BTK inhibitor (e.g., ibrutinib). In embodiments, a CAR-expressing cell described herein is administered to a subject in combination with ibrutinib (also called PCI-32765). The structure of ibrutinib (1-[(3*R*)-3-[4-Amino-3-(4-phenoxyphenyl)-1*H*-pyrazolo[3,4-d]pyrimidin-1-yl]piperidin-1-yl]prop-2-en-1-one) is shown below.

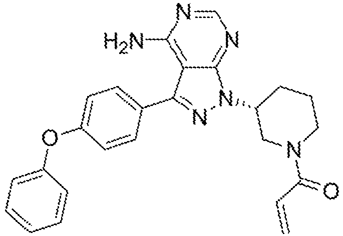

In embodiments, the subject has CLL, mantle cell lymphoma (MCL), or small lymphocytic lymphoma (SLL). For example, the subject has a deletion in the short arm of chromosome 17 (del(17p), e.g., in a leukemic cell). In other examples, the subject does not have a del(17p). In embodiments, the subject has relapsed CLL or SLL, e.g., the subject has previously been administered a cancer therapy (e.g., previously been administered one, two, three, or four prior cancer therapies). In embodiments, the subject has refractory CLL or SLL. In other embodiments, the subject has follicular lymphoma, e.g., relapse or refractory follicular lymphoma. In some embodiments, ibrutinib is administered at a dosage of about 300-600 mg/day (e.g., about 300-350, 350-400, 400-450, 450-500, 500-550, or 550-600 mg/day, e.g., about 420 mg/day or about 560 mg/day), e.g., orally. In embodiments, the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of ibrutinib are administered. Without being bound by theory, it is thought that the addition of ibrutinib enhances the T cell proliferative response and may shift T cells from a T-helper-2 (Th2) to T-helper-1 (Th1) phenotype. Th1 and Th2 are phenotypes of helper T cells, with Th1 versus Th2 directing different immune response pathways. A Th1 phenotype is associated with proinflammatory responses, e.g., for killing cells, such as intracellular pathogens/viruses or cancerous cells, or perpetuating autoimmune responses. A Th2 phenotype is associated with eosinophil accumulation and anti-inflammatory responses.

In one embodiment, the kinase inhibitor is an mTOR inhibitor selected from temsirolimus; ridaforolimus (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3S)-3-methylmorpholin-4-yl]pyrido[2,3-*d*]pyrimidin-7-yl}-2-methoxyphenyl)methanol (AZD8055); 2-amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126) (SEQ ID NO: 1262); and XL765.

In one embodiment, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

In one embodiment, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4-*d*] pyrimidine.

In embodiments, a CAR-expressing cell described herein is administered to a subject in combination with a phosphoinositide 3-kinase (PI3K) inhibitor (e.g., a PI3K inhibitor described herein, e.g., idelalisib or duvelisib) and/or rituximab. In embodiments, a CAR-expressing cell described herein is administered to a subject in combination with idelalisib and rituximab. In embodiments, a CAR-expressing cell described herein is administered to a subject in combination with duvelisib and rituximab. Idelalisib (also called GS-1101 or CAL-101; Gilead) is a small molecule that blocks the delta isoform of PI3K. The structure of idelalisib (5-Fluoro-3-phenyl-2-[(1*S*)-1-(7*H*-purin-6-ylamino)propyl]-4(3*H*)-quinazolinone) is shown below.

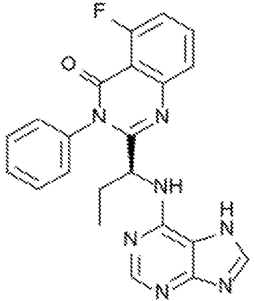

Duvelisib (also called IPI-145; Infinity Pharmaceuticals and Abbvie) is a small molecule that blocks PI3K-δ,γ. The structure of duvelisib (8-Chloro-2-phenyl-3-[(1S)-1-(9H-purin-6-ylamino)ethyl]-1(2H)-isoquinolinone) is shown below.

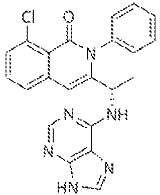

In embodiments, the subject has CLL. In embodiments, the subject has relapsed CLL, e.g., the subject has previously been administered a cancer therapy (e.g., previously been administered an anti-CD20 antibody or previously been administered ibrutinib). For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region (*IgV_{H}* ) gene. In other embodiments, the subject does not comprise a leukemic cell comprising a mutation in the immunoglobulin heavy-chain variable-region (*I_{G}V_{H}*) gene. In embodiments, the subject has a deletion in the long arm of chromosome 11 (del(11q)). In other embodiments, the subject does not have a del(11q). In embodiments, idelalisib is administered at a dosage of about 100-400 mg (e.g., 100-125, 125-150, 150-175, 175-200, 200-225, 225-250, 250-275, 275-300, 325-350, 350-375, or 375-400 mg), e.g., BID. In embodiments, duvelisib is administered at a dosage of about 15-100 mg (e.g., about 15-25, 25-50, 50-75, or 75-100 mg), e.g., twice a day. In embodiments, rituximab is administered at a dosage of about 350-550 mg/m² (e.g., 350-375, 375-400, 400-425, 425-450, 450-475, or 475-500 mg/m²), e.g., intravenously.

In one embodiment, the kinase inhibitor is a dual phosphatidylinositol 3-kinase (PI3K) and mTOR inhibitor selected from 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF-04691502); *N*-[4-[[4-(Dimethylamino)-1-piperidinyl]carbonyl]phenyl]-*N*'-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl}benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In embodiments, a CAR-expressing cell described herein is administered to a subject in combination with an anaplastic lymphoma kinase (ALK) inhibitor. Exemplary ALK kinases include but are not limited to crizotinib (Pfizer), ceritinib (Novartis), alectinib (Chugai), brigatinib (also called AP26113; Ariad), entrectinib (Ignyta), PF-06463922 (Pfizer), TSR-011 (Tesaro) (see, e.g., Clinical Trial Identifier No. NCT02048488), CEP-37440 (Teva), and X-396 (Xcovery). In some embodiments, the subject has a solid cancer, e.g., a solid cancer described herein, e.g., lung cancer.

The chemical name of crizotinib is 3-[(1*R*)-1-(2,6-dichloro-3-fluorophenyl)ethoxy]-5-(1-piperidin-4-ylpyrazol-4-yl)pyridin-2-amine. The chemical name of ceritinib is 5-Chloro-*N*²-[2-isopropoxy-5-methyl-4-(4-piperidinyl)phenyl]-*N*⁴-[2-(isopropylsulfonyl)phenyl]-2,4-pyrimidinediamine. The chemical name of alectinib is 9-ethyl-6,6-dimethyl-8-(4-morpholinopiperidin-1-yl)-11-oxo-6,11-dihydro-5H-benzo[b]carbazole-3-carbonitrile. The chemical name of brigatinib is 5-Chloro-N²-{4-[4-(dimethylamino)-1-piperidinyl]-2-methoxyphenyl}-N⁴-[2-(dimethylphosphoryl)phenyl]-2,4-pyrimidinediamine. The chemical name of entrectinib is N-(5-(3,5-difluorobenzyl)-1H-indazol-3-yl)-4-(4-methylpiperazin-1-yl)-2-((tetrahydro-2H-pyran-4-yl)amino)benzamide. The chemical name of PF-06463922 is (10R)-7-Amino-12-fluoro-2,10,16-trimethyl-15-oxo-10,15,16,17-tetrahydro-2H-8,4-(metheno)pyrazolo[4,3-h][2,5,11]-benzoxadiazacyclotetradecine-3-carbonitrile. The chemical structure of CEP-37440 is (S)-2-((5-chloro-2-((6-(4-(2-hydroxyethyl)piperazin-1-yl)-1-methoxy-6,7,8,9-tetrahydro-5H-benzo[7]annulen-2-yl)amino)pyrimidin-4-yl)amino)-N-methylbenzamide. The chemical name of X-396 is (R)-6-amino-5-(1-(2,6-dichloro-3-fluorophenyl)ethoxy)-N-(4-(4-methylpiperazine-1-carbonyl)phenyl)pyridazine-3-carboxamide.

Drugs that inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993) can also be used. In a further aspect, the cell compositions of the present invention may be administered to a patient in conjunction with (e.g., before, simultaneously or following) bone marrow transplantation, T cell ablative therapy using chemotherapy agents such as, fludarabine, external-beam radiation therapy (XRT), cyclophosphamide, and/or antibodies such as OKT3 or CAMPATH. In one aspect, the cell compositions of the present invention are administered following B-cell ablative therapy such as agents that react with CD20, e.g., Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

In embodiments, a CAR-expressing cell described herein is administered to a subject in combination with an indoleamine 2,3-dioxygenase (IDO) inhibitor. IDO is an enzyme that catalyzes the degradation of the amino acid, L-tryptophan, to kynurenine. Many cancers overexpress IDO, e.g., prostatic, colorectal, pancreatic, cervical, gastric, ovarian, head, and lung cancer. pDCs, macrophages, and dendritic cells (DCs) can express IDO. Without being bound by theory, it is thought that a decrease in L-tryptophan (e.g., catalyzed by IDO) results in an immunosuppressive milieu by inducing T-cell anergy and apoptosis. Thus, without being bound by theory, it is thought that an IDO inhibitor can enhance the efficacy of a CAR-expressing cell described herein, e.g., by decreasing the suppression or death of a CAR-expressing immune cell. In embodiments, the subject has a solid tumor, e.g., a solid tumor described herein, e.g., prostatic, colorectal, pancreatic, cervical, gastric, ovarian, head, or lung cancer. Exemplary inhibitors of IDO include but are not limited to 1-methyl-tryptophan, indoximod (NewLink Genetics) (see, e.g., Clinical Trial Identifier Nos. NCT01191216; NCT01792050), and INCB024360 (Incyte Corp.) (see, e.g., Clinical Trial Identifier Nos. NCT01604889; NCT01685255)

In embodiments, a CAR-expressing cell described herein is administered to a subject in combination with a modulator of myeloid-derived suppressor cells (MDSCs). MDSCs accumulate in the periphery and at the tumor site of many solid tumors. These cells suppress T cell responses, thereby hindering the efficacy of CAR-expressing cell therapy. Without being bound by theory, it is thought that administration of a MDSC modulator enhances the efficacy of a CAR-expressing cell described herein. In an embodiment, the subject has a solid tumor, e.g., a solid tumor described herein, e.g., glioblastoma. Exemplary modulators of MDSCs include but are not limited to MCS110 and BLZ945. MCS110 is a monoclonal antibody (mAb) against macrophage colony-stimulating factor (M-CSF). See, e.g., Clinical Trial Identifier No. NCT00757757. BLZ945 is a small molecule inhibitor of colony stimulating factor 1 receptor (CSF1R). See, e.g., Pyonteck et al. Nat. Med. 19(2013):1264-72. The structure of BLZ945 is shown below.

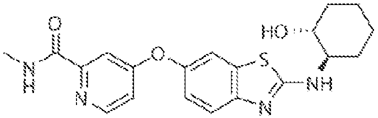

In embodiments, a CAR-expressing cell described herein is administered to a subject in combination with a CD19 CART cell (e.g., CTL019, e.g., as described in WO2012/079000, incorporated herein by reference). In embodiments, the subject has a CD19+ lymphoma, e.g., a CD19+ Non-Hodgkin's Lymphoma (NHL), a CD19+ FL, or a CD19+ DLBCL. In embodiments, the subject has a relapsed or refractory CD19+ lymphoma. In embodiments, a lymphodepleting chemotherapy is administered to the subject prior to, concurrently with, or after administration (e.g., infusion) of CD19 CART cells. In an example, the lymphodepleting chemotherapy is administered to the subject prior to administration of CD19 CART cells. For example, the lymphodepleting chemotherapy ends 1-4 days (e.g,. 1, 2, 3, or 4 days) prior to CD19 CART cell infusion. In embodiments, multiple doses of CD19 CART cells are administered, e.g., as described herein. For example, a single dose comprises about 5 x 10⁸ CD19 CART cells. In embodiments, a lymphodepleting chemotherapy is administered to the subject prior to, concurrently with, or after administration (e.g., infusion) of a CAR-expressing cell described herein, e.g., a non-CD19 CAR-expresing cell. In embodiments, a CD19 CART is administered to the subject prior to, concurrently with, or after administration (e.g., infusion) of a non-CD19 CAR-expressing cell, e.g., a non-CD19 CAR-expressing cell described herein.

In some embodiments, a CAR-expressing cell described herein is administered to a subject in combination with a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15 (Admune Therapeutics, LLC). hetIL-15 is a heterodimeric non-covalent complex of IL-15 and IL-15Ra. hetIL-15 is described in, e.g., U.S. 8,124,084, U.S. 2012/0177598, U.S. 2009/0082299, U.S. 2012/0141413, and U.S. 2011/0081311, incorporated herein by reference. In embodiments, het-IL-15 is administered subcutaneously. In embodiments, the subject has a cancer, e.g., solid cancer, e.g., melanoma or colon cancer. In embodiments, the subject has a metastatic cancer.

In one embodiment, the subject can be administered an agent which reduces or ameliorates a side effect associated with the administration of a CAR-expressing cell. Side effects associated with the administration of a CAR-expressing cell include, but are not limited to CRS, and hemophagocytic lymphohistiocytosis (HLH), also termed Macrophage Activation Syndrome (MAS). Symptoms of CRS include high fevers, nausea, transient hypotension, hypoxia, and the like. CRS may include clinical constitutional signs and symptoms such as fever, fatigue, anorexia, myalgias, arthalgias, nausea, vomiting, and headache. CRS may include clinical skin signs and symptoms such as rash. CRS may include clinical gastrointestinal signs and symsptoms such as nausea, vomiting and diarrhea. CRS may include clinical respiratory signs and symptoms such as tachypnea and hypoxemia. CRS may include clinical cardiovascular signs and symptoms such as tachycardia, widened pulse pressure, hypotension, increased cardac output (early) and potentially diminished cardiac output (late). CRS may include clinical coagulation signs and symptoms such as elevated d-dimer, hypofibrinogenemia with or without bleeding. CRS may include clinical renal signs and symptoms such as azotemia. CRS may include clinical hepatic signs and symptoms such as transaminitis and hyperbilirubinemia. CRS may include clinical neurologic signs and symptoms such as headache, mental status changes, confusion, delirium, word finding difficulty or frank aphasia, hallucinations, tremor, dymetria, altered gait, and seizures.

Accordingly, the methods described herein can comprise administering a CAR-expressing cell described herein to a subject and further administering one or more agents to manage elevated levels of a soluble factor resulting from treatment with a CAR-expressing cell. In one embodiment, the soluble factor elevated in the subject is one or more of IFN-γ, TNFα, IL-2 and IL-6. In an embodiment, the factor elevated in the subject is one or more of IL-1, GM-CSF, IL-10, IL-8, IL-5 and fraktalkine. Therefore, an agent administered to treat this side effect can be an agent that neutralizes one or more of these soluble factors. In one embodiment, the agent that neutralizes one or more of these soluble forms is an antibody or antigen binding fragment thereof. Examples of such agents include, but are not limited to a steroid (e.g., corticosteroid), an inhibitor of TNFα, and an inhibitor of IL-6. An example of a TNFα inhibitor is an anti-TNFα antibody molecule such as, infliximab, adalimumab, certolizumab pegol, and golimumab. Another example of a TNFα inhibitor is a fusion protein such as entanercept. Small molecule inhibitors of TNFα include, but are not limited to, xanthine derivatives (e.g. pentoxifylline) and bupropion. An example of an IL-6 inhibitor is an anti-IL-6 antibody molecule or an anti-IL-6 receptor antibody molecule such as tocilizumab (toc), sarilumab, elsilimomab, CNTO 328, ALD518/BMS-945429, CNTO 136, CPSI-2364, CDP6038, VX30, ARGX-109, FE301, and FM101. In one embodiment, the anti-IL-6 receptor antibody molecule is tocilizumab. An example of an IL-1R based inhibitor is anakinra.

In one embodiment, the subject can be administered an agent which enhances the activity of a CAR-expressing cell. For example, in one embodiment, the agent can be an agent which inhibits an inhibitory molecule. Inhibitory molecules, e.g., Programmed Death 1 (PD-1), can, in some embodiments, decrease the ability of a CAR-expressing cell to mount an immune effector response. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGF beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used to inhibit expression of an inhibitory molecule in the CAR-expressing cell. In an embodiment the inhibitor is an shRNA. In an embodiment, the inhibitory molecule is inhibited within a CAR-expressing cell. In these embodiments, a dsRNA molecule that inhibits expression of the inhibitory molecule is linked to the nucleic acid that encodes a component, e.g., all of the components, of the CAR. In one embodiment, the inhibitor of an inhibitory signal can be, e.g., an antibody or antibody fragment that binds to an inhibitory molecule. For example, the agent can be an antibody or antibody fragment that binds to PD-1, PD-L1, PD-L2 or CTLA4 (e.g., ipilimumab (also referred to as MDX-010 and MDX-101, and marketed as Yervoy^{®}; Bristol-Myers Squibb; Tremelimumab (IgG2 monoclonal antibody available from Pfizer, formerly known as ticilimumab, CP-675,206).). In an embodiment, the agent is an antibody or antibody fragment that binds to TIM3. In an embodiment, the agent is an antibody or antibody fragment that binds to CEACAM (CEACAM-1, CEACAM-3, and/or CEACAM-5). In an embodiment, the agent is an antibody or antibody fragment that binds to LAG3.

PD-1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD-1, PD-L1 and PD-L2 have been shown to downregulate T cell activation upon binding to PD-1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD-1 with PD-L1. Antibodies, antibody fragments, and other inhibitors of PD-1, PD-L1 and PD-L2 are available in the art and may be used combination with a cars of the present invention described herein. For example, nivolumab (also referred to as BMS-936558 or MDX1106; Bristol-Myers Squibb) is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168. Pidilizumab (CT-011; Cure Tech) is a humanized IgGlk monoclonal antibody that binds to PD-1. Pidilizumab and other humanized anti-PD-1 monoclonal antibodies are disclosed in WO2009/101611. Pembrolizumab (formerly known as lambrolizumab, and also referred to as MK03475; Merck) is a humanized IgG4 monoclonal antibody that binds to PD-1. Pembrolizumab and other humanized anti-PD-1 antibodies are disclosed in US 8,354,509 and WO2009/114335. MEDI4736 (Medimmune) is a human monoclonal antibody that binds to PDL1, and inhibits interaction of the ligand with PD1. MDPL3280A (Genentech / Roche) is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906. Other anti-PD-L1 binding agents include YW243.55.S70 (heavy and light chain variable regions are shown in SEQ ID NOs 20 and 21 in WO2010/077634) and MDX-1 105 (also referred to as BMS-936559, and, e.g., anti-PD-L1 binding agents disclosed in WO2007/005874). AMP-224 (B7-DCIg; Amplimmune; e.g., disclosed in WO2010/027827 and WO2011/066342), is a PD-L2 Fc fusion soluble receptor that blocks the interaction between PD-1 and B7-H1. Other anti-PD-1 antibodies include AMP 514 (Amplimmune), among others, e.g., anti-PD-1 antibodies disclosed in US 8,609,089, US 2010028330, and/or US 20120114649.

TIM-3 (T cell immunoglobulin-3) also negatively regulates T cell function, particularly in IFN-g-secreting CD4+ T helper 1 and CD8+ T cytotoxic 1 cells, and plays a critical role in T cell exhaustion. Inhibition of the interaction between TIM3 and its ligands, e.g., galectin-9 (Gal9), phosphotidylserine (PS), and HMGB1, can increase immune response. Antibodies, antibody fragments, and other inhibitors of TIM3 and its ligands are available in the art and may be used combination with a CD19 CAR described herein. For example, antibodies, antibody fragments, small molecules, or peptide inhibitors that target TIM3 binds to the IgV domain of TIM3 to inhibit interaction with its ligands. Antibodies and peptides that inhibit TIM3 are disclosed in WO2013/006490 and US20100247521. Other anti-TIM3 antibodies include humanized versions of RMT3-23 (disclosed in Ngiow et al., 2011, Cancer Res, 71:3540-3551), and clone 8B.2C12 (disclosed in Monney et al., 2002, Nature, 415:536-541). Bi-specific antibodies that inhibit TIM3 and PD-1 are disclosed in US20130156774.

In other embodiments, the agent that enhances the activity of a CAR-expressing cell is a CEACAM inhibitor (*e.g*., CEACAM-1, CEACAM-3, and/or CEACAM-5 inhibitor). In one embodiment, the inhibitor of CEACAM is an anti-CEACAM antibody molecule. Exemplary anti-CEACAM-1 antibodies are described in WO 2010/125571, WO 2013/082366 WO 2014/059251 and WO 2014/022332, e.g., a monoclonal antibody 34B1, 26H7, and 5F4; or a recombinant form thereof, as described in, *e.g.,* US 2004/0047858, US 7,132,255 and WO 99/052552. In other embodiments, the anti-CEACAM antibody binds to CEACAM-5 as described in, *e.g.,* Zheng et al. PLoS One. 2010 Sep 2;5(9). pii: e12529 (DOI:10:1371/journal.pone.0021146), or crossreacts with CEACAM-1 and CEACAM-5 as described in, *e.g.,* WO 2013/054331 and US 2014/0271618.

Without wishing to be bound by theory, carcinoembryonic antigen cell adhesion molecules (CEACAM), such as CEACAM-1 and CEACAM-5, are believed to mediate, at least in part, inhibition of an anti-tumor immune response (see e.g., Markel et al. J Immunol. 2002 Mar 15;168(6):2803-10; Markel et al. J Immunol. 2006 Nov 1;177(9):6062-71; Markel et al. Immunology. 2009 Feb;126(2):186-200; Markel et al. Cancer Immunol Immunother. 2010 Feb;59(2):215-30; Ortenberg et al. Mol Cancer Ther. 2012 Jun;11(6):1300-10; Stern et al. J Immunol. 2005 Jun 1;174(11):6692-701; Zheng et al. PLoS One. 2010 Sep 2;5(9). pii: e12529). For example, CEACAM-1 has been described as a heterophilic ligand for TIM-3 and as playing a role in TIM-3-mediated T cell tolerance and exhaustion (see e.g., WO 2014/022332; Huang, et al. (2014) Nature doi:10.1038/nature13848). In embodiments, co-blockade of CEACAM-1 and TIM-3 has been shown to enhance an anti-tumor immune response in xenograft colorectal cancer models (see e.g., WO 2014/022332; Huang, et al. (2014), *supra*). In other embodiments, co-blockade of CEACAM-1 and PD-1 reduce T cell tolerance as described, *e.g.*, in WO 2014/059251. Thus, CEACAM inhibitors can be used with the other immunomodulators described herein (e.g., anti-PD-1 and/or anti-TIM-3 inhibitors) to enhance an immune response against a cancer, *e.g.,* a melanoma, a lung cancer (e.g., NSCLC), a bladder cancer, a colon cancer an ovarian cancer, and other cancers as described herein.

LAG-3 (lymphocyte activation gene-3 or CD223) is a cell surface molecule expressed on activated T cells and B cells that has been shown to play a role in CD8+ T cell exhaustion. Antibodies, antibody fragments, and other inhibitors of LAG-3 and its ligands are available in the art and may be used combination with a CD19 CAR described herein. For example, BMS-986016 (Bristol-Myers Squib) is a monoclonal antibody that targets LAG3. IMP701 (Immutep) is an antagonist LAG-3 antibody and IMP731 (Immutep and GlaxoSmithKline) is a depleting LAG-3 antibody. Other LAG-3 inhibitors include IMP321 (Immutep), which is a recombinant fusion protein of a soluble portion of LAG3 and Ig that binds to MHC class II molecules and activates antigen presenting cells (APC). Other antibodies are disclosed, e.g., in WO2010/019570.

In some embodiments, the agent which enhances the activity of a CAR-expressing cell can be, e.g., a fusion protein comprising a first domain and a second domain, wherein the first domain is an inhibitory molecule, or fragment thereof, and the second domain is a polypeptide that is associated with a positive signal, e.g., a polypeptide comrpsing an antracellular signaling domain as described herein. In some embodiments, the polypeptide that is associated with a positive signal can include a costimulatory domain of CD28, CD27, ICOS, e.g., an intracellular signaling domain of CD28, CD27 and/or ICOS, and/or a primary signaling domain, e.g., of CD3 zeta, e.g., described herein. In one embodiment, the fusion protein is expressed by the same cell that expressed the CAR. In another embodiment, the fusion protein is expressed by a cell, e.g., a T cell that does not express a CAR of the present invention.

In one embodiment, the agent which enhances activity of a CAR-expressing cell described herein is miR-17-92.

In one embodiment, the agent which enhances activity of a CAR-described herein is a cytokine. Cytokines have important functions related to T cell expansion, differentiation, survival, and homeostatis. Cytokines that can be administered to the subject receiving a CAR-expressing cell described herein include: IL-2, IL-4, IL-7, IL-9, IL-15, IL-18, and IL-21, or a combination thereof. In preferred embodiments, the cytokine administered is IL-7, IL-15, or IL-21, or a combination thereof. The cytokine can be administered once a day or more than once a day, e.g., twice a day, three times a day, or four times a day. The cytokine can be administered for more than one day, e.g. the cytokine is administered for 2 days, 3 days, 4 days, 5 days, 6 days, 1 week, 2 weeks, 3 weeks, or 4 weeks. For example, the cytokine is administered once a day for 7 days.

In embodiments, the cytokine is administered in combination with CAR-expressing T cells. The cytokine can be administered simultaneously or concurrently with the CAR-expressing T cells, e.g., administered on the same day. The cytokine may be prepared in the same pharmaceutical composition as the CAR-expressing T cells, or may be prepared in a separate pharmaceutical composition. Alternatively, the cytokine can be administered shortly after administration of the CAR-expressing T cells, e.g., 1 day, 2 days, 3 days, 4 days, 5 days, 6 days, or 7 days after administration of the CAR-expressing T cells. In embodiments where the cytokine is administered in a dosing regimen that occurs over more than one day, the first day of the cytokine dosing regimen can be on the same day as administration with the CAR-expressing T cells, or the first day of the cytokine dosing regimen can be 1 day, 2 days, 3 days, 4 days, 5 days, 6 days, or 7 days after administration of the CAR-expressing T cells. In one embodiment, on the first day, the CAR-expressing T cells are administered to the subject, and on the second day, a cytokine is administered once a day for the next 7 days. In a preferred embodiment, the cytokine to be administered in combination with CAR-expressing T cells is IL-7, IL-15, or IL-21.

In other embodiments, the cytokine is administered a period of time after administration of CAR-expressing cells, e.g., at least 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells. In one embodiment, the cytokine is administered after assessment of the subject's response to the CAR-expressing cells. For example, the subject is administered CAR-expressing cells according to the dosage and regimens described herein. The response of the subject to CAR-expressing cell therapy is assessed at 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells, using any of the methods described herein, including inhibition of tumor growth, reduction of circulating tumor cells, or tumor regression. Subjects that do not exhibit a sufficient response to CAR-expressing cell therapy can be administered a cytokine. Administration of the cytokine to the subject that has sub-optimal response to the CAR-expressing cell therapy improves CAR-expressing cell efficacy or anti-cancer activity. In a preferred embodiment, the cytokine administered after administration of CAR-expressing cells is IL-7.

### Combination with a low dose of an mTOR inhibitor

In one embodiment, the cells expressing a CAR molecule, e.g., a CAR molecule described herein, are administered in combination with a low, immune enhancing dose of an mTOR inhibitor.

In an embodiment, a dose of an mTOR inhibitor is associated with, or provides, mTOR inhibition of at least 5 but no more than 90%, at least 10 but no more than 90%, at least 15, but no more than 90%, at least 20 but no more than 90%, at least 30 but no more than 90%, at least 40 but no more than 90%, at least 50 but no more than 90%, at least 60 but no more than 90%, or at least 70 but no more than 90%.

In an embodiment, a dose of an mTOR inhibitor is associated with, or provides, mTOR inhibition of at least 5 but no more than 80%, at least 10 but no more than 80%, at least 15, but no more than 80%, at least 20 but no more than 80%, at least 30 but no more than 80%, at least 40 but no more than 80%, at least 50 but no more than 80%, or at least 60 but no more than 80%.

In an embodiment, a dose of an mTOR inhibitor is associated with, or provides, mTOR inhibition of at least 5 but no more than 70%, at least 10 but no more than 70%, at least 15, but no more than 70%, at least 20 but no more than 70%, at least 30 but no more than 70%, at least 40 but no more than 70%, or at least 50 but no more than 70%.

In an embodiment, a dose of an mTOR inhibitor is associated with, or provides, mTOR inhibition of at least 5 but no more than 60%, at least 10 but no more than 60%, at least 15, but no more than 60%, at least 20 but no more than 60%, at least 30 but no more than 60%, or at least 40 but no more than 60%.

In an embodiment, a dose of an mTOR inhibitor is associated with, or provides, mTOR inhibition of at least 5 but no more than 50%, at least 10 but no more than 50%, at least 15, but no more than 50%, at least 20 but no more than 50%, at least 30 but no more than 50%, or at least 40 but no more than 50%.

In an embodiment, a dose of an mTOR inhibitor is associated with, or provides, mTOR inhibition of at least 5 but no more than 40%, at least 10 but no more than 40%, at least 15, but no more than 40%, at least 20 but no more than 40%, at least 30 but no more than 40%, or at least 35 but no more than 40%.

In an embodiment, a dose of an mTOR inhibitor is associated with, or provides, mTOR inhibition of at least 5 but no more than 30%, at least 10 but no more than 30%, at least 15, but no more than 30%, at least 20 but no more than 30%, or at least 25 but no more than 30%.

In an embodiment, a dose of an mTOR inhibitor is associated with, or provides, mTOR inhibition of at least 1, 2, 3, 4 or 5 but no more than 20%, at least 1, 2, 3, 4 or 5 but no more than 30%, at least 1, 2, 3, 4 or 5, but no more than 35, at least 1, 2, 3, 4 or 5 but no more than 40%, or at least 1, 2, 3, 4 or 5 but no more than 45%.

In an embodiment, a dose of an mTOR inhibitor is associated with, or provides, mTOR inhibition of at least 1, 2, 3, 4 or 5 but no more than 90%.

As is discussed herein, the extent of mTOR inhibition can be expressed as the extent of P70 S6 kinase inhibition, e.g., the extent of mTOR inhibition can be determined by the level of decrease in P70 S6 kinase activity, e.g., by the decrease in phosphorylation of a P70 S6 kinase substrate. The level of mTOR inhibition can be evaluated by a method described herein, e.g. by the Boulay assay, or measurement of phosphorylated S6 levels by western blot.

### Exemplary mTOR Inhibitors

As used herein, the term "mTOR inhibitor" refers to a compound or ligand, or a pharmaceutically acceptable salt thereof, which inhibits the mTOR kinase in a cell. In an embodiment an mTOR inhibitor is an allosteric inhibitor. In an embodiment an mTOR inhibitor is a catalytic inhibitor.

Allosteric mTOR inhibitors include the neutral tricyclic compound rapamycin (sirolimus), rapamycin-related compounds, that is compounds having structural and functional similarity to rapamycin including, e.g., rapamycin derivatives, rapamycin analogs (also referred to as rapalogs) and other macrolide compounds that inhibit mTOR activity.

Rapamycin is a known macrolide antibiotic produced by Streptomyces hygroscopicus having the structure shown in Formula A.

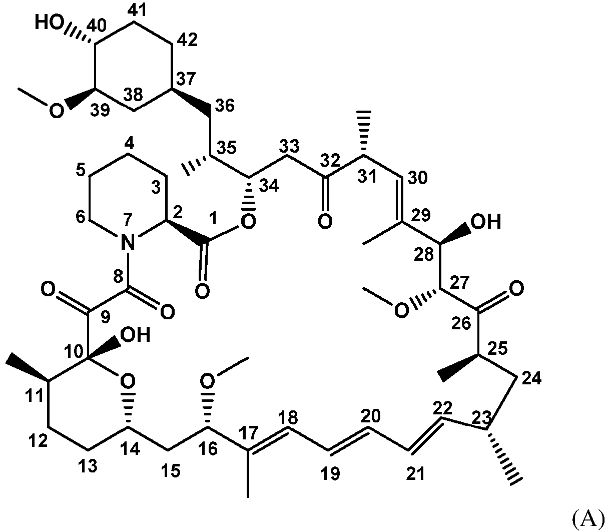

See, e.g., McAlpine, J.B., et al., J. Antibiotics (1991) 44: 688; Schreiber, S.L., et al., J. Am. Chem. Soc. (1991) 113: 7433; U.S. Patent No. 3,929,992. There are various numbering schemes proposed for rapamycin. To avoid confusion, when specific rapamycin analogs are named herein, the names are given with reference to rapamycin using the numbering scheme of formula A.

Rapamycin analogs useful in the invention are, for example, O-substituted analogs in which the hydroxyl group on the cyclohexyl ring of rapamycin is replaced by OR₁ in which R₁ is hydroxyalkyl, hydroxyalkoxyalkyl, acylaminoalkyl, or aminoalkyl; e.g. RAD001, also known as, everolimus as described in US 5,665,772 and WO94/09010 the contents of which are incorporated by reference. Other suitable rapamycin analogs include those substituted at the 26- or 28-position. The rapamycin analog may be an epimer of an analog mentioned above, particularly an epimer of an analog substituted in position 40, 28 or 26, and may optionally be further hydrogenated, e.g. as described in US 6,015,815, WO95/14023 and WO99/15530 the contents of which are incorporated by reference, e.g. ABT578 also known as zotarolimus or a rapamycin analog described in US 7,091,213, WO98/02441 and WO01/14387 the contents of which are incorporated by reference, e.g. AP23573 also known as ridaforolimus.

Examples of rapamycin analogs suitable for use in the present invention from US 5,665,772 include, but are not limited to, 40-O-benzyl-rapamycin, 40-O-(4'-hydroxymethyl)benzyl-rapamycin, 40-O-[4'-(1,2-dihydroxyethyl)]benzyl-rapamycin, 40-O-allyl-rapamycin, 40-O-[3'-(2,2-dimethyl-1,3-dioxolan-4(S)-yl)-prop-2'-en-1'-yl]-rapamycin, (2'E,4'S)-40-O-(4',5'-dihydroxypent-2'-en-1'-yl)-rapamycin, 40-O-(2-hydroxy)ethoxycarbonylmethyl-rapamycin, 40-O-(2-hydroxy)ethyl-rapamycin , 40-O-(3-hydroxy)propyl-rapamycin, 40-O-(6-hydroxy)hexyl-rapamycin, 40-O-[2-(2-hydroxy)ethoxy]ethyl-rapamycin, 40-O-[(3S)-2,2-dimethyldioxolan-3-yl]methyl-rapamycin, 40-O-[(2S)-2,3-dihydroxyprop-1-yl]-rapamycin, 40-O-(2-acetoxy)ethyl-rapamycin, 40-O-(2-nicotinoyloxy)ethyl-rapamycin, 40-O-[2-(N-morpholino)acetoxy]ethyl-rapamycin, 40-O-(2-N-imidazolylacetoxy)ethyl-rapamycin, 40-O-[2-(N-methyl-N'-piperazinyl)acetoxy]ethyl-rapamycin, 39-O-desmethyl-39,40-O,O-ethylene-rapamycin, (26R)-26-dihydro-40-O-(2-hydroxy)ethyl-rapamycin, 40-O-(2-aminoethyl)-rapamycin, 40-O-(2-acetaminoethyl)-rapamycin, 40-O-(2-nicotinamidoethyl)-rapamycin, 40-O-(2-(N-methyl-imidazo-2'-ylcarbethoxamido)ethyl)-rapamycin, 40-O-(2-ethoxycarbonylaminoethyl)-rapamycin, 40-O-(2-tolylsulfonamidoethyl)-rapamycin and 40-O-[2-(4',5'-dicarboethoxy-1',2',3'-triazol-1'-yl)-ethyl]-rapamycin.

Other rapamycin analogs useful in the present invention are analogs where the hydroxyl group on the cyclohexyl ring of rapamycin and/or the hydroxy group at the 28 position is replaced with an hydroxyester group are known, for example, rapamycin analogs found in US RE44,768, e.g. temsirolimus.

Other rapamycin analogs useful in the preset invention include those wherein the methoxy group at the 16 position is replaced with another substituent, preferably (optionally hydroxy-substituted) alkynyloxy, benzyl, orthomethoxybenzyl or chlorobenzyl and/or wherein the mexthoxy group at the 39 position is deleted together with the 39 carbon so that the cyclohexyl ring of rapamycin becomes a cyclopentyl ring lacking the 39 position methyoxy group; e.g. as described in WO95/16691 and WO96/41807 the contents of which are incorporated by reference. The analogs can be further modified such that the hydroxy at the 40-position of rapamycin is alkylated and/or the 32-carbonyl is reduced.

Rapamycin analogs from WO95/16691 include, but are not limited to, 16-demthoxy-16-(pent-2-ynyl)oxy-rapamycin, 16-demthoxy-16-(but-2-ynyl)oxy-rapamycin, 16-demthoxy-16-(propargyl)oxy-rapamycin, 16-demethoxy-16-(4-hydroxy-but-2-ynyl)oxy-rapamycin, 16-demthoxy-16-benzyloxy-40-O-(2-hydroxyethyl)-rapamycin, 16-demthoxy-16-benzyloxy-rapamycin, 16-demethoxy-16-ortho-methoxybenzyl-rapamycin, 16-demethoxy-40-O-(2-methoxyethyl)-16-pent-2-ynyl)oxy-rapamycin, 39-demethoxy-40-desoxy-39-formyl-42-nor-rapamycin, 39-demethoxy-40-desoxy-39-hydroxymethyl-42-nor-rapamycin, 39-demethoxy-40-desoxy-39-carboxy-42-nor-rapamycin, 39-demethoxy-40-desoxy-39-(4-methyl-piperazin-1-yl)carbonyl-42-nor-rapamycin, 39-demethoxy-40-desoxy-39-(morpholin-4-yl)carbonyl-42-nor-rapamycin, 39-demethoxy-40-desoxy-39-[N-methyl, N-(2-pyridin-2-yl-ethyl)]carbamoyl-42-nor-rapamycin and 39-demethoxy-40-desoxy-39-(p-toluenesulfonylhydrazonomethyl)-42-nor-rapamycin.

Rapamycin analogs from WO96/41807 include, but are not limited to, 32-deoxo-rapamycin, 16-O-pent-2-ynyl-32-deoxo-rapamycin, 16-O-pent-2-ynyl-32-deoxo-40-O-(2-hydroxy-ethyl)-rapamycin, 16-O-pent-2-ynyl-32-(S)-dihydro-40-O-(2-hydroxyethyl)-rapamycin, 32(S)-dihydro-40-O-(2-methoxy)ethyl-rapamycin and 32(S)-dihydro-40-O-(2-hydroxyethyl)-rapamycin.

Another suitable rapamycin analog is umirolimus as described in US2005/0101624 the contents of which are incorporated by reference.

RAD001, otherwise known as everolimus (Afinitor^{®}), has the chemical name (1R,9S,12S,15R,16E,18R,19R,21R,23S,24E,26E,28E,30S,32S,35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone
Further examples of allosteric mTOR inhibitors include sirolimus (rapamycin, AY-22989), 40-[3-hydroxy-2-(hydroxymethyl)-2-methylpropanoate]-rapamycin (also called temsirolimus or CCI-779) and ridaforolimus (AP-23573/MK-8669). Other examples of allosteric mTor inhibtors include zotarolimus (ABT578) and umirolimus.

Alternatively or additionally, catalytic, ATP-competitive mTOR inhibitors have been found to target the mTOR kinase domain directly and target both mTORC1 and mTORC2. These are also more effective inhibitors of mTORC1 than such allosteric mTOR inhibitors as rapamycin, because they modulate rapamycin-resistant mTORC1 outputs such as 4EBP1-T37/46 phosphorylation and cap-dependent translation.

Catalytic inhibitors include: BEZ235 or 2-methyl-2-[4-(3-methyl-2-oxo-8-quinolin-3-yl-2,3-dihydro-imidazo[4,5-c]quinolin-1-yl)-phenyl]-propionitrile, or the monotosylate salt form. the synthesis of BEZ235 is described in WO2006/122806; CCG168 (otherwise known as AZD-8055, Chresta, C.M., et al., Cancer Res, 2010, 70(1), 288-298) which has the chemical name {5-[2,4-bis-((S)-3-methyl-morpholin-4-yl)-pyrido[2,3d]pyrimidin-7-yl]-2-methoxy-phenyl}-methanol; 3-[2,4-bis[(3S)-3-methylmorpholin-4-yl]pyrido[2,3-d]pyrimidin-7-yl]-N-methylbenzamide (WO09104019); 3-(2-aminobenzo[d]oxazol-5-yl)-1-isopropyl-1H-pyrazolo[3,4-d]pyrimidin-4-amine (WO10051043 and WO2013023184); A N-(3-(N-(3-((3,5-dimethoxyphenyl)amino)quinoxaline-2-yl)sulfamoyl)phenyl)-3-methoxy-4-methylbenzamide (WO07044729 and WO12006552); PKI-587 (Venkatesan, A.M., J. Med.Chem., 2010, 53, 2636-2645) which has the chemical name 1-[4-[4-(dimethylamino)piperidine-1-carbonyl]phenyl]-3-[4-(4,6-dimorpholino-1,3,5-triazin-2-yl)phenyl]urea; GSK-2126458 (ACS Med. Chem. Lett., 2010, 1, 39-43) which has the chemical name 2,4-difluoro-N-{2-methoxy-5-[4-(4-pyridazinyl)-6-quinolinyl]-3-pyridinyl } benzenesulfonamide; ; 5-(9-isopropyl-8-methyl-2-morpholino-9H-purin-6-yl)pyrimidin-2-amine (WO10114484); (E)-N-(8-(6-amino-5-(trifluoromethyl)pyridin-3-yl)-1-(6-(2-cyanopropan-2-yl)pyridin-3-yl)-3-methyl-1H-imidazo[4,5-c]quinolin-2(3H)-ylidene)cyanamide (WO12007926).

Further examples of catalytic mTOR inhibitors include 8-(6-methoxy-pyridin-3-yl)-3-methyl-1-(4-piperazin-1-yl-3-trifluoromethyl-phenyl)-1,3-dihydro-imidazo[4,5-c]quinolin-2-one (WO2006/122806) and Ku-0063794 (Garcia-Martinez JM, et al.,Biochem J., 2009, 421(1), 29-42.. Ku-0063794 is a specific inhibitor of the mammalian target of rapamycin (mTOR).) WYE-354 is another example of a catalytic mTor inhibitor (Yu K, et al. (2009). Biochemical, Cellular, and In vivo Activity of Novel ATP-Competitive and Selective Inhibitors of the Mammalian Target of Rapamycin. Cancer Res. 69(15): 6232-6240).
mTOR inhibitors useful according to the present invention also include prodrugs, derivatives, pharmaceutically acceptable salts, or analogs thereof of any of the foregoing.
mTOR inhibitors, such as RAD001, may be formulated for delivery based on well-established methods in the art based on the particular dosages described herein. In particular, US Patent 6,004,973 (incorporated herein by reference) provides examples of formulations useable with the mTOR inhibitors described herein.

### Evaluation of mTOR Inhibition

mTOR phosphorylates the kinase P70 S6, thereby activating P70 S6 kinase and allowing it to phosphorylate its substrate. The extent of mTOR inhibition can be expressed as the extent of P70 S6 kinase inhibition, e.g., the extent of mTOR inhibition can be determined by the level of decrease in P70 S6 kinase activity, e.g., by the decrease in phosphorylation of a P70 S6 kinase substrate. One can determine the level of mTOR inhibition, by measuring P70 S6 kinase activity (the ability of P70 S6 kinase to phsophorylate a substrate), in the absence of inhibitor, e.g., prior to administration of inhibitor, and in the presences of inhibitor, or after the administration of inhibitor. The level of inhibition of P70 S6 kinase gives the level of mTOR inhibition. Thus, if P70 S6 kinase is inhibited by 40%, mTOR activity, as measured by P70 S6 kinase activity, is inhibited by 40%. The extent or level of inhibition referred to herein is the average level of inhibition over the dosage interval. By way of example, if the inhibitor is given once per week, the level of inhibition is given by the average level of inhibition over that interval, namely a week.

Boulay et al., Cancer Res, 2004, 64:252-61, hereby incorporated by reference, teaches an assay that can be used to assess the level of mTOR inhibition (referred to herein as the Boulay assay). In an embodiment, the assay relies on the measurement of P70 S6 kinase activity from biological samples before and after administration of an mTOR inhibitor, e.g., RAD001. Samples can be taken at preselected times after treatment with an mTOR ihibitor, e.g., 24, 48, and 72 hours after treatment. Biological samples, e.g., from skin or peripheral blood mononuclear cells (PBMCs) can be used. Total protein extracts are prepared from the samples. P70 S6 kinase is isolated from the protein extracts by immunoprecipitation using an antibody that specifically recognizes the P70 S6 kinase. Activity of the isolated P70 S6 kinase can be measured in an in vitro kinase assay. The isolated kinase can be incubated with 40S ribosomal subunit substrates (which is an endogenous substrate of P70 S6 kinase) and gamma-³²P under conditions that allow phosphorylation of the substrate. Then the reaction mixture can be resolved on an SDS-PAGE gel, and ³²P signal analyzed using a PhosphorImager. A ³²P signal corresponding to the size of the 40S ribosomal subunit indicates phosphorylated substrate and the activity of P70 S6 kinase. Increases and decreases in kinase activity can be calculated by quantifying the area and intensity of the ³²P signal of the phosphorylated substrate (e.g., using ImageQuant, Molecular Dynamics), assigning arbitrary unit values to the quantified signal, and comparing the values from after administration with values from before administration or with a reference value. For example, percent inhibition of kinase activity can be calculated with the following formula: 1-(value obtained after administration/value obtained before administration) X 100. As described above, the extent or level of inhibition referred to herein is the average level of inhibition over the dosage interval.

Methods for the evaluation of kinase activity, e.g., P70 S6 kinase activity, are also provided in US 7,727,950, hereby incorporated by reference.

The level of mTOR inhibition can also be evaluated by a change in the ration of PD1 negative to PD1 positive T cells. T cells from peripheral blood can be identified as PD1 negative or positive by art-known methods.

### Low-Dose mTOR Inhibitors

Methods described herein use low, immune enhancing, dose mTOR inhibitors, doses of mTOR inhibitors, e.g., allosteric mTOR inhibitors, including rapalogs such as RAD001. In contrast, levels of inhibitor that fully or near fully inhibit the mTOR pathway are immunosuppressive and are used, e.g., to prevent organ transplant rejection. In addition, high doses of rapalogs that fully inhibit mTOR also inhibit tumor cell growth and are used to treat a variety of cancers (See, e.g., Antineoplastic effects of mammalian target of rapamycine inhibitors. Salvadori M. World J Transplant. 2012 Oct 24;2(5):74-83; Current and Future Treatment Strategies for Patients with Advanced Hepatocellular Carcinoma: Role of mTOR Inhibition. Finn RS. Liver Cancer. 2012 Nov;1(3-4):247-256; Emerging Signaling Pathways in Hepatocellular Carcinoma. Moeini A, Cornellà H, Villanueva A. Liver Cancer. 2012 Sep;1(2):83-93; Targeted cancer therapy - Are the days of systemic chemotherapy numbered? Joo WD, Visintin I, Mor G. Maturitas. 2013 Sep 20.; Role of natural and adaptive immunity in renal cell carcinoma response to VEGFR-TKIs and mTOR inhibitor. Santoni M, Berardi R, Amantini C, Burattini L, Santini D, Santoni G, Cascinu S. Int J Cancer. 2013 Oct 2).

The present invention is based, at least in part, on the surprising finding that doses of mTOR inhibitors well below those used in current clinical settings had a superior effect in increasing an immune response in a subject and increasing the ratio of PD-1 negative T cells/PD-1 positive T cells. It was surprising that low doses of mTOR inhibitors, producing only partial inhibition of mTOR activity, were able to effectively improve immune responses in human human subjects and increase the ratio of PD-1 negative T cells/PD-1 positive T cells.

Alternatively, or in addition, without wishing to be bound by any theory, it is believed that low, a low, immune enhancing, dose of an mTOR inhibitor can increase naive T cell numbers, e.g., at least transiently, e.g., as compared to a non-treated subject. Alternatively or additionally, again while not wishing to be bound by theory, it is believed that treatment with an mTOR inhibitor after a sufficient amount of time or sufficient dosing results in one or more of the following:
an increase in the expression of one or more of the following markers: CD62L^{hign}, CD127^{high}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{high}, increased CD127^{high}, increased CD27⁺, decreased KLRG1, and increased BCL2;
and wherein any of the changes described above occurs, e.g., at least transiently, e.g., as compared to a non-treated subject (Araki, K et al. (2009) Nature 460:108-112). Memory T cell precursors are memory T cells that are early in the differentiation program. For example, memory T cells have one or more of the following characteristics: increased CD62L^{high}, increased CD127^{high}, increased CD27⁺, decreased KLRG1, and/or increased BCL2.

In an embodiment, the invention relates to a composition, or dosage form, of an mTOR inhibitor, e.g., an allosteric mTOR inhibitor, e.g., a rapalog, rapamycin, or RAD001, or a catalytic mTOR inhibitor, which, when administered on a selected dosing regimen, e.g., once daily or once weekly, is associated with: a level of mTOR inhibition that is not associated with complete, or significant immune suppression, but is associated with enhancement of the immune response.

An mTOR inhibitor, e.g., an allosteric mTOR inhibitor, e.g., a rapalog, rapamycin, or RAD001, or a catalytic mTOR inhibitor, can be provided in a sustained relase formulation. Any of the compositions or unit dosage forms described herein can be provided in a sustained release formulation. In some embodiments, a sustained release formulation will have lower bioavailability than an immediate release formulation. E.g., in embodiments, to attain a similar therapeutic effect of an immediate release forlation a sustained release formulation will have from about 2 to about 5, about 2.5 to about 3.5, or about 3 times the amount of inhibitor provided in the immediate release formulation.

In an embodiment, immediate release forms, e.g., of RAD001, typically used for one administration per week, having 0.1 to 20, 0.5 to 10, 2.5 to 7.5, 3 to 6, or about 5, mgs per unit dosage form, are provided. For once per week administrations, these immediate release formulations correspond to sustained release forms, having, respectively, 0.3 to 60, 1.5 to 30, 7.5 to 22.5, 9 to 18, or about 15 mgs of an mTOR inhibitor, e.g., an allosteric mTOR inhibitor, e.g., rapamycin or RAD001. In embodiments both forms are administered on a once/week basis.

In an embodiment, immediate release forms, e.g., of RAD001, typically used for one administration per day, having having 0.005 to 1.5, 0.01 to 1.5, 0.1 to 1.5, 0.2 to 1.5, 0.3 to 1.5, 0.4 to 1.5, 0.5 to 1.5, 0.6 to 1.5, 0.7 to 1.5, 0.8 to 1.5, 1.0 to 1.5, 0.3 to 0.6, or about 0.5 mgs per unit dosage form, are provided. For once per day administrations, these immediate release forms correspond to sustained release forms, having, respectively, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0 to 4.5, 0.9 to 1.8, or about 1.5 mgs of an mTOR inhibitor, e.g., an allosteric mTOR inhibitor, e.g., rapamycin or RAD001. For once per week administrations, these immediate release forms correspond to sustained release forms, having, respectively, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of an mTOR inhibitor, e.g., an allosteric mTOR inhibitor, e.g., rapamycin or RAD001.

In an embodiment, immediate release forms, e.g., of RAD001, typically used for one administration per day, having having 0.01 to 1.0 mgs per unit dosage form, are provided. For once per day administrations, these immediate release forms correspond to sustained release forms, having, respectively, 0.03 to 3 mgs of an mTOR inhibitor, e.g., an allosteric mTOR inhibitor, e.g., rapamycin or RAD001.For once per week administrations, these immediate release forms correspond to sustained release forms, having, respectively, 0.2 to 20 mgs of an mTOR inhibitor, e.g., an allosteric mTOR inhibitor, e.g., rapamycin or RAD001.

In an embodiment, immediate release forms, e.g., of RAD001, typically used for one administration per week, having having 0.5 to 5.0 mgs per unit dosage form, are provided. For once per week administrations, these immediate release forms correspond to sustained release forms, having, respectively, 1.5 to 15 mgs of an mTOR inhibitor, e.g., an allosteric mTOR inhibitor, e.g., rapamycin or RAD001.

As described above, one target of the mTOR pathway is the P70 S6 kinase. Thus, doses of mTOR inhibitors which are useful in the methods and compositions described herein are those which are sufficient to achieve no greater than 80% inhibition of P70 S6 kinase activity relative to the activity of the P70 S6 kinase in the absence of an mTOR inhibitor, e.g., as measured by an assay described herein, e.g., the Boulay assay. In a further aspect, the invention provides an amount of an mTOR inhibitor sufficient to achieve no greater than 38% inhibition of P70 S6 kinase activity relative to P70 S6 kinase activity in the absence of an mTOR inhibitor.

In one aspect the dose of mTOR inhibitor useful in the methods and compositions of the invention is sufficient to achieve, e.g., when administered to a human subject, 90 +/-5 % (i.e., 85-95%), 89+/-5 %, 88+/-5 %, 87+/-5 %, 86+/-5 %, 85+/-5 %, 84+/-5 %, 83+/-5 %, 82+/-5 %, 81+/- 5 %, 80+/-5 %, 79+/-5 %, 78+/-5 %, 77+/-5 %, 76+/-5 %, 75+/-5 %, 74+/-5 %, 73+/-5 %, 72 +/-5%, 71 +/-5%, 70 +/-5%, 69 +/-5%, 68 +/-5%, 67 +/-5%, 66 +/-5%, 65 +/-5%, 64 +/-5%, 63 +/-5%, 62 +/-5%, 61 +/-5%, 60 +/-5%, 59 +/-5%, 58 +/-5%, 57 +/-5%, 56 +/-5%, 55 +/-5%, 54 +/-5%, 54 +/-5%, 53 +/-5%, 52 +/-5%, 51 +/-5%, 50 +/-5%, 49 +/-5%, 48 +/-5%, 47 +/-5%, 46 +/-5%, 45 +/-5%, 44 +/-5%, 43 +/-5%, 42 +/-5%, 41 +/-5%, 40 +/-5%, 39 +/-5%, 38 +/-5%, 37 +/-5%, 36 +/-5%, 35 +/-5%, 34 +/-5%, 33 +/-5%, 32 +/-5%, 31 +/-5%, 30 +/-5%, 29 +/-5%, 28 +/-5%, 27 +/-5%, 26 +/-5%, 25 +/-5%, 24 +/-5%, 23 +/-5%, 22 +/-5%, 21 +/-5%, 20 +/-5%, 19 +/-5%, 18 +/-5%, 17 +/-5%, 16 +/-5%, 15 +/-5%, 14 +/-5%, 13 +/-5%, 12 +/-5%, 11 +/-5%, or 10 +/-5%, inhibition of P70 S6 kinase activity , e.g., as measured by an assay described herein, e.g., the Boulay assay.

P70 S6 kinase activity in a subject may be measured using methods known in the art, such as, for example, according to the methods described in U.S. Pat. 7,727,950, by immunoblot analysis of phosphoP70 S6K levels and/or phosphoP70 S6 levels or by in vitro kinase activity assays.

As used herein, the term "about" in reference to a dose of mTOR inhibitor refers to up to a +/- 10% variability in the amount of mTOR inhibitor, but can include no variability around the stated dose.

In some embodiments, the invention provides methods comprising administering to a subject an mTOR inhibitor, e.g., an allosteric inhibitor, e.g., RAD001, at a dosage within a target trough level. In some embodiments, the trough level is significantly lower than trough levels associated with dosing regimens used in organ transplant and cancer patients. In an embodiment mTOR inhibitor, e.g., RAD001, or rapamycin, is administerd to result in a trough level that is less than ½, 1/4, 1/10, or 1/20 of the trough level that results in immunosuppression or an anticancer effect. In an embodiment mTOR inhibitor, e.g., RAD001, or rapamycin, is administerd to result in a trough level that is less than ½, 1/4, 1/10, or 1/20 of the trough level provided on the FDA approved packaging insert for use in immunosuppression or an anticancer indications.

In an embodiment a method disclosed herein comprises administering to a subject an mTOR inhibitor, e.g., an allosteric inhibitor, e.g., RAD001, at a dosage that provides a target trough level of 0.1 to 10 ng/ml, 0.1 to 5 ng/ml, 0.1 to 3ng/ml, 0.1 to 2 ng/ml, or 0.1 to 1 ng/ml.

In an embodiment a method disclosed herein comprises administering to a subject an mTOR inhibitor, e.g., an allosteric inhibitor, e.g., RAD001, at a dosage that provides a target trough level of 0.2 to 10 ng/ml, 0.2 to 5 ng/ml, 0.2 to 3ng/ml, 0.2 to 2 ng/ml, or 0.2 to 1 ng/ml.

In an embodiment a method disclosed herein comprises administering to a subject an mTOR inhibitor, e.g. an, allosteric inhibitor, e.g., RAD001, at a dosage that provides a target trough level of 0.3 to 10 ng/ml, 0.3 to 5 ng/ml, 0.3 to 3ng/ml, 0.3 to 2 ng/ml, or 0.3 to 1 ng/ml.

In an embodiment a method disclosed herein comprises administering to a subject an mTOR inhibitor, e.g., an allosteric inhibitor, e.g., RAD001, at a dosage that provides a target trough level of 0.4 to 10 ng/ml, 0.4 to 5 ng/ml, 0.4 to 3ng/ml, 0.4 to 2 ng/ml, or 0.4 to 1 ng/ml.

In an embodiment a method disclosed herein comprises administering to a subject an mTOR inhibitor, e.g., an allosteric inhibitor, e.g., RAD001, at a dosage that provides a target trough level of 0.5 to 10 ng/ml, 0.5 to 5 ng/ml, 0.5 to 3ng/ml, 0.5 to 2 ng/ml, or 0.5 to 1 ng/ml.

In an embodiment a method disclosed herein comprises administering to a subject an mTOR inhibitor, e.g., an allosteric inhibitor, e.g., RAD001, at a dosage that provides a target trough level of 1 to 10 ng/ml, 1 to 5 ng/ml, 1 to 3ng/ml, or 1 to 2 ng/ml.

As used herein, the term "trough level" refers to the concentration of a drug in plasma just before the next dose, or the minimum drug conce ntration between two doses.

In some embodiments, a target trough level of RAD001 is in a range of between about 0.1 and 4.9 ng/ml. In an embodiment, the target trough level is below 3ng/ml, e.g., is between 0.3 or less and 3 ng/ml. In an embodiment, the target trough level is below 3ng/ml, e.g., is between 0.3 or less and 1 ng/ml.

In a further aspect, the invention can utilize an mTOR inhibitor other than RAD001 in an amount that is associated with a target trough level that is bioequivalent to the specified target trough level for RAD001. In an embodiment, the target trough level for an mTOR inhibitor other than RAD001, is a level that gives the same level of mTOR inhibition (e.g., as measured by a method described herein, e.g., the inhibition of P70 S6) as does a trough level of RAD001 described herein.

### Pharmaceutical compositions: mTOR Inhibitors

In one aspect, the present invention relates to pharmaceutical compositions comprising an mTOR inhibitor, e.g., an mTOR inhibitor as described herein, formulated for use in combination with CAR cells described herein.

In some embodiments, the mTOR inhibitor is formulated for administration in combination with an additional, e.g., as described herein.

In general, compounds of the invention will be administered in therapeutically effective amounts as described above via any of the usual and acceptable modes known in the art, either singly or in combination with one or more therapeutic agents.

The pharmaceutical formulations may be prepared using conventional dissolution and mixing procedures. For example, the bulk drug substance (e.g., an mTOR inhibitor or stabilized form of the compound (e.g., complex with a cyclodextrin derivative or other known complexation agent) is dissolved in a suitable solvent in the presence of one or more of the excipients described herein. The mTOR inhibitor is typically formulated into pharmaceutical dosage forms to provide an easily controllable dosage of the drug and to give the patient an elegant and easily handleable product.

Compounds of the invention can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Where an mTOR inhibitor is administered in combination with (either simultaneously with or separately from) another agent as described herein, in one aspect, both components can be administered by the same route (e.g., parenterally). Alternatively, another agent may be administered by a different route relative to the mTOR inhibitor. For example, an mTOR inhibitor may be administered orally and the other agent may be administered parenterally.

### SUSTAINED RELEASE

mTOR inhibitors, e.g., allosteric mTOR inhibitors or catalytic mTOR inhibitors, disclosed herein can be provided as pharmaceutical formulations in form of oral solid dosage forms comprising an mTOR inhibitor disclosed herein, e.g., rapamycin or RAD001, which satisfy product stability requirements and/or have favorable pharmacokinetic properties over the immediate release (IR) tablets, such as reduced average plasma peak concentrations, reduced inter- and intra-patient variability in the extent of drug absorption and in the plasma peak concentration, reduced Cₘₐₓ / Cₘᵢₙ ratio and/or reduced food effects. Provided pharmaceutical formulations may allow for more precise dose adjustment and/or reduce frequency of adverse events thus providing safer treatments for patients with an mTOR inhibitor disclosed herein, e.g., rapamycin or RAD001.

In some embodiments, the present disclosure provides stable extended release formulations of an mTOR inhibitor disclosed herein, e.g., rapamycin or RAD001, which are multi-particulate systems and may have functional layers and coatings.

The term "extended release, multi-particulate formulation as used herein refers to a formulation which enables release of an mTOR inhibitor disclosed herein, e.g., rapamycin or RAD001, over an extended period of time e.g. over at least 1, 2, 3, 4, 5 or 6 hours. The extended release formulation may contain matrices and coatings made of special excipients, e.g., as described herein, which are formulated in a manner as to make the active ingredient available over an extended period of time following ingestion.

The term "extended release" can be interchangeably used with the terms "sustained release" (SR) or "prolonged release". The term "extended release" relates to a pharmaceutical formulation that does not release active drug substance immediately after oral dosing but over an extended in accordance with the definition in the pharmacopoeias Ph. Eur. (7^{th} edition) mongraph for tablets and capsules and USP general chapter <1151> for pharmaceutical dosage forms. The term "Immediate Release" (IR) as used herein refers to a pharmaceutical formulation which releases 85% of the active drug substance within less than 60 minutes in accordance with the definition of "Guidance for Industry: "Dissolution Testing of Immediate Release Solid Oral Dosage Forms" (FDA CDER, 1997). In some embodiments, the term "immediate release" means release of everolismus from tablets within the time of 30 minutes, e.g., as measured in the dissolution assay described herein.

Stable extended release formulations of an mTOR inhibitor disclosed herein, e.g., rapamycin or RAD001, can be characterized by an in-vitro release profile using assays known in the art, such as a dissolution assay as described herein: a dissolution vessel filled with 900 mL phosphate buffer pH 6.8 containing sodium dodecyl sulfate 0.2% at 37°C and the dissolution is performed using a paddle method at 75 rpm according to USP by according to USP testing monograph 711, and Ph.Eur. testing monograph 2.9.3. respectively.

In some embodiments, stable extended release formulations of an mTOR inhibitor disclosed herein, e.g., rapamycin or RAD001, release the mTOR inhibitor in the in-vitro release assay according to following release specifications:
0.5h: <45%, or <40, e.g., <30%
1h: 20-80%, e.g., 30-60%
2h: >50%, or >70%, e.g., >75%
3h: >60%, or >65%, e.g., >85%, e.g., >90%.

In some embodiments, stable extended release formulations of an mTOR inhibitor disclosed herein, e.g., rapamycin or RAD001, release 50% of the mTOR inhibitor not earlier than 45, 60, 75, 90, 105 min or 120 min in the in-vitro dissolution assay.

### Biopolymer delivery methods

In some embodiments, one or more CAR-expressing cells as disclosed herein can be administered or delivered to the subject via a biopolymer scaffold, e.g., a biopolymer implant. Biopolymer scaffolds can support or enhance the delivery, expansion, and/or dispersion of the CAR-expressing cells described herein. A biopolymer scaffold comprises a biocompatible (e.g., does not substantially induce an inflammatory or immune response) and/or a biodegradable polymer that can be naturally occurring or synthetic.

Examples of suitable biopolymers include, but are not limited to, agar, agarose, alginate, alginate/calcium phosphate cement (CPC), beta-galactosidase (β-GAL), (1 ,2,3,4,6-pentaacetyl a-D-galactose), cellulose, chitin, chitosan, collagen, elastin, gelatin, hyaluronic acid collagen, hydroxyapatite, poly(3-hydroxybutyrate-co-3-hydroxy-hexanoate) (PHBHHx), poly(lactide), poly(caprolactone) (PCL), poly(lactide-co-glycolide) (PLG), polyethylene oxide (PEO), poly(lactic-co-glycolic acid) (PLGA), polypropylene oxide (PPO), polyvinyl alcohol) (PVA), silk, soy protein, and soy protein isolate, alone or in combination with any other polymer composition, in any concentration and in any ratio. The biopolymer can be augmented or modified with adhesion- or migration-promoting molecules, e.g., collagen-mimetic peptides that bind to the collagen receptor of lymphocytes, and/or stimulatory molecules to enhance the delivery, expansion, or function, e.g., anti-cancer activity, of the cells to be delivered. The biopolymer scaffold can be an injectable, e.g., a gel or a semi-solid, or a solid composition.

In some embodiments, CAR-expressing cells described herein are seeded onto the biopolymer scaffold prior to delivery to the subject. In embodiments, the biopolymer scaffold further comprises one or more additional therapeutic agents described herein (e.g., another CAR-expressing cell, an antibody, or a small molecule) or agents that enhance the activity of a CAR-expressing cell, e.g., incorporated or conjugated to the biopolymers of the scaffold. In embodiments, the biopolymer scaffold is injected, e.g., intratumorally, or surgically implanted at the tumor or within a proximity of the tumor sufficient to mediate an anti-tumor effect. Additional examples of biopolymer compositions and methods for their delivery are described in Stephan et al., Nature Biotechnology, 2015, 33:97-101; and WO2014/110591.

### Pharmaceutical compositions and treatments

Pharmaceutical compositions of the present invention may comprise a CAR-expressing cell, e.g., a plurality of CAR-expressing cells, as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are in one aspect formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-tumor effective amount," "a tumor-inhibiting effective amount," or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the immune effector cells (e.g., T cells, NK cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In certain aspects, it may be desired to administer activated immune effector cells (e.g., T cells, NK cells) to a subject and then subsequently redraw blood (or have an apheresis performed), activate immune effector cells (e.g., T cells, NK cells) therefrom according to the present invention, and reinfuse the patient with these activated and expanded immune effector cells (e.g., T cells, NK cells). This process can be carried out multiple times every few weeks. In certain aspects, immune effector cells (e.g., T cells, NK cells) can be activated from blood draws of from 10cc to 400cc. In certain aspects, immune effector cells (e.g., T cells, NK cells) are activated from blood draws of 20cc, 30cc, 40cc, 50cc, 60cc, 70cc, 80cc, 90cc, or 100cc.

The administration of the subject compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection. In one aspect, the T cell compositions of the present invention are administered by i.v. injection. The compositions of immune effector cells (e.g., T cells, NK cells) may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs of the invention may be introduced, thereby creating a CAR T cell of the invention. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present invention. In an additional aspect, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

In one embodiment, the CAR is introduced into immune effector cells (e.g., T cells, NK cells), e.g., using in vitro transcription, and the subject (e.g., human) receives an initial administration of CAR immune effector cells (e.g., T cells, NK cells) of the invention, and one or more subsequent administrations of the CAR immune effector cells (e.g., T cells, NK cells) of the invention, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR immune effector cells (e.g., T cells, NK cells) of the invention are administered to the subject (e.g., human) per week, e.g., 2, 3, or 4 administrations of the CAR immune effector cells (e.g., T cells, NK cells) of the invention are administered per week. In one embodiment, the subject (e.g., human subject) receives more than one administration of the CAR immune effector cells (e.g., T cells, NK cells) per week (e.g., 2, 3 or 4 administrations per week) (also referred to herein as a cycle), followed by a week of no CAR immune effector cells (e.g., T cells, NK cells) administrations, and then one or more additional administration of the CAR immune effector cells (e.g., T cells, NK cells) (e.g., more than one administration of the CAR immune effector cells (e.g., T cells, NK cells) per week) is administered to the subject. In another embodiment, the subject (e.g., human subject) receives more than one cycle of CAR immune effector cells (e.g., T cells, NK cells), and the time between each cycle is less than 10, 9, 8, 7, 6, 5, 4, or 3 days. In one embodiment, the CAR immune effector cells (e.g., T cells, NK cells) are administered every other day for 3 administrations per week. In one embodiment, the CAR immune effector cells (e.g., T cells, NK cells) of the invention are administered for at least two, three, four, five, six, seven, eight or more weeks.

In one aspect, CAR-expressing cells of the present inventions are generated using lentiviral viral vectors, such as lentivirus. Cells, e.g., CARTs, generated that way will have stable CAR expression.

In one aspect, CAR-expressing cells, e.g., CARTs, are generated using a viral vector such as a gammaretroviral vector, e.g., a gammaretroviral vector described herein. CARTs generated using these vectors can have stable CAR expression.

In one aspect, CARTs transiently express CAR vectors for 4, 5, 6, 7, 8, 9, 10, 11, 12, 13, 14, 15 days after transduction. Transient expression of CARs can be effected by RNA CAR vector delivery. In one aspect, the CAR RNA is transduced into the T cell by electroporation.

A potential issue that can arise in patients being treated using transiently expressing CAR immune effector cells (e.g., T cells, NK cells) (particularly with murine scFv bearing CARTs) is anaphylaxis after multiple treatments.

Without being bound by this theory, it is believed that such an anaphylactic response might be caused by a patient developing humoral anti-CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen day break in exposure to antigen.

If a patient is at high risk of generating an anti-CAR antibody response during the course of transient CAR therapy (such as those generated by RNA transductions), CART infusion breaks should not last more than ten to fourteen days.

### EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

### Treatment of Patient with CLL with CART19

Patient UPCC04409-10 was treated with autologous CART19 T cells for CLL. The treatment led to complete remission of the CLL.

### Analysis of CART cell population

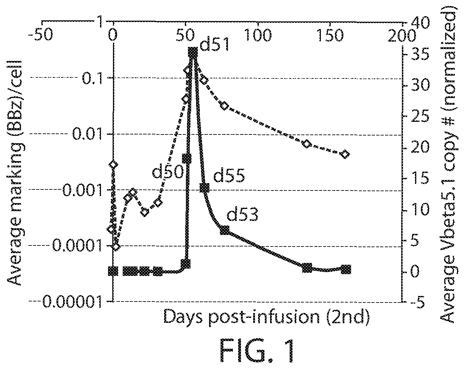

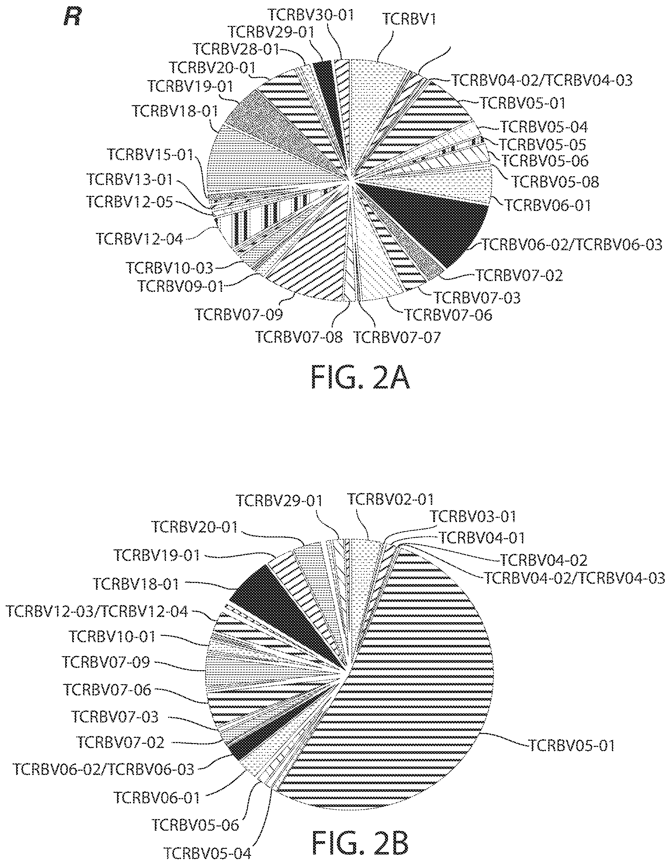

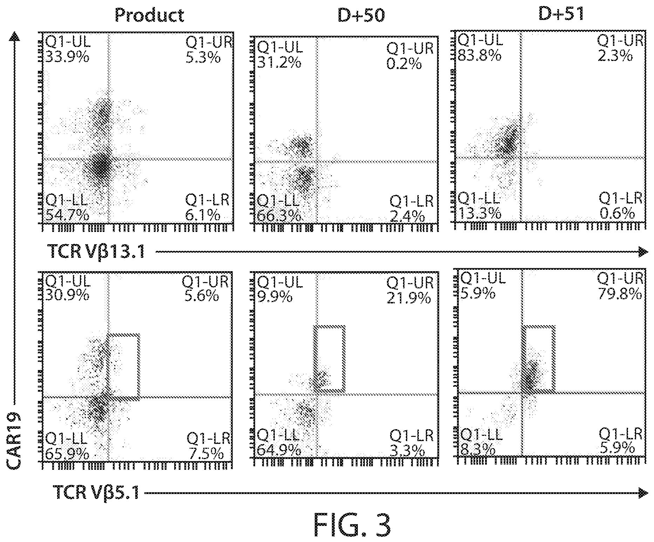

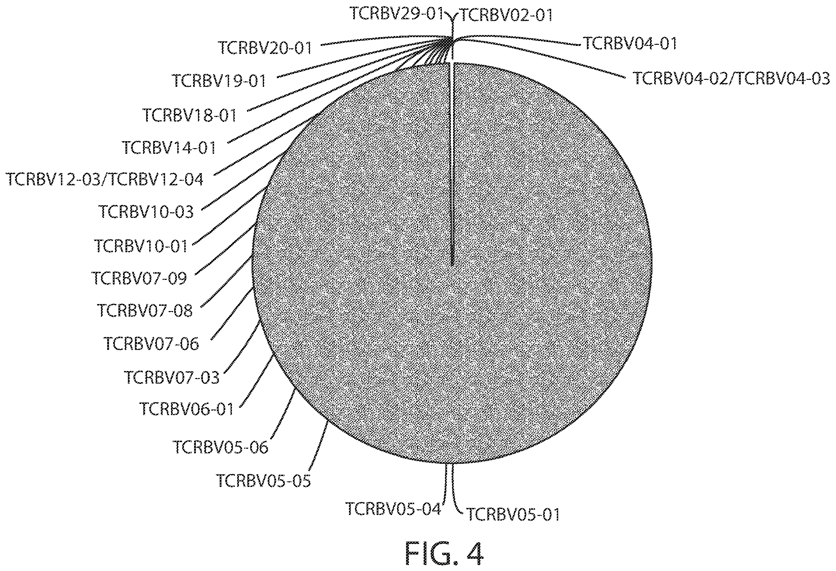

As shown in FIG. 1, CART cells in patient UPCC04409-10 were monitored over time by sampling blood. The amount of BBZ expression in cells was determined (red). The number of copies of sequence from the Vbeta5.1 TCR family was determined (blue). Both measurements were made from samples collected on the indicated days after the second infusion of CART cells. As shown in FIG2, the T-cell receptor repertoire from patient UPCC04409-10 was determined from a sample collected on day 28 (FIG. 2A) or day 51 (FIG. 2B) after CART infusion. This demonstrates the abundance of the TCRBV05-01 family of T-cell receptors at day 51 indicating clonal expansion over time. As shown in FIG. 3, The T-cells isolated from patient UPCC04409-10 were analyzed for the simultaneous expression of CAR19 and 2 different TCR family genes over time (day 50 and day 51) and compared to the input dosed material (product): upper panel is TCR family Vb13.1; the lower panel shows TCR family Vb5.1. The data demonstrate that the CAR19 positive cells contain a single TCR family gene (Vb5.1) that becomes rapidly enriched between days 50 and 51. As shown in FIG. 4, the T-cell receptor repertoire of CD8 positive cells from patient UPCC04409-10 was determined from a sample collected on day 51 after CART infusion. This demonstrates the abundance of the TCRBV05-01 family of T-cell receptors at day 51 indicating clonal expansion of CD8 positive cells over time.

### Analysis of Persisting CART Clone

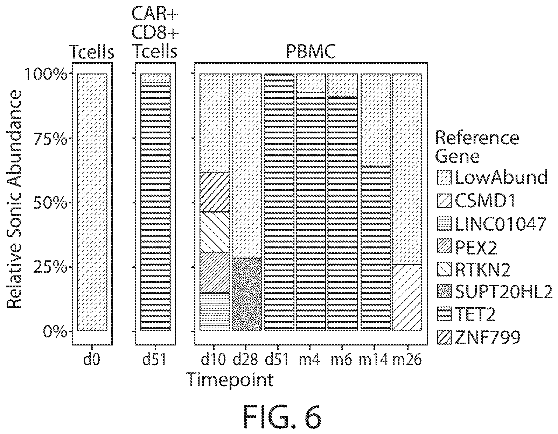

As shown in FIG. 6, sonically fragmented DNA was generated from T-cells from Patient #. This material was used to amplify genomic sequences adjacent to the CAR19 insertion. The genes indicated were identified as being enriched relative to the infused product (D0) adjacent to CAR19 in the genome. At the different time points after CART infusion indicated (d=day; m=month), a different relative abundance of adjacent genes was seen, with Tet2 abundance peaking in both peripheral blood (PBMC) and CAR+CD8+ T-cells samples at day 51.

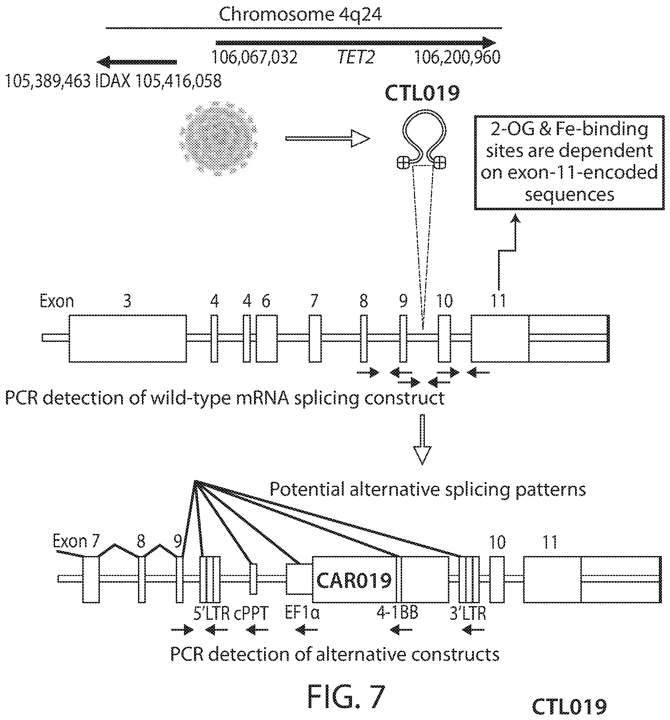

As shown in FIG. 7, the site of insertion of the CAR19 gene was mapped to the Tet2 gene. More specifically, the insertion occurred between exons 9 and 10 of the Tet2 gene. The catalytic domain for Tet2 resides in exon 11. The insertion at this location may lead to expression of aberrant mRNA transcripts or decrease the expression of functional (wild-type) Tet2.

As shown in FIG. 8, transcripts of the Tet2 gene from mRNA isolated from patient UPCC04409-10 were evaluated by RTPCR using primers spanning the indicated regions of Tet2 or CAR19 or both as indicated in the right hand side of the figure. Rxn 3 contains primers designed to amplify the region of the Tet2 transcript spanning exons 9 and 10. Rxn, 6, 7, 8, 9, and 10 are primers designed to amplify the indicated portions of the CAR19 lentivirus. Rxn 12-16 are pairs of primers that contain exon 9 sequence of the Tet2 transcript as well as sequence from the CAR19 lentiviral construct. These data show that transcripts are made from the Tet2 locus that contains both Tet2 sequence as well as CAR19 sequence.

### Analysis of Tet2 Function

As shown in FIG. 10, the enzymatic activity of Tet2 is schematized (FIG. 10A). Tet family protein convert 5-methylcytosine (5-mc) to 5-hydroxymethylcytosine (5-hmc) and then into 5-formylcytosine (5-fmc) resulting in demethylated cytosine. Methylated DNA is an epigenetic state that is known to affect transcriptional profiles. The methylation state of the T-cells from patient UPCC04409-10 was evaluated (FIG. 10B). The patient's T-cells were stained for TCRVb5.1 (which contain the CAR19 insertion at Tet2) and the 5-hmc and 5-fmc were evaluated in TCRVb5.1 positive (red) and TCRVb5.1 negative (blue) populations by flow cytometry. This data indicates that the cells containing the insertion of CAR19 in the Tet2 gene are defective in demethylation.

### Treatment of T cells with shRNA Tet2 Inhibitors

### Materials and methods

### Lenti-viral preparation and infection to the Jurkat cells

Lenti-viruses were prepared from 15cm 293T cells. Briefly, 10 million 293T cells were seeded onto collagen coated 15cm dishes at day -1. At day 0, 15ug shRNA vector (i.e., vector comprising sequence encoding the TET2-targeting or control shRNA), 15ug Gag/pol vector, and 5ug VSV-G vector were transfected using Lipofectamin 2000 (Invitrogen). 24 hours later (day 1), media was changed. After changing media, viral supernants were harvested at day 2 and day 3. Viruses were concentrated with Lenti-X concentrator (3:1 volume ratio, Clonetech, Cat#: 631231). 100ul of viruses were added into either 0.5 million jurkat cells in the presence of 6 ug/ml of polybrene. The cells were spin-infected at 2000 rpm, 90 min at 32°C. After 1 hour incubation at 37 degree incubator, fresh RPMI 1640 media were added and transferred into 24-well plate. At day 6, cells were transferred into 6-well plate in the presence of final concentration 2 ug/ml of puromycin for 6 days.

### Antibodies

Antibodies used for western blotting were as follows: β-actin (clone#: 8H10D10, Cell Signaling); TET2 (clone#: hT2H21F11, Millipore); mouse IgG(H+L) (HRP conjugated, Cat#: 115-035-166, Jackson ImmunoResearch); rabbit IgG(H+L) (HRP conjugated, Cat#: 111-035-114, Jackson ImmunoResearch).

### Western blotting

To examine TET2 shRNA knockdown efficiency at protein level in jurkat cells, cell lysates were prepared in protease inhibitor cocktails (Sigma) containing RIPA buffer. Protein concentration was measured by BCA protein assay kits (Pierce, Item#: 3603904). 20ug of protein was subjected to SDS-PAGE followed by transferring protein onto nitrocellulose membrane using iBot transfer system (Invitrogen, 20V, 11min 30sec). The membrane was blocked in 5% BSA containing TBST for 30min at room temperature. Antibodies were overnight incubated with membranes at 1:000 dilution at 4°C. After incubation with HRP conjugated secondary antibodies, signal was detected using chemiluminescence detection machine (Chemidoc; Bio-Rad).

### Quantitative RT-PCR

To examine TET2 shRNA knock-down efficiency at DNA level in jurkat cells, quantitative reverse transcription polymerase chain reaction (qRT-PCR) was performed. A RNeasy Micro Kit (Qiagen) was used to extract RNA. mRNA was reverse transcribed to single-strand complementary DNA (cDNA) with SuperScript III First-Strand Synthesis System for RT-PCR (Invitrogen). Real-time PCR was performed with C1000 Touch Thermal Cycler (Biorad). A SYBR-based protocol was used to detect gene expression (SsoAdvanced Universal SYBR Green Supermix, Biorad). The PCR reactions were done in 96-well plates and run using the manufacture's recommended cycling parameters with triplicate (95°C for 3 minutes, followed by 40 cycles of 95°C for 15 seconds and 60°C for 30 seconds). Cycle threshold (Ct) values for the genes of interest were normalized to the Ct for β-actin. Primers used for qRT-PCR were as follows: β-actin #1 (fowrard primer: CAT GTA CGT TGC TAT CCA GGC (SEQ ID NO: 1263), reverse primer: CTC CTT AAT GTC ACG CAC GAT (SEQ ID NO: 1264); product size 250 bp); β-actin #2 (fowrard primer: CTC ACC ATG GAT GAT GAT ATC GC (SEQ ID NO: 1265), reverse primer: CCA CAT AGG AAT CCT TCT GAC CC (SEQ ID NO: 1266); product size 169 bp); TET1 (forward primer: CAG AAC CTA AAC CAC CCG TG (SEQ ID NO: 1267), reverse primer: TGC TTC GTA GCG CCA TTG TAA (SEQ ID NO: 1268); product size 141 bp); TET2 (forward primer: ATA CCC TGT ATG AAG GGA AGC C (SEQ ID NO: 1269), reverse primer: CTT ACC CCG AAG TTA CGT CTT TC (SEQ ID NO: 1270); product size 197 bp); TET3 (forward primer: CAC CCG GCT CTA TGA AAC CTT (SEQ ID NO: 1271), reverse primer: CCA GCC ACT CGA GGT AGT CA (SEQ ID NO: 1272); product size 209 bp); RPLP1 (Cat#: PPH17813G-200, Qiagen).

### Flow cytometry

The cells were acquired on a FACS Fortessa (BD). Data processing for presentation was done using FlowJo (Treestar Inc.) program.

### Results

### Validation of Knockdown Efficiency of Tet2 shRNAs

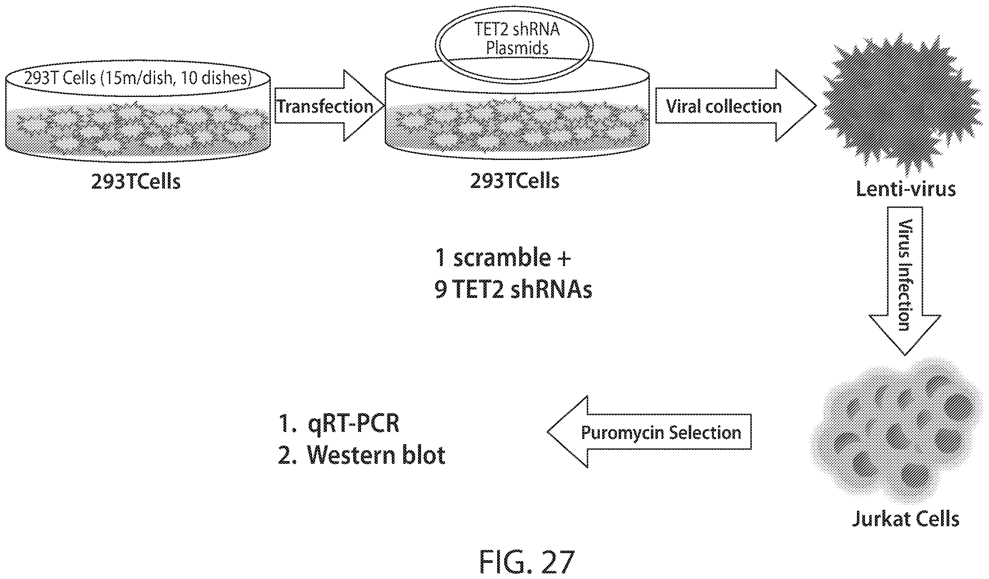

As shown in FIG. 27, the validation of knockdown efficiency of TET2 shRNAs is schematized. TET2 and scramble control shRNA constructs expressing Red Fluorescence Protein (RPF) and puromycin resistant gene were introduced into jurkat cells to validate knockdown efficiency of TET2 shRNAs by qRT-PCR and western blot experiments.

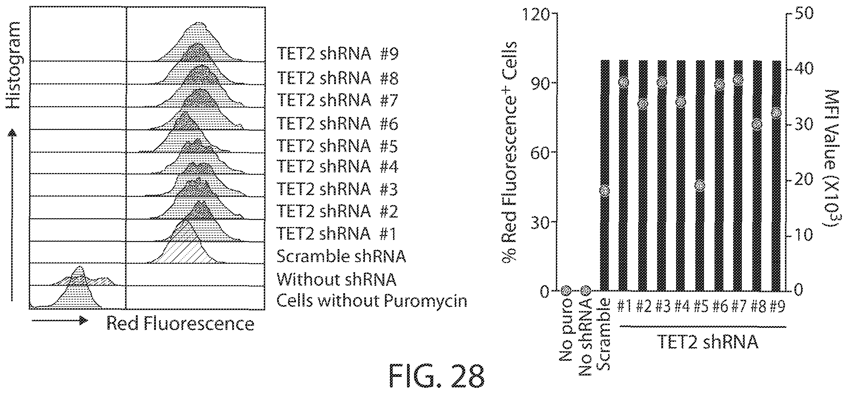

As shown in FIG. 28, shRNA infected jurkat cells express RFP. RFP expression was determined by FACS on day 6 after puromycin treatment. Based on RFP expression, greater than 99% shRNA introduced jurkat cells were selected by puromycin treatment. Of note, TET2 shRNA #3 and #4 infected jurkat cells did not grow in the presence of puromycin. Therefore, TET2 shRNA #3 and #4 infected jurkat cells were not processed further. This data indicates that puromycin is effective to select shRNA infected jurkat cells.

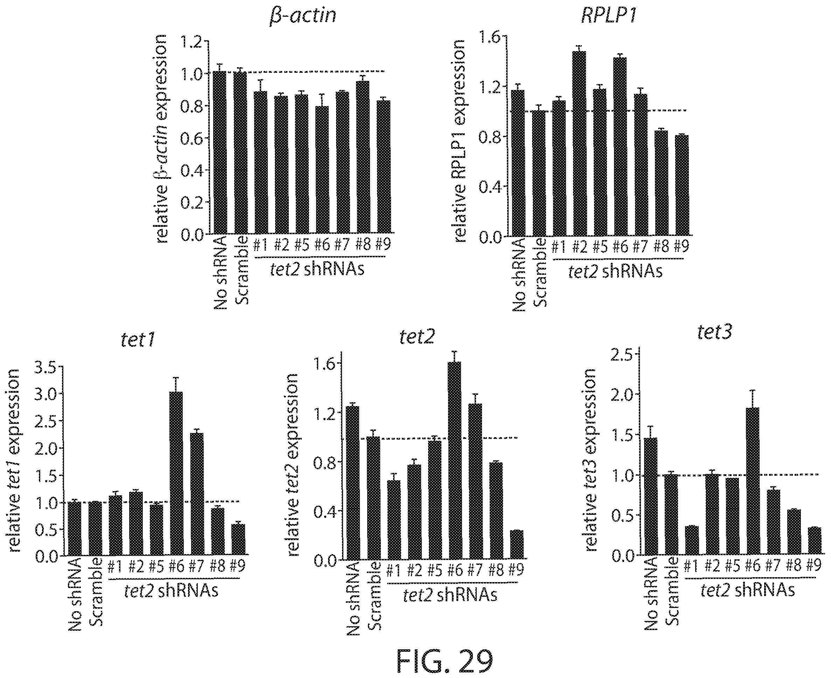

As shown in FIG. 29, knockdown efficiency of *tet2* depends on shRNAs. To determine mRNA expression level of *tet1* and *tet2* and *tet3* in TET2 shRNAs infected jurkat cells, qRT-PCR experiment was performed. Compared to scramble shRNA, TET2 shRNA #1, #2, #8, and #9 knockdown *tet2* gene at 35.6%, 22.7%, 21.6%, and 76.7% respectively. Surprisingly, while TET2 shRNA #9 knocks down *tet2* efficiently, it also down-regulates *tet1* and *tet3* expression at 43.4% and 67.3% respectively. β-actin serves as an internal control to quantify relative gene expression among samples tested. To increase reliability of qRT-PCR, two β-actin primers and one RPLP1 primer were used in this experiment. This data indicates that several TET2-targeting shRNA are capable of reducing mRNA levels of TET2, with shRNA#9 showing the most robust knockdown effect of *tet2,* while also affecting levels of TET1 and TET2.

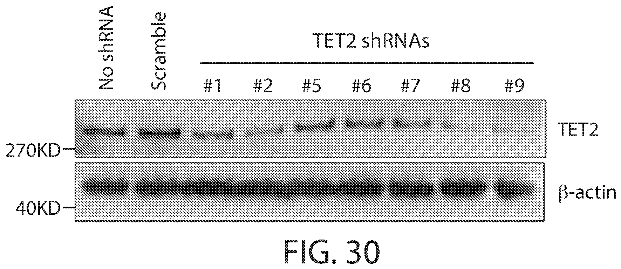

As shown in FIG. 30, knockdown of TET2 protein in response to shRNAs correlates with knockdown of TET2 mRNA levels. To determine protein expression level of TET2 in TET2 shRNAs infected jurkat cells, a western blot experiment was performed. Similar to qRT-PCR data as shown in FIG.29, TET2 shRNA #1, #2, #8, and #9 reduce TET2 protein level compared to scramble shRNA, while β-actin is constitutively expressed in all samples tested. This data indicates that several TET2-targeting shRNA are capable of reducing TET2 protein levels in Jurkat cells, with shRNA#9 showing the most robust knockdown effect of Tet2.

### Treatment of primary T cells with shRNA Tet2 Inhibitors

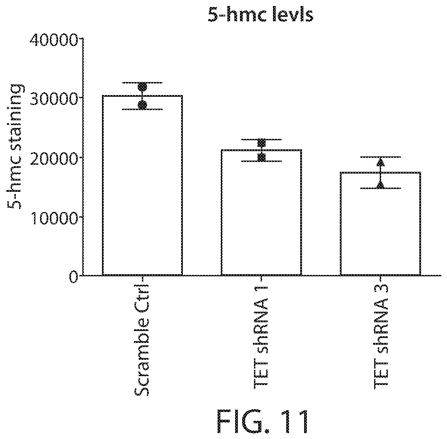

As shown in FIG. 11, TET2 shRNAs reduce 5-hmc levels in normal human T cells. TET2 and scramble control shRNA constructs expressing mCherry were introduced into normal human T cells. 5-hmc levels were determined by intracellular staining by FACS on day 6 following expansion with anti-CD3/CD28 beads. Knockdown of TET2 reduced overall 5-hmc levels.

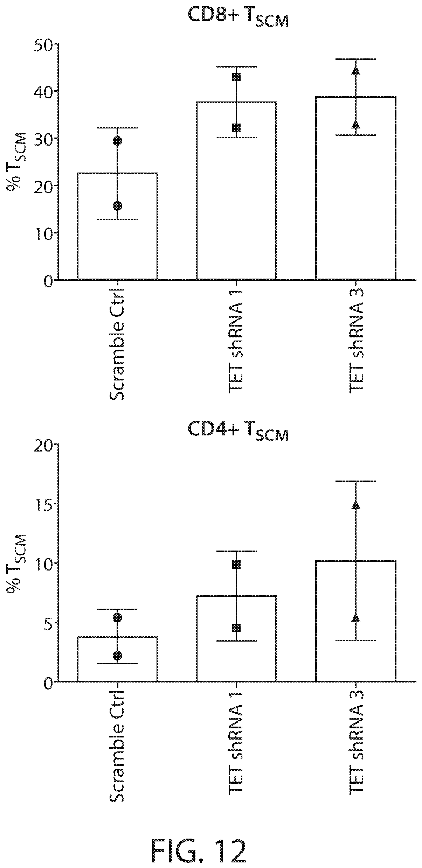

As shown in FIG. 12, TET2 shRNAs expand Tscm T cells. TET2 and scramble control shRNA constructs expressing mCherry were introduced into normal human T cells. CD45RA+CD62L+CCR7+CD27+CD95+ Tscm T cells were determined by FACS staining on day 11 following expansion with anti-CD3/CD28 beads. Knockdown of TET2 promoted the expansion of T cells with a Tscm phenotype.

### Tet2 Inhibition in CAR T Cells Using CRISPR/Cas Gene Editing Systems

In this example, inhibiton of TET2 was explored in chimeric antigen receptor (CAR)-expressing T cells.

### Methods

### Guide RNA molecules

gRNA molecules comprising the targeting sequences listed in Table 5 were used for the experiments described in this subexample. Unless otherwise indicated, all gRNA molecules were tested as dual gRNA molecules comprising the tracr and crRNA sequences described in this subexample.

**Table 5**

| **Target Region** | **TET2 gRNA targeting sequence** | **guide reference** |
|---|---|---|
| Exon 9 | CAGAGCACCAGAGUGCCGUC (SEQ ID NO: 1273) | 9_1 (also referred to as Tet2_E9_1_Tet2) |
| Exon 9 | AGAGCACCAGAGUGCCGUCU (SEQ ID NO: 1274) | 9_2 (also referred to as Tet2_E9_2_Tet2) |
| Exon 9 | UUCAGACCCAGACGGCACUC (SEQ ID NO: 1275) | 9_3 (also referred to as Tet2_E9_3_Tet2) |
| Exon 9 | AUGGCAGCACAUUGGUAAGU (SEQ ID NO: 1276) | 9_4 (also referred to as Tet2_E9_4_Tet2) |
| Exon 9 | CACAUUGGUAAGUUGGGCUG (SEQ ID NO: 1277) | 9_5 (also referred to as Tet2_E9_5_Tet2) |
| Exon 9 | GACUUGCACAACAUGCAGAA (SEQ ID NO: 1278) | 9_6 (also referred to as Tet2_E9_5_Tet2, Ex9-3 or crEx9-3) |
| exon 7 | UCAUGGAGCAUGUACUACAA (SEQ ID NO: 1279) | 7_1 |
| exon 7 | AACUUGCGCCUGUCAGGGGC (SEQ ID NO: 1280) | 7_2 |
| exon 7 | CCAAGGAAGUUUAAGCUGCU (SEQ ID NO: 1281) | 7_3 |
| exon 7 | CCAAGCAGCUUAAACUUCCU (SEQ ID NO: 1282) | 7_4 |
| exon 8 | UUGGUGCCAUAAGAGUGGAC (SEQ ID NO: 1283) | 8_1 |
| exon 8 | GCAAAACCUGUCCACUCUUA (SEQ ID NO: 1284) | 8_2 |
| exon 8 | AUAUGUUGGUGCCAUAAGAG (SEQ ID NO: 1285) | 8_3 |
| exon 10 | AAAACGGAGUGGUGCCAUUC (SEQ ID NO: 1286) | 10_1 |
| exon 10 | GUCUCUGACGUGGAUGAGUU (SEQ ID NO: 1287) | 10_2 |
| exon 10 | UUUAUACAA AGUCUCUGACG (SEQ ID NO: 1288) | 10_3 |
| exon 10 | AGAGAAGACAAtjCGAGAAUU (SEQ ID NO: 1289) | 10_4 |
| exon 10 | ACGUCAGAGACUUUGUAUAA (SEQ ID NO: 1290) | 10_5 |
| exon 3 | GGAUAGAACCAACCAUGUUG (SEQ ID NO: 1291) | 3_1 (also referred to as Tet2_E3_1_Tet2) |
| exon 3 | UUGUAGCCAGAGGUUCUGUC (SEQ ID NO: 1292) | 3_2 (also referred to as Tet2_E3_2_Tet2) |
| exon 3 | UCUGUUGCCCUCAACAUGGU (SEQ ID NO: 1293) | 3_3 (also referred to as Tet2_E3_3_Tet2, Ex3-3 or crEx3-3) |
| exon 3 | GAUAGAACCAACCAUGUUGA (SEQ ID NO: 1294) | 3_4 (also referred to as Tet2_E3_4_Tet2) |
| exon 3 | UUCUGGAGCUUUGUAGCCAG (SEQ ID NO: 1295) | 3_5 (also referred to as Tet2_E3_5_Tet2) |

### Generation of CRISPR CAR T cells

Isolated and frozen Pan T cells were thawed and activated with CD3/CD28 beads (CD3/CD28 CTS Dynabeads^{®} 43205D) on day 0. Activated T cells were transduced with lentivirus encoding either a BCMA CAR (BCMA-10 (139109) as described in WO2016/0046724; referred to herein as BCMA-10 CAR) or CD19 CAR (the CD19 CAR having the amino acid sequence of SEQ ID NO: 12 of WO2012/079000; referred to herein as CD19 CAR) on day 1. On day 3, transduced CAR T cells were electroporated to introduce CRISPR/Cas systems in the form of pre-complexed gRNA/Cas9 ribonuclear protein ("RNP"). To form RNP, all RNA samples were heated at 95C. *S. pyogenes* CAS9 Protein (NLS CAS9 iPROT106154, 37µM) was diluted in buffer before tracrRNA (having the sequence: AACAGCAUAGCAAGUUAAAAUAAGGCUAGUCCGUUAUCAACUUGAAAAAGUGGC ACCGAGUCGGUGUUUUUUU (SEQ ID NO: 1296); AXO Labs) was added to it. After mixing CAS9 Protein with tracrRNA, the CRISPR RNA was added (in each case, each crRNA comprised the sequence nnnnnnnnnnnnnnnnnnnnGUUUUAGAGCUAUGCUGUUUUG (SEQ ID NO: 40), where the n residues represent the 20 ribonucleic acid residues of the indicated targeting sequence). The precomplexed RNPs were then added to a total of 1 million cells, RNP concentration was 3.2µM. Electroporation was done by neon electroporator using *Neon*^{®} *Transfection System 100 µL Kit (MPK10096)* at 1600V, 10ms, 3 pulses. The cells were kept in culture for 7 more days. Cells were then divided, with some used to perform flow cytometry: Staining for CAR (PE), CD3 (PerCP-Cy5.5), CD4 (V450) and CD8 (APC). Remaining T cells were frozen and used for functional assays and next generation sequencing (NGS) sample generation.

### Next Generation Sequencing (NGS) sample generation

Frozen edited cell pellets (described above) were thawed and processed using *DNeasy Blood & Tissue Kit (Qiagen, 69506)* to isolate genomic DNA. Eluted DNA was used to run PCR using *Titanium Taq PCR kit (Clontech Laboratories, 639211)* and TET2 primers (primers designed to flank the expected target site of the gRNA). PCR product was purified using *QIAquick PCR Purification Kit (Qiagen, 28104).* Purified PCR product was then used for T7E1 assay to detect base pair mismatches and confirm gene editing. PCR amplicons were subjected to standard Nextera NGS library prep (Illumina) and sequenced with paired-end reads on an Illumina MiSeq sequencer. Sequencing reads were aligned to the reference genome and variants were called.

### Cytokine Production assay

Effector cells (CAR T cells) are thawed on the day of the assay and counted on Cellometer (Nexelcom). These cells are then co cultured with different target cells at Effector:Target cell ratio of 2:1. 100ul of co-culture supernatant is harvested after 20h. These supernatants are then used measure the cytokines IL-2 and IFN-g released using Meso Scale Discovery, Proinflammatory Panel 1 catalog # N05049A-1 system according to the manufacturer's protocol.

### T cell Proliferation Assay

CAR T cell proliferation in response to BCMA- or CD19-expressing target cells was evaluated. Target cell lines were: BCMA positive multiple myeloma cell lines, NCI-H929-luc, KMS-11-luc, and BCMA-negative Nalm6luc (CD19-positive cell line). CAR T cells were thawed incubated for 2 hours in T cell medium to recover. Cells were counted on a Cellometer. Target cells were irradiated at 10,000 rad. After irradiation, target cells were washed twice in complete T cell medium and counted. 30,000 Irradiated target cells were then co cultured with CART cells at 1:1 ratio. As a negative control, medium alone was added to CAR T cells.

The co-culture was incubated for 4 days at 37°C. On Day 4, coculture cells were stained for 20 mins on ice with CD3-percp cy5.5(Ebioscience:45-0037), CD4-eflor450(Ebioscience:48-0047), and CD8-APC(Ebioscience 17-0087and measured by flow cytometry relative to CountBright Absolute Counting Beads (Life Technologies) to determine relative cell counts. ). CAR expression was measured by two step incubation of 20 mins each on ice: Biotinylated-Protein L + Streptavidin-PE(Jackson immuno research). Flow cytometry data was acquired using BD 5 laser Fortessa and analyzed by FlowJo software.

### Results

FIGS. 13A and 13B show CAR expression in cells electroporated with and without Tet2 CRISPR. FIG. 13A shows the gating strategy for determining CAR+ T cells. Lymphocytes were selected using forward scatter (FSC-A) and side scatter (SSC-A) as encircled. CD3-expressing cells were then selected (middle panel). CAR positive (PE positive cells using CAR detection by biotinylated protein and streptavitin-PE) cells indicated by the bar were then determined by gating relative to the negative control peak. FIG. 13B shows the quantitation of the percentage of CAR positive cells. Cells were transduced with either the BCMA-10 CAR of the CD19 CAR as indicated. Cells were electroporated with RNP containing Cas9 protein, tracer RNA, and the indicated guide RNA targeting Tet2 (Ex3-3 targeting exon 3 or Ex9-6 targeting exon 9), or with no electroporation. CAR expression was determined 10 days after cell activation with beads. These data indicate that editing of Tet2 does not impact CAR expression in T cells.

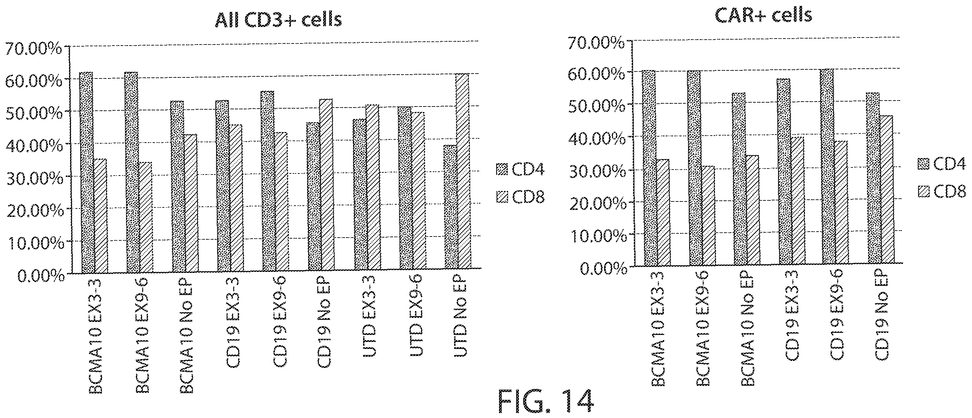

FIG. 14 shows quantitation of CD4 and CD8 positive cells after CAR transduction and Tet2 editing. Cells were stained for CD3, CD4, CD8, and CAR expression at the end of the 10 day bead expansion. The left panel indicates the percentage of CD4 and CD8 positive cells in the total population of CD3+ cells. The right panel indicates the percentage of CD4 and CD8 positive cells in the population of CD3+ cells that are also CAR+. Cells were transduced with either the BCMA-10 CAR of the CD19 CAR or left untransduced (UTD) as indicated. Cells were electroporated with RNP containing Cas9 protein, tracer RNA, and the indicated guide RNA targeting Tet2 (Ex3-3 targeting exon 3 and Ex9-6 targeting exon 9), or with no electroporation. These data indicated that editing of Tet2 causes a small but consistent decrease in the percentage of CD8 cells and increase in the percentage of CD4 during the window of time of the bead-based expansion process.

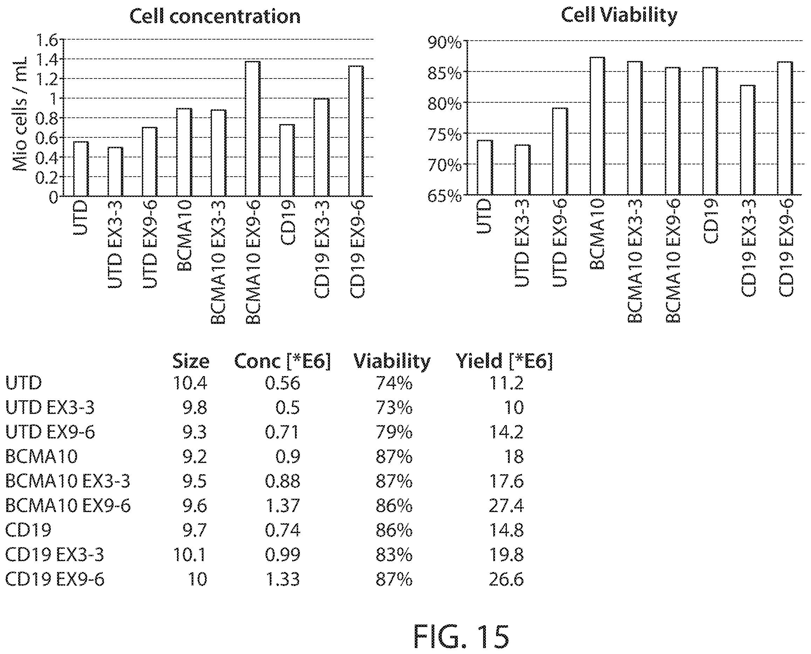

FIG. 15 shows cell yield and viability after bead expansion for 10 days. The number of cells per mL (left panel) and the viability of cells (right panel) were measured by Cellometer after the 10 day bead expansion process. Cells were transduced with either the BCMA-10 CAR of the CD19 CAR or left untransduced (UTD) as indicated. Cells were electroporated with RNP containing Cas9 protein, tracer RNA, and the indicated guide RNA targeting Tet2 (Ex3-3 targeting exon 3 and Ex9-6 targeting exon 9), or with no electroporation. These data indicate that Tet2 editing causes an increase in cell number and viability relative to cells with no CRISPR/Cas9, with the Exon 9 targeting guide (ex9-6) showing the greatest impact in untransduced as well as CAR transduced cells.

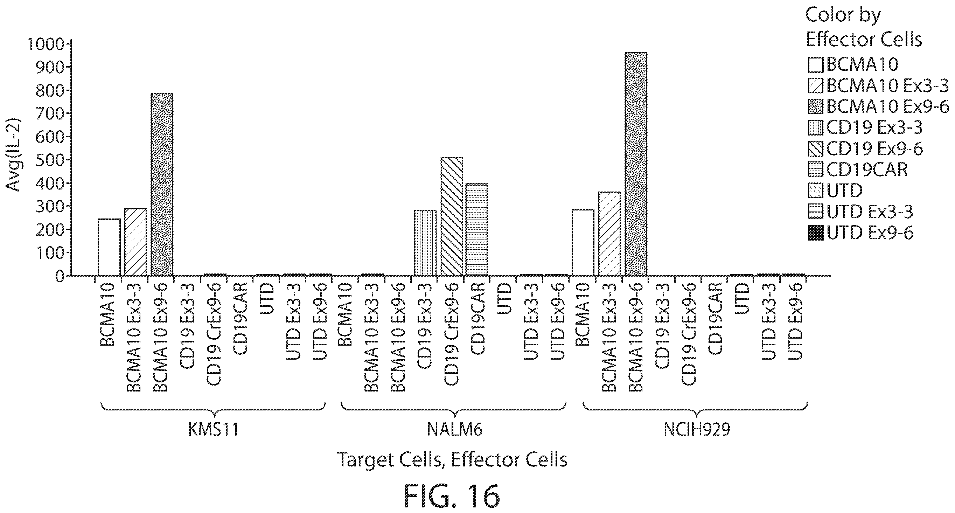

FIG. 16 shows IL-2 production in response to cells either positive or negative for the antigen recognized by the CAR. CART cells or untransduced cells (UTD) were co-cultured with BCMA-positive/ CD19-negative cells (KMS11 and NCIH929) or BCMA-negative/CD19-positive cells (NALM6) and cytokine secretion into the media was measured. IL-2 (pg/ml) levels are shown. Cells were prepared as described above. These data indicated that Tet2 editing causes an increase in IL-2 production by T cells in response to antigen with the Exon 9 targeting guide (Ex9-6) showing the greatest effect.

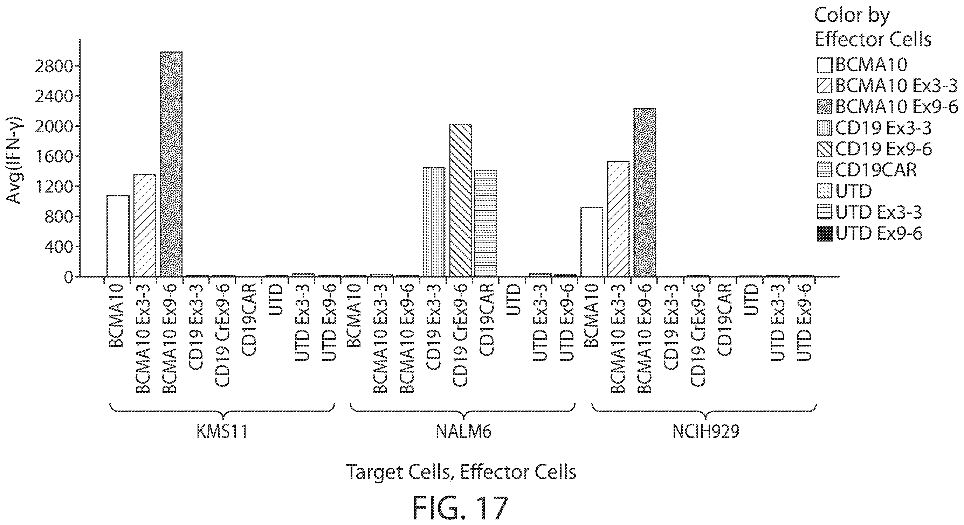

FIG. 17 shows interferon gamma production. CART cells or untransduced cells (UTD) were co-cultured with BCMA-positive/ CD19-negative cells (KMS11 and NCIH929) or BCMA-negative/ CD19-positive cells (NALM6) and cytokine secretion into the media was measured. Interferon gamma (IFN-g) (pg/ml) levels are shown. Cells were prepared as described above. These data indicated that Tet2 editing causes an increase in Interferon gamma production by T cells in response to antigen, with the Exon 9 targeting guide (Ex9-6) showing the greatest effect.

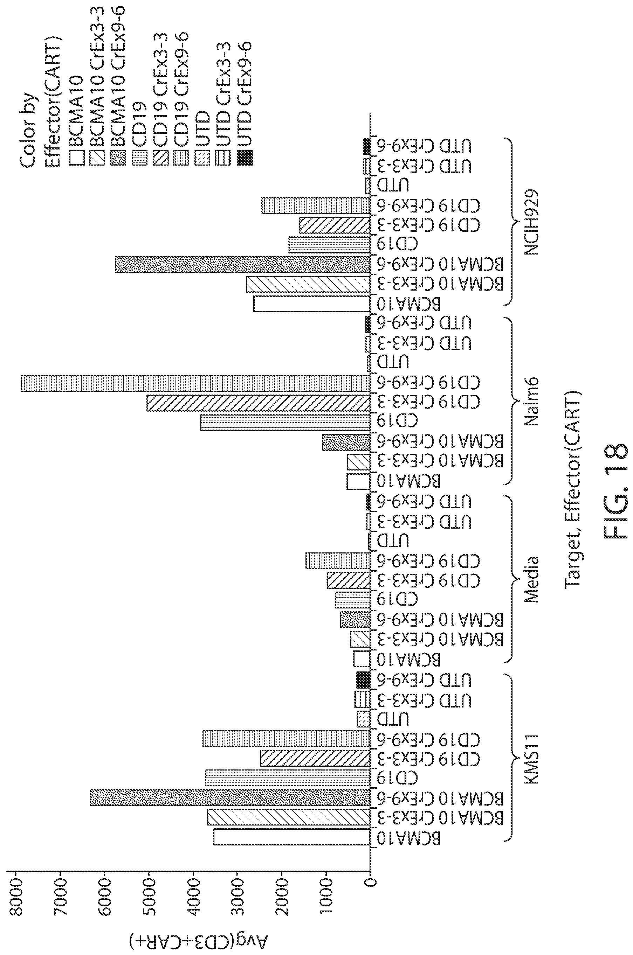

FIG. 18 shows antigen-driven CAR-T cell proliferation. CART cells or untransduced cells (UTD) were co-cultured with BCMA-positive/ CD19-negative cells (KMS11 and NCIH929) or BCMA-negative/CD19-positive cells (NALM6) or no target cells (media) and proliferation of CAR positive T cells was measured. Cells were prepared as described above. These data indicated that Tet2 editing causes an increase in CAR+ T cell proliferation in response to antigen, with the Exon 9 targeting guide (CrEx9-6) showing the greatest effect.

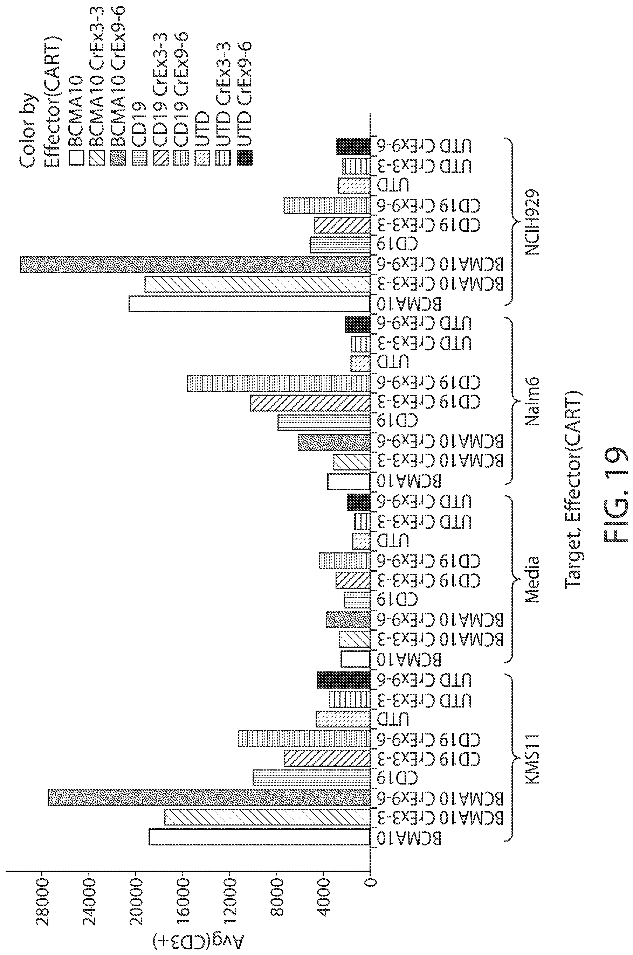

FIG. 19 shows antigen-driven total T cell proliferation. CART cells or untransduced cells (UTD) were co-cultured with BCMA-positive/CD19-negative cells (KMS11 and NCIH929) or BCMA-negative/CD19-positive cells (NALM6) or no target cells (media) and proliferation of all CD3+ T cells was measured. Cells were prepared as described above. These data indicated that Tet2 editing causes an increase in CD3+ T cell proliferation in response to antigen, with the Exon 9 targeting guide (CrEx9-6) showing the greatest effect.

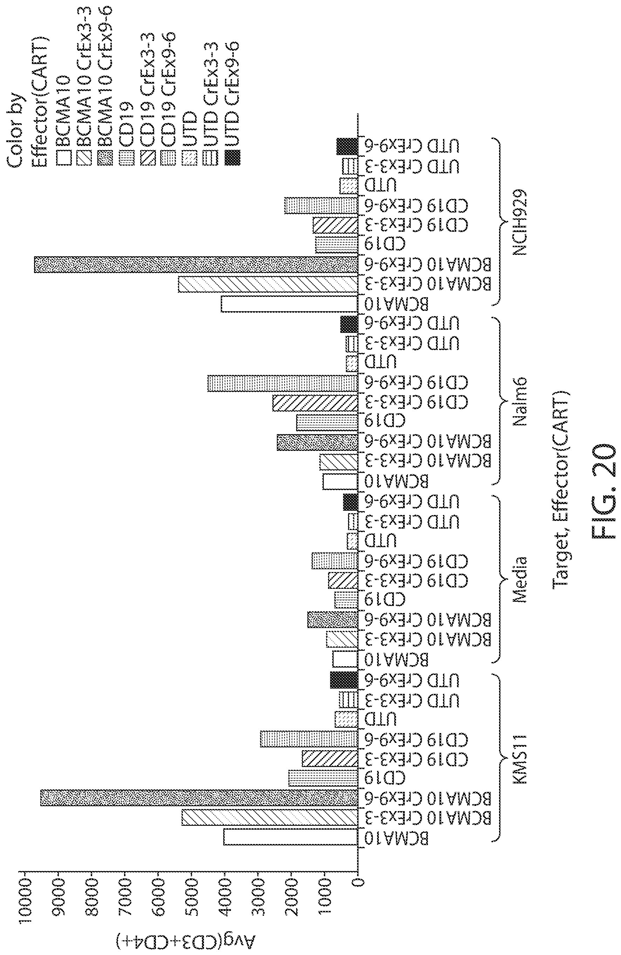

FIG. 20 shows antigen-driven CAR+ T cell proliferation. CART cells or untransduced cells (UTD) were co-cultured with BCMA-positive/CD19-negative cells (KMS11 and NCIH929) or BCMA-negative/CD19-positive cells (NALM6) or no target cells (media) and proliferation of all CD4+ CD3+ T cells was measured. Cells were prepared as described above. These data indicated that Tet2 editing causes an increase in CD4+ T cell proliferation in response to antigen, with the Exon 9 targeting guide (CrEx9-6) showing the greatest effect.

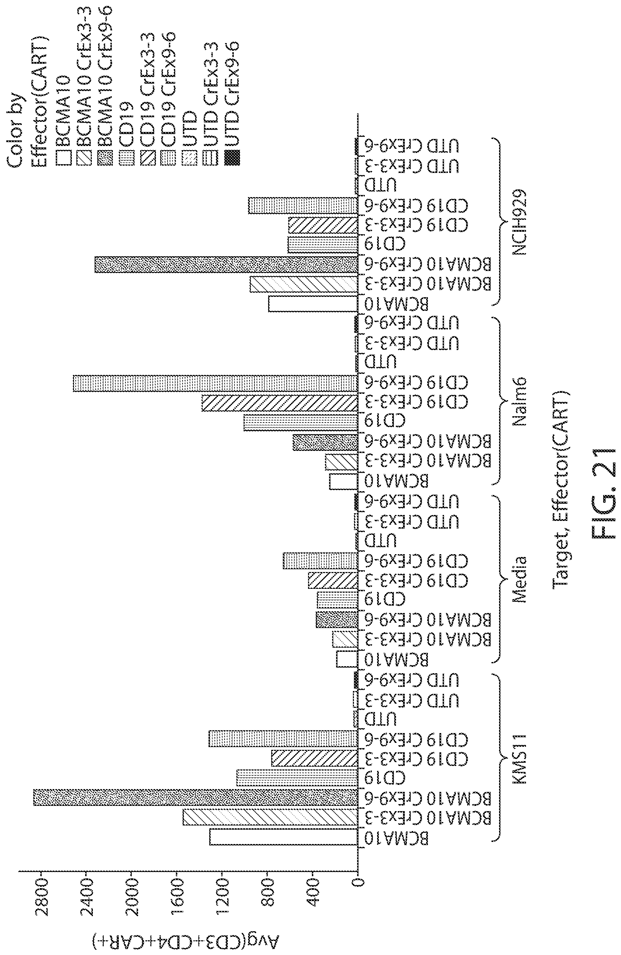

FIG. 21 shows antigen-driven CAR+ CD4+ T cell proliferation. CART cells or untransduced cells (UTD) were co-cultured with BCMA-positive/CD19-negative cells (KMS11 and NCIH929) or BCMA-negative/CD19-positive cells (NALM6) or no target cells (media) and proliferation of all CAR+CD4+ CD3+ T cells was measured. Cells were prepared as described above. These data indicated that Tet2 editing causes an increase in CAR+CD4+ T cell proliferation in response to antigen, with the Exon 9 targeting guide (CrEx9-6) showing the greatest effect.

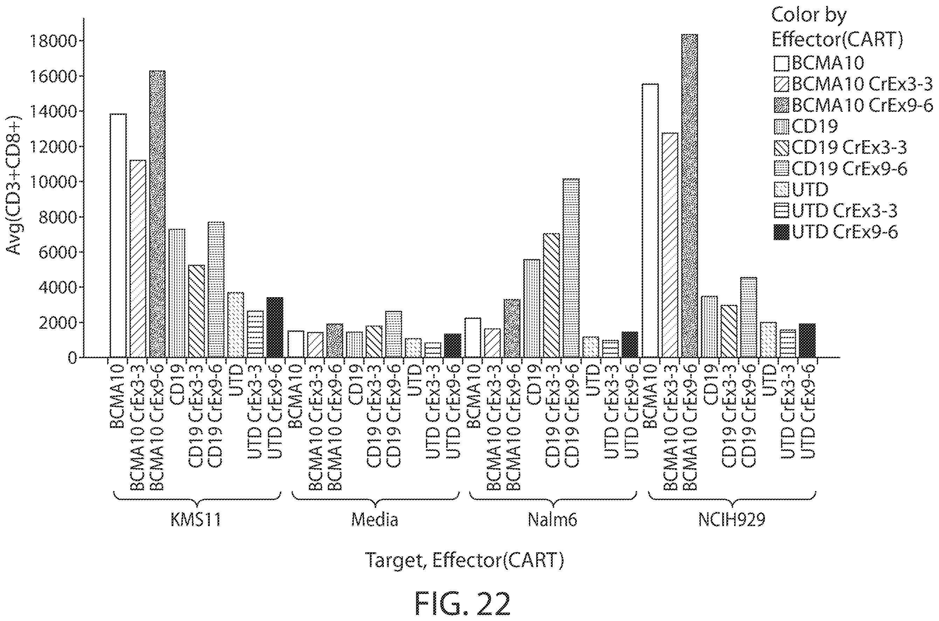

FIG. 22 shows antigen-driven CD8+ T cell proliferation. CART cells or untransduced cells (UTD) were co-cultured with BCMA-positive/CD19-negative cells (KMS11 and NCIH929) or BCMA-negative/CD19-positive cells (NALM6) or no target cells (media) and proliferation of all CD8+ CD3+ T cells was measured. Cells were prepared as described above. These data indicated that Tet2 editing causes an increase in CD8+ T cell proliferation in response to antigen, with the Exon 9 targeting guide (CrEx9-6) showing the greatest effect.

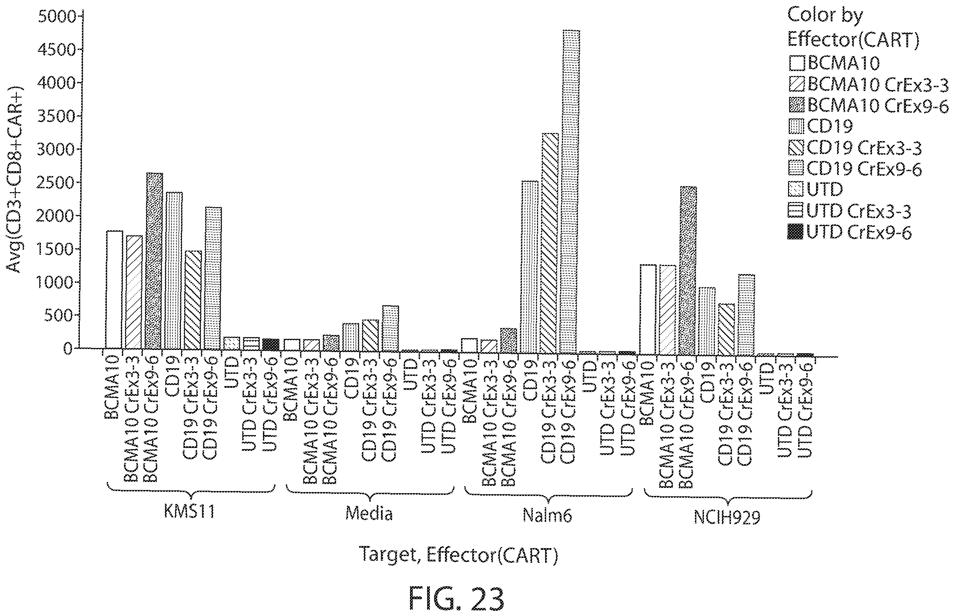

FIG. 23 shows antigen-driven CAR+ CD8+ T cell proliferation. CART cells or untransduced cells (UTD) were co-cultured with BCMA-positive/CD19-negative cells (KMS11 and NCIH929) or BCMA-negative/CD19-positive cells (NALM6) or no target cells (media) and proliferation of all CAR+CD8+ CD3+ T cells was measured. Cells were prepared as described above. These data indicated that Tet2 editing causes an increase in CAR+CD8+ T cell proliferation in response to antigen, with the Exon 9 targeting guide (CrEx9-6) showing the greatest effect.

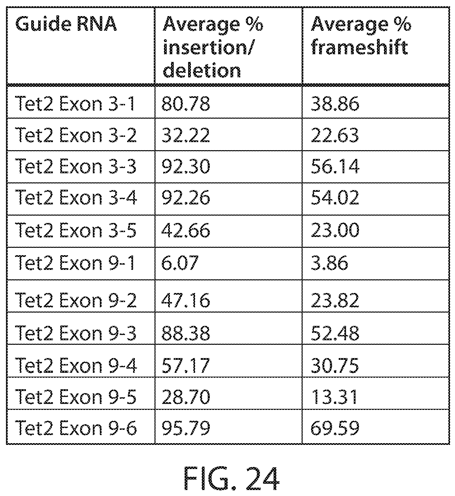

FIG. 24 shows % editing, and % frame shift edit by introduction of Tet2 targeting CRISPR/Cas systems. The level of editing in primary T cells after electroporation of RNP containing Cas9 protein, tracer RNA, and the indicated guide RNAs targeting either exon 3 or exon 9 of Tet2 is shown. The percentage of observed insertions or deletions of nucleotides relative to a reference genome is shown in the middle column (average % insertion/deletion). Editing that results in a shift in the open reading frame is shown in the far right column (average % frameshift). These data are the average of triplicates. These data indicate highly efficient genome editing in primary T cells with these guide RNA sequences.

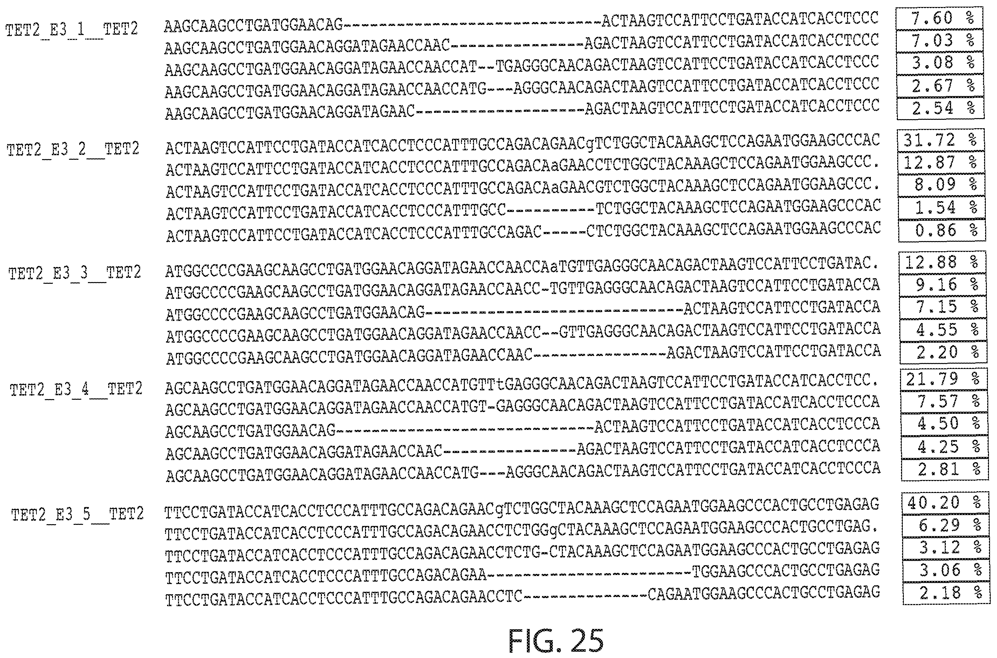

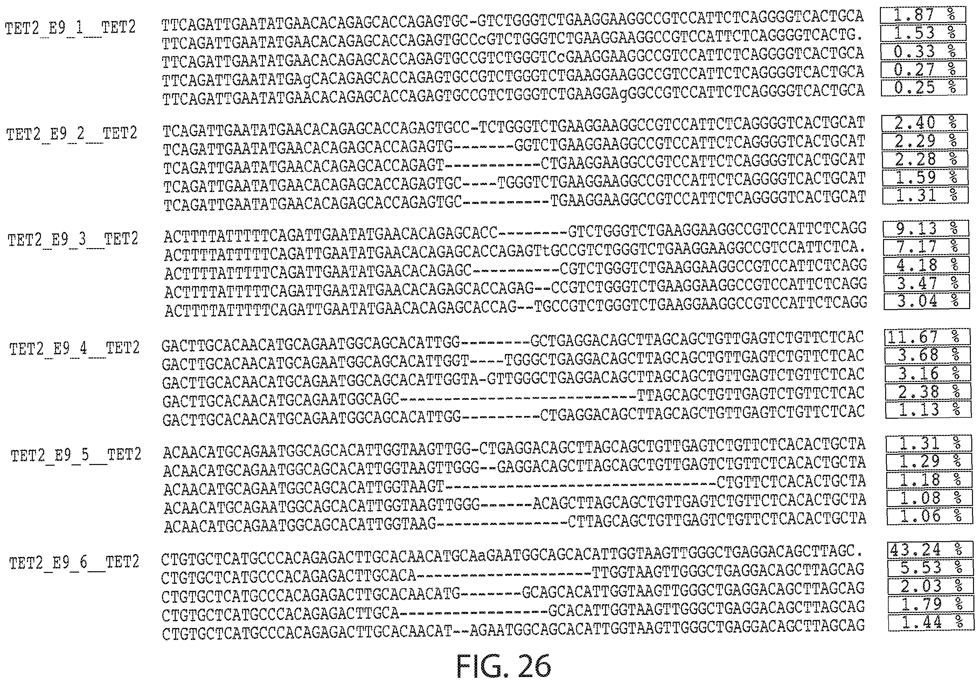

The insertion and deletion pattern present at or near the target site for each gRNA was assessed by next generation sequencing. Briefly, T cells were electroporated with an RNP containing the indicated guide RNAs. After 48 hours, DNA was isolated and processed for sequencing. FIG. 25 shows the 5 most common indels (insertions and/or deletions) observed in primary T cells for the guides RNAs targeting exon 3 of Tet2. FIG. 26 shows the 5 most common indels (insertions and/or deletions) observed in primary T cells for the guides RNAs targeting exon 9 of Tet2. The percentages indicate the frequency with which a given editing pattern was observed. Insertions are shown by lowercase nucleotide letters ("a," "g," "c" or "t"), while deletions are shown by a dash ("-").

### ATAC-Seq Experiments

CD8+ T cells with and without the Tet2 insertion were expanded from a patient's post-infusion sample. Chromatin accessibility was assessed using Assay for Transposase-Accessible Chromatin with high throughput sequencing (ATAC-seq). This is essentially a technique for global chromatin mapping based on the transposition of "barcoded" DNA fragments. These DNA fragments get incorporated into regions of open chromatin, which allow for determination of which chromatin regions are opened versus closed. Based on the location of open or closed regions, pathway analyses can be done under the assumption that "open" equals to "expressed" and "closed" equals to "repressed." FIG. 30A shows Venn diagrams of ATAC peaks in the CAR+CD8+ T cells from a patient with a Tet2 disruption compared to CAR-CD8+ T cells from the same patient at the matched time point without the Tet2 disruption. FIG. 28B show GO terms associated with ATAC peaks more closed in the cell population with the Tet2 disruption, compared to its counterpart. The significance of FIG. 30B is, at least in part, that the chromatin landscape of the CD8+ T cells with the Tet2 disruption is possibly in line with a less differentiated cell that may be more "early memory-like" and less "effector-like." These are the sort of cells that are thought to persist to provide robust and long-term anti-tumor activity.

### ShRNA Studies

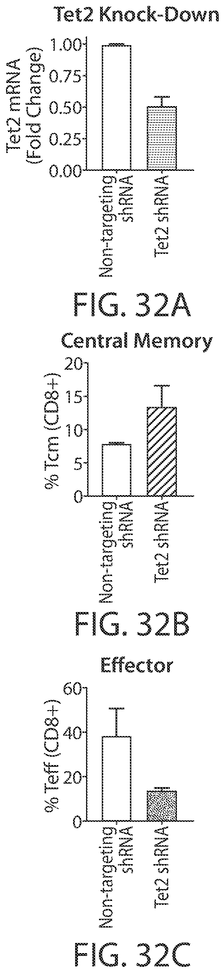

T cells from healthy donors were activated for 24 hours via CD3/CD28-coated beads, followed by lentiviral transduction with either the non-targeting (control) shRNA or the Tet2 shRNA. As shown in FIG. 32A, knock-down efficiency was assessed by qPCR and shown to be 50% (may mirror what happened in the patient in which Tet2 was disrupted via lentiviral integration). The differentiation phenotype was examined at day 14 by examining CCR7, CD45RO. Central memory cells are defined as CCR7+CD45RO+, whereas effector cells are CCR7-CD45RO-. To examine the differentiation phenotype specifically in cells with the 50% Tet2 knockdown, a GFP indicator was used in the shRNA constructs. The results are shown in FIGS. 32B and 32C.

Without further description, it is believed that one of ordinary skill in the art can, using the preceding description and the following illustrative examples, make and utilize the compounds of the present invention and practice the claimed methods. The following working examples specifically point out various aspects of the present invention, and are not to be construed as limiting in any way the remainder of the disclosure.

### EQUIVALENTS

The disclosures of each and every patent, patent application, and publication cited herein are hereby incorporated herein by reference in their entirety. While this invention has been disclosed with reference to specific aspects, it is apparent that other aspects and variations of this invention may be devised by others skilled in the art without departing from the true spirit and scope of the invention. The appended claims are intended to be construed to include all such aspects and equivalent variations.
The following numbered clauses, describing aspects of the invention, are part of the description.
1. A cell (e.g., a population of cells) engineered to express a chimeric antigen receptor (CAR), wherein the CAR comprises an antigen-binding domain, a transmembrane domain, and an intracellular signaling domain, and wherein expression and/or function of Tet1, Tet2 and/or Tet3 in said cell has been reduced or eliminated.
2. The cell of clause 1, wherein the antigen-binding domain binds to a tumor antigen is selected from a group consisting of: TSHR, CD19, CD123, CD22, CD30, CD171, CS-1, CLL-1, CD33, EGFRvIII , GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.
3. The cell of clause 2, wherein the tumor antigen is CD19.
4. The cell of clause 1, wherein the antigen-binding domain is an antibody or antibody fragment as described in, e.g., WO2012/079000 or WO2014/153270.
5. The cell of any of the preceding clauses, wherein the transmembrane domain comprises:
   an amino acid sequence having at least one, two or three modifications but not more than 20, 10 or 5 modifications of an amino acid sequence of SEQ ID NO: 12, or a sequence with 95-99% identity to an amino acid sequence of SEQ ID NO: 12; or
   the sequence of SEQ ID NO: 12.
6. The cell of any of the preceding clauses, wherein the antigen binding domain is connected to the transmembrane domain by a hinge region, wherein said hinge region comprises SEQ ID NO: 2 or SEQ ID NO: 6, or a sequence with 95-99% identity thereof.
7. The cell of any of the preceding clauses, wherein the intracellular signaling domain comprises a primary signaling domain and/or a costimulatory signaling domain, wherein the primary signaling domain comprises a functional signaling domain of a protein chosen from CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, or DAP12.
8. The cell of any of the preceding clauses, wherein the primary signaling domain comprises:
   an amino acid sequence having at least one, two or three modifications but not more than 20, 10 or 5 modifications of an amino acid sequence of SEQ ID NO: 18 or SEQ ID NO: 20, or a sequence with 95-99% identity to an amino acid sequence of SEQ ID NO: 18 or SEQ ID NO: 20; or
   the amino acid sequence of SEQ ID NO:18 or SEQ ID NO: 20.
9. The cell of any of the preceding clauses, wherein the intracellular signaling domain comprises a costimulatory signaling domain, or a primary signaling domain and a costimulatory signaling domain, wherein the costimulatory signaling domain comprises a functional signaling domain of a protein selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D.
10. The cell of any of the preceding clauses, wherein the costimulatory signaling domain comprises an amino acid sequence having at least one, two or three modifications but not more than 20, 10 or 5 modifications of an amino acid sequence of SEQ ID NO:14 or SEQ ID NO: 16, or a sequence with 95-99% identity to an amino acid sequence of SEQ ID NO:14 or SEQ ID NO: 16.
11. The cell of any of the preceding clauses, wherein the costimulatory signaling domain comprises a sequence of SEQ ID NO: 14 or SEQ ID NO: 16.
12. The cell of any of the preceding clauses, wherein the intracellular domain comprises the sequence of SEQ ID NO: 14 or SEQ ID NO: 16, and the sequence of SEQ ID NO: 18 or SEQ ID NO: 20, wherein the sequences comprising the intracellular signaling domain are expressed in the same frame and as a single polypeptide chain.
13. The cell of any of the preceding clauses, further comprising a leader sequence comprises the sequence of SEQ ID NO: 2.
14. The cell of any of the preceding clauses, wherein the cell is an immune effector cell (e.g., a population of immune effector cells).
15. The cell of clause 14, wherein the immune effector cell is a T cell or an NK cell.
16. The cell of clause 15, wherein the immune effector cell is a T cell.
17. The cell of clause 16, wherein the T cell is a CD4+ T cell, a CD8+ T cell, or a combination thereof.
18. The cell of any of the preceding clauses, wherein the cell is a human cell.
19. The cell of any of the preceding clauses, wherein the cell comprises an inhibitor of Tet1, Tet2, and/or Tet3.
20. The cell clause 19, wherein the inhibitor of Tet1, Tet2 and/or Tet3 is (1) a gene editing system targeted to one or more sites within the gene encoding Tet1, Tet2 and/or Tet3 or its regulatory elements, e.g., Tet2, or its regulatory elements; (2) nucleic acid encoding one or more components of said gene editing system; or (3) combinations thereof.
21. The cell of clause 20, wherein the gene editing system is selected from the group consisting of: a CRISPR/Cas9 system, a zinc finger nuclease system, a TALEN system and a meganuclease system.
22. The cell of clause 20 or 21, wherein the gene editing system binds to a target sequence in an early exon or intron of a gene encoding Tet1, Tet2 and/or Tet3, e.g., Tet2.
23. The cell of clause 22, wherein the gene editing system binds a target sequence of a gene encoding tet2, and the target sequence is upstream of exon 4, e.g., in exon1, exon2, or exon3, e.g. in exon 3.
24. The cell of any of clauses 20-23, wherein the gene editing system binds to a target sequence in a late exon or intron of a gene encoding Tet1, Tet2 and/or Tet3, e.g., Tet2.
25. The cell of clause 24, wherein the gene editing system binds a target sequence of a gene encoding tet2, and the target sequence is downstream of exon 8, e.g., is in exon9, exon10, or exon11, e.g. is in exon 9.
26. The cell of any of clauses 18-25, wherein the gene editing system is a CRISPR/Cas system comprising a gRNA molecule comprising a targeting sequence which hybridizes to a target sequence of a Tet2 gene.
27. The cell of clause 26, wherein the targeting sequence is a targeting sequence listed in Table 3.
28. The cell of clause 26, wherein the targeting sequence is a targeting sequence listed in Table 5.
29. The cell of clause 19, wherein the inhibitor of Tet2 is an siRNA or shRNA specific for Tet1, Tet2, Tet3, or nucleic acid encoding said siRNA or shRNA.
30. The cell of clause 29, wherein the siRNA or shRNA comprises a sequence complementary to a sequence of a Tet2 mRNA, e.g., comprises a target sequence of shRNA listed in Table 4.
31. The cell of clause 19, wherein the inhibitor of Tet1, Tet2 and/or Tet3 is a small molecule.
32. The cell of clause 19, wherein the inhibitor of Tet1, Tet2, and/or Tet3 is a protein, e.g., is a dominant negative binding partner of Tet1, Tet2, and/or Tet3 (e.g., a histone deacetylase (HDAC) that interacts with Tet1, Tet2, and/or Tet3), or nucleic acid encoding said dominant negative binding partner of Tet1, Tet2, and Tet3.
33. The cell of clause 19, wherein the inhibitor of Tet1, Tet2, and/or Tet3 is a protein, e.g., is a dominant negative (e.g., catalytically inactive) Tet1, Tet2, or Tet3, or nucleic acid encoding said dominant negative Tet1, Tet2, or Tet3.
34. A method of increasing the therapeutic efficacy of a CAR-expressing cell, e.g., a cell of any of the preceding clauses, e.g., a CAR19-expressing cell (e.g., CTL019), comprising a step of decreasing the level of 5-hydroxymethylcytosine in said cell.
35. A method of increasing the therapeutic efficacy of a CAR-expressing cell, e.g., a cell of any of the preceding clauses, e.g., a CAR19-expressing cell (e.g., CTL019), comprising a step of contacting said cell with Tet inhibitor, e.g., a Tet1, Tet2 and/or Tet3 inhibitor.
36. The method of clause 34, wherein said step comprises contacting said cells with a Tet inhibitor.
37. The method of clause 35 or 36, wherein said Tet inhibitor is a Tet2 inhibitor.
38. The method of clause 36, wherein the Tet inhibitor is selected from the group consisting of: (1) a gene editing system targeted to one or more sites within the gene encoding Tet1, Tet2, or Tet3, or its corresponding regulatory elements; (2) a nucleic acid (e.g., an siRNA or shRNA) that inhibits expression of Tet1, Tet2, or Tet3; (3) a protein (e.g., a dominant negative, e.g., catalytically inactive) Tet1, Tet2, or Tet3, or a binding partner of Tet1, Tet2, or Tet3; (4) a small molecule that inhibits expression and/or function of Tet1, Tet2, or Tet3; (5) a nucleic acid encoding any of (1)-(3); and (6) any combination of (1) -(5).
39. The method of clause 38, wherein the Tet inhibitor is a Tet2 inhibitor.
40. The method of any of clauses 36-39, wherein said contacting occurs *ex vivo.*
41. The method of any of clauses 36-39, wherein the contacting occurs *in vivo.*
42. The method of clause 41, wherein the contacting occurs *in vivo* prior to delivery of nucleic acid encoding a CAR into the cell.
43. The method of clause 41, wherein the contacting occurs *in vivo* after the cells have been administered to a subject in need thereof.
44. A cell for use in a method of treating a subject in need thereof, the method comprising administering to said subject an effective amount of the cell of any of clauses 1-33.
45. The cell for use of clause 44, wherein the method further comprises administering to said subject a Tet1, Tet2, and/or Tet3 inhibitor.
46. A CAR-expressing cell therapy for use in a method of treating a subject in need thereof, the method comprising administering to said subject the CAR-expressing cell therapy and a Tet1, Tet2, and/or Tet3 inhibitor.
47. The CAR-expressing cell therapy for use of clause 46, wherein the subject receives a pre-treatment of the Tet1, Tet2 and/or Tet3 inhibitor, prior to the initiation of the CAR-expressing cell therapy.
48. The CAR-expressing cell therapy for use of clause 46, wherein the subject receives concurrent treatment with a Tet1, Tet2, and/or Tet3 inhibitor and the CAR expressing cell therapy.
49. The CAR-expressing cell therapy for use of clause 46, wherein the subject receives treatment with a Tet1, Tet2, and/or Tet3 inhibitor post-CAR-expressing cell therapy.
50. The CAR-expressing cell therapy for use of any of clauses 44-49, wherein the subject has a disease associated with expression of a tumor antigen, e.g., a proliferative disease, a precancerous condition, a cancer, and a non-cancer related indication associated with expression of the tumor antigen.
51. The CAR-expressing cell therapy for use of clause 50, wherein the cancer is a hematologic cancer chosen from one or more of chronic lymphocytic leukemia (CLL), acute leukemias, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or pre-leukemia.
52. The CAR-expressing cell therapy for use of clause 50, wherein the cancer is selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.
53. A Tet1, Tet2 and/or Tet3 inhibitor, for use in the treatment of a subject, wherein said subject has received, is receiving, or is about to receive therapy comprising a CAR-expressing cell.
54. A method of manufacturing a CAR-expressing cell, comprising introducing nucleic acid encoding a CAR into a cell such that said nucleic acid (or CAR-encoding portion thereof) integrates into the genome of the cell within a Tet1, Tet2 and/or Tet3 gene (e.g., within an intron or exon of a Tet1, Tet2 and/or Tet3 gene), such that Tet1, Tet2 and/or Tet3 expression and/or function is reduced or eliminated.
55. A method of manufacturing a CAR-expressing cell, comprising contacting said CAR-expressing cell *ex vivo* with a Tet1, Tet2 and/or Tet3 inhibitor.
56. The method of any of clauses 40-55, wherein the inhibitor is a Tet2 inhibitor.
57. A vector comprising sequence encoding a CAR and sequence encoding a Tet inhibitor, e.g., a Tet1, Tet2, and/or Tet3 inhibitor.
58. The vector of clause 57, wherein the Tet inhibitor is a (1) a gene editing system targeted to one or more sites within the gene encoding Tet1, Tet2, or Tet3, or its corresponding regulatory elements; (2) a nucleic acid (e.g., an siRNA or shRNA) that inhibits expression of Tet1, Tet2, or Tet3; (3) a protein (e.g., a dominant negative, e.g., catalytically inactive) Tet1, Tet2, or Tet3, or a binding partner of Tet1, Tet2, or Tet3; and (4) a nucleic acid encoding any of (1)-(3), or combinations thereof.
59. The vector of clause 57 or 58, wherein the sequence encoding a CAR and the sequence encoding a Tet inhibitor are separated by a 2A site.
60. A gene editing system that is specific for a sequence of a Tet gene or its regulatory elements, e.g., a Tet1, Tet2 or Tet3 gene or its regulatory elements.
61. The gene editing system of clause 61, wherein the gene editing system is specific for a sequence of a Tet2 gene.
62. The gene editing system of clause 60 or 61, wherein the gene editing system is (1) a CRISPR/Cas gene editing system, (2) a zinc finger nuclease system, a TALEN system and a meganuclease system.
63. The gene editing system of clause 62, wherein the gene editing system is a CRISPR/Cas gene editing system.
64. The gene editing system of clause 63, comprising:
   a gRNA molecule comprising a targeting sequence specific to a sequence of a Tet2 gene or its regulatory elements, and a Cas9 protein;
   a gRNA molecule comprising a targeting sequence specific to a sequence of a Tet2 gene or its regulatory elements, and a nucleic acid encoding a Cas9 protein;
   a nucleic acid encoding a gRNA molecule comprising a targeting sequence specific to a sequence of a Tet2 gene or its regulatory elements, and a Cas9 protein; or
   a nucleic acid encoding a gRNA molecule comprising a targeting sequence specific to a sequence of a Tet2 gene or its regulatory elements, and a nucleic acid encoding a Cas9 protein.
65. The gene editing system of any of clauses 60-64, further comprising a template DNA.
66. The gene editing system of clause 65, wherein the template DNA comprises nucleic acid sequence encoding a CAR, e.g., a CAR as described herein.
67. A composition for the *ex vivo* manufacture of a CAR-expressing cell, comprising a Tet inhibitor, e.g., a Tet1, Tet2, and/or Tet3 inhibitor, e.g., a Tet2 inhibitor.
68. The composition of clause 67, wherein the Tet inhibitor is selected from N-[3-[7-(2,5-dimethyl-2H-pyrazol-3-ylamino)-1-methyl-2-oxo-1,4-dihydro-2H-pyrimido[4,5-d]pyrimidin-3-yl]-4-methylphenyl]-3-trifluoromethyl-benzamide, 2-[(2,6-dichloro-3-methylphenyl)amino]benzoic acid and 2-hydroxyglutarate.
69. A population of cells comprising one or more cells of any of clauses 1-33, wherein the population of cells comprises a higher percentage of Tscm cells (e.g., CD45RA+CD62L+CCR7+CD27+CD95+ T cells) than a population of cells which does not comprise one or more cells in which expression and/or function of Tet1, Tet2 and/or Tet3 in said cell has been reduced or eliminated.

## Claims

1. A gene editing system that is specific for a sequence of a Tet1, Tet2, and/or Tet3 gene or its regulatory elements in a cell engineered to express a chimeric antigen receptor (CAR), wherein the CAR comprises an antigen-binding domain that binds to a tumor antigen, a transmembrane domain, and an intracellular signaling domain.

2. The gene editing system of claim 1, wherein the gene editing system is:
(a) a CRISPR/Cas gene editing system;
(b) a zinc finger nuclease system;
(c) a TALEN system; or
(d) a meganuclease system.

3. The gene editing system of claim 2, wherein the gene editing system is a CRISPR/Cas gene editing system, and wherein the CRISPR/Cas gene editing system comprises:
(a) a gRNA molecule comprising a targeting sequence specific to the sequence of the Tet1, Tet2, and/or Tet3 gene or its regulatory elements, and a Cas protein;
(b) a gRNA molecule comprising a targeting sequence specific to the sequence of the Tet1, Tet2, and/or Tet3 gene or its regulatory elements, and a nucleic acid encoding a Cas protein;
(c) a nucleic acid encoding a gRNA molecule comprising a targeting sequence specific to the sequence of the Tet1, Tet2, and/or Tet3 gene or its regulatory elements, and a Cas protein; or
(d) a nucleic acid encoding a gRNA molecule comprising a targeting sequence specific to the sequence of the Tet1, Tet2, and/or Tet3 gene or its regulatory elements, and a nucleic acid encoding a Cas protein.

4. The gene editing system of claim 3, wherein the targeting sequence hybridizes to a sequence of the Tet1, Tet2, or Tet3 gene, and/or wherein the gRNA molecule further comprises a nucleotide sequence which is capable of binding to the Cas protein, optionally wherein:
(a) the targeting sequence is fully complementary to 15-25 nucleotides (e.g. 20 nucleotides) of the Tet1, Tet2, and/or Tet3 gene, wherein the 15-25 nucleotides (e.g. 20 nucleotides) of the Tet1, Tet2, and/or Tet3gene are disposed immediately 5' to a protospacer adjacent motif (PAM) sequence recognized by the Cas protein of the CRISPR/Cas gene editing system;
(b) the targeting sequence comprises an RNA sequence complementary to a target sequence listed in Table 2, a targeting sequence listed in Table 3, or a nucleotide sequence of any one of SEQ ID NOs: 73-1243; and/or
(c) the Cas protein is a Cas9 protein.

5. The gene editing system of claim 2, wherein the gene editing system is a zinc finger nuclease gene editing system, and wherein the zinc finger nuclease gene editing system comprises a zinc finger nuclease comprising a FokI nuclease domain or derivative thereof fused to a DNA-binding domain.

6. The gene editing system of claim 2, wherein the gene editing system is a TALEN system, and wherein the TALEN system comprises a TAL effector DNA-binding domain fused to a DNA cleavage domain.

7. The gene editing system of any one of claims 1-6, further comprising a template DNA, optionally wherein the template DNA comprises a nucleic acid sequence encoding the CAR.

8. The gene editing system of any one of claims 1-7, wherein:
(a) the gene editing system binds to a target sequence in an early exon or intron of the Tet1, Tet2, and/or Tet3 gene, and wherein the target sequence is upstream of exon 4; or
(b) the gene editing system binds to a target sequence in a late exon or intron of the Tet1, Tet2, and/or Tet3gene, and wherein the target sequence is downstream of exon 8.

9. The gene editing system of any one of claims 1-8, wherein the tumor antigen is selected from a group consisting of: TSHR, CD19, CD123, CD22, CD30, CD171, CS-1, CLL-1, CD33, EGFRvIII , GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-la, MAGE-Al, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

10. The gene editing system of any one of claims 1-9, wherein the transmembrane domain comprises:
(a) an amino acid sequence having at least one, two, or three modifications but not more than 20, 10, or 5 modifications of the amino acid sequence of SEQ ID NO: 12, or a sequence with 95-99% identity to the amino acid sequence of SEQ ID NO: 12; or
(b) the amino acid sequence of SEQ ID NO: 12.

11. The gene editing system of any one of claims 1-10, wherein the antigen binding domain is connected to the transmembrane domain by a hinge region, and wherein the hinge region comprises the amino acid sequence of SEQ ID NO: 4 or SEQ ID NO: 6, or an amino acid sequence with 95-99% identity thereof.

12. The gene editing system of any one of claims 1-11, wherein the intracellular signaling domain comprises a primary signaling domain and/or a costimulatory signaling domain, wherein:
(a) the primary signaling domain comprises:
(i) a functional signaling domain of a protein chosen from CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, or DAP12;
(ii) an amino acid sequence having at least one, two, or three modifications but not more than 20, 10, or 5 modifications of the amino acid sequence of SEQ ID NO: 18 or SEQ ID NO: 20, or a sequence with 95-99% identity to the amino acid sequence of SEQ ID NO: 18 or SEQ ID NO: 20; or
(iii) the amino acid sequence of SEQ ID NO: 18 or SEQ ID NO: 20; and/or
(b) the costimulatory signaling domain comprises:
(i) a functional signaling domain of a protein selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D;
(ii) an amino acid sequence having at least one, two, or three modifications but not more than 20, 10, or 5 modifications of the amino acid sequence of SEQ ID NO:14 or SEQ ID NO: 16, or the amino acid sequence with 95-99% identity to the amino acid sequence of SEQ ID NO:14 or SEQ ID NO: 16; or
(iii) the amino acid sequence of SEQ ID NO:14 or SEQ ID NO: 16.

13. The gene editing system of any one of claims 1-12, wherein the CAR further comprises a leader sequence comprising the amino acid sequence of SEQ ID NO: 2.

14. The gene editing system of any one of claims 1-13, wherein the cell is an immune effector cell and/or a human cell, optionally wherein the immune effector cell is a T cell or an NK cell, and further optionally wherein the T cell is a CD4+ T cell, a CD8+ T cell, or a combination thereof.

15. A cell engineered to express a CAR, wherein the CAR comprises an antigen-binding domain that binds to a tumor antigen, a transmembrane domain, and an intracellular signaling domain, wherein the cell comprises a gene editing system that is specific for a sequence of a Tetl, Tet2, and/or Tet3 gene or its regulatory elements.
